(19) Europäisches Patentamt
European Patent Office
Office européen des brevets

(11) EP 2 394 993 A2

(12) # EUROPEAN PATENT APPLICATION

published in accordance with Art. 153(4) EPC

(43) Date of publication:
**14.12.2011 Bulletin 2011/50**

(21) Application number: **09832072.4**

(22) Date of filing: **04.12.2009**

(51) Int Cl.:
***C07D 213/16*** *(2006.01)* ***C07D 213/53*** *(2006.01)*

(86) International application number:
**PCT/KR2009/007216**

(87) International publication number:
**WO 2010/067987 (17.06.2010 Gazette 2010/24)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **10.12.2008 KR 20080125360**

(71) Applicant: **Dong Wha Pharm. Co., Ltd.**
**Jung-gu**
**Seoul 100-130 (KR)**

(72) Inventors:
- **RYU, Jei Man**
**Anyang-si**
**Gyeonggi-do 431-817 (KR)**
- **LEE, Jin Soo**
**Yongin-si**
**Gyeonggi-do 448-172 (KR)**
- **PARK, Whui Jung**
**Suwon-si**
**Gyeonggi-do 442-150 (KR)**
- **HWANG, Yun Ha**
**Gunpo-si**
**Gyeonggi-do 435-040 (KR)**
- **KIM, Ki Yoon**
**Hwaseong-si**
**Gyeonggi-do 445-360 (KR)**

(74) Representative: **Fabry, Bernd**
**IP2 Patentanwalts GmbH**
**Bruchstrasse 13**
**41352 Korschenbroich (DE)**

(54) **NOVEL 2,6-SUBSTITUTED-3-NITROPYRIDINE DERIVATIVE, METHOD FOR PREPARING SAME, AND PHARMACEUTICAL COMPOSITION INCLUDING SAME**

(57) The present invention relates to a novel 2,6-substituted-3-nitropyridine derivative compound, a method for preparing the same, and a pharmaceutical composition including the same for prevention and treatment of osteoporosis. The 2,6-substituted-3-nitropyridine derivative compound of the present invention increases osteoblast activity and effectively inhibits the differentiation of osteoclasts, and thus can be usefully used for the prevention and treatment of osteoporosis.

EP 2 394 993 A2

## Description

### TECHNICAL FIELD

The present invention relates to a novel 2,6-substituted-3-nitropyridine derivative compound, a method for preparing the same and a pharmaceutical composition containing the same.

### BACKGROUND ART

Bone is a supporting material for the body's framework and serves to conserve the necessary bone mass and structure. Bone also functions as a reservoir of calcium ($Ca^{2+}$) or the like and plays an important role in maintaining blood levels of calcium or the like. To cope with these functions, the growth of bone is a metabolic balance between the activity of osteoblasts and osteoclasts in the bone remodeling cycle. Accordingly, bone is in a steady state, which maintains good balance between bone absorption and bone formation in the process of metabolism by continuously performing both bone absorption and bone formation. When the balance between bone absorption and bone formation is disrupted, the degree of bone absorption is relatively higher than that of bone formation, which may lead to osteoporosis, a condition which causes reduction in bone density or bone mass, resulting in decrease in bone strength. This is a disease which frequently occurs in middle-aged or elderly women.

Osteoporosis is a disease, which results from a disturbance in the balance between bone absorption and bone formation, and is caused by having a higher degree of bone absorption relative to that of bone formation. Osteoporosis reduces calcification of bone tissues, and decreases the level of the compact substances in the bone, which broadens the marrow cavity. As osteoporosis progresses, bone becomes brittle, and bone fracture may easily occur even with a small impact. Bone is a steady state structure, in which the bone formation by osteoblasts and the bone resorption by osteoclasts occur continuously.

Previous studies on osteoporosis have focused mainly on dysmetabolism of bone minerals such as calcium and phosphorus. However, such studies did not provide sufficient findings on the pathogenic mechanism of osteoporosis.

Although bisphosphonate (alendronate, etidronate, etc.), hormone therapy (raloxifen), vitamin D, calcitonin, calcium agents, and the like have been currently used as an anti-osteoporotic agent, they are known to have adverse side effects. Specifically, bisphosphonate agents exhibit low absorptivity, difficulty of administration and risk of causing esophagitis. Hormone agents must be administered throughout a patient's life and long-term administration thereof may result in adverse side effects such as breast cancer, uterus cancer, gallstones and thrombosis. Vitamin D agents are expensive and show little efficacy, and calcitonin agents are also very expensive and have difficulty of administration. Calcium agents have few adverse side effects, but their medicinal effects are restricted to the prevention of osteoporosis, not the treatment thereof.

Osteoporosis cannot be treated with short-term administration of drugs and generally requires long-term administration of drugs. Therefore, there is a need for a novel substance having excellent medicinal efficacy without causing the above-mentioned adverse side effects even upon long-term administration thereof.

As a result of intensive studies and experiments to solve the above-described problems and develop an effective therapeutic agent against osteoporosis, the inventors of the present invention succeeded in the synthesis of novel 2,6-substituted-3-nitropyridine derivatives and discovered that these compounds have excellent effects on the treatment and prevention of osteoporosis, by suppressing the differentiation of osteoclasts to effectively inhibit osteoclastic bone absorption and simultaneously promoting the activity of osteoblasts to thereby increase osteogenesis. The present invention has been completed based on these findings.

### DISCLOSURE OF THE INVENTION

### TECHNICAL PROBLEM

Therefore, the present invention is intended to provide a novel 2,6-substituted-3-nitropyridine derivative compound.

Further, the present invention is intended to provide a method for preparing a 2,6-substituted-3-nitropyridine derivative compound.

Further, the present invention is intended to provide a pharmaceutical composition for the prevention or treatment of osteoporosis, containing a 2,6-substituted-3-nitropyridine derivative compound.

Further, the present invention is intended to provide a method for the prevention or treatment of osteoporosis, including administering an effective amount of a 2,6-substituted-3-nitropyridine derivative compound to a mammal including a human.

Further, the present invention is intended to provide use of a 2,6-substituted-3-nitropyridine derivative compound, for manufacturing a pharmaceutical composition for the prevention or treatment of osteoporosis.

## TECHNICAL SOLUTION

The present invention provides a 2,6-substituted-3-nitropyridine derivative compound represented by the following formula 1:

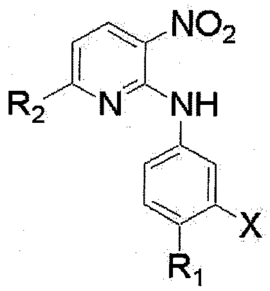

**Formula I**

wherein

$R_1$ represents hydrogen, fluoro, a $C_1$-$C_6$ linear or branched alkyl group, a methoxy group, a methylsulfanyl group, a nitrile group, a hydroxyl group or $NR_3R_4$ wherein $R_3$ and $R_4$ each independently represent H, a methyl group or an ethyl group, or $R_3$ and $R_4$ taken together form a saturated or unsaturated 5-, 6- or 7-membered heterocyclic amino compound which contains 1 to 3 hetero atoms selected from N, O and S and is unsubstituted or substituted by a $C_1$-$C_3$ alkyl group, a hydroxyl group, a $C_1$-$C_3$ hydroxyalkyl group, an amino group, a carboxyl group or a carbamoyl group; when $R_1$ represents a thiazolyl group

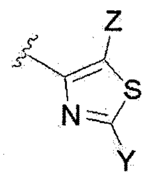

Y is substituted by a $C_1$-$C_5$ linear or branched alkyl group, a $C_1$-$C_3$ alkylamine or dialkylamine group or a $C_5$-$C_6$ saturated or unsaturated cyclic amine group, and Z represents hydrogen or a $C_1$-$C_3$ alkyl group, $R_1$ optionally contains an asymmetric carbon atom,

$R_2$ represents $NR_5(CH_2)_nR_6$ wherein $R_5$ represents H, a $C_1$-$C_6$ linear or branched alkyl group or an unsubstituted or substituted $C_3$-$C_6$ cyclic alkyl group, and $R_6$ represents H, a hydroxyl group, a phenyl group, a $C_1$-$C_2$ alkoxy group, a $C_1$-$C_6$ linear or branched alkylamine group, or a $C_1$-$C_6$ linear or branched alkyl group which is terminally substituted by a saturated or unsaturated 5 to 7-membered heterocyclic compound containing 1 to 3 hetero atoms selected from N, O and S, or $R_5$ and $R_6$ taken together form a saturated or unsaturated 5 to 7-membered heterocyclic amine compound which contains 1 to 3 hetero atoms selected from N, O and S and is unsubstituted or substituted by a $C_1$-$C_3$ alkyl group, an amine group, a hydroxyl group or a $C_1$-$C_2$ hydroxyalkyl group,

n represents an integer of 0 to 3, and

X represents hydrogen, a fluoro group, a hydroxyl group, an amino group, an acetyl group or a nitrile group; or a pharmaceutically acceptable salt thereof.

The compound of formula 1 in accordance with the present invention preferably has the following substituents:

In formula 1,

$R_1$ represents hydrogen, fluoro, a methyl group, an n-butyl group, a t-butyl group, a methoxy group, a methylsulfanyl group, a nitrile group, a hydroxyl group or $NR_3R_4$ wherein $R_3$ and $R_4$ each independently represent H, a methyl group or an ethyl group, or $R_3$ and $R_4$ taken together form a heterocyclic compound which is morpholine, thiomorpholine, piperazine, piperidine, methylpiperidine, hydroxypiperidine, hydroxymethylpiperidine, aminopiperidine, 3- or 4-carbamoylpiperidine, carboxypiperidine, imidazol-1-yl or thiazol-4-yl derivative

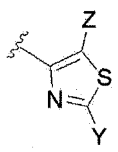

wherein Y represents a methyl group, an isopropyl group, a cyclohexyl group or a dipropylamine group, and Z represents hydrogen or a $C_1$-$C_3$ alkyl group,

$R_2$ represents $NR_5(CH_2)_nR_6$ wherein $R_5$ represents H, a methyl group, an ethyl group, an isopropyl group, a cyclopropyl group, an n-butyl group, an isobutyl group or a t-butyl group, and $R_6$ represents H, a hydroxyl group, a morpholinyl group, a phenyl group, a pyridin-2-yl, pyridin-3-yl, pyridin-4-yl, imidazol-1-yl or 1,3-dioxolan-2-yl, or $R_5$ and $R_6$ taken together form a heterocyclic compound which is morpholine, piperazine, methylpiperazine, aminopiperidine, 2-methyl-4,5-dihydroimidazol-1-yl, 2-methyl- imidazol-1-yl or isopropylimidazol-1-yl,

n represents an integer of 0 to 3, and

X represents hydrogen, a fluoro group, an amino group, an acetyl group or a nitrile group.

Among the compounds of formula 1 in accordance with the present invention, more preferable compounds are as follows:

- 2-(4-methylphenylamino)-6-(methylamino)-3-nitropyridine,
- 2-(4-methylphenylamino)-6-(isopropylamino)-3-nitropyridine,
- 2-(4-methylphenylamino)-6-(isobutylamino)-3-nitropyridine,
- 2-(4-methylphenylamino)-6-[(N-[1,3]-dioxolan-2-ylmethyl)methylamino]-3-nitropyridine,
- 2-(4-methylphenylamino)-6-(4-hydroxypiperidino)-3-nitropyridine,
- 2-(4-methylphenylamino)-6-[(2-methyl-4,5-dihydro)imidazol-1-yl]-3-nitropyridine,
- 2-(4-methylphenylamino)-6-[(2-isopropyl)imidazol-1-yl]-3-nitropyridine,
- 2-(4-methylphenylamino)-6-[(4-pyridyl)methylamino]-3-nitropyridine,
- 2-(4-methylphenylamino)-6-[(3-imidazol-1-yl)propylamino]-3-nitropyridine,
- 2-(4-methylphenylamino)-6-[2-(3-pyridyl)ethylamino]-3-nitropyridine,
- 2-(4-methylphenylamino)-6-(4-methylpiperazin-1-yl)-3-nitropyridine,
- 2-(4-methylphenylamino)-6-(piperazin-1-yl)-3-nitropyridine,
- 2-(4-methylphenylamino)-6-(4-aminopiperidino)-3-nitropyridine,
- 2-(4-methylphenylamino)-6-morpholino-3-nitropyridine,
- 2-(4-methoxyphenylamino)-6-(methylamino)-3-nitropyridine,
- 2-(4-methoxyphenylamino)-6-(isopropylamino)-3-nitropyridine,
- 2-(4-methoxyphenylamino)-6-(isobutylamino)-3-nitropyridine,
- 2-(4-methoxyphenylamino)-6-[(N-[1,3]-dioxolan-2-ylmethyl)methylamino]-3-nitropyridine,
- 2-(4-methoxyphenylamino)-6-(4-hydroxypiperidino)-3-nitropyridine,
- 2-(4-methoxyphenylamino)-6-[(2-methyl-4,5-dihydro)imidazol-1-yl]-3-nitropyridine,
- 2-(4-methoxyphenylamino)-6-[(2-isopropyl)imidazol-1-yl]-3-nitropyridine,
- 2-(4-methoxyphenylamino)-6-[(4-pyridyl)methylamino]-3-nitropyridine,
- 2-(4-methoxyphenylamino)-6-(t-butylamino)-3-nitropyridine,
- 2-(4-methoxyphenylamino)-6-[(N-methyl-2-hydroxy)ethylamino]-3-nitropyridine,
- 2-(4-methoxyphenylamino)-6-[(3-imidazol-1-yl)propylamino]-3-nitropyridine,
- 2-(4-methoxyphenylamino)-6-(4-methylpiperazin-1-yl)-3-nitropyridine,
- 2-(4-methoxyphenylamino)-6-(piperazin-1-yl)-3-nitropyridine,
- 2-(4-methoxyphenylamino)-6-(4-aminopiperidino)-3-nitropyridine,

- 2-(4-methoxyphenylamino)-6-morpholino-3-nitropyridine,
- 2-[4-(t-butyl)phenylamino]-6-(methylamino)-3-nitropyridine,
- 2-[4-(t-butyl)phenylamino]-6-(isopropylamino)-3-nitropyridine,
- 2-[4-(t-butyl)phenylamino]-6-(isobutylamino)-3-nitropyridine,
- 2-[4-(t-butyl)phenylamino]-6-[(N-[1,3]-dioxolan-2-ylmethyl)methylamino]-3-nitropyridine,
- 2-[4-(t-butyl)phenylamino]-6-(4-hydroxypiperidino)-3-nitropyridine,
- 2-[4-(t-butyl)phenylamino]-6-[(2-methyl-4,5-dihydro)imidazol-1-yl]-3-nitropyridine,
- 2-[4-(t-butyl)phenylamino]-6-[(2-isopropyl)imidazol-1-yl]-3-nitropyridine,
- 2-[4-(t-butyl)phenylamino]-6-[(3-pyridyl)methylamino]-3-nitropyridine,
- 2-[4-(t-butyl)phenylamino]-6-[(4-pyridyl)methylamino]-3-nitropyridine,
- 2-[4-(t-butyl)phenylamino]-6-[(3-imidazol-1-yl)propylamino]-3-nitropyridine,
- 2-[4-(t-butyl)phenylamino]-6-[2-(2-pyridyl)ethylamino]-3-nitropyridine,
- 2-[4-(t-butyl)phenylamino]-6-(4-methylpiperazin-1-yl)-3-nitropyridine,
- 2-[4-(t-butyl)phenylamino]-6-(piperazin-1-yl)-3-nitropyridine,
- 2-[4-(t-butyl)phenylamino]-6-(4-aminopiperidino)-3-nitropyridine,
- 2-[4-(t-butyl)phenylamino]-6-morpholino-3-nitropyridine,
- 2-(4-cyanophenylamino)-6-(methylamino)-3-nitropyridine,
- 2-(4-cyanophenylamino)-6-(isobutylamino)-3-nitropyridine,
- 2-(4-cyanophenylamino)-6-(4-hydroxypiperidino)-3-nitropyridine,
- 2-(4-cyanophenylamino)-6-[(2-methyl-4,5-dihydro)imidazol-1-yl]-3-nitropyridine,
- 2-(4-cyanophenylamino)-6-[(2-isopropyl)imidazol-1-yl]-3-nitropyridine,
- 2-(4-cyanophenylamino)-6-[(4-pyridyl)methylamino]-3-nitropyridine,
- 2-(4-cyanophenylamino)-6-[(N-ethyl-2-hydroxy)ethylamino]-3-nitropyridine,
- 2-(4-cyanophenylamino)-6-[(3-imidazol-1-yl)propylamino]-3-nitropyridine,
- 2-[3-cyanophenylamino]-6-[(3-imidazol-1-yl)propylamino]-3-nitropyridine,
- 2-(4-hydroxyphenylamino)-6-[(3-imidazol-1-yl)propylamino]-3-nitropyridine,
- 2-[4-(methylsulfanyl)phenylamino]-6-[(3-imidazol-1-yl)propylamino]-3-nitropyridine,
- 2-[4-(n-butyl)phenylamino]-6-[(3-imidazol-1-yl)propylamino]-3-nitropyridine,
- 2-[4-(amino)phenylamino]-6-(methylamino)-3-nitropyridine,
- 2-[4-(amino)phenylamino]-6-(isopropylamino)-3-nitropyridine,
- 2-[4-(amino)phenylamino]-6-(isobutylamino)-3-nitropyridine,
- 2-[4-(amino)phenylamino]-6-(t-butylamino)-3-nitropyridine,
- 2-[4-(amino)phenylamino]-6-(4-hydroxypiperidino)-3-nitropyridine,
- 2-[4-(amino)phenylamino]-6-(piperazin-1-yl)-3-nitropyridine,
- 2-[4-(amino)phenylamino]-6-(4-methylpiperazin-1-yl)-3-nitropyridine,
- 2-[4-(amino)phenylamino]-6-morpholino-3-nitropyridine,
- 2-[4-(amino)phenylamino]-6-(4-aminopiperidino)-3-nitropyridine,
- 2-[4-(amino)phenylamino]-6-[(4-pyridyl)methylamino]-3-nitropyridine,
- 2-[4-(amino)phenylamino]-6-[(3-imidazol-1-yl)propylamino]-3-nitropyridine,
- 2-[4-(amino)phenylamino]-6-[2-(morpholin-1-yl)ethylamino]-3-nitropyridine,
- 2-[4-(amino)phenylamino]-6-[3-(morpholin-1-yl)propylamino]-3-nitropyridine,
- 2-[3-(amino)phenylamino]-6-(methylamino)-3-nitropyridine,
- 2-[3-(amino)phenylamino]-6-(isopropylamino)-3-nitropyridine,
- 2-[3-(amino)phenylamino]-6-(isobutylamino)-3-nitropyridine,
- 2-[3-(amino)phenylamino]-6-(t-butylamino)-3-nitropyridine,
- 2-[3-(amino)phenylamino]-6-(4-hydroxypiperidino)-3-nitropyridine,
- 2-[3-(amino)phenylamino]-6-[(2-isopropyl)imidazol-1-yl]-3-nitropyridine,
- 2-[3-(amino)phenylamino]-6-(piperazin-1-yl)-3-nitropyridine,
- 2-[3-(amino)phenylamino]-6-(4-methylpiperazin-1-yl)-3-nitropyridine,
- 2-[3-(amino)phenylamino]-6-morpholino-3-nitropyridine,
- 2-[3-(amino)phenylamino]-6-(4-aminopiperidino)-3-nitropyridine,
- 2-[3-(amino)phenylamino]-6-[(3-pyridyl)methylamino]-3-nitropyridine,
- 2-[3-(amino)phenylamino]-6-[(4-pyridyl)methylamino]-3-nitropyridine,
- 2-[3-(amino)phenylamino]-6-[(3-imidazol-1-yl)propylamino]-3-nitropyridine,
- 2-[3-(amino)phenylamino]-6-[2-(morpholin-1-yl)ethylamino]-3-nitropyridine,
- 2-[3-(amino)phenylamino]-6-[3-(morpholin-1-yl)propylamino]-3-nitropyridine,
- 2-[3-(amino)phenylamino]-6-[(2-methyl)imidazol-1-yl]-3-nitropyridine,
- 2-[4-(imidazol-1-yl)phenylamino]-6-(methylamino)-3-nitropyridine,

- 2-[4-(imidazol-1-yl)phenylamino]-6-(isopropylamino)-3-nitropyridine,
- 2-[4-(imidazol-1-yl)phenylamino]-6-(isobutylamino)-3-nitropyridine,
- 2-[4-(imidazol-1-yl)phenylamino]-6-[(N-[1,3]-dioxolan-2-ylmethyl)methylamino]-3-nitropyridine,
- 2-[4-(imidazol-1-yl)phenylamino]-6-(4-hydroxypiperidino)-3-nitropyridine,
- 2-[4-(imidazol-1-yl)phenylamino]-6-[(2-methyl-4,5-dihydro)imidazol-1-yl]-3-nitropyridine,
- 2-[4-(imidazol-1-yl)phenylamino]-6-[(2-isopropyl)imidazol-1-yl]-3-nitropyridine,
- 2-[4-(imidazol-1-yl)phenylamino]-6-[(3-pyridyl)methylamino]-3-nitropyridine,
- 2-[4-(imidazol-1-yl)phenylamino]-6-[(4-pyridyl)methylamino]-3-nitropyridine,
- 2-[4-(imidazol-1-yl)phenylamino]-6-[(3-imidazol-1-yl)propylamino]-3-nitropyridine,
- 2-(3-acetylphenylamino)-6-(methylamino)-3-nitropyridine,
- 2-(3-acetylphenylamino)-6-(isopropylamino)-3-nitropyridine,
- 2-(3-acetylphenylamino)-6-(isobutylamino)-3-nitropyridine,
- 2-(3-acetylphenylamino)-6-(4-hydroxypiperidino)-3-nitropyridine,
- 2-(3-acetylphenylamino)-6-[(2-methyl-4,5-dihydro)imidazol-1-yl]-3-nitropyridine,
- 2-(3-acetylphenylamino)-6-[(2-isopropyl)imidazol-1-yl]-3-nitropyridine,
- 2-(3-acetylphenylamino)-6-[(3-pyridyl)methylamino]-3-nitropyridine,
- 2-(3-acetylphenylamino)-6-[(4-pyridyl)methylamino]-3-nitropyridine,
- 2-(3-acetylphenylamino)-6-(t-butylamino)-3-nitropyridine,
- 2-(3-acetylphenylamino)-6-(4-methylpiperazin-1-yl)-3-nitropyridine,
- 2-(3-acetylphenylamino)-6-(piperazin-1-yl)-3-nitropyridine,
- 2-(3-acetylphenylamino)-6-morpholino-3-nitropyridine,
- 2-(4-morpholinophenylamino)-6-(methylamino)-3-nitropyridine,
- 2-(4-morpholinophenylamino)-6-(isopropylamino)-3-nitropyridine,
- 2-(4-morpholinophenylamino)-6-(isobutylamino)-3-nitropyridine,
- 2-(4-morpholinophenylamino)-6-[(N-[1,3]-dioxolan-2-ylmethyl)methylamino]-3-nitropyridine,
- 2-(4-morpholinophenylamino)-6-(4-hydroxypiperidino)-3-nitropyridine,
- 2-(4-morpholinophenylamino)-6-[(2-methyl-4,5-dihydro)imidazol-1-yl]-3-nitropyridine,
- 2-(4-morpholinophenylamino)-6-[(2-isopropyl)imidazol-1-yl]-3-nitropyridine,
- 2-(4-morpholinophenylamino)-6-[(3-pyridyl)methylamino]-3-nitropyridine,
- 2-(4-morpholinophenylamino)-6-[(4-pyridyl)methylamino]-3-nitropyridine,
- 2-(4-morpholinophenylamino)-6-(t-butylamino)-3-nitropyridine,
- 2-(4-morpholinophenylamino)-6-[(N-ethyl-2-hydroxy)ethylamino]-3-nitropyridine,
- 2-(4-morpholinophenylamino)-6-[(3-imidazol-1-yl)propylamino]-3-nitropyridine,
- 2-(4-morpholinophenylamino)-6-(piperazin-1-yl)-3-nitropyridine,
- 2-(4-morpholinophenylamino)-6-(4-aminopiperidino)-3-nitropyridine,
- 2-[(3,4-difluoro)phenylamino]-6-(methylamino)-3-nitropyridine,
- 2-[(3,4-difluoro)phenylamino]-6-(isopropylamino)-3-nitropyridine,
- 2-[(3,4-difluoro)phenylamino]-6-(isobutylamino)-3-nitropyridine,
- 2-[(3,4-difluoro)phenylamino]-6-(t-butylamino)-3-nitropyridine,
- 2-[(3,4-difluoro)phenylamino]-6-(4-hydroxypiperidino)-3-nitropyridine,
- 2-[(3,4-difluoro)phenylamino]-6-[(N-[1,3]-dioxolan-2-ylmethyl)-methylamino]-3-nitropyridine,
- 2-[(3,4-difluoro)phenylamino]-6-(4-methylpiperazin-1-yl)-3-nitropyridine,
- 2-[(3,4-difluoro)phenylamino]-6-morpholino-3-nitropyridine,
- 2-[(3,4-difluoro)phenylamino]-6-(4-aminopiperidino)-3-nitropyridine,
- 2-[(3,4-difluoro)phenylamino]-6-[(4-pyridyl)methylamino]-3-nitropyridine,
- 2-[4-(2-methylthiazol-4-yl)phenylamino]-6-(methylamino)-3-nitropyridine,
- 2-[4-(2-methylthiazol-4-yl)phenylamino]-6-(isopropylamino)-3-nitropyridine,
- 2-[4-(2-methylthiazol-4-yl)phenylamino]-6-(isobutylamino)-3-nitropyridine,
- 2-[4-(2-methylthiazol-4-yl)phenylamino]-6-(4-hydroxypiperidino)-3-nitropyridine,
- 2-[4-(2-methylthiazol-4-yl)phenylamino]-6-[(2-methyl-4,5-dihydro)imidazol-1-yl]-3-nitropyridine,
- 2-[4-(2-methylthiazol-4-yl)phenylamino]-6-[(2-isopropyl)imidazol-1-yl]-3-nitropyridine,
- 2-[4-(2-methylthiazol-4-yl)phenylamino]-6-[(3-pyridyl)methylamino]-3-nitropyridine,
- 2-[4-(2-methylthiazol-4-yl)phenylamino]-6-[(4-pyridyl)methylamino]-3-nitropyridine,
- 2-[4-(2-methylthiazol-4-yl)phenylamino]-6-(t-butylamino)-3-nitropyridine,
- 2-[4-(2-methylthiazol-4-yl)phenylamino]-6-[(N-ethyl-2-hydroxy)ethylamino]-3-nitropyridine,
- 2-[4-(2-methylthiazol-4-yl)phenylamino]-6-(4-methylpiperazin-1-yl)-3-nitropyridine,
- 2-[4-(2-methylthiazol-4-yl)phenylamino]-6-(piperazin-1-yl)-3-nitropyridine,
- 2-[4-(2-methylthiazol-4-yl)phenylamino]-6-(4-aminopiperidino)-3-nitropyridine,

- 2-[4-(2-methylthiazol-4-yl)phenylamino]-6-morpholino-3-nitropyridine,
- 2-[4-(2-isopropylthiazol-4-yl)phenylamino]-6-(isobutylamino)-3-nitropyridine,
- 2-[4-(2-isopropylthiazol-4-yl)phenylamino]-6-(4-hydroxypiperidino)-3-nitropyridine,
- [2-[4-(2-isopropylthiazol-4-yl)phenylamino]-6-[(N-ethyl-2-hydroxyethyl)amino]-3-nitropyridine,
- 2-[4-(2-isopropylthiazol-4-yl)phenylamino]-6-(4-methylpiperazin-1-yl)-3-nitropyridine,
- 2-[4-(2-isopropylthiazol-4-yl)phenylamino]-6-(4-aminopiperidino)-3-nitropyridine,
- 2-[4-(2-cyclohexylthiazol-4-yl)phenylamino]-6-(methylamino)-3-nitropyridine,
- 2-[4-(2-cyclohexylthiazol-4-yl)phenylamino]-6-(isopropylamino)-3-nitropyridine,
- 2-[4-(2-cyclohexylthiazol-4-yl)phenylamino]-6-(isobutylamino)-3-nitropyridine,
- 2-[4-(2-cyclohexylthiazol-4-yl)phenylamino]-6-(t-butylamino)-3-nitropyridine,
- 2-[4-(2-cyclohexylthiazol-4-yl)phenylamino]-6-(4-hydroxypiperidino)-3-nitropyridine,
- 2-[4-(2-cyclohexylthiazol-4-yl)phenylamino]-6-[(N-ethyl-2-hydroxyethyl)amino]-3-nitropyridine,
- 2-[4-(2-cyclohexylthiazol-4-yl)phenylamino]-6-[(2-isopropyl)imidazol-1-yl]-3-nitropyridine,
- 2-[4-(2-cyclohexylthiazol-4-yl)phenylamino]-6-(piperazin-1-yl)-3-nitropyridine,
- 2-[4-(2-cyclohexylthiazol-4-yl)phenylamino]-6-(4-methyl)piperazin-1-yl)-3-nitropyridine,
- 2-[4-(2-cyclohexylthiazol-4-yl)phenylamino]-6-morpholino-3-nitropyridine,
- 2-[4-(2-cyclohexylthiazol-4-yl)phenylamino]-6-(4-aminopiperidino)-3-nitropyridine,
- 2-[4-(2-cyclohexylthiazol-4-yl)phenylamino]-6-[(3-pyridyl)methylamino]-3-nitropyridine,
- 2-[4-(2-cyclohexylthiazol-4-yl)phenylamino]-6-[(4-pyridyl)methylamino]-3-nitropyridine,
- 2-[4-(2-cyclohexylthiazol-4-yl)phenylamino]-6-[2-(2-pyridyl)ethylamino]-3-nitropyridine,
- 2-[4-(2-cyclohexylthiazol-4-yl)phenylamino]-6-(n-butylamino)-3-nitropyridine,
- 2-[4-(2-dipropylaminothiazol-4-yl)phenylamino]-6-(methylamino)-3-nitropyridine,
- 2-[4-(2-dipropylaminothiazol-4-yl)phenylamino]-6-(isopropylamino)-3-nitropyridine,
- 2-[4-(2-dipropylaminothiazol-4-yl)phenylamino]-6-(isobutylamino)-3-nitropyridine,
- 2-[4-(2-dipropylaminothiazol-4-yl)phenylamino]-6-(4-hydroxypiperidino)-3-nitropyridine,
- 2-[4-(2-dipropylaminothiazol-4-yl)phenylamino]-6-[(N-ethyl-2-hydroxyethyl)amino]-3-nitropyridine,
- 2-[4-(2-dipropylaminothiazol-4-yl)phenylamino]-6-(piperazin-1-yl)-3-nitropyridine,
- 2-[4-(2-dipropylaminothiazol-4-yl)phenylamino]-6-(4-methylpiperazin-1-yl)-3-nitropyridine,
- 2-[4-(2-dipropylaminopropylthiazol-4-yl)phenylamino]-6-(4-aminopiperidino)-3-nitropyridine,
- 2-[4-(2-dipropylaminothiazol-4-yl)phenylamino]-6-[(3-pyridyl)methylamino]-3-nitropyridine,
- 2-[(3-fluoro-4-diethylamino)phenylamino]-6-(methylamino)-3-nitropyridine,
- 2-[(3-fluoro-4-diethylamino)phenylamino]-6-(isopropylamino)-3-nitropyridine,
- 2-[(3-fluoro-4-diethylamino)phenylamino]-6-(isobutylamino)-3-nitropyridine,
- 2-[(3-fluoro-4-diethylamino)phenylamino]-6-(t-butylamino)-3-nitropyridine,
- 2-[(3-fluoro-4-diethylamino)phenylamino]-6-(4-hydroxypiperidino)-3-nitropyridine,
- 2-[(3-fluoro-4-diethylamino)phenylamino]-6-[(2-isopropyl)imidazol-1-yl]-3-nitropyridine,
- 2-[(3-fluoro-4-diethylamino)phenylamino]-6-[(2-methyl-4,5-dihydro)imidazol-1-yl]-3-nitropyridine,
- 2-[(3-fluoro-4-diethylamino)phenylamino]-6-(piperazin-1-yl)-3-nitropyridine,
- 2-[(3-fluoro-4-diethylamino)phenylamino]-6-(4-methylpiperazin-1-yl)-3-nitropyridine,
- 2-[(3-fluoro-4-diethylamino)phenylamino]-6-morpholino-3-nitropyridine,
- 2-[(3-fluoro-4-diethylamino)phenylamino]-6-[(3-pyridyl)methylamino]-3-nitropyridine,
- 2-[(3-fluoro-4-diethylamino)phenylamino]-6-[(4-pyridyl)methylamino]-3-nitropyridine,
- 2-[(3-fluoro-4-diethylamino)phenylamino]-6-(4-aminopiperidino)-3-nitropyridine,
- 2-[(3-fluoro-4-diethylamino)phenylamino]-6-[2-(morpholin-1-yl)ethylamino]-3-nitropyridine,
- 2-[(3-fluoro-4-diethylamino)phenylamino]-6-[(3-imidazol-1-yl)propylamino]-3-nitropyridine,
- 2-[(3-fluoro-4-diethylamino)phenylamino]-6-[(3-morpholin-1-yl)propylamino]-3-nitropyridine,
- 2-[(3-fluoro-4-morpholino)phenylamino]-6-(methylamino)-3-nitropyridine,
- 2-[(3-fluoro-4-morpholino)phenylamino]-6-(isopropylamino)-3-nitropyridine,
- 2-[(3-fluoro-4-morpholino)phenylamino]-6-(isobutylamino)-3-nitropyridine,
- 2-[(3-fluoro-4-morpholino)phenylamino]-6-(4-hydroxypiperidino)-3-nitropyridine,
- 2-[(3-fluoro-4-morpholino)phenylamino]-6-[(2-methyl-4,5-dihydro)imidazol-1-yl]-3-nitropyridine,
- 2-[(3-fluoro-4-morpholino)phenylamino]-6-[(2-isopropyl)imidazol-1-yl]-3-nitropyridine,
- 2-[(3-fluoro-4-morpholino)phenylamino]-6-[(3-pyridyl)methylamino]-3-nitropyridine,
- 2-[(3-fluoro-4-morpholino)phenylamino]-6-[(4-pyridyl)methylamino]-3-nitropyridine,
- 2-[(3-fluoro-4-morpholino)phenylamino]-6-(t-butylamino)-3-nitropyridine,
- 2-[(3-fluoro-4-morpholino)phenylamino]-6-(4-methylpiperazin-1-yl)-3-nitropyridine,
- 2-[(3-fluoro-4-morpholino)phenylamino]-6-(piperazin-1-yl)-3-nitropyridine,
- 2-[(3-fluoro-4-morpholino)phenylamino]-6-(4-aminopiperidino)-3-nitropyridine,

- 2-[(3-fluoro-4-thiomorpholino)phenylamino]-6-(methylamino)-3-nitropyridine,
- 2-[(3-fluoro-4-thiomorpholino)phenylamino]-6-(isopropylamino)-3-nitropyridine,
- 2-[(3-fluoro-4-thiomorpholino)phenylamino]-6-(isobutylamino)-3-nitropyridine,
- 2-[(3-fluoro-4-thiomorpholino)phenylamino]-6-(4-hydroxypiperidino)-3-nitropyridine,
- 2-[(3-fluoro-4-thiomorpholino)phenylamino]-6-[(2-methyl-4,5-dihydro)imidazol-1-yl]-3-nitropyridine,
- 2-[(3-fluoro-4-thiomorpholino)phenylamino]-6-[(2-isopropyl)imidazol-1-yl]-3-nitropyridine,
- 2-[(3-fluoro-4-thiomorpholino)phenylamino]-6-[(3-pyridyl)methylamino]-3-nitropyridine,
- 2-[(3-fluoro-4-thiomorpholino)phenylamino]-6-[(4-pyridyl)methylamino]-3-nitropyridine,
- 2-[(3-fluoro-4-thiomorpholino)phenylamino]-6-(t-butylamino)-3-nitropyridine,
- 2-[(3-fluoro-4-thiomorpholino)phenylamino]-6-(4-methylpiperazin-1-yl)-3-nitropyridine,
- 2-[(3-fluoro-4-thiomorpholino)phenylamino]-6-(piperazin-1-yl)-3-nitropyridine,
- 2-[(3-fluoro-4-thiomorpholino)phenylamino]-6-(4-aminopiperidino)-3-nitropyridine,
- 2-[(3-fluoro-4-piperazino)phenylamino]-6-(methylamino)-3-nitropyridine,
- 2-[(3-fluoro-4-piperazino)phenylamino]-6-(isopropylamino)-3-nitropyridine,
- 2-[(3-fluoro-4-piperazino)phenylamino]-6-(isobutylamino)-3-nitropyridine,
- 2-[(3-fluoro-4-piperazino)phenylamino]-6-(4-hydroxypiperidino)-3-nitropyridine,
- 2-[(3-fluoro-4-piperazino)phenylamino]-6-[(2-isopropyl)imidazol-1-yl]-3-nitropyridine,
- 2-[(3-fluoro-4-piperazino)phenylamino]-6-[(3-pyridyl)methylamino]-3-nitropyridine,
- 2-[(3-fluoro-4-piperazino)phenylamino]-6-[(4-pyridyl)methylamino]-3-nitropyridine,
- 2-[(3-fluoro-4-piperazino)phenylamino]-6-(t-butylamino)-3-nitropyridine,
- 2-[(3-fluoro-4-piperidino)phenylamino]-6-(methylamino)-3-nitropyridine,
- 2-[(3-fluoro-4-piperidino)phenylamino]-6-(isopropylamino)-3-nitropyridine,
- 2-[(3-fluoro-4-piperidino)phenylamino]-6-(isobutylamino)-3-nitropyridine,
- 2-[(3-fluoro-4-piperidino)phenylamino]-6-(4-hydroxypiperidino)-3-nitropyridine,
- 2-[(3-fluoro-4-piperidino)phenylamino]-6-[(2-methyl-4,5-dihydro)imidazol-1-yl]-3-nitropyridine,
- 2-[(3-fluoro-4-piperidino)phenylamino]-6-[(2-isopropyl)imidazol-1-yl]-3-nitropyridine,
- 2-[(3-fluoro-4-piperidino)phenylamino]-6-[(3-pyridyl)methylamino]-3-nitropyridine,
- 2-[(3-fluoro-4-piperidino)phenylamino]-6-[(4-pyridyl)methylamino]-3-nitropyridine,
- 2-[(3-fluoro-4-piperidino)phenylamino]-6-(t-butylamino)-3-nitropyridine,
- 2-[(3-fluoro-4-piperidino)phenylamino]-6-(4-methylpiperazin-1-yl)-3-nitropyridine,
- 2-[(3-fluoro-4-piperidino)phenylamino]-6-(piperazin-1-yl)-3-nitropyridine,
- 2-[(3-fluoro-4-piperidino)phenylamino]-6-(4-aminopiperidino)-3-nitropyridine,
- 2-[(3-fluoro-4-piperidino)phenylamino]-6-morpholino-3-nitropyridine,
- 2-{[3-fluoro-4-(4-hydroxypiperidino)]phenylamino}-6-(methylamino)-3-nitropyridine,
- 2-{[3-fluoro-4-(4-hydroxypiperidino)]phenylamino}-6-(isopropylamino)-3-nitropyridine,
- 2-{[3-fluoro-4-(4-hydroxypiperidino)]phenylamino}-6-(isobutylamino)-3-nitropyridine,
- 2-{[3-fluoro-4-(4-hydroxypiperidino)]phenylamino}-6-(4-hydroxypiperidino)-3-nitropyridine,
- 2-{[3-fluoro-4-(4-hydroxypiperidino)]phenylamino}-6-[(2-methyl-4,5-dihydro)imidazol-1-yl]-3-nitropyridine,
- 2-{[3-fluoro-4-(4-hydroxypiperidino)]phenylamino}-6-[(3-pyridyl)methylamino]-3-nitropyridine,
- 2-{[3-fluoro-4-(4-hydroxypiperidino)]phenylamino}-6-[(4-pyridyl)methylamino]-3-nitropyridine,
- 2-{[3-fluoro-4-(4-hydroxypiperidino)]phenylamino}-6-(t-butylamino)-3-nitropyridine,
- 2-{[3-fluoro-4-(4-hydroxypiperidino)]phenylamino}-6-(4-methylpiperazin-1-yl)-3-nitropyridine,
- 2-{[3-fluoro-4-(4-hydroxypiperidino)]phenylamino}-6-(piperazin-1-yl)-3-nitropyridine,
- 2-{[3-fluoro-4-(4-hydroxypiperidino)]phenylamino}-6-(4-aminopiperidino)-3-nitropyridine,
- 2-{[3-fluoro-4-(4-hydroxypiperidino)]phenylamino}-6-morpholino-3-nitropyridine,
- 2-{[3-fluoro-4-(4-aminopiperidino)]phenylamino}-6-(methylamino)-3-nitropyridine,
- 2-{[3-fluoro-4-(4-aminopiperidino)]phenylamino}-6-(isopropylamino)-3-nitropyridine,
- 2-{[3-fluoro-4-(4-aminopiperidino)]phenylamino}-6-(isobutylamino)-3-nitropyridine,
- 2-{[3-fluoro-4-(4-aminopiperidino)]phenylamino}-6-(4-hydroxypiperidino)-3-nitropyridine,
- 2-{[3-fluoro-4-(4-aminopiperidino)]phenylamino}-6-[(2-methyl-4,5-dihydro)imidazol-1-yl]-3-nitropyridine,
- 2-{[3-fluoro-4-(4-aminopiperidino)]phenylamino}-6-(piperazin-1-yl)-3-nitropyridine,
- 2-{[3-fluoro-4-(4-aminopiperidino)]phenylamino}-6-(4-methylpiperazin-1-yl)-3-nitropyridine,
- 2-{[3-fluoro-4-(4-aminopiperidino)]phenylamino}-6-morpholino-3-nitropyridine,
- 2-{[3-fluoro-4-(4-aminopiperidino)]phenylamino}-6-(4-aminopiperidino-3-nitropyridine,
- 2-{[3-fluoro-4-(4-aminopiperidino)]phenylamino}-6-[(3-pyridyl)methylamino]-3-nitropyridine,
- 2-{[3-fluoro-4-(4-aminopiperidino)]phenylamino}-6-[(4-pyridyl)methylamino]-3-nitropyridine,
- 2-{[3-fluoro-4-(4-aminopiperidino)]phenylamino}-6-[2-(morpholin-1-yl)ethylamino]-3-nitropyridine,
- 2-{[3-fluoro-4-(4-aminopiperidino)]phenylamino}-6-[(3-morpholin-1-yl)propylamino]-3-nitropyridine,

- 2-{[3-fluoro-4-(2-methylpiperidino)]phenylamino}-6-(methylamino)-3-nitropyridine,
- 2-{[3-fluoro-4-(2-methylpiperidino)]phenylamino}-6-(isopropylamino)-3-nitropyridine,
- 2-{[3-fluoro-4-(2-methylpiperidino)]phenylamino}-6-(isobutylamino)-3-nitropyridine,
- 2-{[3-fluoro-4-(2-methylpiperidino)]phenylamino}-6-(t-butylamino)-3-nitropyridine,
- 2-{[3-fluoro-4-(2-methylpiperidino)]phenylamino}-6-(4-hydroxypiperidino)-3-nitropyridine,
- 2-{[3-fluoro-4-(2-methylpiperidino)]phenylamino}-6-[(2-methyl-4,5-dihydro)imidazol-1-yl]-3-nitropyridine,
- 2-{[3-fluoro-4-(2-methylpiperidino)]phenylamino}-6-(piperazin-1-yl)-3-nitropyridine,
- 2-{[3-fluoro-4-(2-methylpiperidino)]phenylamino}-6-(4-methylpiperazin-1-yl)-3-nitropyridine,
- 2-{[3-fluoro-4-(2-methylpiperidino)]phenylamino}-6-morpholino-3-nitropyridine,
- 2-{[3-fluoro-4-(2-methylpiperidino)]phenylamino}-6-(4-aminopiperidino)-3-nitropyridine,
- 2-{[3-fluoro-4-(2-methylpiperidino)]phenylamino}-6-[(4-pyridyl)methylamino]-3-nitropyridine
- 2-{[3-fluoro-4-(2-methylpiperidino)]phenylamino}-6-[(3-imidazol-1-yl)propylamino]-3-nitropyridine,
- 2-{[3-fluoro-4-(2-methylpiperidino)]phenylamino}-6-[2-(morpholin-1-yl)ethylamino]-3-nitropyridine,
- 2-{[3-fluoro-4-(2-methylpiperidino)]phenylamino}-6-[(3-morpholin-1-yl)propylamino]-3-nitropyridine,
- 2-{[3-fluoro-4-(3-hydroxymethylpiperidino)]phenylamino}-6-(methylamino)-3-nitropyridine,
- 2-{[3-fluoro-4-(3-hydroxymethylpiperidino)]phenylamino}-6-(isopropylamino)-3-nitropyridine,
- 2-{[3-fluoro-4-(3-hydroxymethylpiperidino)]phenylamino}-6-(isobutylamino)-3-nitropyridine,
- 2-{[3-fluoro-4-(3-hydroxymethylpiperidino)]phenylamino}-6-(t-butylamino)-3-nitropyridine,
- -{[3-fluoro-4-(3-hydroxymethylpiperidino)]phenylamino}-6-(4-hydroxypiperidino)-3-nitropyridine,
- 2-{[3-fluoro-4-(3-hydroxymethylpiperidino)]phenylamino}-6-[(2-methyl-4,5-dihydro)imidazol-1-yl]-3-nitropyridine,
- 2-{[3-fluoro-4-(3-hydroxymethylpiperidino)]phenylamino}-6-(piperazin-1-yl)-3-nitropyridine,
- 2-{[3-fluoro-4-(3-hydroxymethylpiperidino)]phenylamino}-6-(4-methylpiperazin-1-yl)-3-nitropyridine,
- 2-{[3-fluoro-4-(3-hydroxymethylpiperidino)]phenylamino}-6-morpholino-3-nitropyridine,
- 2-{[3-fluoro-4-(3-hydroxymethylpiperidino)]phenylamino}-6-(4-aminopiperidino)-3-nitropyridine,
- 2-{[3-fluoro-4-(3-hydroxymethylpiperidino)]phenylamino}-6-[(3-pyridyl)methylamino]-3-nitropyridine,
- 2-{[3-fluoro-4-(3-hydroxymethylpiperidino)]phenylamino}-6-[(4-pyridyl)methylamino]-3-nitropyridine,
- 2-{[3-fluoro-4-(3-hydroxymethylpiperidino)]phenylamino}-6-[2-(2-pyridyl)ethylamino]-3-nitropyridine,
- 2-{[3-fluoro-4-(3-hydroxymethylpiperidino)]phenylamino}-6-(cyclopropylamino)-3-nitropyridine,
- 2-{[3-fluoro-4-(4-carbamoylpiperidino)]phenylamino}-6-(methylamino)-3-nitropyridine,
- 2-{[3-fluoro-4-(4-carbamoylpiperidino)]phenylamino}-6-(isopropylamino)-3-nitropyridine,
- 2-{[3-fluoro-4-(4-carbamoylpiperidino)]phenylamino}-6-(isobutylamino)-3-nitropyridine,
- 2-{[3-fluoro-4-(4-carbamoylpiperidino)]phenylamino}-6-(t-butylamino)-3-nitropyridine,
- 2-{[3-fluoro-4-(4-carbamoylpiperidino)]phenylamino}-6-(4-hydroxypiperidino)-3-nitropyridine,
- 2-{[3-fluoro-4-(4-carbamoylpiperidino)]phenylamino}-6-(piperazin-1-yl)-3-nitropyridine,
- 2-{[3-fluoro-4-(4-carbamoylpiperidino)]phenylamino}-6-(4-methylpiperazin-1-yl)-3-nitropyridine,
- 2-{[3-fluoro-4-(4-carbamoylpiperidino)]phenylamino}-6-morpholino-3-nitropyridine,
- 2-{[3-fluoro-4-(4-carbamoylpiperidino)]phenylamino}-6-(4-aminopiperidino)-3-nitropyridine,
- 2-{[3-fluoro-4-(4-carbamoylpiperidino)]phenylamino}-6-[(4-pyridyl)methylamino]-3-nitropyridine,
- 2-{[3-fluoro-4-(4-carbamoylpiperidino)]phenylamino}-6-[(3-imidazol-1-yl)propylamino]-3-nitropyridine,
- 2-{[3-fluoro-4-(4-carbamoylpiperidino)]phenylamino}-6-[2-(morpholin-1-yl)ethylamino]-3-nitropyridine,
- 2-{[3-fluoro-4-(3-carbamoylpiperidino)]phenylamino}-6-(methylamino)-3-nitropyridine,
- 2-{[3-fluoro-4-(3-carbamoylpiperidino)]phenylamino}-6-(isopropylamino)-3-nitropyridine,
- 2-{[3-fluoro-4-(3-carbamoylpiperidino)]phenylamino}-6-(isobutylamino)-3-nitropyridine,
- 2-{[3-fluoro-4-(3-carbamoylpiperidino)]phenylamino}-6-(t-butylamino)-3-nitropyridine,
- 2-{[3-fluoro-4-(3-carbamoylpiperidino)]phenylamino}-6-(4-hydroxypiperidino-3-nitropyridine,
- 2-{[3-fluoro-4-(3-carbamoylpiperidino)]phenylamino}-6-(piperazin-1-yl)-3-nitropyridine,
- 2-{[3-fluoro-4-(3-carbamoylpiperidino)]phenylamino}-6-(4-methylpiperazin-1-yl)-3-nitropyridine,
- 2-{[3-fluoro-4-(3-carbamoylpiperidino)]phenylamino}-6-morpholino-3-nitropyridine,
- 2-{[3-fluoro-4-(3-carbamoylpiperidino)]phenylamino}-6-(4-aminopiperidino)-3-nitropyridine,
- 2-{[3-fluoro-4-(3-carbamoylpiperidino)]phenylamino}-6-[(3-pyridyl)methylamino]-3-nitropyridine,
- 2-{[3-fluoro-4-(3-carbamoylpiperidino)]phenylamino}-6-[(4-pyridyl)methylamino]-3-nitropyridine,
- 2-{[3-fluoro-4-(3-carbamoylpiperidino)]phenylamino}-6-[(3-imidazol-1-yl)propylamino]-3-nitropyridine,
- 2-{[3-fluoro-4-(3-carbamoylpiperidino)]phenylamino}-6-[2-(morpholin-1-yl)ethylamino]-3-nitropyridine,
- 2-{[3-fluoro-4-(3-carbamoylpiperidino)]phenylamino}-6-[(3-morpholin-1-yl)propylamino]-3-nitropyridine,
- 2-{[3-fluoro-4-(3-carbamoylpiperidino)]phenylamino}-6-(diethylamino)-3-nitropyridine,
- 2-{[3-fluoro-4-(4-carboxypiperidino)]phenylamino}-6-(methylamino)-3-nitropyridine,
- 2-{[3-fluoro-4-(4-carboxypiperidino)]phenylamino}-6-(isopropylamino)-3-nitropyridine,
- 2-{[3-fluoro-4-(4-carboxypiperidino)]phenylamino}-6-(isobutylamino)-3-nitropyridine,

- 2-{[3-fluoro-4-(4-carboxypiperidino)]phenylamino}-6-(4-hydroxypiperidino)-3-nitropyridine,
- 2-{[3-fluoro-4-(4-carboxypiperidino)]phenylamino}-6-(4-methylpiperazin-1-yl)-3-nitropyridine,
- 2-{[3-fluoro-4-(4-carboxypiperidino)]phenylamino}-6-[(3-pyridyl)methylamino]-3-nitropyridine,
- 2-{[3-fluoro-4-(4-carboxypiperidino)]phenylamino}-6-[(4-pyridyl)methylamino]-3-nitropyridine,
- 2-{[3-fluoro-4-(4-carboxypiperidino)]phenylamino}-6-[(3-imidazol-1-yl)propylamino]-3-nitropyridine,
- 2-{[3-fluoro-4-(4-carboxypiperidino)]phenylamino}-6-[2-(morpholin-1-yl)ethylamino]-3-nitropyridine, and
- 2-{[3-fluoro-4-(4-carboxypiperidino)]phenylamino}-6-[(3-morpholin-1-yl)propylamino]-3-nitropyridine.

With regard to the compound of formula 1 in accordance with the present invention, the pharmaceutically acceptable salt refers to a salt with a pharmaceutically acceptable free acid. The free acid may be an inorganic or organic acid. Examples of the inorganic acid include hydrochloric acid, hydrobromic acid, sulfuric acid, and phosphoric acid. Examples of the organic acid include citric acid, acetic acid, lactic acid, tartaric acid, fumaric acid, formic acid, propionic acid, oxalic acid, trifluoroacetic acid, methanesulfonic acid, benzenesulfonic acid, maleic acid, benzoic acid, gluconic acid, glycolic acid, succinic acid, 4-morpholine-ethanesulfonic acid, camphorsulfonic acid, 4-nitrobenzenesulfonic acid, hydroxy-O-sulfonic acid, 4-toluenesulfonic acid, galacturonic acid, embonic acid, glutamic acid, and aspartic acid. Preferably, the inorganic acid is hydrochloric acid, and the organic acid is methanesulfonic acid.

Further, the present invention provides a method for preparing a 2,6-substituted-3-nitropyridine derivative compound of formula 1,

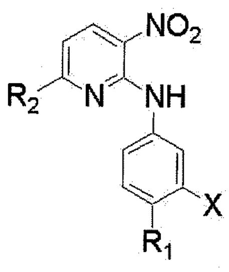

**Formula 1**

which includes the following steps:

(a) a step of reacting 2,6-dichloro-3-nitropyridine with an aniline compound of formula 3

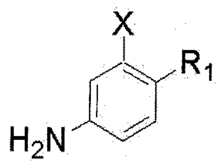

**Formula 3**

in the presence of a base to prepare a 6-chloro-3-nitropyridine derivative compound of formula 4,

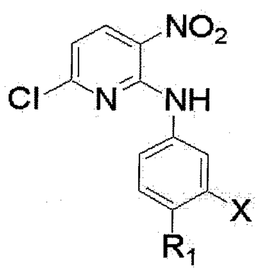

**Formula 4**

and

(b) a step of reacting the compound of formula 4 prepared in Step a) with an amine compound of formula 5

$HNR_5(CH_2)_n,R_6$ Formula 5

to prepare a 2,6-substituted-3-nitropyridine derivative compound of formula 1.

In the above formulae, $R_1$, $R_2$, $R_5$, $R_6$, n and X are as defined in the compound of formula 1 hereinbefore.

In Step a) of the above-mentioned preparation method, 2,6-dichloro-3-nitropyridine and the aniline compound of formula 3 used as a starting material and a reactant are easily commercially available or may be prepared by a known method.

In Step a) of the above-mentioned preparation method, the base may be appropriately selected and used from an organic base and an inorganic base. For example, a common tertiary organic base such as triethylamine, N,N-diisopropylethylamine, N-methylmorpholine, N-methylpiperidine, 4-dimethylaminopyridine, N,N-dimethylaniline, 2,6-lutidine or pyridine is preferably used as the organic base, and sodium hydroxide or sodium hydride is preferably used as the inorganic base.

In Step a) or Step b) of the above-mentioned preparation method, the reaction solvent used is preferably selected from alcohols such as methanol, ethanol and isopropanol, acetonitrile, chloroform, methylene chloride, tetrahydrofuran, N,N-dimethylformamide, N-methylpyrrolidinone and any combination thereof. Although the reaction temperature of Step a) or Step b) may vary depending on the type of the reaction solvent or amine of formula 5, it is preferably in the range of 25 to 80°C.

Further, the present invention provides a method for preparing a 2,6-substituted-3-nitropyridine derivative compound of formula 1, wherein the compound of formula 3 is prepared by a preparation method including the following steps:

(a) a step of subjecting a 4-nitrophenone compound of formula 6

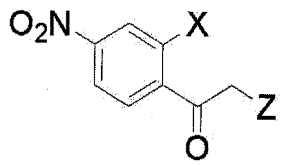

**Formula 6**

to bromination at the alpha position with respect to the carboxyl group thereof to prepare a compound of formula 7;

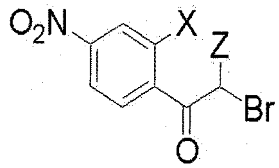

**Formula 7**

(b) a step of reacting the compound of formula 7 prepared in Step a) with a thioamide compound of formula 8

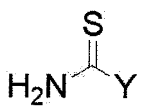

**Formula 8**

to prepare a compound of formula 9

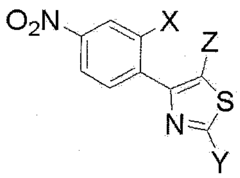

**Formula 9**

and

(c) a step of subjecting the compound of formula 9 prepared in Step b) to hydrogenation, thereby preparing the compound of formula 3.

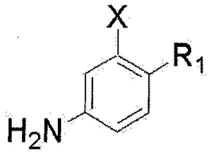

**Formula 3**

In the above formulae, X, Z and Y are as defined in the compound of formula 1 hereinbefore, and $R_1$ represents a thiazolyl group

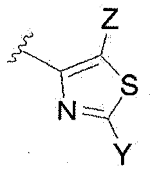

In the above-mentioned preparation method, the reagent used for the bromination reaction of Step a) is preferably copper (II) bromide or bromine. Further, the reaction temperature is preferably in a range of 20 to 80°C, and the reaction time is preferably in a range of 8 to 24 hours. The reaction solvent used may be ethyl acetate, dichloromethane or the like. Ethyl acetate is more preferable.

In the above-mentioned preparation method, the compound of formula 8 in Step b) is commercially available or may be prepared by a known method. Examples of such a compound include thioacetamide, thiopropionamide, thioisobutyramide, trimethylthioacetamide, thiohexanoamide, cyclohexancarbothioic acid amide, piperidine-4-carbothioic acid amide, thiourea, N-methylthiourea, N-ethylthiourea, N,N-dipropylthiourea, and thiobenzamide.

In the above-mentioned preparation method, the reaction temperature and time of Step b) may vary depending on the type of the thioamide compound of formula 8. The reaction is preferably carried out at a temperature of 60 to 90°C for 5 to 24 hours. Ethanol as a single solvent or a mixed solvent of ethanol and water is preferably used as the reaction solvent.

In the above-mentioned preparation method, the hydrogenation reaction of Step c) is preferably carried out under hydrogen gas in the presence of a Pd/C catalyst or a Raney nickel catalyst. For example, the reaction is preferably carried out using 10% palladium/active carbon or Raney nickel in an amount of 10% to 20% of the weight of the compound of formula 9 prepared in Step b) at room temperature under 3 to 5 bar of hydrogen gas for 2 hours to 8 hours. The solvent used is preferably ethyl acetate, methanol, ethanol or any combination thereof.

Further, the present invention provides a method for preparing a 2,6-substituted-3-nitropyridine derivative compound of formula 1, wherein the compound of formula 3 is prepared by a preparation method including the following steps:

(a) a step of reacting a 3,4-difluoronitrobenzene compound with a compound of formula 10

$HR_1$ Formula 10

in the presence of an organic base to prepare a nitrobenzene compound of formula 11;

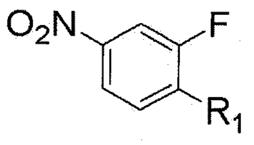

**Formula 11**

and

(b) a step of subjecting the compound of formula 11 prepared in Step a) to hydrogenation, thereby preparing the compound of formula 3

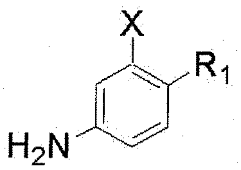

**Formula 3.**

In the above formulae,

$R_1$ represents $NR_3R_4$ wherein $R_3$ and $R_4$ taken together form a saturated or unsaturated 5-, 6- or 7-membered heterocyclic amino compound which contains 1 to 3 hetero atoms selected from N, O and S and is unsubstituted or substituted by a $C_1$-$C_3$ alkyl group, a hydroxyl group, a $C_1$-$C_3$ hydroxyalkyl group, an amino group, a carbamoyl group or a carboxyl group, and

X represents a fluoro group.

In the above-mentioned preparation method, the compound of formula 10 of Step a) is preferably diethylamine, morpholine, thiomorpholine, unsubstituted or substituted piperazine, piperidine, methylpiperidine, hydroxypiperidine, hydroxymethylpiperidine, hydroxyethylpiperidine, aminopiperidine, 3- or 4-carbamoylpiperidine, carboxypiperidine or pyrrolidine, each of which is commercially available or may be conveniently synthesized by a method known to those skilled in the art.

In the above-mentioned preparation method, the reaction temperature and time of Step a) may vary depending on the type of the substituted amine compound of formula 10. The reaction is preferably carried out at a temperature of 60 to 90°C for 5 to 24 hours. The reaction solvent is preferably an alcohol solvent such as methanol or ethanol.

In the above-mentioned preparation method, the organic base of Step a) is preferably at least one selected from triethylamine, N,N-diisopropylethylamine, N-methylmorpholine, N-methylpiperidine, 4-dimethylaminopyridine, N, N-dimethylaniline, 2,6-lutidine and pyridine.

In the above-mentioned preparation method, the hydrogenation reaction of Step b) is preferably carried out under hydrogen gas in the presence of a Pd/C catalyst or a Raney nickel catalyst. For example, the reaction is preferably carried out using, as a catalyst, 10% palladium/active carbon or Raney nickel in an amount of 10% to 20% of the weight of the compound of formula 11 prepared in Step a) at room temperature under 3 to 5 bar of hydrogen gas for 2 hours to 8 hours. The solvent used is preferably ethyl acetate, methanol, ethanol or any combination thereof.

Further, the present invention provides a pharmaceutical composition for the prevention or treatment of osteoporosis, containing the 2,6-substituted-3-nitropyridine derivative compound of the present invention or a pharmaceutically acceptable salt thereof as an active ingredient.

Here, the pharmaceutically acceptable salt is the same as illustrated in the pharmaceutically acceptable salt of the 2,6-substituted-3-nitropyridine derivative compound of the present invention hereinbefore.

Further, the present invention provides a method for the prevention or treatment of osteoporosis, including

administering an effective amount of the above-mentioned compound of formula 1 or a pharmaceutically acceptable salt thereof to a mammal including a human in need thereof.

Further, the present invention provides use of the above-mentioned compound of formula 1 or a pharmaceutically acceptable salt thereof, for manufacturing a pharmaceutical preparation for the prevention or treatment of osteoporosis.

The term "osteoporosis" as used herein means the state that minerals and matrices for forming the bone are reduced abnormally in large amounts, even without any defect in the structure of the remaining bone, so that many pores are generated in the bone, making it like a sponge and more likely to fracture. This condition is also referred to as "osteopenia". In specific embodiments, the 2,6-substituted-3-nitropyridine derivative compound of formula 1 in accordance with the present invention not only promotes the activity of osteoblasts to thereby effectively increase osteogenesis, but also suppresses the formation of osteoclasts to inhibit osteoclastic bone absorption. Thus, the 2,6-substituted-3-nitropyridine derivative compound of the present invention or a pharmaceutically acceptable salt thereof can be beneficially used for the prevention and treatment of osteoporosis.

The composition of the present invention may contain one or more active ingredients which are equivalent or similar in function to the nitropyridine derivative of the present invention, in addition to the 2,6-substituted-3-nitropyridine derivative or a pharmaceutically acceptable salt thereof.

The composition of the present invention which further contains one or more pharmaceutically acceptable carriers in addition to the above-described ingredients may be prepared. The pharmaceutically acceptable carrier may be saline, sterile water, a Ringer's solution, buffered saline, a dextrose solution, a maltodextrin solution, glycerol, ethanol or any combination thereof, and may be, if necessary, further supplemented with other typical additives such as an antioxidant, a buffer and a bacteriostatic agent. In combination with a diluent, a dispersant, a surfactant, a binder and a lubricant, the composition of the present invention may also be formulated into injectable dosage forms, such as an aqueous solution, a suspension and an emulsion, pills, capsules, granules, or tablets. Moreover, depending on the kind of the ingredient or the disease, the formulation may be preferably prepared using an appropriate method known in the art or disclosed in Remington's Pharmaceutical Sciences (latest edition), Mack Publishing Company, Easton, PA.

The composition of the present invention may be administered orally or parenterally (e.g., intravenously, subcutaneously, intraperitoneally or topically) depending on applications. The dosage varies depending on body weight, age, gender, and health state of the patient, diet, administration time period, administration route, excretion rate, and severity of disease. The derivative compound of formula 1 in accordance with the present invention is administered once or several times at a daily dose of approximately 10 to 1,000 mg/kg and preferably at a daily dose of approximately 50 to 500 mg/kg.

For the prevention and treatment of osteoporosis, the composition of the present invention may be used alone or in combination with surgery, hormone therapy, chemical therapy, and use of a biological response modulator.

## ADVANTAGEOUS EFFECTS

A novel 2,6-substituted-3-nitropyridine derivative compound of the present invention not only promotes the activity of osteoblasts to thereby effectively facilitate osteogenesis but also suppresses the formation of osteoclasts to inhibit osteoclastic bone absorption and therefore can be beneficially used for the prevention and treatment of osteoporosis.

## MODE FOR INVENTION

A better understanding of the present invention may be obtained through the following preferable Preparation Examples and Examples, which are set forth to illustrate, but are not to be construed as the limit of the present invention.

In the experimental part the following abbreviations are used:

"()" in column of "Amine" of the following tables means amounts used based on the starting material.

A = In the following tables (number) means equivalents used based on the starting material, "#" means additional use of triethylamine, and "X" means no additional use of triethylamine.

M+ = Molecular weight

S = Solvent

T = Temperature

Y = Yield

Unless otherwise specified, reagents and solvents referred hereinafter were purchased from Aldrich or Cambridge Isotope Laboratories, and $^1$H-NMR data were measured by a JNM-LA400 spectrometer (manufactured by JEOL) and Mass data were measured by a 1100MSD spectrometer (manufactured by Hewlett Packard).

**Preparation Example 1:**

**Preparation of a compound according to formula 4**

**Example 1-1**

**Preparation of 2-(4-methylphenylamino)-6-chloro-3-nitropyridine**

To 100ml of methanol were added 3g (15.5mmol) of 2,6-dichloronitropyridine and 2.6ml (18.7mmol) of triethylamine and 1.75g (16.03mmol) of p-toluidine was then added thereto, followed by reaction at room temperature (20 to 30°C) for about 5 hours. After the reaction was complete, 20ml of water was slowly added thereto, followed by stirring at room temperature for 1 hour. The reactant was filtered, washed with 20ml of a 4:1 (v/v) solution of methanol and water, and then dried under vacuum at about 40°C to afford 2.9g (yield: 71%) of the desired compound.
Mass (M+): 264.1
$^1$H-NMR (DMSO-$d_6$): 2.30(s, 3H), 6.94(d, 2H), 7.18(d, 2H), 7.45(d, 2H), 8.50(d, 1H), 10.07(s, 1H).

**Example 1-2**

**Preparation of 2-(4-methoxyphenylamino)-6-chloro-3-nitropyridine**

To 100ml of methanol were added 3g (15.5mmol) of 2,6-dichloronitropyridine and 2.6ml (18.7mmol) of triethylamine and 2g (16.3mmol) of p-anisidine was then added thereto, followed by reaction at room temperature (20 to 30°C) for about 5 hours. After the reaction was complete, the reactant was filtered, washed with 20ml of methanol and then dried under vacuum at about 40°C to afford 3.1g (yield: 72%) of the desired compound.
Mass (M+): 280.0
$^1$H-NMR (DMSO-$d_6$): 3.80(s, 3H), 6.95(m, 3H), 7.46(d, 2H), 8.51(d, 1H), 10.62(s, 1H).

**Example 1-3**

**Preparation of 2-[4-(t-butyl)phenylamino]-6-chloro-3-nitropyridine**

To 50ml of methanol were added 1.5g (7.77mmol) of 2,6-dichloronitropyridine and 1.2ml (8.55mmol) of triethylamine and 1.2ml (7.77mmol) of p-(t-butyl)aniline was then added thereto, followed by reaction at room temperature (20 to 30°C) for about 5 hours. After the reaction was complete, 5ml of water was slowly added thereto, followed by stirring at room temperature for 1 hour. The reactant was filtered, washed with 10ml of a 4:1 (v/v) solution of methanol and water, and then dried under vacuum at about 40°C to afford 1.8g (yield: 76%) of the desired compound.
Mass (M+): 306.1
$^1$H-NMR (DMSO-$d_6$): 1.29(s, 9H), 6.97(d, 1H), 7.40(d, 2H), 7.51(d, 2H), 8.52(d, 1H), 10.08(s, 1H).

**Example 1-4**

**Preparation of 2-(4-cyanophenylamino)-6-chloro-3-nitropyridine**

To 50ml of acetonitrile were added 1.35g (11.4mmol) of 4-aminobenzonitrile and 460mg (11.4mmol) of sodium hydroxide, followed by stirring at a temperature of 55 to 60°C for about 1 hour, and 2g (10.4mmol) of 2,6-dichloronitropyridine was added thereto. This solution was allowed to react at a temperature of 55 to 60°C for 20 hours, cooled to room temperature, extracted with 100ml of water and 100ml of methylene chloride, dried over anhydrous magnesium sulfate, filtered, and purified by column chromatography with a 4:1 (v/v) solution of n-hexane and ethyl acetate as a developing solvent to afford 1.3g (yield: 46%) of the desired compound.
Mass (H+): 275.0
$^1$H-NMR (DMSO-$d_6$): 7.14(d, 1H), 7.85(m, 4H), 8.58(d, 1H), 10.26(s, 1H).

**Example 1-5**

**Preparation of 2-[3-cyanophenylamino]-6-chloro-3-nitropyridine**

To 30ml of acetonitrile were added 650mg (5.5mmol) of 3-aminobenzonitrile and 230mg (5.5mmol) of sodium hydroxide, followed by stirring at a temperature of 55 to 60°C for about 1 hour, and 1g (5.2mmol) of 2,6-dichloronitropyridine was added thereto. This solution was allowed to react at a temperature of 55 to 60°C for 20 hours, cooled to room temperature, extracted with 100ml of water and 100ml of dichloromethane, dried over anhydrous magnesium sulfate, filtered, and purified by column chromatography with a 4:1 (v/v) solution of n-hexane and ethyl acetate as a developing solvent to afford 600mg (yield: 43%) of the desired compound.

Mass (M+): 275.0

$^1$H-NMR (DMSO-$d_6$): 7.16(d, 1H), 7.88(m, 4H), 8.54(d, 1H), 10.33(s, 1H).

**Example 1-6**

**Preparation of 2-(4-hydroxyphenylamino)-6-chloro-3-nitropyridine**

To 10ml of methanol were added 600mg (3.11mmol) of 2,6-dichloronitropyridine and 0.52ml (3.73mmol) of triethylamine and 355mg (3.27mmol) of 4-aminophenol was added thereto, followed by reaction at room temperature (20 to 30°C) for about 2 hours. The reaction solvent was removed, followed by column chromatography purification with a 3:1 (v/v) solution of n-hexane and ethyl acetate as a developing solvent and vacuum drying at about 40°C to afford 640mg (yield: 78%) of the desired compound.

Mass (M+): 266.0

$^1$H-NMR (DMSO-$d_6$): 6.78((d, 2H), 6.91(d, 1H), 7.31(d, 2H), 8.50(d, 2H), 9.47(s, 1H), 10.00(s, 1H).

**Example 1-7**

**Preparation of 2-(4-methylsulfanylphenylamino)-6-chloro-3-nitropyridine**

To 20ml of methanol were added 500mg (2.59mmol) of 2,6-dichloronitropyridine and 0.4ml (2.85mmol) of triethylamine and 0.34ml (2.72mmol) of 4-(methylthio)aniline was then added thereto, followed by reaction at room temperature (20 to 30°C) for about 23 hours. After the reaction was complete, 5ml of water was slowly added thereto, followed by stirring at room temperature for 1 hour. The reactant was filtered, washed with 10ml of a 1:1 (v/v) solution of methanol and water, and then dried under vacuum at about 40°C to afford 480mg (yield: 63%) of the desired compound.

Mass (M+): 296.0

$^1$H-NMR (DMSO-$d_6$): 2.48(s, 3H), 6.99(d, 1H), 7.30(dd, 1H), 7.55(dd, 2H), 8.53(d, 1H), 10.11(s, 1H).

**Example 1-8**

**Preparation of 2-[4-(n-butyl)phenylamino]-6-chloro-3-nitropyridine**

To 30ml of methanol were added 600mg (3.11mmol) of 2,6-dichloronitropyridine and 0.48ml (3.42mmol) of triethylamine and 0.48ml (3.11mmol) of 4-(n-butyl)aniline was then added thereto, followed by reaction at room temperature (20 to 30°C) for about 19 hours. After the reaction was complete, 5ml of water was slowly added thereto, followed by stirring at room temperature for 1 hour. The reactant was filtered, washed with 10ml of water, and then dried under vacuum at about 40°C to afford 653mg (yield: 69%) of the desired compound.

Mass (M+): 306.0

$^1$H-NMR (DMSO-$d_6$): 0.90(t, 3H), 1.32(q, 2H), 1.55(m, 2H), 2.58(t, 2H), 6.98(d, 1H), 7.21(d, 2H), 7.48(d, 2H), 8.53(d, 1H), 10.09(s, 1H).

**Example 1-9**

**Preparation of 2-(4-aminophenylamino)-6-chloro-3-nitropyridine**

To 100ml of methanol were added 5g (26mmol) of 2,6-dichloronitropyridine and 4ml (28.6mmol) of triethylamine and 2.8ml (26mmol) of p-phenylenediamine was added thereto at a temperature of 0 to 5°C, followed by reaction at the same temperature for about 2 hours. After the reaction was complete, 50ml of water was slowly added thereto, followed by stirring at room temperature for 1 hour. The reactant was filtered, washed with 10ml of water, and then dried under

vacuum at about 40°C to afford 6.52g (yield: 95%) of the desired compound.

Mass (M+): 265.0

$^1$H-NMR (DMSO-$d_6$): 5.47(s, 2H), 6.61(d, 2H), 6.86(d, 1H), 7.18(d, 2H), 8.47(d, 1H), 9.96(s, 1H).

**Example 1-10**

**Preparation of 2-(3-aminophenylamino)-6-chloro-3-nitropyridine**

To 200ml of methanol were added 10g (52mmol) of 2,6-dichloronitropyridine and 7.9ml (57mmol) of triethylamine and 6.2g (57mmol) of m-phenylenediamine was then added thereto at a temperature of 0 to 5°C, followed by reaction at the same temperature for about 2 days. After the reaction was complete, 50ml of water was slowly added thereto, followed by stirring at room temperature for 1 hour. The reactant was filtered, washed with 10ml of water, and then dried under vacuum at about 40°C to afford 8g (yield: 59%) of the desired compound.

Mass (M+): 265.0

$^1$H-NMR (DMSO-$d_6$): 5.39(m, 2H), 6.43(d, 1H), 6.77(s, 1H), 6.80(d, 1H), 6.96(d, 1H), 7.04(t, 1H), 8.52(d, 1H), 9.97(s, 1H).

**Example 1-11**

**Preparation of 2-[4-(imidazol-1-yl-)phenylamino]-6-chloro-3-nitropyridine**

To 150ml of methanol were added 4.12g (25.9mmol) of 4-(1H-imidazol-1-yl)aniline and 7.22ml (51.8mmol) of triethylamine, followed by stirring at room temperature (20 to 30°C) for about 30 minutes, and 5g (25.9mmol) of 2,6-dichloronitropyridine was added thereto, followed by reaction at a temperature of 30 to 35°C for 3 days. After being cooled to room temperature, the resulting solid was filtered and removed. The remaining solution was distilled under reduced pressure and purified by column chromatography with a 10:5:1 (v/v/v) mixed solvent of n-hexane:ethyl acetate: methanol as a developing solvent to afford 1.53g (yield: 19%) of the desired compound.

Mass (M+): 316.0

$^1$H-NMR (DMSO-$d_6$): 6.94(d, 1H), 7.48(s, 1H), 7.61(m, 3H), 7.96(d, 2H), 8.52(d, 1H), 9.22(s, 1H), 10.44(s, 1H).

**Example 1-12**

**Preparation of 2-(3-acetylphenylamino)-6-chloro-3-nitropyridine**

To 100ml of methanol were added 3g (15.5mmol) of 2,6-dichloronitropyridine and 2.4ml (17.1mmol) of triethylamine and 2.1g (15.5mmol) of 3-aminoacetophenone was then added thereto, followed by reaction at room temperature (20 to 30°C) for about 5 hours. After the reaction was complete, the reactant was filtered, washed with 20ml of methanol and then dried under vacuum at about 40°C to afford 3.7g (yield: 82%) of the desired compound.

Mass (M+): 292.0

$^1$H-NMR (DMSO-$d_6$): 2.60(s, 3H), 7.05(d, 2H), 7.56(m, 1H), 7.77(d, 2H), 7.87(d, 2H), 8.22(s, 2H), 8.56(d, 1H), 10.23(s, 1H).

**Example 1-13**

**Preparation of 2-(4-morpholinophenylamino)-6-chloro-3-nitropyridine**

To 50ml of methanol were added 2g (10.4mmol) of 2,6-dichloronitropyridine and 1.73ml (12.4mmol) of triethylamine and 1.94g (10.4mmol) of 4-morpholinoaniline was then added thereto, followed by reaction at room temperature (20 to 30°C) for about 5 hours. After the reaction was complete, the reactant was filtered, washed with 20ml of methanol and then dried under vacuum at about 40°C to afford 3.17g (yield: 91%) of the desired compound.

Mass (M+): 335.0

$^1$H-NMR (DMSO-$d_6$): 3.13(m, H), 3.74(brm, 4H), 6.93(d, 1H), 6.97(d, 2H), 7.42(d, 1H), 8.50(d, 1H), 10.05(s, 1H).

**Example 1-14**

**Preparation of 2-(3,4-difluorophenylamino)-6-chloro-3-nitropyridine**

To 200ml of methanol were added 3.5g (17.6mmol) of 2,6-dichloronitropyridine and 2.9ml (21mmol) of triethylamine and 3.5ml (19mmol) of 3,4-difluoroaniline was added thereto at room temperature (20 to 30°C), followed by reaction at the same temperature for about 24 hours. After the reaction was complete, 50ml of water was slowly added thereto, followed by stirring at room temperature for 1 hour. The reactant was filtered, washed with 10ml of water, and then dried under vacuum at about 40°C to afford 3g (yield: 60%) of the desired compound.

Mass (M+): 265.0

$^1$H-NMR (DMSO-$d_6$): 6.99(d, 1H), 7.19(t, 1H), 7.34(m, 1H), 7.54(d, 1H), 8.52(d, 1H), 10.07(s, 1H).

**Preparation Example 2**

**Preparation of a compound according to formula 4, wherein $R_1$ represents thiazole**

**Example 2-1-1**

**Preparation of a-bromo-4-nitroacetophenone**

5g (30.3mmol) of 4-nitroacetophenone was dissolved in 150ml of ethyl acetate and 13.5g (60.6mmol) of copper (II) bromide was added thereto, followed by stirring at a temperature of 60 to 65°C for 8 hours. After the reaction was complete, the reaction liquid was cooled to room temperature and the salt formed during the reaction was filtered off. The filtrate was washed three times with a sodium bicarbonate saturated solution. This solution was dried over anhydrous magnesium sulfate, filtered under reduced pressure, distilled under reduced pressure and then dried under vacuum at about 40°C to afford 7.3g (yield: 99%) of the desired compound which was then directly subjected to the subsequent reaction.

Mass (M+): 245.1

**Example 2-1-2**

**Preparation of 4-(2-methylthiazol-4-yl)nitrobenzene**

To 150ml of ethanol were added 7.3g (29.9mmol) of α-bromo-4-nitroacetophenone synthesized in Preparation Example 2-1-1 and 2.5g (32.3mmol) of thioacetamide, followed by reaction at a temperature of 60 to 65°C for 16 hours. After the reaction was complete, the reaction liquid was cooled to room temperature, and the resulting solid was filtered, washed with 50ml of methanol and then dried under vacuum at about 40°C to afford 4.3g (yield: 65%) of the desired compound.

Mass (M+): 221.2

$^1$H-NMR (DMSO-$d_6$): 2.74(s, 3H), 8.19(d, 2H), 8.28(m, 3H).

**Example 2-1-3**

**Preparation of 4-(2-methylthiazol-4-yl)aniline**

To 120ml of ethyl acetate were sequentially added 4g (18.2 mmol) of 4-(2-methylthiazol-4-yl)nitrobenzene synthesized in Preparation Example 2-1-2 and 400mg (10W%) of Pd/C, followed by reaction in a hydrogen reactor under hydrogen pressure of 4bar for 5 hours. After the reaction was complete, Pd/C was filtered through celite, and the filtrate was distilled under reduced pressure, purified by recrystallization from ethyl acetate and n-hexane and then dried under vacuum at about 40°C to afford 3.4g (yield: 99%) of the desired compound.

Mass (M+): 191.0

$^1$H-NMR (DMSO-$d_6$): 2.66(s, 3H), 5.27(s, 1H), 6.58(d, 2H), 7.47(s, 1H), 7.60(d, 2H).

**Example 2-1-4**

**Preparation of 2-[4-(2-methylthiazol-4-yl)phenylamino]-6-chloro-3-nitropyridine**

To 100ml of methanol were added 3.5g (18.1mmol) of 2,6-dichloronitropyridine and 3ml (21.7mmol) of triethylamine and 3.44g (18.2mmol) of 4-(2-methylthiazol-4-yl)aniline obtained in Preparation Example 2-1-3 was then added thereto, followed by reaction at room temperature (20 to 30°C) for about 24 hours. After the reaction was complete, the reactant was filtered, washed with 20ml of methanol, and then dried under vacuum at about 40°C to afford 4.4g (yield: 70%) of the desired compound.

Mass (M+): 347.0

$^{1}H$-NMR (DMSO-$d_6$): 2.71(s, 3H), 7.00(d, 1H), 7.67(d, 2H), 7.88(s, 1H), 7.94(d, 2H), 8.53(d, 1H), 10.18(s, 1H).

**Example 2-2-1**

**Preparation of 4-(2-isopropylthiazol-4-yl)nitrobenzene**

To 100ml of ethanol were added 5g (20.5mmol) of α-bromo-4-nitroacetophenone synthesized in Preparation Example 2-1-1 and 4.23g (41mmol) of thioisopropylamide, followed by reaction at a temperature of 60 to 65°C for 6 hours. After the reaction was complete, the reaction liquid was cooled to room temperature, and the resulting solid was filtered, washed with 50ml of methanol and then dried under vacuum at about 40°C to afford 4.85g (yield: 95%) of the desired compound.

Mass (M+): 249.1

$^{1}H$-NMR (DMSO-$d_6$): 1.37(d, 6H), 3.34(m, 1H), 8.22(d, 2H), 8.23(d, 2H), 8.28(s, 1H).

**Example 2-2-2**

**Preparation of 4-(2-isopropylthiazol-4-yl)aniline**

To 120ml of ethyl acetate were sequentially added 4.5g (18.1 mmol) of 4-(2-isopropylthiazol-4-yl)nitrobenzene synthesized in Preparation Example 2-2-1 and 450mg (10W%) of Pd/C, followed by reaction in a hydrogen reactor under hydrogen pressure of 4bar for 5 hours. After the reaction was complete, Pd/C was filtered through celite. The filtrate was distilled under reduced pressure and dried under vacuum at about 40°C to afford 3.9g (yield: 99%) of the desired compound.

Mass (M+): 218.0

$^{1}H$-NMR (DMSO-$d_6$): 1.34(d, 6H), 3.28(m, 1H), 5.26(d, 1H), 6.58(d, 2H), 7.51(s, 1H), 7.61(d, 2H).

**Example 2-2-3**

**Preparation of 2-[4-(2-isopropylthiazol-4-yl)phenylamino]-6-chloro-3-nitropyridine**

To 100ml of methanol were added 1.8g (9.33mmol) of 2,6-dichloronitropyridine and l.5ml (11.2mmol) of triethylamine and 2g (9.33mmol) of 4-(2-isopropylthiazol-4-yl)aniline obtained in Preparation Example 2-2-2 was added thereto, followed by reaction at room temperature (20 to 30°C) for about 24 hours. After the reaction was complete, the reactant was filtered, washed with 20ml of methanol and then dried under vacuum at about 40°C to afford 969mg (yield: 38%) of the desired compound.

Mass (M+): 375.1

$^{1}H$-NMR (DMSO-$d_6$): 1.38(d, 6H), 3.34(m, 1H), 7.04(d, 1H), 7.69(d, 2H), 7.96(m,3H), 8.56(d, 1H), 10.20(s, 1H).

**Example 2-3-1**

**Preparation of 4-(2-cyclohexylthiazol-4-yl)nitrobenzene**

To 100ml of ethanol were added 4.5g (18.44mmol) of α-bromo-4-nitroacetophenone synthesized in Preparation Example 2-1-1 and 5.3g (36.88mmol) of cyclohexylthioamide, followed by reaction at a temperature of 60 to 65°C for 18 hours. After the reaction was complete, the reaction liquid was cooled to room temperature. The resulting solid was filtered, washed with 50ml of methanol and then dried under vacuum at about 40°C to afford 3.8g (yield: 71%) of the desired compound.

Mass (M+): 289.1

$^{1}H$-NMR (DMSO-$d_6$): 1.28(m, 1H), 1.42(m, 2H), 1.51(m, 2H), 1.70(m, 1H), 1.77(m,2H), 3.07(m, 1H), 8.21(d, 2H), 8.29(d, 2H), 8.34(s, 1H).

**Example 2-3-2**

**Preparation of 4-(2-cyclohexylthiazol-4-yl)aniline**

To 150m1 of methanol were sequentially added 4.5g (18.1 mmol) of 4-(2-cyclohexylthiazol-4-yl)nitrobenzene synthesized in Preparation Example 2-3-1 and 450mg (10W%) of Pd/C, followed by reaction in a hydrogen reactor under hydrogen pressure of 4bar for 5 hours. After the reaction was complete, Pd/C was filtered through celite. The filtrate

was distilled under reduced pressure and dried under vacuum at about 40°C to afford 3.9g (yield: 99%) of the desired compound.

Mass (M+): 259.1

$^1$H-NMR (DMSO-$d_6$): 1.38(m, 1H), 1.44(m, 4H), 1.67(d, 1H), 1.80(m, 2H), 2.07(m, 2H), 2.99(m, 1H), 6.02(brs, 2H), 6.68(d, 2H), 7.56(s, 1H), 7.65(d, 2H)

**Example 2-3-3**

**Preparation of 2-[4-(2-cyclohexylthiazol-4-yl)phenylamino]-6-chloro-3-nitropyridine**

To 50ml of methanol were added 1g (5.18mmol) of 2,6-dichloronitropyridine and 0.87ml (6.22mmol) of triethylamine and 1.49g (5.18mmol) of 4-(2-cyclohexylthiazol-4-yl)aniline obtained in Preparation Example 2-3-2 was then added thereto, followed by reaction at room temperature (20 to 30°C) for about 32 hours. After the reaction was complete, the reactant was filtered, washed with 20ml of methanol and then dried under vacuum at about 40°C to afford 1.8g (yield: 84%) of the desired compound.

Mass (M+): 415.1

$^1$H-NMR (DMSO-$d_6$): 1.38(m, 1H), 1.51(m, 4H), 1.72(m, 1H), 1.80(m, 2H), 2.10(m, 2H), 3.04(m, 1H), 7.04(d, 1H), 7.70(d, 2H), 7.96(t, 3H), 8.56(d, 1H), 10.20(s, 1H).

**Example 2-4-1**

**Preparation of 4-(2-dipropylaminothiazol-4-yl)nitrobenzene**

To 100ml of ethanol were added 4g (18.44mmol) of α-bromo-4-nitroacetophenone synthesized in Preparation Example 2-1-1 and 3.15g (19.7mmol) of 1,1-dipropylthiourea, followed by reaction at a temperature of 60 to 65°C for 5 hours. After the reaction was complete, the reaction liquid was cooled to room temperature and 50ml of water was slowly added thereto. The resulting solid was filtered and washed with 50ml of a 1:1 (v/v) mixture of methanol and water to afford 3.85g (yield: 77%) of the desired compound.

Mass (M+): 376.1

$^1$H-NMR (DMSO-$d_6$): 0.91(t, 6H), 1.6(m, 4H), 3.40(t, 4H), 7.52(s, 1H), 8.09(d, 2H), 8.25(d, 2H).

**Example 2-4-2**

**Preparation of 4-(2-dipropylaminothiazol-4-yl)aniline**

To 150ml of methanol were sequentially added 3.8g (12.4 mmol) of 4-(2-dipropyl-aminothiazol-4-yl)nitrobenzene synthesized in Preparation Example 2-4-1 and 570mg (15W%)of Pd/C, followed by reaction in a hydrogen reactor under hydrogen pressure of 4bar for 5 hours. After the reaction was complete, Pd/C was filtered through celite. The filtrate was distilled under reduced pressure and purified by column chromatography with a 4:1 (v/v) mixed solvent of n-hexane and ethyl acetate as a developing solvent. The resulting compound was distilled under reduced pressure and dried under vacuum at about 40°C to afford 1.38g (yield: 41%) of the desired compound.

Mass (M+): 276.2

$^1$H-NMR (DMSO-$d_6$): 0.89(t, 6H), 1.63(m, 4H), 3.37(t, 4H), 5.18(s, 2H), 6.53(d, 2H), 6.68(s, 1H), 7.50(d, 2H).

**Example 2-4-3**

**Preparation of 2-[4-(2-dipropylaminothiazol-4-yl)phenylamino]-6-chloro-3-nitropyridine**

To 50ml of methanol were added 1.1g (5.7mmol) of 2,6-dichloronitropyridine and 1.2ml (8.55mmol) of triethylamine and 1.74g (5.7mmol) of 4-(2-dipropylaminothiazol-4-yl)aniline obtained in Preparation Example 2-4-2 was then added thereto, followed by reaction at room temperature (20 to 30°C) for about 32 hours. After the reaction was complete, the reactant was filtered, washed with 20ml of methanol and then dried under vacuum at about 40°C to afford 1.98g (yield: 81%) of the desired compound.

Mass (M+): 432.1

$^1$H-NMR (DMSO-$d_6$): 0.91(t, 6H), 1.68(m, 4H), 3.42(t, 4H), 7.03(d, 1H), 7.12(s, 1H), 7.63(d, 2H), 7.86(d, 2H), 8.56(d, 1H), 10.18(s, 1H).

**Preparation Example 3**

**Preparation of formula 4 wherein X represents fluoro**

**Example 3-1-1**

**Preparation of (3-fluoro-4-diethylamino)nitrobenzene**

To 50ml of methanol were added 5g (31.4mmol) of 3,4-difluoronitrobenzene, 3.6ml (40.8mmol) of triethylamine and 5.3ml (34.5mmol) of diethylamine, followed by reaction at a temperature of 50 to 60°C for 24 hours. After the reaction was complete, the reaction liquid was cooled to room temperature and 30ml of water was slowly added dropwise thereto. The resulting solid was filtered, washed with 100ml of water and then dried under vacuum at about 40°C to afford 5.4g (yield: 81%) of the desired compound.

Mass (M+): 213.1

$^1$H-NMR (DMSO-$d_6$): 1.16(t, 6H), 3.45(m, 4H), 6.97(t, 1H), 7.93(t, 2H).

**Example 3-1-2**

**Preparation of (3-fluoro-4-diethylamino)aniline**

To 150ml of ethyl acetate were sequentially added 5.4g (25.4 mmol) of (3-fluoro-4-diethylamino)nitrobenzene synthesized in Preparation Example 3-1-1 and 540mg (10W%) of Pd/C, followed by reaction in a hydrogen reactor under hydrogen pressure of 4bar for 5 hours. After the reaction was complete, Pd/C was filtered through celite. The filtrate was distilled under reduced pressure, purified by recrystallization from ethyl acetate and n-hexane, and then dried under vacuum at about 40°C to afford 3.2g (yield: 88%) of the desired compound.

Mass (M+): 183.1

$^1$H-NMR (DMSO-$d_6$): 0.87(m, 6H), 2.88(m, 4H), 5.02(s, 2H), 6.31(t, 2H), 6.78(t, 1H).

**Example 3-1-3**

**Preparation of 2-[(3-fluoro-4-diethylamino)phenylamino]-6-chloro-3-nitropyridine**

To 100ml of methanol were added 3.92g (20.3mmol) of 2,6-dichloronitropyridine and 5.66ml (40.6mmol) of triethylamine and 3.7g (20.3mmol) of (3-fluoro-4-diethylamino)aniline obtained in Preparation Example 3-1-2 was then added thereto, followed by reaction at room temperature (20 to 30°C) for about 24 hours. After the reaction was complete, the reactant was filtered, washed with 20ml of methanol and then dried under vacuum at about 40°C to afford 3.44g (yield: 50%) of the desired compound.

Mass (M+): 339.1

$^1$H-NMR (DMSO-$d_6$): 1.03(t, 6H), 3.16(q, 4H), 7.35(d, 2H), 8.50(m, 3H), 10.06(s, 1H).

**Example 3-2-1**

**Preparation of (3-fluoro-4-morpholino)nitrobenzene**

To 100ml of methanol were added 3g (18.9mmol) of 3,4-difluoronitrobenzene and 8ml (94.3mmol) of morpholine, followed by reaction at a temperature of 50 to 60°C for 16 hours. After the reaction was complete, the reaction liquid was cooled to room temperature. The resulting solid was filtered, washed with 20ml of methanol and then dried under vacuum at about 40°C to afford 4.2g (yield: 98%) of the desired compound.

Mass (M+): 227.0

$^1$H-NMR (DMSO-$d_6$): 3.28(m, 4H), 3.75(t, 1H), 7.18(t, 1H), 8.04(m, 2H).

**Example 3-2-2**

**Preparation of (3-fluoro-4-morpholino)aniline**

To 120ml of ethyl acetate were sequentially added 4.2g (18.6 mmol) of (3-fluoro-4-morpholino)nitrobenzene synthesized in Preparation Example 3-2-1 and 420mg (10W%) of Pd/C, followed by reaction in a hydrogen reactor under hydrogen pressure of 4bar for 5 hours. After the reaction was complete, Pd/C was filtered through celite. The filtrate

was distilled under reduced pressure, purified by recrystallization from ethyl acetate and n-hexane and then dried under vacuum at about 40°C to afford 3.2g (yield: 88%) of the desired compound.

Mass (M+): 197.1

$^1$H-NMR (DMSO-$d_6$): 2.80(brm, 4H), 3.68(brm, 4H), 4.99(brs, 2H), 6.33(m, 2H), 6.76(t, 1H).

**Example 3-2-3**

**Preparation of 2-[(3-fluoro-4-morpholino)phenylamino]-6-chloro-3-nitropyridine**

To 50ml of methanol were added 2.5g (13.0mmol) of 2,6-dichloronitropyridine and 2.2ml (15.5mmol) of triethylamine and 2.54g (13.0mmol) of (3-fluoro-4-morpholino)aniline obtained in Preparation Example 3-2-2 was then added thereto, followed by reaction at room temperature (20 to 30°C) for about 24 hours. After the reaction was complete, the reactant was filtered, washed with 20ml of methanol and then dried under vacuum at about 40°C to afford 3.6g (yield: 79%) of the desired compound.

Mass (M+): 353.1

$^1$H-NMR (DMSO-$d_6$): 3.00(t, 4H), 3.74(t, 4H), 7.01(m, 2H), 7.33(d, 1H), 7.52(dd, 1H), 8.53(d, 1H), 10.08(s, 1H).

**Example 3-3-1**

**Preparation of 3-fluoro-4-thiomorpholinonitrobenzene**

To 100ml of methanol were sequentially added 3g (18.9mmol) of 3,4-difluoronitrobenzene, 3.15ml (22.6mmol) of triethylamine and 2.15ml (20.8mmol) of thiomorpholine, followed by reaction at a temperature of 50 to 60°C for 24 hours. After the reaction was complete, the reaction liquid was cooled to room temperature, followed by removal of the solvent, extracted with ethyl acetate, purified by column chromatography with a 6:1 (v/v) solution of n-hexane and ethyl acetate as a developing solvent and then dried under vacuum at about 40°C to afford 4.48g (yield: 98%) of the desired compound.

Mass (M+): 243.0

$^1$H-NMR (DMSO-$d_6$): 2.80(m, 4H), 3.53(m, 4H), 6.97(d, 1H), 7.88(dd, 1H), 8.01(s, 1H).

**Example 3-3-2**

**Preparation of (3-fluoro-4-thiomorpholino)aniline**

To 100ml of ethyl acetate were sequentially added 4.45g (18.4 mmol) of (3-fluoro-4-thiomorpholino)nitrobenzene synthesized in Preparation Example 3-3-1 and 450mg (10W%) of Pd/C, followed by reaction in a hydrogen reactor under hydrogen pressure of 4bar for 6 hours. After the reaction was complete, Pd/C was filtered through celite. The filtrate was distilled under reduced pressure and purified by recrystallization from ethyl acetate and n-hexane. The resulting solid was dried under vacuum at about 40°C to afford 3.86g (yield: 99%) of the desired compound.

Mass (M+): 213.0

$^1$H-NMR (DMSO-$d_6$): 2.69(brm, 4H), 3.00(brm, 4H), 5.03(d, 2H), 6.30(d, 2H), 6.78(t, 1H).

**Example 3-3-3**

**Preparation of 2-[(3-fluoro-4-thiomorpholino)phenylamino]-6-chloro-3-nitropyridine**

To 100ml of methanol were added 2.5g (13.0mmol) of 2,6-dichloronitropyridine and 2.2ml (15.5mmol) of triethylamine and 2.75g (13.0mmol) of (3-fluoro-4-thiomorpholino)aniline obtained in Preparation Example 3-3-2 was then added thereto, followed by reaction at room temperature (20 to 30°C) for about 24 hours. After the reaction was complete, the reactant was filtered, washed with 20ml of methanol and then dried under vacuum at about 40°C to afford 3.7g (yield: 77%) of the desired compound.

Mass (M+): 369.0

$^1$H-NMR (DMSO-$d_6$): 2.75(t, 4H), 3.25(t, 4H), 7.00(d, 1H), 7.09(d, 1H), 7.45(d, 1H), 7.52(dd, 1H), 8.52(d, 1H), 10.07(s, 1H).

**Example 3-4-1**

**Preparation of [3-fluoro-4-(BOC-piperazino)]nitrobenzene**

To 100ml of methanol were sequentially added 5g (31.4mmol) of 3,4-difluoronitrobenzene, 5.3ml (37.7mmol) of triethylamine and 6.4g (34.5mmol) of BOC-piperazine, followed by reaction at a temperature of 50 to 60°C for 17 hours. After the reaction was complete, the reaction liquid was cooled to room temperature and 20ml of water was slowly added dropwise thereto, followed by stirring for 4 hours. The resulting solid was filtered, washed with a 1:1 (v/v) solution of water and methanol and then dried under vacuum at about 40°C to afford 9.3g (yield: 91%) of the desired compound.

$^1$H-NMR (DMSO-$d_6$): 1.42(s, 9H), 3.25(m, 4H), 3.48(m, 4H), 7.18(3,1H), 8.03(m, 2H).

**Example 3-4-2**

**Preparation of [3-fluoro-4-(BOC-piperazino)]aniline**

To 150ml of ethyl acetate were sequentially added 9.3g (28.6 mmol) of [3-fluoro-4-(BOC-piperazino)]nitrobenzene synthesized in Preparation Example 3-4-1 and 930mg (10W%) of Pd/C, followed by reaction in a hydrogen reactor under hydrogen pressure of 4bar for 6 hours. After the reaction was complete, Pd/C was filtered through celite. The filtrate was distilled under reduced pressure and dried under vacuum at about 40°C to afford 8.22g (yield: 97%) of the desired compound.

Mass (M+): 296.1

$^1$H-NMR (DMSO-$d_6$): 1.42(s, 9H), 2.76(brm, 4H), 3.43(brm, 4H), 5.02(s, 2H), 6.33(m, 2H), 6.79(m, 1H).

**Example 3-4-3**

**Preparation of 2-[3-fluoro-4-(BOC-piperazino)]phenylamino-6-chloro-3-nitropyridine**

To 100ml of methanol were added 2.75g (14.2mmol) of 2,6-dichloronitropyridine and 2.38ml (17.0mmol) of triethylamine and 4.2g (14.2mmol) of [3-fluoro-4-(BOC-piperazino)]aniline obtained in Preparation Example 3-4-2 was then added thereto, followed by reaction at room temperature (20 to 30°C) for about 24 hours. After the reaction was complete, the reactant was filtered, washed with 20ml of methanol and then dried under vacuum at about 40°C to afford 4.47g (yield: 70%) of the desired compound.

Mass (M+): 452.0

$^1$H-NMR (DMSO-$d_6$): 1.42(s, 9H), 2.96(t, 4H), 3.48(m, 4H), 7.01(d, 1H), 7.07(t, 1H), 7.34(d, 1H), 7.53(d, 1H), 8.53(d, 1H), 10.08(s, 1H).

**Example 3-5-1**

**Preparation of (3-fluoro-4-piperidino)nitrobenzene**

To 100ml of methanol were sequentially added 4g (25.1mmol) of 3,4-difluoronitrobenzene, 4.2ml (30.2mmol) of triethylamine and 2.7ml (27.6mmol) of piperidine, followed by reaction at a temperature of 50 to 60°C for 17 hours. After the reaction was complete, the reaction liquid was cooled to room temperature, extracted with ethyl acetate and water, dried over anhydrous magnesium sulfate, filtered, distilled under reduced pressure, and then dried under vacuum at about 40°C to afford 5.5g (yield: 97%) of the desired compound.

Mass (M+): 225.1

$^1$H-NMR (DMSO-$d_6$):1.70(m, 6H), 3.26(m, 4H), 6.94(s, 1H), 7.93(m, 2H).

**Example 3-5-2**

**Preparation of (3-fluoro-4-piperidino)aniline**

To 100ml of ethyl acetate were sequentially added 5.4g (24.1 mmol) of (3-fluoro-4-piperidino)nitrobenzene synthesized in Preparation Example 3-5-1 and 540mg (10W%) of Pd/C, followed by reaction in a hydrogen reactor under hydrogen pressure of 4bar for 6 hours. After the reaction was complete, Pd/C was filtered through celite. The filtrate was distilled under reduced pressure and dried under vacuum at about 40°C to afford 4.54g (yield: 97%) of the desired compound.

Mass (M+): 191.0

$^1$H-NMR (DMSO-$d_6$): 1.46(m, 2H), 1.60(brm, 4H), 2.76(brm, 4H), 4.91(s, 2H), 6.32(m, 2H), 6.74(t,1H).

**Example 3-5-3**

**Preparation of 2-[(3-fluoro-4-piperidino)phenylamino]-6-chloro-3-nitropyridine**

To 80ml of methanol were added 4g (15.5mmol) of 2,6-dichloronitropyridine and 2.6ml (18.6mmol) of triethylamine and 3.02g (15.5mmol) of (3-fluoro-4-piperidino)aniline obtained in Preparation Example 3-5-2 was then added thereto, followed by reaction at room temperature (20 to 30°C) for about 24 hours. After the reaction was complete, the reactant was filtered, washed with 20ml of methanol and then dried under vacuum at about 40°C to afford 4.2g (yield: 77%) of the desired compound.
Mass (M+): 351.1
$^1$H-NMR (DMSO-$d_6$): 1.51(m, 2H), 1.65(brm, 4H), 2.95(m, 4H), 6.98(m, 2H), 7.30(d, 1H), 7.46(dd, 1H), 8.50 (d, 1H), 10.06(s, 1H).

**Example 3-6-1**

**Preparation of [3-fluoro-4-(4-hydroxypiperidino)]nitrobenzene**

To 100ml of methanol were sequentially added 3g (18.9mmol) of 3,4-difluoronitrobenzene, 4.2ml (30.2mmol) of triethylamine and 2.79ml (27.6mmol) of 4-hydroxypiperidine, followed by reaction at a temperature of 50 to 60°C for 24 hours. After the reaction was complete, the reaction liquid was cooled to room temperature. The resulting solid was filtered, washed with 20ml of methanol and then dried under vacuum at about 40°C to afford 5.13g (yield: 85%) of the desired compound.
Mass (M+): 241.1
$^1$H-NMR (DMSO-$d_6$): 1.51(m, 2H), 1.87(m, 2H), 3.06(m, 2H), 3.52(m, 2H), 3.81(m, 1H), 4.80(d, 1H), 7.14(t, 1H), 7.95(d, 1H), 7.98(s, 1H).

**Example 3-6-2**

**Preparation of [3-fluoro-4-(4-hydroxypiperidino)]aniline**

To 100ml of ethyl acetate were sequentially added 5.1g (21.3 mmol) of [3-fluoro-4-(4-hydroxypiperidino)]nitrobenzene synthesized in Preparation Example 3-6-1 and 510mg (10W%) of Pd/C, followed by reaction in a hydrogen reactor under hydrogen pressure of 4bar for 5 hours. After the reaction was complete, Pd/C was filtered through celite. The filtrate was distilled under reduced pressure, purified by recrystallization from ethyl acetate and n-hexane and then dried under vacuum at about 40°C to afford 4.37g (yield: 98%) of the desired compound.
Mass (M+): 195.1
$^1$H-NMR (DMSO-$d_6$):1.51(m, 2H), 1.79(m, 2H), 2.58(m, 2H), 3.00(m, 2H), 3.53(m, 1H), 4.66(m, 1H), 4.93(m, 2H), 6.30(m, 2H), 6.75(m, 1H).

**Example 3-6-3**

**Preparation of 2-{[3-fluoro-4-(4-hydroxypiperidino)]phenylamino}-6-chloro-3-nitropyridine**

To 100ml of methanol were added 3g (15.5mmol) of 2,6-dichloronitropyridine and 2.6ml (18.7mmol) of triethylamine and 3.28g (15.5mmol) of [3-fluoro-4-(4-hydroxypipe-ridino)]-aniline obtained in Preparation Example 3-6-2 was then added thereto, followed by reaction at room temperature (20 to 30°C) for about 24 hours. After the reaction was complete, the reactant was filtered, washed with 20ml of methanol and then dried under vacuum at about 40°C to afford 4.1g (yield: 72%) of the desired compound.
Mass (M+): 367.1
$^1$H-NMR (DMSO-$d_6$): 1.54(m, 2H), 1.83(m, 2H), 2.77(m, 2H), 3.24(m, 2H), 3.61(m, 1H), 4.71(brm, 1H), 6.98 (m, 2H), 7.30(d, 1H), 7.48(dd, 1H), 8.52(d, 1H), 10.61(s, 1H).

**Example 3-7-1**

**Preparation of [3-fluoro-4-(4-aminopiperidino)]nitrobenzene**

To 100ml of methanol were sequentially added 3g (18.9mmol) of 3,4-difluoronitrobenzene, 3.15ml (22.6mmol) of triethylamine and 2.4ml (22.6mmol) of 4-aminopiperidine, followed by reaction at a temperature of 50 to 60°C for 19 hours. After the reaction was complete, the reaction liquid was cooled to room temperature, followed by distillation of the solvent under reduced pressure, extracted with dichloromethane and water, dried over anhydrous magnesium sulfate, and filtered. The filtrate was distilled under reduced pressure and dried under vacuum at about 40°C without purification to afford 4.3g (yield: 95%) of the desired compound.

Mass (M+): 240.1

$^1$H-NMR (DMSO-$d_6$): 1.36(m, 2H), 1.79(m, 2H), 2.78(m, 1H), 2.96(t, 2H), 3.62(m, 2H), 7.15(t, 1H), 7.96(m, 2H).

**Example 3-7-2**

**Preparation of [3-fluoro-4-(BOC-amino)piperidino]nitrobenzene**

To 150ml of dichloromethane were sequentially added 4.3g (17.9mmol) of 3-fluoro-4-(4-aminopiperidino)nitrobenzene synthesized in Preparation Example 3-7-1 and 4.7g (21.5mmol) of t-dibutoxydicarboxylate, followed by reaction at a temperature of 20 to 30°C for 3 hours. After the reaction was complete, the reaction liquid was cooled to room temperature, followed by distillation of the solvent under reduced pressure, extracted with dichloromethane and water, and dried over anhydrous magnesium sulfate. The filtrate was distilled under reduced pressure, purified by recrystallization from ethyl acetate and n-hexane and then dried under vacuum at about 40°C to afford 5g (yield: 82%) of the desired compound.

Mass (M+): 340.1

$^1$H-NMR (DMSO-$d_6$): 1.37(s, 9H), 1.47(m, 2H), 1.83(m, 2H), 2.98(t, 2H), 3.49(m, 1H), 3.63(m, 2H), 6.93(d, 1H), 7.15(t, 1H), 8.00(m, 2H).

**Example 3-7-3**

**Preparation of [3-fluoro-4-(BOC-amino)piperidino]aniline**

To 100ml of ethyl acetate were sequentially added 5g (14.7 mmol) of [3-fluoro-4-(BOC-amino)piperidino]nitrobenzene synthesized in Preparation Example 3-7-2 and 500mg (10W%) of Pd/C, followed by reaction in a hydrogen reactor under hydrogen pressure of 4bar for 5 hours. After the reaction was complete, Pd/C was filtered through celite. The filtrate was distilled under reduced pressure, purified by column chromatography with a 10:5:1 (v/v/v) solution of n-hexane, ethyl acetate and methanol as a developing solvent and then dried under vacuum at about 40°C to afford 4g (yield: 88%) of the desired compound.

Mass (M+): 310.1

$^1$H-NMR (DMSO-$d_6$): 1.41(s, 9H), 1.53(m, 2H), 1.76(m, 2H), 2.56(m, 2H), 3.05(m, 2H), 3.25(m, 1H), 4.93(brs, 2H), 6.30(m, 2H), 6.78(t, 1H), 6.86(d, 1H).

**Example 3-7-4**

**Preparation of 2-[3-fluoro-4-(4-BOC-aminopiperidino)phenylamino]-6-chloro-3-nitro**pyridine

To 100ml of methanol were added 1g (15.5mmol) of 2,6-dichloro-3-nitropyridine and 0.72ml (6.22mmol) of triethylamine and 1.6g (5.18mmol) of [3-fluoro-4-(4-BOC-aminopiperidino)]aniline obtained in Preparation Example 3-7-3 was then added thereto, followed by reaction at room temperature (20 to 30°C) for about 24 hours. After the reaction was complete, the reactant was filtered, washed with 20ml of methanol and then dried under vacuum at about 40°C to afford 1.7g (yield: 70%) of the desired compound.

Mass (M+): 466.2

$^1$H-NMR (DMSO-$d_6$): 1.34(s, 9H), 1.53(m, 2H), 1.82(m, 2H), 2.70(t, 2H), 3.31(m, 3H), 6.90(d, 1H), 7.00(d, 1H), 7.05(t, 1H), 7.30(d, 1H), 7.49(d, 1H), 8.53(d, 1H), 10.07(s, 1H).

**Example 3-8-1**

**Preparation of [3-fluoro-4-(2-methylpiperidino)]nitrobenzene**

To 150ml of methanol were sequentially added 5g (31.4mmol) of 3,4-difluoronitrobenzene, 5.26ml (37.7mmol) of triethylamine and 4.06ml (34.6mmol) of 2-methylpiperidine, followed by reaction at a temperature of 50 to 60°C for 28 hours. After the reaction was complete, the reaction liquid was cooled to room temperature, concentrated under reduced pressure, diluted with dichloromethane and then washed three times with 100ml of water. This solution was dried over anhydrous magnesium sulfate, filtered, concentrated under reduced pressure and then dried under vacuum at about 40°C to afford 7.4g (yield: 99%) of the desired compound.
Mass (M+): 239.2
$^{1}$H-NMR (DMSO-$d_6$): 1.08(d, 3H), 1.52(m, 3H), 1.67(m, 2H), 3.18(m, 2H), 3.98(m, 2H), 7.07(t, 1H), 7.91(m, 2H).

**Example 3-8-2**

**Preparation of [3-fluoro-(2-methylpiperidino)]aniline**

To 60ml of ethyl acetate were sequentially added 6g (25.2 mmol) of [3-fluoro-4-(2-methylpiperidino)]nitrobenzene synthesized in Preparation Example 3-8-1 and 900mg (15w%) of Pd/C, followed by reaction in a hydrogen reactor under hydrogen pressure of 4bar for 5 hours. After the reaction was complete, Pd/C was filtered through celite. The filtrate was distilled under reduced pressure, purified by recrystallization from ethyl acetate and n-hexane and then dried under vacuum at about 40°C to afford 4.37g (yield: 98%) of the desired compound.
Mass (M+): 209.2
$^{1}$H-NMR (DMSO-$d_6$): 0.76(d, 3H), 1.24(m, 2H), 1.54(m, 2H), 1.67(m, 2H), 2.67(m, 1H), 2.86(m, 2H), 5.09(s, 2H), 6.27(m, 2H), 6.84(m, 1H).

**Example 3-8-3**

**Preparation 2-{[3-fluoro-4-(2-methylpiperidino)]phenylamino}-6-chloro-3-nitropyridine**

To 100ml of methanol were added 3.5g (17.6mmol) of 2,6-dichloronitropyridine and 2.94ml (21.1mmol) of triethylamine and 4.03g (19.4mmol) of [3-fluoro-4-(2-methylpiperidino)]aniline obtained in Preparation Example 3-8-2 was then added thereto, followed by reaction at room temperature (20 to 30°C) for about 25 hours. After the reaction was complete, the reactant was filtered, washed with 20ml of methanol and then dried under vacuum at about 40°C to afford 3.5g (yield: 54%) of the desired compound.
Mass (M+): 365.1
$^{1}$H-NMR (DMSO-$d_6$): 0.89(d, 3H), 1.43(m, 2H), 1.62(m, 3H), 1.80(m, 1H), 2.78(m, 1H), 3.05(m, 1H), 3.33(m, 1H), 7.02(d, 1H), 7.14(t, 1H), 7.34(dd, 1H), 7.55(dd, 1H), 8.54(d, 1H), 10.09(s, 1H),

**Example 3-9-1**

**Preparation of [3-fluoro-4-(3-hydroxymethylpiperidino)]nitrobenzene**

To 200ml of methanol were sequentially added 5g (31.4mmol) of 3,4-difluoronitrobenzene, 5.26ml (37.7mmol) of triethylamine and 3.62ml (31.4mmol) of 3-hydroxymethylpiperidine, followed by reaction at a temperature of 50 to 60°C for 24 hours. After the reaction was complete, the reaction liquid was cooled to room temperature, concentrated under reduced pressure, diluted with ethyl acetate and washed three times with 100ml of water. This solution was dried over anhydrous magnesium sulfate, filtered, concentrated under reduced pressure and then dried under vacuum at about 40°C to afford 7.7g (yield: 96%) of the desired compound which was subjected to the subsequent reaction without further purification.
Mass (M+): 256.1

**Example 3-9-2**

**Preparation of [3-fluoro-4-(3-hydroxymethylpiperidino)]aniline**

To 100ml of ethyl acetate were sequentially added 7.7g (30.1 mmol) of [3-fluoro-4-(3-hydroxymethylpiperidino)] nitrobenzene synthesized in Preparation Example 3-9-1 and 770mg (10W%) of Pd/C, followed by reaction in a hydrogen

reactor under hydrogen pressure of 4bar for 5 hours. After the reaction was complete, Pd/C was filtered through celite. The filtrate was distilled under reduced pressure, purified by recrystallization from ethyl acetate and n-hexane and then dried under vacuum at about 40°C to afford 4.9g (yield: 73%) of the desired compound.

Mass (M+): 225.2

$^1$H-NMR (DMSO-$d_6$): 0.97(m, 1H), 1.56(m, 1H), 1.65(m, 2H), 1.69(m, 1H), 2.22(t, 1H), 2.46(td, 1H), 2.98(d, 1H), 3.12(dd, 1H), 3.24(m, 1H), 3.31(m, 1H), 4.44(t, 1H), 4.93(s, 2H), 6.29(m, 2H), 6.74(t,1H).

**Example 3-9-3**

**Preparation of 2-{[3-fluoro-4-(3-hydroxymethylpiperidino)]phenylamino}-6-chloro-3-**nitropyridine

To 100ml of methanol were added 3.57g (18.5mmol) of 2,6-dichloronitropyridine and 3.1ml (22.2mmol) of triethylamine and 4.15g (18.5mmol) of 3-fluoro-4-(3-hydroxymethylpiperidino)aniline obtained in Preparation Example 3-9-2 was then added thereto, followed by reaction at room temperature (20 to 30°C) for about 24 hours. After the reaction was complete, the reactant was filtered, washed with 20ml of methanol and then dried under vacuum at about 40°C to afford 5g (yield: 71%) of the desired compound.

Mass (M+): 381.2

$^1$H-NMR (DMSO-$d_6$): 1.04(m, 1H), 1.62(m, 1H), 1.73(m, 3H), 2.38(t, 1H), 2.63(td, 1H), 3.27(m, 2H), 3.36(m, 2H), 4.51(t, 1H), 6.99(d, 1H), 7.03(t, 1H), 7.29(dd, 1H), 7.48(dd, 1H), 8.53(d, 1H).

**Example 3-10-1**

**Preparation of [3-fluoro-4-(4-carbamoylpiperidino)]nitrobenzene**

To 50ml of methanol were sequentially added 5g (31.4mmol) of 3,4-difluoronitrobenzene, 5.26ml (37.7mmol) of triethylamine and 4.4g (34.6mmol) of isonipecotamide, followed by reaction at a temperature of 50 to 60°C for 24 hours. After the reaction was complete, the reaction liquid was cooled to room temperature. The resulting solid was filtered, washed with about 50ml of methanol and then dried under vacuum at about 40°C to afford 6.7g (yield: 80%) of the desired compound which was subjected to the subsequent reaction without further purification.

Mass (M+): 268.1

$^1$H-NMR (DMSO-$d_6$): 1.66(m, 2H), 1.81(m, 2H), 2.33(m, 1H), 2.94(t, 2H), 3.69(d, 2H), 6.85(s, 1H), 7.16(t, 1H), 7.33(s, 1H), 7.98(d, 2H).

**Example 3-10-2**

**Preparation of [3-fluoro-4-(4-carbamoylpiperidino)]aniline**

To 100ml of ethyl acetate were sequentially added 5g (18.7 mmol) of [3-fluoro-4-(4-carbamoylpiperidino)] nitrobenzene synthesized in Preparation Example 3-10-1 and 750mg (15W%) of Pd/C, followed by reaction in a hydrogen reactor under hydrogen pressure of 4bar for 5 hours. After the reaction was complete, Pd/C was filtered through celite. The filtrate was distilled under reduced pressure, purified by recrystallization from ethyl acetate and n-hexane and then dried under vacuum at about 40°C to afford 4g (yield: 90%) of the desired compound.

Mass (M+): 238.1

$^1$H-NMR (DMSO-$d_6$): 1.65(m, 2H), 1.72(m, 2H), 2.12(m, 1H), 2.49(m, 1H), 2.54(s, 1H), 3.68(d, 2H), 4.97(s, 2H), 6.30(m, 2H), 6.76(m, 2H), 7.27(s, 1H).

**Example 3-10-3**

**Preparation of 2-{[3-fluoro-4-(4-carbamoylpiperidino)]phenylamino}-6-chloro-3-**nitropyridine

To 70ml of methanol were added 3.5g (18.1mmol) of 2,6-dichloronitropyridine and 3ml (21.8mmol) of triethylamine and 4.7g (19.9mmol) of [3-fluoro-4-(4-carbamoylpiperidino)]aniline obtained in Preparation Example 3-10-2 was then added thereto, followed by reaction at room temperature (20 to 30°C) for about 24 hours. After the reaction was complete, the reactant was filtered, washed with 20ml of methanol and then dried under vacuum at about 40°C to afford 6.3g (yield: 88%) of the desired compound.

Mass (M+): 394.2

$^1$H-NMR (DMSO-$d_6$): 1.69(m, 2H), 1.79(m, 2H), 2.22(m, 2H), 2.66(t, 2H), 3.32(s, 1H), 3.37(s, 1H), 6.81(s, 1H), 7.00(d, 1H), 7.07(t, 1H), 7.31(m 2H), 7.49(d, 1H), 8.53(d, 1H), 10.08(s, 1H).

**Example 3-11-1**

**Preparation of [3-fluoro-4-(3-carbamoylpiperidino)]nitrobenzene**

To 50ml of methanol were sequentially added 5g (31.4mmol) of 3,4-difluoronitrobenzene, 5.26ml (37.7mmol) of triethylamine and 4.4g (34.6mmol) of nipecotamide, followed by reaction at a temperature of 50 to 60°C for 24 hours. After the reaction was complete, the reaction liquid was cooled to room temperature. The resulting solid was filtered, washed with about 50ml of methanol and then dried under vacuum at about 40°C to afford 5.7g (yield: 76%) of the desired compound.
Mass (M+): 268.1
$^1$H-NMR (DMSO-$d_6$): 1.56(t, 2H), 1.74(m, 1H), 1.89(m, 1H), 2.48(m, 1H), 2.88(m, 1H), 2.96(m, 1H), 3.64(m, 2H), 6.91(s, 1H), 7.15(m, 1H), 7.38(s, 1H), 7.95(m, 2H).

**Example 3-11-2**

**Preparation of [3-fluoro-4-(3-carbamoylpiperidino)]aniline**

To 100ml of ethyl acetate were sequentially added 5g (18.7 mmol) of [3-fluoro-4-(3-carbamoylpiperidino)] nitrobenzene synthesized in Preparation Example 3-11-1 and 750mg (15w%) of Pd/C, followed by reaction in a hydrogen reactor under hydrogen pressure of 4bar for 5 hours. After the reaction was complete, Pd/C was filtered through celite. The filtrate was distilled under reduced pressure, purified by recrystallization from ethyl acetate and n-hexane and then dried under vacuum at about 40°C to afford 4g (yield: 90%) of the desired compound.
Mass (M+): 238.2
$^1$H-NMR (DMSO-$d_6$):1.40(m, 1H), 1.56(m, 1H), 1.70(m, 1H), 1.80(m, 1H), 2.46(m, 1H), 2.49(m, 1H), 2.57(m, 1H), 2.97(m, 1H), 3.07(m, 1H), 4.97(s, 2H), 6.29(m, 2H), 6.79(m, 2H), 7.32(s, 1H).

**Example 3-11-3**

**Preparation of 2-{[3-fluoro-4-(3-carbamoylpiperidino)]phenylamino}-6-chloro-3-**nitropyridine

To 100ml of methanol were added 3.26g (16.9mmol) of 2,6-dichloronitropyridine and 4.7ml (33.8mmol) of triethylamine and 4g (16.9mmol) of [3-fluoro-4-(3-carbamoylpiperidino)]aniline obtained in Preparation Example 3-11-2 was then added thereto, followed by reaction at room temperature (20 to 30°C) for about 24 hours. After the reaction was complete, the reactant was filtered, washed with 20ml of methanol and then dried under vacuum at about 40°C to afford 4g (yield: 60%) of the desired compound.
Mass (M+): 394.1
$^1$H-NMR (DMSO-$d_6$): 1.46(m, 1H), 1.73(m, 1H), 1.84(m, 1H), 1.87(m, 1H), 2.65(m, 2H), 3.32(m, 3H), 6.85(s, 1H), 6.97(s, 1H), 7.00(t, 1H), 7.35(m, 2H), 7.47(d, 1H), 8.52(d, 1H), 10.06(s, 1H).

**Example 3-12-1**

**Preparation of [3-fluoro-4-(4-carboxypiperidino)]nitrobenzene**

To 100ml of methanol were sequentially added 5g (31.4mmol) of 3,4-difluoronitrobenzene, 5.26ml (37.7mmol) of triethylamine and 4.5g (34.6mmol) of isonipecotic acid, followed by reaction at a temperature of 50 to 60°C for 5 hours. After the reaction was complete, the reaction liquid was cooled to room temperature. The resulting solid was filtered, washed with about 50ml of methanol and then dried under vacuum at about 40°C to afford 8.09g (yield: 96%) of the desired compound.
Mass (M+): 269.1
$^1$H-NMR (DMSO-$d_6$):1.67(m, 2H), 1.91(m, 2H), 2.50(m, 1H), 3.00(m, 2H), 3.67(m, 2H), 7.15(m, 1H), 7.96(m, 2H).

**Example 3-12-2**

**Preparation of [3-fluoro-4-(4-carboxypiperidino)]aniline**

To 150ml of ethyl acetate were sequentially added 8g (18.7 mmol) of [3-fluoro-4-(4-carboxypiperidino)]nitrobenzene synthesized in Preparation Example 3-12-1 and 800mg (10W%) of Pd/C, followed by reaction in a hydrogen reactor under hydrogen pressure of 4bar for 5 hours. After the reaction was complete, Pd/C was filtered through celite. The

filtrate was distilled under reduced pressure, purified by recrystallization from ethyl acetate and n-hexane and then dried under vacuum at about 40°C to afford 7g (yield: 99%) of the desired compound.

Mass (M+): 239.1

$^1$H-NMR (DMSO-$d_6$): 1.65(m, 2H), 1.83(m, 2H), 2.14(m, 1H), 2.52(m, 2H), 3.03(d, 2h), 5.05(brs, 1H), 6.29(m, 2H), 7.40(t, 1H).

### Example 3-12-3

### Preparation of 2-{[3-fluoro-4-(4-carboxypiperidino)]phenylamino}-6-chloro-3-nitropyridine

To 150ml of methanol were added 5.68g (29.4mmol) of 2,6-dichloronitropyridine and 8.2ml (58.8mmol) of triethylamine and 7g (29.4mmol) of [3-fluoro-4-(4-carboxypiperidino)]aniline obtained in Preparation Example 3-12-2 was then added thereto, followed by reaction at a temperature of 40 to 50°C for about 24 hours. After the reaction was complete, the reactant was filtered, washed with 100ml of methanol and then dried under vacuum at about 40°C to afford 7.8g (yield: 67%) of the desired compound.

Mass (M+): 395.1

$^1$H-NMR (DMSO-$d_6$): 1.70(m, 2H), 1.92(m, 2H), 2.37(m, 1H), 2.73(t, 2H), 3.28(m, 2H), 7.00(d, 1H), 7.05(t, 1H), 7.31(dd, 1H), 7.50(dd, 1H), 8.53(d, 1H), 10.08(s, 1H).

### Example 1:

### Preparation of 2-(4-methylphenylamino)-6-(methylamino)-3-nitropyridine

To 10 ml of acetonitrile were added 200 mg (0.76 mmol) of the 6-chloro-2-(4-methylphenylamino)-3-nitropyridine compound obtained in Preparation Example 1-1 and 5ml of a 40% (wt/v) methylamine-methanol solution, followed by reaction at room temperature for 4 hours. After the reaction was complete, the solvent was distilled under reduced pressure, followed by stirring in 5ml of methanol for 1 hour at a temperature of 40 to 45 °C. The resulting solid was filtered, washed with 5ml of methanol and then dried under vacuum at about 40 °C to afford 168 mg (yield: 86 %) of the desired compound.

Mass (M+): 259.1

$^1$H-NMR (DMSO-$d_6$) (ppm): 2.30(s, 3H), 2.89(d, 3H), 6.10(d, 1H), 7.17(d, 2H), 7.66(d, 2H), 8.06(d, 1H), 8.26 (brm, 1H), 10.88(s, 1H).

### Examples 2 to 14

In the same manner as in Example 1 and using amine compounds described in the following Table 1 in place of "40 % methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 1 were obtained.

**Table 1**

| Compounds according to Examples 2 to 14, Solvent S = $CH_3CN$ (all examples),<br>Starting material: 2-(4-methylphenylamino)-6-chloro-3-nitropyridine according to Preparation Example 1-1 | | | | | | | |
|---|---|---|---|---|---|---|---|
| Ex. | Amine | A | Compound | NMR(DMSO-d6) | T[°C] | Y [%] | M(+) |
| 2 | Isopropylamine (excess) | X | 2-(4-methylphenylamino)-6-(isopropylamino)-3-nitropyridine | 1.18(d, 6H), 2.29(s, 3H), 4.10(m 1H), 6.08(d, 1H), 7.16(d, 2H), 7.61(d, 2H), 8.07(d, 1H), 8.19(m, 1H), 10.86(s, 1H) | 20~ 30 | 68 | 287.1 |

(continued)

| Compounds according to Examples 2 to 14, Solvent S = $CH_3CN$ (all examples), Starting material: 2-(4-methylphenylamino)-6-chloro-3-nitropyridine according to Preparation Example 1-1 | | | | | | | |
|---|---|---|---|---|---|---|---|
| Ex. | Amine | A | Compound | NMR(DMSO-d6) | T[°C] | Y [%] | M(+) |
| 3 | Isobutylamine (excess) | X | 2-(4-methylphenyl-amino)-6-(isobutyl-amino)-3-nitropyridine | 0.88(d, 6H), 1.85(m, 1H), 2.29(s, 3H), 3.17(t, 2H), 6.13(d, 1H), 7.16(d, 2H), 7.62(d, 2H), 8.07(d, 1H), 8.41(t, 1H), 10.85(s, 1H) | 20~ 30 | 63 | 301.1 |
| 4 | 2-methylamino-methyl-1-1,3-dioxolane (2 equivalents) | 2 # | 2-(4-methylphenyl-amino)-6-[(N-[1,3]-dioxolan-2-ylmethyl)-methylamino]-3-nitropyridine | 2.29(s, 3H), 3.16(s, 3H), 3.80(m, 4H), 3.89(m, 2H), 5.04 (m, 1H), 6.40(m, 1H). 7.15(d, 2H), 7.56(m, 2H), 8.21 (brs, 1H), 10.55~10.65(m, 1H) | 60~ 70 | 68 | 345.1 |
| 5 | 4-hydroxy-piperidine (1.5 equivalents) | 1.5 # | 2-(4-methylphenylamino)-6-(4-hydroxypiperidino)-3-nitropyridine | 1.39(m, 2H), 1.79 (m, 2H), 2.29(s, 3H), 3.36(m, 2H), 3.79 (m, 1H),4.01(m, 2H), 4.79(d, 1H), 6.52(d, 1H), 7.17(d, 2H), 7.51(d, 2H), 8.15(d, 1H), 10.56 (s, 1H) | 20~ 30 | 68 | 329.1 |
| 6 | 2-methyl-2-imidazoline (2 equivalents) | 2 # | 2-(4-methylphenylamino)-6-[(2-methyl-4,5-dihydro)imidazol-1-yl]-3-nitropyridine | 2.06(s, 3H), 2.29(s, 3H), 3.69(t, 2H), 3.84(t, 2H), 6.08(d, 1H), 7.19(d, 2H), 7.33(d,2H), 8.36(d, 1H), 10.17(s, 1H) | 60~ 70 | 73 | 312.2 |
| 7 | 2-isopropyl-imidazole (5 equivalents) | 5 # | 2-(4-methylphenylamino)-6-[(2-isopropyl) imidazol-1-yl]-3-nitropyridine | 0.88(d, 6H), 2.31(s, 3H), 3.31(m, 1H), 6.89(s, 1H), 7.05(d, 1H), 7.21(m, 2H), 7.30(d, 2H), 7.58(s, 1H), 8.62(d, 1H), 10.07(s, 1H) | 60~ 70 | 47 | 338.1 |
| 8 | 4-aminomethyl-pyridine (1.5 equivalents) | 1.5 # | 2-(4-methylphenylamino)-6-[(4-pyridyl) methylamino] -3-nitropyridine | 2.26(s, 3H), 4.56(d, 2H), 6.24(d, 2H), 7.02(d, 2H), 7.23(d, 2H), 7.32(d, 2H), 8.15(d, 1H), 8.51(d, 2H), 8.83(m, 1H), 10.69(s, 1H) | 60~ 70 | 33 | 335.3 |

(continued)

| Compounds according to Examples 2 to 14, Solvent S = $CH_3CN$ (all examples), Starting material: 2-(4-methylphenylamino)-6-chloro-3-nitropyridine according to Preparation Example 1-1 | | | | | | | |
|---|---|---|---|---|---|---|---|
| Ex. | Amine | A | Compound | NMR(DMSO-d6) | T[°C] | Y [%] | M(+) |
| 9 | 1-(3-aminopropyl) imidazole (1.5 equivalents) | 1.5 # | 2-(4-methylphenylamino)-6-[(3-imidazol-1-yl) propylamino]-3-nitropyridine | 1.98(t, 2H), 2.29(s, 3H), 3.27(m, 2H), 4.00(t, 2H), 6.11(d, 1H), 6.89(s, 1H), 7.15(d, 2H), 7.18(s, 1H), 7.56(d, 2H), 7.60(s, 1H), 8.09(d, 1H), 8.32(t, 1H), 10.81(s, 1H) | 60~ 70 | 78 | 353.1 |
| 10 | 3-(2-amino-ethyl) pyridine (2 equivalents) | 2 # | 2-(4-methylphenyl-amino)-6-[2-(3-pyridyl) ethylamino]-3-nitropyridine | 2.27(s, 3H), 2,82(m, 2H), 3.56(m, 4H), 6.10(m, 1H), 7.14 (m. 2H), 7.30(m, 1H), 7.54(m, 3H), 8.09(d, 1H), 8.43(m, 3H), 10.71(s, 1H) | 60~ 70 | 55 | 350.1 |
| 11 | 1-methyl-piperazine (3 equivalents) | X | 2-(4-methylphenyl-amino)-6-(4-methyl-piperazin-1-yl)-3-nitropyridine | 2.19(s, 3H), 2.29(s, 3H), 2.38(brm, 4H), 3.69(brm, 4H), 6.50 (d, 1H), 7.17(d, 2H), 7.49(d, 2H), 8.18(d, 1H), 10.54(s, 1H) | 20~ 30 | 56 | 328.1 |
| 12 | Piperazine (5 equivalents) | X | 2-(4-methylphenyl-amino)-6-(piperazin-1-yl)-3-nitropyridine | 2.29(s, 3H), 2.73(t, 4H), 3.62(m, 4H), 6.45(d, 1H), 7.17(d, 2H), 7.50(d, 2H), 8.16(d, 1H), 10.57 (s, 1H) | 20~ 30 | 63 | 314.2 |
| 13 | 4-amino-piperidine (2 equivalents) | 2 # | 2-(4-methylphenyl-amino)-6-(4-amino-piperidino)-3-nitropyridine | 1.46(m, 2H), 1.99 (m, 2H), 2.30(s, 3H), 3.11(m, 2H), 3.35 (m, 1H), 4.44(brm, 2H), 6.54(d, 1H), 7.19(d, 2H), 7.50(d, 2H), 8.23(d, 1H), 10.53(s, 1H) | 20~ 30 | 475 | 328.2 |
| 14 | Morpholine (3 equivalents) | X | 2-(4-methylphenyl-amino)-6-morpholino-3-nitropyridine | 2.29(s, 3H), 3.67 (brm, 8H), 6.49(d, 1H), 7.17(d, 2H), 7.50(d, 2H), 8.22(d, 1H), 10.54(s, 1H) | 20~ 30 | 75 | 315.1 |

**Example 15:**

**Preparation of 2-(4-methoxyphenylamino)-6-(methylamino)-3-nitropyridine**

To 10 ml of acetonitrile were added 200 mg (0.72 mmol) of the 2-(4-methoxyphenylamino)-6-chloro-3-nitropyridine compound obtained in Preparation Example 1-2 and 3ml of a 40% (wt/v) methylamine-methanol solution, followed by reaction at room temperature for 4 hours. After the reaction was complete, the solvent was distilled under reduced pressure, followed by stirring in 5ml of methanol for 1 hour at a temperature of 35 to 40 °C. The resulting solid was

filtered, washed with 5 ml of methanol and then dried under vacuum at about 40 °C to afford 146 g (yield: 52 %) of the desired compound.

Mass (M+): 275.1

$^1$H-NMR(DMSO-$d_6$) (ppm) 2.87(d, 3H), 3.75(s, 3H), 6.08(d, 1H), 6.94(d, 2H), 7.68(d, 2H), 8.05(d, 1H), 8.25(s, 1H), 10.84(s, 1H).

**Examples 16 to 29**

In the same manner as in Example 15 and using amine compounds described in the following Table 2 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 2 were obtained.

The following Table 2 shows the name of compounds prepared in Examples 16 to 29, the name and equivalents of amine compounds used in the reaction, use/nonuse and equivalents of triethylamine in the reaction, the reaction temperature, the reaction solvent, yield, Mass analysis results and NMR analysis results.

**Table 2**

| Compounds according to Examples 16 to 29, Solvent S = $CH_3CN$ (all examples)<br>Starting material: 2-(4-methoxyphenylamino)-6-chloro-3-nitropyridine according to Preparation Example 1-2 | | | | | | | |
|---|---|---|---|---|---|---|---|
| EX | Amine | A | Compound | NMR (DMSO-d6) | T[°C] | Y[%] | M(+) |
| 16 | Isopropylamine (excess) | X | 2-[(4-methoxy)-phenyl-amino]-6-(isopropylamino)-3-nitropyridine | 1.17(d, 6H), 3.75(s, 3H), 4.05(m, 1H), 6.05(d, 1H), 6.93(d, 2H), 7.62(d, 2H), 8.04(d, 1H), 8.14 (m, 1H), 10.79(s, 1H) | 20~ 30 | 43 | 303.1 |
| 17 | Isobutylamine (excess) | X | 2-(4-methoxy-phenylamino)-6-(isobutylamino)-3-nitropyridine | 0.86(d, 6H), 1.83 (m, 1H), 3.12(m, 2H), 3.75(s, 3H), 6.10(d, 1H), 6.93(d, 2H), 7.61(d, 2H), 8.05(d, 1H), 8.34 (m, 1H), 10.76(s, 1H) | 20~ 30 | 34 | 317.1 |
| 18 | 2-methyl-amino-methyl-1-1,3-dioxolane (2 equivalents) | 2 # | 2-(4-methoxy-phenylamino)-6-[(N-[l,3]-dioxolan-2-ylmethyl) methylamin o]-3-nitropyridine | 3.15(s, 3H), 3.72(m, 2H), 3.75(s, 3H), 3.78(m, 2H), 3.88 (brm, 2H), 5.02(brs, 1H), 6.34(m, 1H), 6.92(brm, 2H), 7.56 (brm, 2H), 8.21 (brm, 1H), 10.49 (brm, 1H) | 60~ 70 | 51 | 361.1 |
| 19 | 4-hydroxy-piperidine (2 equivalents) | 2 # | 2-(4-methoxy-phenylamino)-6-(4-hydroxypiperidino)-3-nitropyridine | 1.35(brm, 2H), 1.76 (m, 2H), 3.37(m, 2H), 3.75(s, 3H), 4.02(m, 2H), 4.79 (d, 1H), 6.49(d, 1H), 6.94(d, 2H), 7.51(d, 2H), 8.15(d, 1H), 10.49(s, 1H) | 20~ 30 | 72 | 345.1 |

(continued)

| Compounds according to Examples 16 to 29, Solvent S = $CH_3CN$ (all examples)<br>Starting material: 2-(4-methoxyphenylamino)-6-chloro-3-nitropyridine according to Preparation Example 1-2 | | | | | | | |
|---|---|---|---|---|---|---|---|
| EX | Amine | A | Compound | NMR (DMSO-d6) | T[°C] | Y[%] | M(+) |
| 20 | 2-methyl-2-imidazoline (2 equivalents) | 2 # | 2-(4-methoxy-phenylamino)-6-[(2-methyl-4,5-dihydro)imidazol-1-yl]-3-nitropyridine | 1.92(s, 3H), 3.68(t, 2H), 3.76(s, 3H), 3.82(t, 2H), 6.34(d, 1H), 6.95(d, 2H), 7.33(d, 2H), 8.37(d, 1H), 10.10(s, 1H) | 60~ 70 | 47 | 354.1 |
| 21 | 2-isopropyl-imidazole (5 equivalents) | 5 # | 2-(4-methoxy-phenylamino)-6-[(2-isopropyl) imidazol-1-yl]-3-nitropyridine | 0.88(d, 6H), 3.31 (m, 1H), 3.77(s, 3H), 6.89(s, 1H), 6.98(d, 2H), 7.04(d, 1H), 7.32(d, 2H), 7.60(s, 1H), 8.64(d, 1H), 10.04(s, 1H) | 60~ 70 | 47 | 354.1 |
| 22 | 4-amino-methyl-pyridine (2 equivalents) | 2 # | 2-(4-methoxy-phenylamino)-6-[(4-pyridyl) methylamino] -3-nitropyridine | 3.73(s, 3H), 4.52(d, 2H), 6.21(d, 1H), 6.77(d, 2H), 7.20(d, 2H), 7.33(d, 2H), 8.14(d, 1H), 8.50(d, 2H), 8.80(t, 1H), 10.62(s, 1H) | 60~ 70 | 62 | 352.1 |
| 23 | t-butylamine (excess) | X | 2-(4-methoxy-phenylamino)-6-(t-butylamino)-3-nitropyridine | 1.21(s, 9H), 3.75(s, 3H), 6.09(d, 1H), 6.94(d, 2H), 7.38(d, 2H), 7.78(s, 1H), 7.99(d, 2H), 10.52 (s, 1H) | 60~ 70 | 69 | 317.1 |
| 24 | 2-methylamino-ethanol (2 equivalents) | 2 # | 2-(4-methoxy-phenylamino)-6-[(N-methyl-2-hydroxy-ethyl) amino]-3-nitropyridine | 3.15(s,3H),3.58(m, 4H), 3.76(s, 3H), 4.80(d, 1H), 6.37(d, 1H), 6.93(d, 2H), 7.59(brm, 2H), 8.16 (s, 1H), 10.58(d, 1H) | 20~ 30 | 82 | 319.1 |
| 25 | 1-(3-aminopropyl) imidazole (1.5 equivalents) | 2 # | 2-(4-methoxy-phenylamino)-6-[(3-imidazol-1-yl) propyl-amino]-3-nitropyridine | 1.95(m, 2H), 3.22 (q, 2H), 3.75(s, 3H), 3.97(t, 2H), 6.07(d, 1H), 6.88(s, 1H), 6.92(d, 2H), 7.13(s, 1H), 7.55(d, 2H), 7.59(s, 1H), 8.06(d, 1H), 8.29(m, 1H), 10.74(s, 1H), | 60~ 70 | 86 | 369.2 |
| 26 | 1-methyl-piperazine (3 equivalents) | X | 2-(4-methoxy-phenylamino)-6-(4-methylpiperazin-1-yl)-3-nitropyridine | 2.19(s, 3H), 2.35 (brt, 4H), 3.67(brm, 4H), 3.75(s, 3H), 6.48(d, 1H), 6.94(d, 2H), 7.50(d, 2H), 8.17(d, 1H), 10.47 (s, 1H) | 60~ 70 | 66 | 344.2 |

(continued)

| Compounds according to Examples 16 to 29, Solvent S = $CH_3CN$ (all examples)<br>Starting material: 2-(4-methoxyphenylamino)-6-chloro-3-nitropyridine according to Preparation Example 1-2 | | | | | | | |
|---|---|---|---|---|---|---|---|
| EX | Amine | A | Compound | NMR (DMSO-d6) | T[°C] | Y[%] | M(+) |
| 27 | Piperazine (5 equivalents) | X | 2-(4-methoxy-phenylamino)-6-(piperazin-1-yl)-3-nitropyridine | 2.78(brm, 4H), 3.63 (brm, 4H), 3.75(s, 3H), 6.46(d, 1H) 6.94(d,2H),7.51(d, 1H), 8.17(d, 1H), 10.50(s, 1H) | 20~ 30 | 66 | 330.2 |
| 28 | 4-amino-piperidine (2 equivalents) | 2 # | 2-(4-methoxy-phenylamino)-6-(4-aminopiperidino)-3-nitropyridine | 1.48(brm, 2H), 2.10 (m, 2H), 3.09(m, 2H), 3.35(m, 3H), 3.76(s, 3H), 4.42 (brm, 2H), 6.52(d, 1H), 6.95(d, 2H), 7.52(d, 2H), 8.20(d, 1H), 10.47(s, 1H) | 20~ 30 | 45 | 344.2 |
| 29 | Morpholine (3 equivalents) | X | 2-(4-methoxy-phenylamino)-6-morpholino-3-nitropyridine | 3.66(m, 8H), 3.75 (S, 3H), 6.47(d, 1H), 6.94(d, 2H), 7.50(d, 2H), 8.21(d, 1H), 10.48(s, 1H) | 20~ 30 | 64 | 331.1 |

**Example 30**

**Preparation of 2-[4-(t-butyl)phenylamino]-6-(methylamino)-3-nitropyridine**

To 10ml of acetonitrile were added 200mg (0.65mmol) of the 2-[4-(t-butyl)phenylamino]-6-chloro-3-nitropyridine compound obtained in Preparation Example 1-3 and 3ml of a 40% (wt/v) methylamine-methanol solution, followed by reaction at room temperature for 4 hours. After the reaction was complete, the solvent was distilled under reduced pressure, followed by stirring in 5ml of methanol for 1 hour at room temperature. The resulting solid was filtered, washed with 5ml of methanol and then dried under vacuum at about 40°C to afford 151mg (yield: 77%) of the desired compound.

Mass (M+): 275.1

$^1$H-NMR(DMSO-$d_6$) (ppm) 1.28(s, 9H), 2.93(d, 3H), 6.11(d, 1H), 7.38(d, 2H), 7.74(d, 2H), 8.07(d, 1H), 8.31(m, 1H), 10.96(s, 1H).

**Examples 31 to 44**

In the same manner as in Example 30 and using amine compounds described in the following Table 3 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 3 were obtained.

The following Table 3 shows the name of compounds prepared in Examples 31 to 44, the name and equivalents of amine compounds used in the reaction, use/nonuse and equivalents of triethylamine in the reaction, the reaction temperature, the reaction solvent, yield, Mass analysis results and NMR analysis results.

**Table 3**

| Compounds according to Examples 31 to 44, Solvent S = $CH_3CN$ (all examples)<br>Starting material: 2-[4-(t-butyl)phenylamino]-6-chloro-3-nitropyridine according to Preparation Example 1-3 | | | | | | | |
|---|---|---|---|---|---|---|---|
| Ex | Amine | A | Compound | NMR(DMSO-d6) | T [°C] | Y [%] | M(+) |
| 31 | Isopropylamine (excess) | X | 2-[4-(t-butyl)phenyl-amino]-6-(isopropyl-amino)-3-nitro-pyridine | 1.20(d, 6H), 1.28(s, 9H), 4.13(m, 1H), 6.08(d, 1H), 7.38(d, 2H), 7.68(d, 2H), 8.06(d, 1H), 8.21(d, 1H),10.95(s,1H) | 20~ 30 | 69 | 329.1 |
| 32 | Isobutylamine (excess) | X | 2-[4-(t-butyl)phenyl-amino]-6-(isobutyl-amino)-3-nitro-pyridine | 0.86(d, 6H), 1.28(s, 9H), 1.85(m, 1H), 3.14(t, 2H), 6.12(d, 1H), 7.36(d, 2H), 7.63(d, 2H), 8.06(d, 1H), 8.40(t, 1H), 10.82(s, 1H) | 20~ 30 | 46 | 343.1 |
| 33 | 2-methylamino-methyl-1-1,3-dioxolane (2 equivalents) | 2 # | 2-[4-(t-butyl)phenyl-amino]-6-[(N-[1,3]-dioxolan-2-ylmethyl)-methylamino] -3-nitropyridine | 1.28(s, 9H), 3.17 (brs, 3H), 3.77(m, 4H), 3.87(m, 2H), 5.05(s, 1H), 6.35~6.48(m, 1H), 7.36(m, 1H), 7.58(m, 2H),8.23(brs,1H), 10.56~10.74(m, 1H) | 60~ 70 | 52 | 131 |
| 34 | 4-hydroxy-piperidine (2 equivalents) | 2 # | 2-[4-(t-butyl)phenyl-amino]-6-(4-hydroxypiperidino)-3-nitropyridine | 1.26(s, 9H), 1.40(m, 2H), 1.80(m, 2H), 3.43(t, 2H), 3.81(m, 1H), 4.06(brm, 2H), 4.80(d, 1H), 6.52(d, 1H), 7.38(d, 2H), 7.57(d, 2H), 8.17(d, 1H), 10.64(s, 1H) | 20~ 30 | 59 | 371.1 |
| 35 | 2-methyl-2-imidazoline (2 equivalents) | 2 # | 2-[4-(t-butyl)phenyl-amino]-6-[(2-methyl-4,5-dihydro)imidazol-1-yl]-3-nitropyridine | 1.29(s, 9H), 1.87(s, 3H), 3.70(t, 2H), 3.86 (t, 2H), 6.38(d, 1H), 7.35(d, 2H), 7.41(d, 2H), 8.38(d, 1H), 10.19(s, 1H) | 60~ 70 | 56 | 354.1 |
| 36 | 2-isopropyl-imidazole (5 equivalents) | 2 # | 2-[4-(t-butyl)phenyl-amino]-6-[(2-isopropyl) imidazol-1-yl]-3-nitropyridine | 0.88(d, 6H), 1.34(s, 9H), 3.35(m, 1H), 6.90(s, 1H), 7.07(d, 1H), 7.33(d, 2H), 7.43(d, 2H), 7.62(s, 1H), 8.64(d, 1H), 10.08(s, 1H) | 60~ 70 | 45 | 380.1 |
| 37 | 3-amino-methylpyridine (1.5 equivalents) | 2 # | 2-[4-(t-butyl)phenyl-amino]-6-[(3-pyridyl) methylamino] -3-nitropyridine | 1.27(s, 9H), 4.56(d, 2H), 6.19(d, 1H), 7.29(m, 3H), 7.44(d, 2H), 7.46(d, 1H), 8.13(d, 1H), 8.45(s, 2H), 8.79(t, 1H), 10.73(s. 1H) | 60~ 70 | 67 | 378.2 |

(continued)

| Ex | Amine | A | Compound | NMR(DMSO-d6) | T [°C] | Y [%] | M(+) |
|---|---|---|---|---|---|---|---|
| Compounds according to Examples 31 to 44, Solvent S = $CH_3CN$ (all examples)<br>Starting material: 2-[4-(t-butyl)phenylamino]-6-chloro-3-nitropyridine according to Preparation Example 1-3 | | | | | | | |
| 38 | 4-amino-methylpyridine (1.5 equivalents) | X | 2-[4-(t-butyl)phenyl-amino]-6-[(4-pyridyl) methylamino] -3-nitropyridine | 1.27(s, 9H), 4.55(d, 2H), 6.24(d, 1H), 7.20(m, 4H), 7.34(d, 2H), 8.15(d, 1H), 8.48(d, 2H), 8.86(t, 1H), 10.69(s, 1H) | 60~ 70 | 74 | 378.0 |
| 39 | 1-(3-amino-propyl) imidazole) (2 equivalents) | 2 # | 2-[4-(t-butyl)phenyl-amino]-6-[(3-imidazol-1-yl)propyl-amino]-3-nitro-pyridine | 1.27(s, 9H), 1.98(m, 2H), 3.28(m, 2H), 3.99(t, 2H), 6.10(d, 1H), 6.87(s, 1H), 7.13(s, 1H), 7.36(d, 1H), 7.61(m, 3H), 8.08(s, 1H), 8.35(m, 1H), 10.86(s, 1H) | 20~ 30 | 77 | 395.4 |
| 40 | 2-(2-aminoethyl)-pyridine (1.5 equivalents) | 1.5 # | 2-[4-(t-butyl)phenyl-amino]-6-[2-(2-pyridyl) ethylamino]-3-nitropyridine | 1.28(s, 9H), 3.04(t, 2H), 3.77(m, 2H), 6.11(d, 1H), 7.27(m, 4H), 7.70(m, 3H), 8.08(d, 1H), 8.50(t, 1H), 8.53(d, 1H), 10.90(s, 1H) | 20~ 30 | 56 | 392.0 |
| 41 | 1-methyl-piperazine (1.5 equivalents) | X | 2-[4-(t-butyl)phenyl-amino]-6-(4-methyl-piperazin-1-yl)-3-nitropyridine | 1.28(s, 9H), 2.20(s, 3H), 2.38(brm, 4H), 3.72(brm, 4H), 6.51 (d, 1H), 7.38(d, 2H), 7.56(d, 2H), 8.19(d, 1H),10.63(s,1H) | 60~ 70 | 49 | 370.0 |
| 42 | Piperazine (5 equivalents) | X | 2-[4-(t-butyl)phenyl-amino]-6-(piperazin-1-yl)-3-nitropyridine | 1.28(s, 9H), 2.76 (brm, 4H), 3.65(brm, 4H), 6.49(d, 1H), 7.38(d, 1H), 7.57(d, 1H), 8.18(d, 1H), 10.67(s, 1H) | 20~ 30 | 62 | 356.2 |
| 43 | 4-amino-piperidine (2 equivalents) | 2 # | 2-[4-(t-butyl)phenyl-amino]-6-(4-amino-piperidino)-3-nitropyridine | 1.95(m, 2H), 1.28(s, 9H), 1.73(m, 2H), 1.77(m, 2H), 2.89(m, 1H), 3.19(t, 2H), 4.28 (brm, 2H), 6.52(d, 1H), 7.37(d, 2H), 7.57(d, 2H), 8.16(d, 1H), 10.65(s, 1H) | 20~ 30 | 46 | 370.3 |
| 44 | Morpholine (3 equivalents) | X | 2-[4-(t-butyl)phenyl-amino]-6-morpholino-3-nitropyridine | 1.29(s, 9H), 3.70(m, 8H), 6.51(d, 1H), 7.39(d, 2H), 7.58(d, 2H), 8.23(d, 1H), 10.65(s, 1H) | 20~ 30 | 59 | 357.2 |

**Example 45:**

**Preparation of 2-[4-cyanophenylamino]-6-(methylamino)-3-nitropyridine**

To 10ml of acetonitrile were added 200mg (0.55mmol) of the 2-[4-cyanophenylamino]-6-chloro-3-nitropyridine compound obtained in Preparation Example 1-4 and 3ml of a 40% (wt/v) methylamine-methanol solution, followed by reaction at room temperature for 4 hours. After the reaction was complete, the solvent was distilled under reduced pressure, followed by stirring in 5ml of methanol for 1 hour at room temperature. The resulting solid was filtered, washed with 5ml of methanol and then dried under vacuum at about 40°C to afford 124mg (yield: 62%) of the desired compound.
Mass (M+): 270.1
$^1$H-NMR(DMSO-$d_6$) (ppm) 1.28(s, 9H), 2.93(d, 3H), 6.11(d, 1H), 7.38(d, 2H), 7.74(d, 2H), 8.07(d, 1H), 8.31(m, 1H), 10.96(s, 1H).

<u>Examples 46 to 52</u>

In the same manner as in Example 45 and using amine compounds described in the following Table 4 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 4 were obtained.
The following Table 4 shows the name of compounds prepared in Examples 46 to 52, the name and equivalents of amine compounds used in the reaction, use/nonuse and equivalents of triethylamine in the reaction, the reaction temperature, the reaction solvent, yield, Mass analysis results and NMR analysis results.

**Table 4**

| Compounds according to Examples 46 to 52, Solvent S = $CH_3CN$ (all examples)<br>Starting material: 2-[4-cyanophenylamino]-6-chloro-3-nitropyridine compound according to Preparation Example 1-4 | | | | | | | |
|---|---|---|---|---|---|---|---|
| Ex. | Amine | A | Compound | NMR(DMSO-d6) | T [°C] | Y [%] | M(+) |
| 46 | Amine Isobutylamine (excess) | X | 2-(4-cyanophenyl-amino)-6-(isobutyl-amino)-3-nitro-pyridine | 0.90(d, 6H), 1.87 (m, 1H), 3.19(t, 2H), 6.22(d, 1H), 7.98(d, 2H), 8.12 (d, 1H), 8.52(t, 1H), 10.98(s, 1H) | 20~ 30 | 62 | 312.1 |
| 47 | 4-hydroxypiperid ine (1.5 equivalents) | 2 # | 2-(4-cyanophenyl-amino)-6-(4-hydroxy-piperidino)-3-nitropyridine | 1.41(m, 2H), 1.80(m, 2H), 3.45(m, 2H), 3.80(m, 1H), 4.02(m, 2H), 4.83(d, 1H), 6.61 (d, 1H), 7.82(d, 2H), 7.85(d, 2H), 8.21(d, 1H), 10.73(s,1H) | 20~ 30 | 70 | 340.1 |
| 48 | 2-methyl-2-imidazoline (2 equivalents) | 2 # | 2-(4-cyanophenyl-amino)-6-[(2-methyl-4,5-dihydro) imidazol-1-yl]-3-nitropyridine | 2.14(s, 3H), 3.71 (t, 2H), 3.91(t, 2H), 6.59(d, 1H), 7.76(d, 2H), 7.83 (d, 2H), 8.42(d, 1H), 10.40(s, 1H) | 60~ 70 | 50 | 323.1 |

(continued)

| Compounds according to Examples 46 to 52, Solvent S = $CH_3CN$ (all examples) Starting material: 2-[4-cyanophenylamino]-6-chloro-3-nitropyridine compound according to Preparation Example 1-4 | | | | | | | |
|---|---|---|---|---|---|---|---|
| Ex. | Amine | A | Compound | NMR(DMSO-d6) | T [°C] | Y [%] | M(+) |
| 49 | 2-isopropylimid-azole (5 equivalents) | 5 # | 2-(4-cyanophenyl-amino)-6-[(2-isopropyl) imidazol-1-yl]-3-nitropyridine | 1.00(d, 6H), 3.39 (m, 1H), 6.95(s, 1H), 7.21(d, 1H), 7.60(s, 1H), 7.75 (d, 1H), 7.85(s, 1H), 7.90(d, 2H), 8.71(d, 1H), 10.28(s, 1H) | 60~ 70 | 57 | 349.1 |
| 50 | 4-aminomethylp yridine (1.5 equivalents) | 1.5 # | 2-(4-cyanophenyl-amino)-6-[(4-pyridyl) methylamino] -3-nitropyridine | 4.61(d, 2H), 6.36 (d, 2H), 7.30(d, 2H), 7.34(d, 2H), 7.66(d, 2H), 8.20 (d, 1H), 8.53(d, 2H), 8.95(t, 1H), 10.84(s, 1H) | 60~ 70 | 87 | 347.0 |
| 51 | 2-(ethylamino)-ethanol (2 equivalents) | 2 # | 2-(4-cyanophenyl-amino)-6-[(N-ethyl-2-hydroxyethyl) amino]-3-nitropyridine | 1.15(t, 3H), 3.62 (m, 6H), 4.88(m, 1H), 6.47(m, 1H), 7.80(m, 2H), 7.93(d, 2H), 8.22 (m, 1H), 10.82(s, 1H) | 60~ 70 | 61 | 328.1 |
| 52 | 1-(3-aminopropyl)-imidazol e (1.5 equivalents) | 2 # | 2-(4-cyanophenyl-amino)-6-[(3-imidazol-1-yl) propyl-amino]-3-nitropyridine | 2.01(m, 2H), 3.28(m, 2H), 4.05(m, 2H), 6.20(d, 1H), 6.90 (d, 1H), 7.18(s, 1H), 7.65(s, 1H), 7.80(d, 1H), 7.93 (d, 1H), 8.14(d, 1H), 8.45(t, 1H), 10.96(s. 1H) | 60~ 70 | 76 | 365.1 |

**Example 53**

**Preparation of 2-(3-cyanophenylamino)-6-[(3-imidazol-1-yl)propylamino]-3-nitropyridine**

To 10ml of acetonitrile were added 200mg (0.55mmol) of the 2-(3-cyanophenylamino)-6-chloro-3-nitropyridine compound obtained in Preparation Example 1-5, 0.11ml (0.83mmol) of triethylamine and 0.1ml (0.83mmol) of 1-(3-aminopropyl)imidazole, followed by reaction at a temperature of 70 to 80°C for 4 hours. After the reaction was complete, the solvent was distilled under reduced pressure, followed by stirring in 5ml of methanol for 1 hour at room temperature. The resulting solid was filtered, washed with 5ml of methanol and then dried under vacuum at about 40°C to afford 96mg (yield: 48%) of the desired compound.

Mass (M+): 365.1

$^1$H-NMR(DMSO-$d_6$) (ppm) 1.99(m, 2H), 3.29(m, 2H), 4.01(m, 2H), 6.17(d, 1H), 6.87(s, 1H), 7.15(s, 1H), 7.55 (t, 1H), 7.59(d, 1H), 7.96(d, 1H), 8.12(d, 1H), 8.31(s, 1H), 8.43(t, 1H), 10.84(s, 1H).

**Example 54**

**Preparation of 2-(4-hydroxyphenylamino)-6-[(3-imidazol-1-yl)propylamino]-3-nitropyridine**

To 10ml of acetonitrile were added 477mg (1.8mmol) of the 2-(4-hydroxyphenylamino)-6-chloro-3-nitropyridine compound obtained in Preparation Example 1-6, 0.3ml (2.15mmol) of triethylamine and 0.26ml (2.16mmol) of 1-(3-aminopropyl)imidazole, followed by reaction at a temperature of 70 to 80°C for 4 hours. After the reaction was complete, the solvent was distilled under reduced pressure, followed by stirring in 5ml of methanol for 1 hour at room temperature. The resulting solid was filtered, washed with 5ml of methanol and then dried under vacuum at about 40°C to afford 450mg (yield: 71%) of the desired compound.

Mass (M+): 355.1

$^1$H-NMR(DMSO-$d_6$) (ppm) 1.94(m, 2H), 3.23(m, 2H), 3.96(t, 2H), 6.07(d, 1H), 6.76(d, 2H), 6.89(s, 1H), 7.13 (s, 1H), 7.43(d, 2H), 7.59(s, 1H), 8.06(s, 1H), 8.28(t, 1H), 9.40(s, 1H).

**Example 55**

**Preparation of 2-[(4-methylsulfanyl)phenylamino]-6-[(3-imidazol-1-yl)propylamino]-3-nitropyridine**

To 10ml of acetonitrile were added 250mg (0.84mmol) of the 2-(4-methylsulfanyl-phenylamino)-6-chloro-3-nitropyridine compound obtained in Preparation Example 1-7, 0.14ml (1.01mmol) of triethylamine and 0.12ml (1.01mmol) of 1-(3-aminopropyl)imidazole, followed by reaction at a temperature of 70 to 80°C for 4 hours. After the reaction was complete, the solvent was distilled under reduced pressure, followed by column chromatography purification with a 12: 1 (v/v) solution of chloroform and methanol as a developing solvent and vacuum drying at about 40°C to afford 245mg (yield: 76%) of the desired compound.

Mass (M+): 385.1

$^1$H-NMR(DMSO-$d_6$) (ppm) 1.99(t, 2H), 2.48(s, 3H), 3.25(m, 2H), 4.01(t, 2H), 6.11(d, 1H), 6.89(s, 1H), 7.16(s, 1H), 7.26(d, 2H), 7.63(m, 3H), 8.09(d, 1H), 8.35(t, 1H), 10.83(s, 1H).

**Example 56**

**Preparation of 2-(4-n-butylphenylamino)-6-[(3-imidazol-1-yl)propylamino]-3-nitropyridine**

To 10ml of acetonitrile were added 280mg (0.92mmol) of the 2-(4-n-butylphenylamino)-6-chloro-3-nitropyridine compound obtained in Preparation Example 1-8, 0.14ml (1.01mmol) of triethylamine and 0.12ml (1.01mmol) of 1-(3-aminopropyl)imidazole, followed by reaction at a temperature of 70 to 80°C for 20 hours. After the reaction was complete, the solvent was distilled under reduced pressure, followed by column chromatography purification with a 15:1 (v/v) solution of chloroform and methanol as a developing solvent and vacuum drying at about 40°C to afford 245mg (yield: 76%) of the desired compound.

Mass (M+): 395.0

$^1$H-NMR(DMSO-$d_6$) (ppm) 0.90(t, 3H), 1.31(m, 2H), 1.54(m, 2H), 1.99(m, 2H), 2.50(m, 2H), 3.27(m, 2H), 3.99 (t, 2H), 6.11(d, 1H), 6.88(s, 1H), 7.17(m, 3H), 7.60(m, 3H), 8.09(d, 1H), 8.34(t, 1H), 10.84(s,1H).

**Example 57**

**Preparation of 2-(4-aminophenylamino)-6-(methylamino)-3-nitropyridine**

To 10ml of acetonitrile were added 300mg (1.13mmol) of the 2-(4-aminophenylamino)-6-chloro-3-nitropyridine compound obtained in Preparation Example 1-9 and 3ml of a 40% (wt/v) methylamine-methanol solution, followed by reaction at room temperature for 4 hours. After the reaction was complete, the solvent was distilled under reduced pressure, followed by stirring in 5ml of methanol for 1 hour at room temperature. The resulting solid was filtered, washed with 5ml of methanol and then dried under vacuum at about 40°C to afford 150mg (yield: 51%) of the desired compound.

Mass (M+): 260.1

$^1$H-NMR(DMSO-$d_6$) (ppm) 2.86(d, 3H), 5.04(s, 2H), 6.03(d, 1H), 6.56(d, 2H), 7.40(d, 2H), 8.02(d, 1H), 8.20(s, 1H), 10.80(s, 1H).

**Examples 58 to 69**

In the same manner as in Example 57 and using amine compounds described in the following Table 5 in place

of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 5 were obtained.

The following Table 5 shows the name of compounds prepared in Examples 58 to 69, the name and equivalents of amine compounds used in the reaction, use/nonuse and equivalents of triethylamine in the reaction, the reaction temperature, the reaction solvent, yield, Mass analysis results and NMR analysis results.

**Table 5**

| Compounds according to Examples 58 to 69, Solvent S = $CH_3CN$ (all examples)<br>Starting material: 2-[4-aminophenylamino]-6-chloro-3-nitropyridine compound according to Preparation Example 1-9 | | | | | | | |
|---|---|---|---|---|---|---|---|
| Ex. | Amine | A | Compound | NMR(DMSO-d6) | T [°C] | Y [%] | M(+) |
| 58 | Isopropylamine (excess) | X | 2-[4-(amino) phenyl-amino]-6-(isopropyl-amino)-3-nitropyridine | 1.16(d, 6H), 4.07(m, 1H), 5.04(s, 2H), 6.01(d, 1H), 6.56(d, 2H), 7.34(d, 2H), 8.01(d, 1H), 8.12(d, 1H), 10.75(s, 1H) | 20~ 30 | 81 | 288.1 |
| 59 | Isobutylamine (excess) | X | 2-[4-(amino) phenyl-amino]-6-(isobutyl-amino)-3-nitropyridine | 0.89(d, 6H), 1.85(m, 1H), 3.16(m, 2H), 5.05(s, 2H), 6.06(d, 1H), 6.56(d, 1H), 7.36(d, 2H), 8.02(d, 1H), 8.34(s, 1H), 10.77(s, 1H) | 20~ 30 | 77 | 302.2 |
| 60 | t-butylamine (excess) | X | 2-[4-(amino) phenyl-amino]-6-(t-butylamino)-3-nitropyridine | 1.24(s, 9H), 5.17(s, 2H), 6.06(d, 1H), 6.57(d, 2H), 7.11(d, 2H), 7.76(s, 1H), 7.96(d, 1H), 10.49(s, 1H) | 20~ 30 | 22 | 302.2 |
| 61 | 4-hydroxy-piperidine (1.5 equivalents) | 1.5 # | 2-[4-(amino) phenylamino] -6-(4-hydroxy-piperidino)-3-nitropyridine | 1.38(m,2H), 1.77(m, 2H), 3.34(m, 2H), 3.79(m, 1H), 4.02(brm, 2H), 4.80(s, 1H), 5.04(s, 2H), 6.44(d, 1H), 6.56(d, 2H), 7.23(d, 2H), 8.11(d, 1H), 10.42(s, 1H) | 20~ 30 | 50 | 330.2 |

(continued)

| Compounds according to Examples 58 to 69, Solvent S = $CH_3CN$ (all examples)<br>Starting material: 2-[4-aminophenylamino]-6-chloro-3-nitropyridine compound according to Preparation Example 1-9 | | | | | | | |
|---|---|---|---|---|---|---|---|
| Ex. | Amine | A | Compound | NMR(DMSO-d6) | T [°C] | Y [%] | M(+) |
| 62 | Piperazine (5 equivalents) | X | 2-[4-(amino) phenyl-amino]-6-(piperazin-1-yl)-3-nitropyridine | 3.41(brm, 4H), 3.45(brm, 4H). 5.07(s, 2H), 6.42(d, 1H), 6.56(d, 2H), 7.22(d, 2H), 8.13(d, 1H), 10.42(s, 1H) | 20~ 30 | 79 | 315.2 |
| 63 | 1-methyl-piperazine (1.5 equivalents) | 1.5 # | 2-[4-(amino) phenylamino] -6-(4-methylpiperazin-1-yl)-3-nitropyridine | 2.19(s, 3H), 2.45(brm, 4H), 3.67(brm, 4H), 5.06(s, 2H), 6.45(d, 1H), 6.56(d, 2H), 7.22(d, 2H), 8.14(d, 1H), 10.40(s, 1H) | 20~ 30 | 36 | 329.2 |
| 64 | Morpholine (3 equivalents) | X | 2-[4-(amino) phenyl-amino]-6-morpholino-3-nitropyridine | 3.65(brm, 8H), 5.06(s, 2H), 6.42(d, 1H), 6.56(d, 2H), 7.21(d, 2H), 8.17(d, 1H), 10.40(s, 1H) | 20~ 30 | 62 | 316.3 |
| 65 | 4-amino-piperidine (1.5 equivalents | 1.5 # | 2-[4-(amino) phenyl-amino]-6-(4-aminopiperidino)-3-nitropyridine | 1.16(m, 2H), 1.75(m, 2H), 2.85(s, 1H), 3.10(m, 2H), 3.16(m, 2H), 4.26(s, 1H), 5.06(s, 1H), 6.45(d, 1H), 6.56(d, 1H), 7.23(d, 2H), 8.09(d, 1H), 10.43(s, 1H) | 60~ 70 | 57 | 329.2 |
| 66 | 4-amino-methyl-pyridine (1.5 equivalents) | 1.5 # | 2-[4-(amino) phenyl-amino]-6-[(4-pyridyl) methylamino] -3-nitropyridine | 4.52(d, 2H), 5.04(s, 2H), 6.18(d, 1H), 6.45(d, 2H), 7.06(d, 2H), 7.20(d, 2H), 8.10(d, 1H), 8.49(d, 2H), 8.90(s, 1H), 10.60(s, 1H) | 60~ 70 | 68 | 337.2 |

(continued)

| Compounds according to Examples 58 to 69, Solvent S = $CH_3CN$ (all examples)<br>Starting material: 2-[4-aminophenylamino]-6-chloro-3-nitropyridine compound according to Preparation Example 1-9 | | | | | | | |
|---|---|---|---|---|---|---|---|
| Ex. | Amine | A | Compound | NMR(DMSO-d6) | T [°C] | Y [%] | M(+) |
| 67 | 1-(3-aminopropyl)-imidazole (1.5 equivalents) | 1.5 # | 2-[4-(amino) phenyl-amino]-6-[(3-imidazol-1-yl)-propylamino]-3-nitropyridine | 1.95(t, 2H), 3.24(m, 2H), 3.97(t, 2H), 5.07(s, 2H), 6.05(d, 1H), 6.57(d, 2H), 6.91(s, 1H), 7.14(s, 1H), 7.30(d, 2H), 7.60(s, 1H), 8.04(d, 1H), 8.28(s, 1H), 10.71(s, 1H) | 60~ 70 | 75 | 354.2 |
| 68 | 4-(2-aminoethyl)-morpholine (1.5 equivalents) | 1.5 # | 2-[4-(amino) phenyl-amino]-6-[2-(morpholin-1-yl)-ethylamino]-3-nitropyridine | 2.35(brm, 4H), 2.45(m, 2H), 3.35(m, 2H), 3.55(m, 4H), 5.07(s, 2H), 6.05(d, 1H), 6.55(d,2H), 7.29(d, 2H), 8.02(d, 1H), 8.20(s, 1H), 10.68(s, 1H) | 60~ 70 | 48 | 359.2 |
| 69 | 4-(3-aminopropyl)-morpholine (1.5 equivalents) | 1.5 # | 2-[4-(amino) phenyl-amino]-6-[3-(morpholin-1-yl)-propylamino]-3-nitropyridine | 1.67(t, 2H), 2.26(m,2H), 2.31(m, 5H), 3.36(t, 2H), 3.55(brm, 4H), 5.06(s, 2H), 6.03(d, 1H), 6.56(d, 2H), 7.35(d, 2H), 8.02(d, 1H), 8.25(s, 1H), 10.75(s, 1H) | 60~ 70 | 53 | 373.2 |

**Example 70**

**Preparation of 2-(3-aminophenylamino)-6-(methylamino)-3-nitropyridine**

To 10ml of acetonitrile were added 300mg (1.13mmol) of the 2-(3-aminophenylamino)-6-chloro-3-nitropyridine compound obtained in Preparation Example 1-10 and 3ml of a 40% (wt/v) methylamine-methanol solution, followed by reaction at room temperature for 4 hours. After the reaction was complete, the solvent was distilled under reduced pressure, followed by stirring in 5ml of methanol for 1 hour at room temperature. The resulting solid was filtered, washed with 5ml of methanol and then dried under vacuum at about 40°C to afford 176mg (yield: 60%) of the desired compound.
Mass (M+): 260.1
$^1$H-NMR(DMSO-$d_6$) (ppm) 2.90(d, 3H), 5.09(s, 2H), 6.08(d, 1H), 6.29(s, 1H), 6.97(m, 3H), 7.99(m, 1H), 8.03 (m, 1H), 10.87(s, 1H).

**Examples 71 to 85**

In the same manner as in Example 70 and using amine compounds described in the following Table 6 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 6 were obtained.

The following Table 6 shows the name of compounds prepared in Examples 71 to 85, the name and equivalents of amine compounds used in the reaction, use/nonuse and equivalents of triethylamine in the reaction, the reaction temperature, the reaction solvent, yield, Mass analysis results and NMR analysis results.

**Table 6**

| Compounds according to Examples 71 to 85, Solvent S = $CH_3CN$ (all examples)<br>Starting material: 2-[3-aminophenylamino]-6-chloro-3-nitropyridine compound according to Preparation Example 1-10 | | | | | | | |
|---|---|---|---|---|---|---|---|
| Ex. | Amine | A | Compound | NMR(DMSO-d6) | T [°C] | Y [%] | M(+) |
| 71 | Isopropylamine (excess) | X | 2-[3-(amino)phenyl-amino]-6-(isopropyl-amino)-3-nitropyridine | 1.20(d, 6H), 4.14(m, 1H), 5.08(s, 2H), 6.07(d, 1H), 6.34(s, 1H), 6.88(s, 1H), 6.99(d, 2H), 8.05(d, 1H), 8.21(d, 1H), 10.88(s, 1H) | 20~ 30 | 95 | 288.1 |
| 72 | Isobutylamine (excess) | X | 2-[3-(amino)phenyl-amino]-6-(isobutyl-amino)-3-nitropyridine | 0.90(d, 6H), 1.88(m, 1H), 3.20(m, 2H), 5.09(s, 2H), 6.13(d, 1H), 6.34(d, 1H), 6.78(s, 1H), 6.98(t, 1H), 7.09 (d, 1H), 8.05(d, 1H), 8.41(s, 1H), 10.83(s, 1H) | 20~ 30 | 95 | 302.2 |
| 73 | t-butylamine (excess) | X | 2-[3-(amino)phenyl-amino]-6-(t-butyl-amino)-3-nitropyridine | 1.22(s, 9H), 5.01(s, 2H), 6.08(d, 1H), 6.35(d, 1H), 6.59(m, 1H), 6.73(d, 1H), 6.97(m, 1H), 7.79(m, 1H), 7.93(m, 1H), 10.60(s, 1H) | 20~ 30 | 78 | 302.2 |

(continued)

| Compounds according to Examples 71 to 85, Solvent S = $CH_3CN$ (all examples)<br>Starting material: 2-[3-aminophenylamino]-6-chloro-3-nitropyridine compound according to Preparation Example 1-10 | | | | | | | |
|---|---|---|---|---|---|---|---|
| Ex. | Amine | A | Compound | NMR(DMSO-d6) | T [°C] | Y [%] | M(+) |
| 74 | 4-hydroxy-piperidine (1.5 equivalents) | 1.5 # | 2-[3-(amino)phenyl-amino]-6-(4-hydroxy-piperidino)-3-nitropyridine | 1.39(m, 2H), 1.78(m, 2H), 3.43(m, 2H), 3.79(m, 1H), 4.10(m, 2H), 4.82(d, 1H), 5.11(s, 2H), 6.34(d, 1H), 6.52(d, 1H), 6.82(m, 2H), 6.99(t, 1H), 8.16 (d, 1H), 10.56(s, 1H) | 20~ 30 | 89 | 330.1 |
| 75 | 2-isopropyl-imidazole (5 equivalents) | 5 # | 2-[3-(amino)phenyl-amino]-6-[(2-isopropyl)imidazol-1-yl]-3-nitropyridine | 0.97(d, 6H), 3.51(m, 1H), 5.19(s, 2H), 6.49(d, 1H), 6.58(d, 2H), 6.91(s, 1H), 7.04(m, 2H), 7.62(s, 1H), 8.63(d, 1H), 9.97(s, 1H) | 60~ 70 | 66 | 339.2 |
| 76 | Amine Piperazine (5 equivalents) | X | 2-[3-(amino)phenyl-amino]-6-(piperazin-1-yl)-3-nitropyridine | 3.70(brm, 8H), 5.14(brs, 2H), 6.34(d, 1H). 6.49(d, 1H), 6.77(d, 1H), 6.84(s, 1H), 6.99(t, 1H), 8.21 (d, 1H), 10.54(s, 1H) | 20~ 30 | 85 | 315.2 |
| 77 | 1-methyl-piperazine (1.5 equivalents) | 1.5 # | 2-[3-(amino)phenyl-amino]-6-(4-methyl-piperazin-1-yl)-3-nitropyridine | 2.20(s, 3H), 2.39(brm, 4H), 3.73(brm, 4H), 5.13(brm, 2H), 6.35(d, 1H), 6.49(d, 1H) 6.83 (t,2H),7.00(d, 1H), 8.17(d, 1H), 10.54(s, 1H) | 20~ 30 | 59 | 329.2 |

(continued)

| Compounds according to Examples 71 to 85, Solvent S = $CH_3CN$ (all examples)<br>Starting material: 2-[3-aminophenylamino]-6-chloro-3-nitropyridine compound according to Preparation Example 1-10 | | | | | | | |
|---|---|---|---|---|---|---|---|
| Ex. | Amine | A | Compound | NMR(DMSO-d6) | T [°C] | Y [%] | M(+) |
| 78 | Morpholine (3 equivalents) | X | 2-[3-(amino)phenyl-amino]-6-morpholino-3-nitropyridine | 3.07(brm, 4H), 3.87(brm, 4H), 5.17(brs, 2H), 6.35(d, 1H), 6.52(d, 1H), 6.76(d, 1H), 6.84(s, 1H), 7.00(t, 1H), 8.24 (d, 1H), 10.51(s, 1H) | 20~ 30 | 77 | 316.2 |
| 79 | 4-amino-piperidine (1.5 equivalents | 1.5 # | 2-[3-(amino) phenylamino] -6-(4-aminopiperidino)-3-nitropyridine | 1.72(m, 2H), 1.88(m, 2H), 2.83(m, 1H), 2.94(m, 2H), 3.17(m, 2H), 5.22(brs, 2H), 6.34(d, 2H), 6.47(d, 1H), 6.77(s, 1H), 6.99(d, 1H), 8.26(d, 1H), 10.69(s, 1H) | 60~ 70 | 73 | 329.2 |
| 80 | 3-aminomethylp yridine (1.5 equivalents) | 1.5 # | 2-[3-(amino)phenyl-amino]-6-[(3-pyridyl)-methylamino]-3-nitropyridine | 4.61(d, 2H), 5.10(s, 2H), 6.17(d, 1H), 6.34(d, 1H), 6.83(t, 2H), 6.92 (t, 1H), 7.32(m, 1H), 7.65(d, 1H), 8.11(d, 1H), 8.44(d, 1H), 8.53(s, 1H), 8.80(s, 1H), 10.76(s, 1H) | 60~ 70 | 68 | 337.2 |
| 81 | 4-amino-methylpyridine (1.5 equivalents) | 1.5 # | 2-[3-(amino)phenyl-amino]-6-[(4-pyridyl)-methylamino]-3-nitropyridine | 4.60(d, 2H), 5.03(d, 2H), 6.21(d ,1H), 6.31(d, 1H), 6.71(m, 2H), 6.83(t, 1H), 7.24 (d, 2H), 8.13(d, 1H), 8.47(d, 2H), 8.82(t, 1H), 10.69(s, 1H), | 60~ 70 | 88 | 337.2 |

(continued)

| Compounds according to Examples 71 to 85, Solvent S = $CH_3CN$ (all examples)<br>Starting material: 2-[3-aminophenylamino]-6-chloro-3-nitropyridine compound according to Preparation Example 1-10 | | | | | | | |
|---|---|---|---|---|---|---|---|
| Ex. | Amine | A | Compound | NMR(DMSO-d6) | T [°C] | Y [%] | M(+) |
| 82 | 1-(3-amino-propyl) imidazo le (1.5 equivalents) | 1.5 # | 2-[3-(amino)phenyl-amino]-6-[(3-imidazol-1-yl)-propylamino]-3-nitropyridine | 1.99(m, 2H), 3.34(m, 2H), 4.00(m, 2H), 5.14(brs, 2H), 6.10(d, 1H), 6.35(d, 1H), 6.87(s, 1H), 6.91(d, 2H), 7.00(t, 1H), 7.15 (s, 1H), 7.60(s, 1H), 8.07(d, 1H), 8.36(t, 1H), 10.81(s, 1H), | 60~ 70 | 89 | 354.1 |
| 83 | 4-(2-amino-ethyl)-morpholine (1.5 equivalents) | 1.5 # | 2-[3-(amino)phenyl-amino]-6-[2-(morpholin-1-yl)-ethylamino]-3-nitropyridine | 2.36(brm, 4H), 2.49(m, 2H), 3.54(m, 6H), 5.15(S, 2H), 6.12 (d, 1H), 6.34(d, 1H), 6.89 (d, 1H), 6.97(m, 2H), 8.06(d, 1H), 8.29(t, 1H), 10.79(S, 1H), | 60~ 70 | 55 | 359.2 |
| 84 | 4-(3-amino-propyl)-morpholine (1.5 equivalents) | 1.5 # | 2-[3-(amino)phenyl-amino]-6-[3-(morpholin-1-yl)-propylamino]-3-nitropyridine | 1.71(m, 2H), 2.30(m, 6H), 3.41(m, 2H), 3.53(m, 4H), 5.10(brs, 2H), 6.09(d, 1H), 6.34(d, 1H), 6.88(s, 1H), 7.00(m, 2H), 8.05(d, 1H), 8.35 (t, 1H), 10.86(s, 1H), | 60~ 70 | 62 | 373.2 |
| 85 | 2-methyl-imidazole (5 equivalents) | 1.5 # | 2-[3-(amino)phenyl-amino]-6-[(2-methyl) imidazol-1-yl]-3-nitropyridine | 2.32(s, 3H), 5.16(brs, 2H), 6.43(dd, 1H), 6.63(dd, 1H), 6.69(d, 1H), 6.91(t, 1H), 7.03 (t, 1H), 7.09(d, 1H), 7.68(s, 1H), 8.63(d, 1H), 9.99(s, 1H), | 60~ 70 | 88 | 311.2 |

## Example 86

### Preparation of 2-[4-(imidazol-1-yl)phenylamino]-6-(methylamino)-3-nitropyridine

To 10ml of acetonitrile were added 200mg (0.63mmol) of the 2-[4-(imidazol-1-yl)phenyl-amino]-6-chloro-3-nitropyridine compound obtained in Preparation Example 1-11 and 3ml of a 40% (wt/v) methylamine-methanol solution, followed by reaction at room temperature for 4 hours. After the reaction was complete, the solvent was distilled under reduced pressure, followed by stirring in 5ml of methanol for 1 hour at about 40°C. The resulting solid was filtered, washed with 5ml of methanol and then dried under vacuum at about 40°C to afford 100mg (yield: 51%) of the desired compound.

Mass (M+): 311.1

$^1$H-NMR(DMSO-$d_6$) (ppm) 2.92(d, 3H), 6.14(d, 1H), 7.10(s, 1H), 7.67(m, 2H), 7.75(s, 1H), 7.96(d, 2H), 8.11 (d, 1H), 8.27(s, 1H), 8.34(s, 1H), 10.98(s, 1H).

## Examples 87 to 95

In the same manner as in Example 86 and using amine compounds described in the following Table 7 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 7 were obtained.

The following Table 7 shows the name of compounds prepared in Examples 87 to 95, the name and equivalents of amine compounds used in the reaction, use/nonuse and equivalents of triethylamine in the reaction, the reaction temperature, the reaction solvent, yield, Mass analysis results and NMR analysis results.

**Table 7**

| Compounds according to Examples 87 to 95, Solvent S = $CH_3CN$ (all examples) Starting material : 2-[4-(imidazol-1-yl)phenylamino]-6-chloro-3-nitropyridine compound according to Preparation Example 1-11 | | | | | | | |
|---|---|---|---|---|---|---|---|
| Ex | Amine | A | Compound | NMR (DMSO-d6) | T [°C] | Y [%] | M(+) |
| 87 | Isopropylamine (excess) | X | 2-[4-(imidazol-1-yl) phenylamino]-6-(isopropylamino)-3-nitropyridine | 1.19(d, 6H), 4.10(m, 1H), 6.11(d, 1H), 7.09(s, 1H), 7.65(d, 2H), 7.75(m, 1H), 7.89(d, 2H), 8.08(d, 1H), 8.25(m, 2H), 10.94(s, 1H), | 20~ 30 | 70 | 339.1 |

(continued)

| Compounds according to Examples 87 to 95, Solvent S = $CH_3CN$ (all examples) Starting material : 2-[4-(imidazol-1-yl)phenylamino]-6-chloro-3-nitropyridine compound according to Preparation Example 1-11 | | | | | | | |
|---|---|---|---|---|---|---|---|
| Ex | Amine | A | Compound | NMR (DMSO-d6) | T [°C] | Y [%] | M(+) |
| 88 | Isobutylamine (excess) | X | 2-[4-(imidazol-1-yl) phenylamino]-6-(isobutylamino)-3-nitropyridine | 0.93(d, 6H), 1.87(m, 1H), 3.19(t, 2H), 6.17(d, 1H), 7.10(s, 1H), 7.66(m, 2H), 7.75(s, 1H), 7.89(d, 2H), 8.08(d, 1H), 8.25(s, 1H), 8.44(m, 1H), 10.92(s, 1H), | 20~ 30 | 85 | 353.2 |

(continued)

| Compounds according to Examples 87 to 95, Solvent S = $CH_3CN$ (all examples) Starting material : 2-[4-(imidazol-1-yl)phenylamino]-6-chloro-3-nitropyridine compound according to Preparation Example 1-11 | | | | | | | |
|---|---|---|---|---|---|---|---|
| Ex | Amine | A | Compound | NMR (DMSO-d6) | T [°C] | Y [%] | M(+) |
| 89 | 2-methylamino-methyl-1-1,3-dioxolane (2 equivalents) | 2 # | 2-[4-(imidazol-1-yl) phenylamino]-6-[(N-[1,3]-dioxolan-2-ylmethyl) methylamin o]-3-nitropyridine | 3.14 (brs, 3H), 3.72(m, 3H), 3.76(m, 1H), 3.86(m, 2H), 5.03(m, 1H), 6.27(m, 1H), 7.68(d, 2H), 7.70(s, 1H), 7.93 (brs, 2H), 8.12(s, 1H), 8.21(s, 1H), 9.64(s, 1H) | 60~ 70 | 75 | 397.1 |

(continued)

| Compounds according to Examples 87 to 95, Solvent S = $CH_3CN$ (all examples) Starting material : 2-[4-(imidazol-1-yl)phenylamino]-6-chloro-3-nitropyridine compound according to Preparation Example 1-11 | | | | | | | |
|---|---|---|---|---|---|---|---|
| Ex | Amine | A | Compound | NMR (DMSO-d6) | T [°C] | Y [%] | M(+) |
| 90 | 4-hydroxy-piperidine (1.5 equivalents) | 1.5 # | 2-[4-(imidazol-1-yl) phenylamino]-6-(4-hydroxy-piperidino)-3-nitro-pyridine | 1.40 (brm,H), 1.79 (brm, 2H), 3.42(m, 2H), 3.80 (brm, 1H), 4.03 (brm, 2H), 4.80(d, 1H), 6.55(d, 1H), 7.09(s, 1H), 7.66(m, 2H), 7.76(m, 3H), 8.19(d, 1H), 8.25(s, 1H), 10.65 (s, 1H), | 20~ 30 | 64 | 371.2 |

(continued)

| Compounds according to Examples 87 to 95, Solvent S = $CH_3CN$ (all examples) Starting material : 2-[4-(imidazol-1-yl)phenylamino]-6-chloro-3-nitropyridine compound according to Preparation Example 1-11 | | | | | | | |
|---|---|---|---|---|---|---|---|
| Ex | Amine | A | Compound | NMR (DMSO-d6) | T [°C] | Y [%] | M(+) |
| 91 | 2-methyl-2-imidazoline (2 equivalents) | 2 # | 2-[4-(imidazol-1-yl) phenylamino]-6-[(2-methyl-4,5-dihydro) imidazol-1-yl]-3-nitropyridine | 2.03(s, 3H), 3.68(t, 2H), 3.87(t, 2H), 6.45(d, 1H), 7.10(s, 1H), 7.55(d, 2H), 7.64(d, 2H), 7.75(s, 1H), 8.26(s, 1H), 8.40(d, 1H), 10.29(s, 1H), | 60~ 70 | 47 | 364.1 |
| 92 | 2-isopropyl-imidazole (5 equivalents) | 5 # | 2-[4-(imidazol-1-yl) phenylamino]-6-[(2-isopropyl)-imidazol-1-yl]-3-nitropyridine | 0.91(d, 6H), 3.35(m, 1H), 6.91(s, 1H), 7.12(m, 2H), 7.60(m, 3H), 7.72(d, 2H), 7.76(s, 1H), 8.27(s, 1H), 8.67(d, 1H), 10.21(s, 1H), | 60~ 70 | 45 | 390.1 |

(continued)

| Compounds according to Examples 87 to 95, Solvent S = $CH_3CN$ (all examples) Starting material : 2-[4-(imidazol-1-yl)phenylamino]-6-chloro-3-nitropyridine compound according to Preparation Example 1-11 | | | | | | | |
|---|---|---|---|---|---|---|---|
| Ex | Amine | A | Compound | NMR (DMSO-d6) | T [°C] | Y [%] | M(+) |
| 93 | Amine 3-amino-methylpyridine (1.5 equivalents) | 1.5 # | 2-[4-(imidazol-1-yl) phenylamino]-6-[(3-pyridyl)methyl-amino]-3-nitro-pyridine | 4.58(d, 2H), 6.24(d, 2H), 7.10(s, 1H), 7.35(m, 1H), 7.55(d, 2H), 7.64(d, 1H), 7.68(s, 1H), 7.74(s, 2H), 8.16(d, 1H), 8.23(t, 1H), 8.45(d, 1H), 8.49(s, 1H), 8.82(t, 1H), 10.80(s, 1H), | 60~ 70 | 79 | 388.1 |

(continued)

| Compounds according to Examples 87 to 95, Solvent S = $CH_3CN$ (all examples) Starting material : 2-[4-(imidazol-1-yl)phenylamino]-6-chloro-3-nitropyridine compound according to Preparation Example 1-11 | | | | | | | |
|---|---|---|---|---|---|---|---|
| Ex | Amine | A | Compound | NMR (DMSO-d6) | T [°C] | Y [%] | M(+) |
| 94 | 4-amino-methylpyridine (1.5 equivalents) | 2 # | 2-[4-(imidazol-1-yl) phenylamino]-6-[(4-pyridyl)methyl-amino]-3-nitro-pyridine | 4.58(d, 2H), 6.28(d, 1H), 7.10(s, 1H), 7.27(d, 2H), 7.48(d, 2H), 7.60(d, 2H), 7.71(s, 1H), 8.18(d, 1H), 8.22(s, 1H), 8.50(d, 2H), 8.88(t, 1H), 10.76(s, 1H), | 60~ 70 | 57 | 388.1 |

(continued)

| Compounds according to Examples 87 to 95, Solvent S = $CH_3CN$ (all examples) Starting material : 2-[4-(imidazol-1-yl)phenylamino]-6-chloro-3-nitropyridine compound according to Preparation Example 1-11 | | | | | | | |
|---|---|---|---|---|---|---|---|
| Ex | Amine | A | Compound | NMR (DMSO-d6) | T [°C] | Y [%] | M(+) |
| 95 | 1-(3-amino-propyl)-imidazole (2 equivalents) | 2 # | 2-[4-(imidazol-1-yl) phenylamino]-6-[(3-imidazol-1-yl) propylamino]-3-nitropyridine | 2.00(t, 3H), 3.29(m, 2H), 4.04(m, 2H), 6.15(d, 1H), 6.88(s, 1H), 7.12(s, 1H), 7.17(s, 1H), 7.63(m, 3H), 7.78(s, 1H), 7.83(d, 1H), 8.11(d, 1H), 8.28(s, 1H), 8.39(t, 1H), 10.89(s, 1H), | 60~ 70 | 70 | 405.1 |

**Example 96**

**Preparation of 2-(3-acetylphenylamino)-6-(methylamino)-3-nitropyridine**

To 10ml of acetonitrile were added 200mg (0.69mmol) of the 2-(3-acetylphenylamino)-6-chloro-3-nitropyridine compound obtained in Preparation Example 1-12 and 3ml of a 40% (wt/v) methylamine-methanol solution, followed by reaction at room temperature for 4 hours. After the reaction was complete, the solvent was distilled under reduced pressure, followed by stirring in 5ml of methanol for 1 hour at room temperature. The resulting solid was filtered, washed with 5ml of methanol and then dried under vacuum at about 40°C to afford 126mg (yield: 64%) of the desired compound.

Mass (M+): 270.1

$^1$H-NMR(DMSO-$d_6$) (ppm) 1.28(s, 9H), 2.93(d, 3H), 6.11(d, 1H), 7.38(d, 2H), 7.74(d, 2H), 8.07(d, 1H), 8.31(m, 1H), 10.96(s, 1H).

**Examples 97 to 107**

In the same manner as in Example 96 and using amine compounds described in the following Table 8 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 8 were obtained.

The following Table 8 shows the name of compounds prepared in Examples 97 to 107, the name and equivalents of amine compounds used in the reaction, use/nonuse and equivalents of triethylamine in the reaction, the reaction temperature, the reaction solvent, yield, Mass analysis results and NMR analysis results.

**Table 8** Compounds according to Examples 97 to 107, Solvent S = $CH_3CN$ (all examples) Starting material 2-(3-acetylphenylamino)-6-chloro-3-nitropyridine compound obtained in Preparation Example 1-12

| Ex | Amine | A | Compound | NMR (DMSO-d6) | T [°C] | Y [%] | M(+) |
|---|---|---|---|---|---|---|---|
| 97 | Isopropylamine (excess) | X | 2-(3-acetylphenyl-amino)-6-(isopropyl-amino)-3-nitro-pyridine | 1.17(d, 6H), 2.59(s, 3H), 4.21(m, 1H), 6.12(d, 1H), 7.50(t, 1H), 7.72(d, 1H), 7.86(d, 1H), 8.09(d, 1H), 8.24(d, 1H), 8.48(s, 1H), 10.96(s, 1H), | 20~ 30 | 85 | 315.1 |
| 98 | Isobutylamine (excess) | X | 2-(3-acetylphenyl-amino)-6-(isobutyl-amino)-3-nitro-pyridine | 0.87(d, 6H), 1.82(m, 1H), 2.59(s, 3H), 3.20(t, 2H), 6.18(d, 1H), 7.50(t, 1H), 7.73(d, 1H), 7.90(d, 1H), 8.10(d, 1H), 8.36(t, 1H), 8.40(s, 1H), 10.92(s, 1H), | 20~ 30 | 44 | 329.1 |

(continued)

| Ex | Amine | A | Compound | NMR (DMSO-d6) | T [°C] | Y [%] | M(+) |
|---|---|---|---|---|---|---|---|
| 99 | 4-hydroxypiperidine (1.5 equivalents) | 2 # | 2-(3-acetylphenyl-amino)-6-(4-hydroxy-piperidino)-3-nitro-pyridine | 1.41(m, 2H), 1.80(m, 2H), 2.59(s, 3H), 3.43(m, 2H), 3.80(m, 1H), 4.06 (brm, 2H), 4.80(d, 1H), 6.56(d, 1H), 7.52(t, 1H), 7.71(m, 2H), 8.20(d, 1H), 8.41(s, 1H), 10.70(s, 1H), | 20~ 30 | 77 | 357.1 |
| 100 | 2-methyl-2-imidazoline (1.5 equivalents) | 1.5 # | 2-(3-acetylphenyl-amino)-6-[(2-methyl-4,5-dihydro) imidazol-1-yl]-3-nitropyridine | 1.97(s, 3H), 2.59(s, 3H), 3.69(t, 2H), 3.86(t, 2H), 6.47(d, 1H), 7.54(t, 1H), 7.74(d, 1H), 7.80(d, 1H), 8.88(s, 1H), 8.41(d, 1H), 10.33 (s, 1H), | 60~ 70 | 47 | 340.1 |

(continued)

| Ex | Amine | A | Compound | NMR (DMSO-d6) | T [°C] | Y [%] | M(+) |
|---|---|---|---|---|---|---|---|
| 101 | 2-isopropy-limidazole (5 equivalents) | 5 # | 2-(3-acetylphenyl-amino)-6-[(2-isopropyl)-imidazol-1-yl]-3-nitropyridine | 0.86(d, 6H), 2.58(s, 3H), 3.29(m, 1H), 6.91(d, 1H), 7.12(d, H), 7.57 (t, 1H), 7.62(d, 1H), 7.73(dd, 1H), 7.86(d, 1H), 8.05(m, 1H), 8.68(d, 1H), 10.24(s, 1H), | 60~ 70 | 85 | 366.1 |
| 102 | 3-amino-methylpyridine (1.5 equivalents) | 2 # | 2-(3-acetylphenyl-amino)-6-[(3-pyridyl) methylamino] -3-nitropyridine | 2.52(s, 3H), 4.61(s, 2H), 6.23(d, 1H), 7.30(m, 1H), 7.43(t, 1H), 7.60(d, 1H), 7.71(d, 1H), 7.78(d, 1H), 8.16(d, 1H), 8.44(m, 2H), 8.78(t, 1H), 10.83(s, 1H), | 60~ 70 | 73 | 364.1 |

(continued)

| Ex | Amine | A | Compound | NMR (DMSO-d6) | T [°C] | Y [%] | M(+) |
|---|---|---|---|---|---|---|---|
| 103 | 4-amino-methylpyridine (1.5 equivalents) | 1.5 # | 2-(3-acetylphenyl-amino)-6-[(4-pyridyl) methylamino] -3-nitropyridine | 2.50(s, 3H), 4.61(d, 2H), 6.28(d, 1H), 7.20(d, 2H), 7.34(t, 1H), 7.69(m, 2H), 8.19(m, 2H), 8.45(d, 2H), 8.82(t, 1H), 10.78(s, 1H) | 60~ 70 | 77 | 364.2 |
| 104 | t-butylamine (excess) | X | 2-(3-acetylphenyl-amino)-6-(t-butyl-amino)-3-nitro-pyridine | 1.20(s, 9H), 2.57(s, 3H), 6.15(d, 1H), 7.52(t, 1H), 7.77(m, 2H), 7.83(s, 1H), 8.03(d, 2H), 10.69(s, 1H) | 20~ 30 | 45 | 329.1 |

(continued)

| Ex | Amine | A | Compound | NMR (DMSO-d6) | T [°C] | Y [%] | M(+) |
|---|---|---|---|---|---|---|---|
| 105 | 1-methyl-piperazine (3 equivalents) | X | 2-(3-acetylphenyl-amino)-6-(4-methylpiperazin-1-yl)-3-nitropyridine | 2.21(s, 3H), 2.39 (brm, 4H), 2.58(s, 3H), 3.72 (brm, 4H), 6.55(d, 1H), 7.50(t, 1H), 7.73(m, 2H), 8.21(d, 1H), 8.43(s, 1H), 10.67(s, 1H) | 20~ 30 | 71 | 356.1 |
| 106 | Piperazine (5 equivalents) | X | 2-(3-acetylphenyl-amino)-6-(piperazin-1-yl)-3-nitropyridine | 2.58(s, 3H), 2.75 (brm, 4H), 3.66 (brm, 4H), 6.53(d, 1H), 7.52(t, 1H), 7.73(m, 2H), 8.21(d, 1H), 8.42(s, 1H), 10.70(s, 1H) | 20~ 30 | 62 | 342.2 |

(continued)

| Ex | Amine | A | Compound | NMR (DMSO-d6) | T [°C] | Y [%] | M(+) |
|---|---|---|---|---|---|---|---|
| 107 | Morpholine (3 equivalents) | X | 2-(3-acetylphenyl-amino)-6-morpholino-3-nitropyridine | 2.58(s, 3H), 3.70(t, 8H), 6.54(d, 1H), 7.51(t, 1H), 7.74(dd, 2H), 8.25(d, 1H), 8.40(s, 1H), 10.66(s, 1H) | 20~ 30 | 66 | 343.2 |

**Example 108**

**Preparation of 2-(4-morpholinophenylamino)-6-(methylamino)-3-nitropyridine**

To 10ml of acetonitrile were added 200mg (0.60mmol) of the 2-(4-morpholinophenylamino)-6-chloro-3-nitropyridine compound obtained in Preparation Example 1-13 and 3ml of a 40% (wt/v) methylamine-methanol solution, followed by reaction at room temperature for 4 hours. After the reaction was complete, the solvent was distilled under reduced pressure, followed by stirring in 5ml of methanol for 1 hour at about 40°C. The resulting solid was filtered, washed with 5ml of methanol and then dried under vacuum at about 40°C to afford 129mg (yield: 65%) of the desired compound.

Mass (M+): 330.2

$^1$H-NMR(DMSO-$d_6$) (ppm) 2.88(d, 3H), 3.21(brm, 4H), 3.73(t, 4H), 6.08(d, 1H), 6.95(d, 2H), 7.65(d, 2H), 8.05 (d, 1H), 8.25(brs, 1H). 10.88(s, 1H).

**Examples 109 to 121**

In the same manner as in Example 108 and using amine compounds described in the following Table 9 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 9 were obtained.

The following Table 9 shows the name of compounds prepared in Examples 109 to 121, the name and equivalents of amine compounds used in the reaction, use/nonuse and equivalents of triethylamine in the reaction, the reaction temperature, the reaction solvent, yield, Mass analysis results and NMR analysis results.

**Table 9** Compounds according to Examples 109 to 121, Solvent S = $CH_3CN$ (all examples) Starting material 2-(4-morpholinophenylamino)-6-chloro-3-nitropyridine compound obtained in Preparation Example 1-13

| Ex | Amine | A | Compound | NMR (DMSO -d6) | T [°C] | Y [%] | M(+) |
|---|---|---|---|---|---|---|---|
| 109 | Isopropylamine (excess) | 2 # | 2-(4-morpholino-phenylamino)-6-(isopropylamino)-3-nitropyridine | 1.18(d, 6H), 3.09(t, 4H), 3.74(t, 4H), 4.09(m, 1H), 6.05(d, 1H), 6.95(d, 2H), 7.60(d, 2H), 8.04(d, 1H), 8.16(d, 1H), 10.85 (s, 1H) | 20~ 30 | 79 | 358.2 |
| 110 | Isobutylamine (excess) | 1.5 # | 2-(4-morpholino-phenylamino)-6-(isobutylamino)-3-nitropyridine | 0.89(d, 6H), 1.85(m, 1H), 3.07(m, 4H), 3.13(m, 2H), 3.74(d, 4H), 6.10(d, 1H), 6.93(d, 2H), 7.59(d, 2H), 8.06(d, 1H), 8.35(t, 1H), 10.80 (s, 1H) | 20~ 30 | 55 | 372.1 |

(continued)

| Ex | Amine | A | Compound | NMR (DMSO -d6) | T [°C] | Y [%] | M(+) |
|---|---|---|---|---|---|---|---|
| 111 | 2-methyl-aminomethyl-1-1,3-dioxolane (2 equivalents) | 2 # | 2-(4-morpholino-phenylamino)-6-[(N-[l, 3]-dioxolan-2-ylmethyl) methylamin o]-3-nitropyridine | 3.08 (brm, 4H), 3.16 (brs, 3H), 3.74 (brm, 6H), 3.82 (brm, 2H), 3.87 (brm, 2H), 5.04(m, 1H), 6.31(m, 1H), 6.29 (brm, 2H), 7.52 (brm, 2H), 8.18 (brm, 1H), 10.49 (s, 1H) | 60~ 70 | 48 | 416.2 |

(continued)

| Ex | Amine | A | Compound | NMR (DMSO -d6) | T [°C] | Y [%] | M(+) |
|---|---|---|---|---|---|---|---|
| 112 | Amine 4-hydroxy-piperidine (1.5 equivalents) | 1.5 # | 2-(4-morpholinophenylamino) -6-(4-hydroxypiperidino)-3-nitropyridine | 1.39 (brm, 2H), 1.78 (brm, 2H), 3.10(t, 4H), 3.39(t, 2H), 3.73(t, 4H), 3.80(m, 1H), 4.02 (brm, 2H), 4.79(d, 1H), 6.49(d, 1H), 6.95(d, 2H), 7.49(d, 2H), 8.15(d, 1H), 10.54 (s, 1H) | 20~ 30 | 61 | 400.2 |
| 113 | 2-methyl-2-imidazoline (2 equivalents) | 2# | 2-(4-morpholino-phenylamino)-6-[(2-methyl-4,5-dihydro-) imidazol-1-yl]-3-nitropyridine | 1.95(s, 3H), 3.09(t, 4H), 3.69(m, 2H), 3.75(t, 4H), 3.84(t, 2H), 6.35(d, 2H), 6.96(d, 2H), 7.29(d, 2H), 8.35(d, 1H), 10.11 (s, 1H) | 60~ 70 | 42 | 383.2 |

(continued)

| Ex | Amine | A | Compound | NMR (DMSO -d6) | T [°C] | Y [%] | M(+) |
|---|---|---|---|---|---|---|---|
| 114 | 2-isopropyl-imidazole (5 equivalents) | 5 # | 2-(4-morpholino-phenylamino)-6-[(2-isopropyl)imidazol-1-yl]-3-nitropyridine | 0.90(d, 6H), 3.11 (brm, 4H), 3.38(m, 1H), 3.74(t, 4H), 6.89(s, 1H), 7.00(m, 3H), 7.27(d, 2H), 7.61(s, 1H), 8.62(d, 1H), 10.02 (s, 1H) | 60~ 70 | 62 | 409.2 |
| 115 | 3-amino-methylpyridine (1.5 equivalents) | 2 # | 2-(4-morpholino-phenylamino)-6-[(3-pyridyl)methylamino] -3-nitropyridine | 3.07(t, 4H), 3.73(t, 4H), 4.54(d, 2H), 6.16(d, 1H), 6.86(d, 2H), 7.34 (dd, 1H), 7.40(d, 2H), 7.59(d, 1H), 8.10(d, 1H), 8.46(m, 1H), 8.75(t, 1H), 10.69 (s, 1H) | 60~ 70 | 78 | 407.2 |

(continued)

| Ex | Amine | A | Compound | NMR (DMSO -d6) | T [°C] | Y [%] | M(+) |
|---|---|---|---|---|---|---|---|
| 116 | 4-aminomethyl-pyridine (1.5 equivalents) | 1.5 # | 2-(4-morpholino-phenylamino)-6-[(4-pyridyl)methylamino] -3-nitropyridine | 3.06 (brm, 4H), 3.74 (brm, 4H), 4.53(d, 2H), 6.20(d, 1H), 6.78(d, 2H), 7.22(d, 2H), 7.30(d, 2H), 8.13(d. 1H), 8.49(d, 2H), 8.82(t, 1H), 10,66 (s, 1H) | 60~ 70 | 63 | 407.1 |
| 117 | t-butylamine (excess) | 2 # | 2-(4-morpholino-phenylamino)-6-(t-butylamino)-3-nitropyridine | 1.19(s, 9H), 3.08(t, 4H), 3.74(t, 4H), 6.09(d, 1H), 6.95(d, 2H), 7.36(d, 2H), 7.78(s, 1H), 7.99(d, 1H), 10.59 (s, 1H) | 20~ 30 | 65 | 372.2 |

(continued)

| Ex | Amine | A | Compound | NMR (DMSO -d6) | T [°C] | Y [%] | M(+) |
|---|---|---|---|---|---|---|---|
| 118 | 2-(ethylamino)-ethanol (2 equivalents) | 2 # | 2-(4-morpholino-phenylamino)-6-[(N-ethyl-2-hydroxy-ethyl)-amino]-3-nitropyridine | 3.08(t, 4H), 3.17(s, 3H). 3.65(m, 4H), 3.74(t, 4H), 4.08(d, 1H), 6.36(d, 1H), 6.94(d, 2H), 7.57 (brm, 2H), 8.15 (brm, 1H), 10.63 (m, 1H) | 20~ 30 | 85 | 374.1 |
| 119 | 1-(3-aminopropyl)-imidazole (1.5 equivalents) | 2 # | 2-(4-morpholino-phenylamino)-6-[(3-imidazol-1-yl)propyl-amino]-3-nitro-pyridine | 1.96(m, 2H), 3.25(m, 2H), 3.73 (brm, 4H), 3.80 (brm, 4H), 3.98(t, 2H), 6.07(d, 1H), 6.88(s, 1H), 6.92(d, 2H), 7.14(s, 1H), 7.53(d, 2H), 7.60(s, 1H), 8.05(d, 1H), 8.30(t, 1H), 10.78 (s, 1H) | 60~ 70 | 83 | 424.4 |

(continued)

| Ex | Amine | A | Compound | NMR (DMSO -d6) | T [°C] | Y [%] | M(+) |
|---|---|---|---|---|---|---|---|
| 120 | Piperazine (5 equivalents) | X | 2-(4-morpholino-phenylamino)-6-(piperazin-1-yl)-3-nitropyridine | 2.73 (brm, 4H), 3.09 (brm, 4H), 3.63 (brm, 4H), 3.74 (brm, 4H), 6.45(d, 1H), 6.95(d, 2H), 7.48(d, 2H), 8.15(d, 1H), 10.56 (s, 1H) | 20~ 30 | 59 | 385.2 |
| 121 | 4-amino-piperidine (2 equivalents) | 2 # | 2-(4-morpholino-phenylamino)-6-(4-aminopiperidino)-3-nitropyridine | 1.20(m, 2H), 1.61(m, 2H), 1.79(m, 2H), 2.87(m, 1H), 3.14(m, 6H), 3.74 (brm, 4H), 4.28 (brm, 2H)., 6.49(d, 1H), 6.95(d, 2H), 7.49(d, 2H), 8.14(d, 1H), 10.55 (s, 1H) | 20~ 30 | 73 | 399.2 |

**Example 122**

**Preparation of 2-[(3,4-difluoro)phenylamino]-6-(methylamino)-3-nitropyridine**

To 10ml of acetonitrile were added 300mg (1.05mmol) of the 2-[(3,4-difluoro)phenylamino]-6-chloro-3-nitropyridine compound obtained in Preparation Example 1-14 and 3ml of a 40% (wt/v) methylamine-methanol solution, followed by reaction at room temperature for 4 hours. After the reaction was complete, the solvent was distilled under reduced pressure, followed by stirring in 5ml of methanol for 1 hour at about 40°C. The resulting solid was filtered, washed with 5ml of methanol and then dried under vacuum at about 40°C to afford 270mg (yield: 93%) of the desired compound.

Mass (M+): 281.2

$^1$H-NMR(DMSO-$d_6$) (ppm) 2.88(d, 3H), 6.12(d, 1H), 7.42(m, 1H), 7.50(m, 1H), 8.07(m, 1H), 8.34(m, 1H), 10.86 (s, 1H).

**Examples 123 to 131**

In the same manner as in Example 122 and using amine compounds described in the following Table 10 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 10 were obtained.

The following Table 10 shows the name of compounds prepared in Examples 123 to 131, the name and equivalents of amine compounds used in the reaction, use/nonuse and equivalents of triethylamine in the reaction, the reaction temperature, the reaction solvent, yield, Mass analysis results and NMR analysis results.

**Table 10**

| Compounds according to Examples 123 to 131, Solvent S = $CH_3CN$ (all examples) Starting material 2-[(3,4-difluoro) phenylamino]-6-chloro-3-nitropyridine compound obtained in Preparation Example 1-14 | | | | | | | |
|---|---|---|---|---|---|---|---|
| Ex | Amine | A | Compound | NMR (DMSO -d6) | T [°C] | Y [%] | M(+) |
| 123 | Isopropylamine (excess) | 2 # | 2-[(3,4-difluoro)-phenylamino]-6-(isopropylamino)-3 nitropyridine | 1.19(d, 6H), 4.04(m, 1H), 6.12(d, 1H), 7.42(m, 2H), 8.06(m, 1H), 8.24(m, 1H), 10.82 (s, 1H) | 20~ 30 | 96 | 309.1 |

(continued)

| Compounds according to Examples 123 to 131, Solvent S = $CH_3CN$ (all examples) Starting material 2-[(3,4-difluoro) phenylamino]-6-chloro-3-nitropyridine compound obtained in Preparation Example 1-14 | | | | | | | |
|---|---|---|---|---|---|---|---|
| Ex | Amine | A | Compound | NMR (DMSO -d6) | T [°C] | Y [%] | M(+) |
| 124 | Isobutylamine (excess) | 1.5 # | 2-[(3,4-difluoro-phenyl-amino]-6-(isobutylamino)-3-nitropyridine | 0.89(d, 6H), 1.86(m, 1H), 3.14(t, 2H), 6.17(d, 1H), 7.40(m, 2H), 8.09(m, 2H), 8.46(m, 1H), 10.82 (s, 1H) | 20~ 30 | 88 | 323.2 |
| 125 | t-butylamine (excess) | 2 # | 2-[(3,4-difluoro)-phenylamino]-6-(t-butylamino)-3-nitro-pyridine | 1.24(s, 9H), 6.15(d, 1H), 7.27(m, 1H) 7,43(m, 1H), 7.74(m, 1H), 8.01(m, 1H), 8.03(d, 1H), 10,57 (s, 1H) | 20~ 30 | 29 | 323.1 |

(continued)

| Compounds according to Examples 123 to 131, Solvent S = $CH_3CN$ (all examples) Starting material 2-[(3,4-difluoro) phenylamino]-6-chloro-3-nitropyridine compound obtained in Preparation Example 1-14 | | | | | | | |
|---|---|---|---|---|---|---|---|
| Ex | Amine | A | Compound | NMR (DMSO -d6) | T [°C] | Y [%] | M(+) |
| 126 | 4-hydroxy-piperidine (1.5 equivalents) | 1.5 # | 2-[(3,4-difluoro-)-phenylamino] -6-(4-hydroxypiperidino)-3-nitropyridine | 1.39(m, 2H), 1.79(m, 2H), 3.41(m, 2H), 3.79(m, 1H), 4.01(m, 2H), 4.83(d, 1H), 6.55(s, 1H), 7.41(m, 2H), 7.80(m, 1H), 8.16(d, 1H), 10.53 (s, 1H) | 20~ 30 | 86 | 351.1 |
| 127 | 2-methylamino-methyl-1-1,3-dioxolane (2 equivalents) | 2 # | 2-[(3,4-difluoro)-phenylamino]-6-[(N-[1,3]-dioxolan-2-ylmethyl)-methylamino] -3-nitro-pyridine | 1.80(s, 3H), 3.23(m, 2H), 3.40(m, 2H), 6.14(d, 1H), 7.42(m, 1H), 7.52(m, 1H), 7.94(m, 1H), 8.12(m, 1H), 10.79 (s, 1H) | 60~ 70 | 55 | 334.1 |

(continued)

| Compounds according to Examples 123 to 131, Solvent S = $CH_3CN$ (all examples) Starting material 2-[(3,4-difluoro) phenylamino]-6-chloro-3-nitropyridine compound obtained in Preparation Example 1-14 | | | | | | | |
|---|---|---|---|---|---|---|---|
| Ex | Amine | A | Compound | NMR (DMSO -d6) | T [°C] | Y [%] | M(+) |
| 128 | 1-methyl-piperazine (1.5 equivalents) | 1.5 # | 2-[(3,4-difluoro)-phenylamino]-6-(4-methylpiperazin-1-yl)-3-nitropyridine | 2.01(s, 3H), 2.37(m, 4H), 3.68(m, 4H), 6.54(d, 1H), 7.42(m, 2H), 7.80(m, 1H), 8.20(d, 1H), 10.50 (s, 1H) | 20~ 30 | 89 | 350.1 |
| 129 | Morpholine (3 equivalents) | X | 2-[(3,4-difluoro)-phenylamino]-6-morpholino-3-nitropyridine | 3.67 (brm 8H) 6.51(d 1H) 7.41(m, 2H), 7.77(m, 1H), 8.22(d, 1H), 10.49 (s, 1H) | 20~ 30 | 93 | 318.2 |
| 130 | 4-amino-piperidine (1.5 equivalents) | 1.5 # | 2-[(3,4-difluoro)-phenylamino]-6-(4-aminopiperidino)-3-nitropyridine | 1.22(m, 2H), 1.77(m, 2H), 2.88(m, 1H), 3.18(m, 2H), 4.22(m, 2H), 6.54(d, 1H), 7.40(m, 2H), 7.81(m, 1H), 8.16(d, 1H), 10.54 (s, 1H) | 20~ 30 | 89 | 350.1 |

(continued)

| Compounds according to Examples 123 to 131, Solvent S = $CH_3CN$ (all examples) Starting material 2-[(3,4-difluoro) phenylamino]-6-chloro-3-nitropyridine compound obtained in Preparation Example 1-14 | | | | | | | |
|---|---|---|---|---|---|---|---|
| Ex | Amine | A | Compound | NMR (DMSO -d6) | T [°C] | Y [%] | M(+) |
| 131 | 4-amino-methylpyridine (1.5 equivalents) | 2 # | 2-[(3,4-difluoro)-phenylamino]-6-[(4-pyridyl) methylamino] -3-nitropyridine | 4.57(m, 2H), 6.28(d, 1H), 7.23(m, 7.67(m, 4H), 8.17(d, 1H), 8.49(m, 2H), 8.88(m, 1H), 10.66 (s, 1H) | 60~ 70 | 75 | 358.1 |

**Example 132**

**Preparation of 2-[4-(2-methylthiazol-4-yl)phenylamino]-6-(methylamino)-3-nitropyridine**

To 10ml of acetonitrile were added 200mg (0.58 mmol) of the 2-[4-(2-methylthiazol-4-yl)phenylamino]-6-chloro-3-nitropyridine compound obtained in Preparation Example 2-1-4 and 10ml of a 40% (wt/v) methylamine-methanol solution, followed by reaction at room temperature for 4 hours. After the reaction was complete, the solvent was distilled under reduced pressure, followed by stirring in 10ml of methanol for 1 hour at room temperature. The resulting solid was filtered, washed with 5ml of methanol and then dried under vacuum at about 40°C to afford 175mg (yield: 88%) of the desired compound.

Mass (M+): 342.1

$^1$H-NMR(DMSO-$d_6$) (ppm) 2.71(s, 3H), 2.95(d, 3H), 6.14(d, 1H), 7.89(m, 3H), 7.95(d, 2H), 8.08(d, 1H), 8.39 (m, 1H), 11.03(s, 1H).

**Examples 133 to 145**

In the same manner as in Example 132 and using amine compounds described in the following Table 11 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 11 were obtained.

The following Table 11 shows the name of compounds prepared in Examples 133 to 145, the name and equivalents of amine compounds used in the reaction, use/nonuse and equivalents of triethylamine in the reaction, the reaction temperature, the reaction solvent, yield, Mass analysis results and NMR analysis results.

**Table 11**

| Compounds according to Examples 133 to 145, Solvent S = $CH_3CN$ (all examples) Starting material 2-[4-(2-methylthiazol-4-yl)phenylamino]-6-chloro-3-nitropyridine compound obtained in Preparation Example 2-1-4 | | | | | | | |
|---|---|---|---|---|---|---|---|
| Ex | Amine | A | Compound | NMR (DMSO -d6) | T [°C] | Y [%] | M(+) |
| 133 | Isopropylamine (excess) amine (excess) | X | 2-[4-(2-methyl-thiazol-4-yl)phenyl-amino]-6-(isopropyl-amino)-3-nitro-pyridine | 1.22(d, 6H), 2.72(s, 3H), 4.16(m, 1H), 6.12(d, 1H), 7.84(d, 2H), 7.89(s, 1H), 7.94(d, 2H), 8.10(d, 1H), 8.26(d, 1H), 11.02 (s, 1H) | 20~ 30 | 84 | 370.1 |
| 134 | Isobutylamine (excess) | X | 2-[4-(2-methylthiazol-4-yl)phenylamino]-6-(isobutyl-amino)-3-nitro-pyridine | 0.93(d, 6H), 1.91(m, 1H), 2.72(s, 3H), 3.21(t, 1H), 6.18(d, 1H), 7.84(d, 2H), 7,92(m, 3H), 8.10(d, 1H), 8.47(t, 1H), 11.01 (s, 1H) | 20~ 30 | 77 | 384.2 |

(continued)

| Compounds according to Examples 133 to 145, Solvent S = $CH_3CN$ (all examples) Starting material 2-[4-(2-methylthiazol-4-yl)phenylamino]-6-chloro-3-nitropyridine compound obtained in Preparation Example 2-1-4 | | | | | | | |
|---|---|---|---|---|---|---|---|
| Ex | Amine | A | Compound | NMR (DMSO -d6) | T [°C] | Y [%] | M(+) |
| 135 | 4-hydroxy-piperidine (2 equivalents) | 2 # | 2-[4-(2-methyl-thiazol-4-yl)phenyl-amino]-6-(4-hydroxy-piperidino)-3-nitro-pyridine | 1.41(m, 2H), 1,81(m, 2H), 2.72(s, 3H), 3.44(m, 2H), 3.81(m, 1H), 4.02 (brm, 2H), 4.83(s, 1H), 6.56(d, 1H), 7.72(d, 2H), 7.90(s, 1H), 7.95(d, 2H), 8.19(d, 1H), 10.72 (s, 1H) | 20~ 30 | 60 | 412.2 |
| 136 | 2-methyl-2-imidazoline (2 equivalents) | 2 # | 2-[4-(2-methylthiazol-4-yl)phenylamino]-6-[(2-methyl-4,5-dihydro)imidazol-1-yl]-3-nitropyridine | 2.08(s, 3H), 2,72(s, 3H), 3.70(t, 1H), 3.90(t, 2H), 6.48(d, 1H), 7.57(d, 2H), 7.91(s, 1H), 7.96(d, 2H), 8.40(d, 1H), 10.33 (s, 1H) | 60~ 70 | 71 | 395.1 |

(continued)

| Compounds according to Examples 133 to 145, Solvent S = $CH_3CN$ (all examples) Starting material 2-[4-(2-methylthiazol-4-yl)phenylamino]-6-chloro-3-nitropyridine compound obtained in Preparation Example 2-1-4 | | | | | | | |
|---|---|---|---|---|---|---|---|
| Ex | Amine | A | Compound | NMR (DMSO -d6) | T [°C] | Y [%] | M(+) |
| 137 | 2-isopropylimidazole (5 equivalents) | 5 # | 2-[4-(2-methylthiazol-4-yl)phenylamino]-6-[(2-isopropylimidazol-1-yl]-3-nitropyridine | 0.92(d, 6H), 2.73(s, 3H), 3.44(m, 1H), 6.92(s, 1H), 7.11(d, 1H), 7.54(d, 2H), 60~ 39 7.62(s, 1H), 7.94(s, 1H), 7.98(d, 1H), 8.68(d, 1H), 10.21 (s, 1H) | 70 | | 421.1 |
| 138 | 3-aminomethyl-pyridine (1.5 equivalents) | 2 # | 2-[4-(2-methylthiazol-4-yl)phenylamino]-6-[(3-pyridyl)-methylamino]-3-nitropyridine | 2.71(s, 3H), 4.63(d, 2H), 6.23(s, 1H), 7.34(m, 1H), 7.68(d, 3H), 7.86( (m, 3H), 7.16(d, 1H), 8.46(s, 1H), 8.53(s, 1H), 8.84(t, 1H), 10.89 (s, 1H) | 60~ 70 | 68 | 419.1 |

(continued)

| Compounds according to Examples 133 to 145, Solvent S = $CH_3CN$ (all examples) Starting material 2-[4-(2-methylthiazol-4-yl)phenylamino]-6-chloro-3-nitropyridine compound obtained in Preparation Example 2-1-4 | | | | | | | |
|---|---|---|---|---|---|---|---|
| Ex | Amine | A | Compound | NMR (DMSO -d6) | T [°C] | Y [%] | M(+) |
| 139 | 4-aminomethylpyridine (1.5 equivalents) | 1.5 # | 2-[4-(2-methylthiazol-4-yl)phenylamion]-6-[(4-pyridyl) methylamino]-3-nitropyridine | 2.72(s, 3H), 4.63 (d, 2H), 6.28(d, 1H), 7.29(d, 2H), 7.56(d, 2H), 7.78(d, 2H), 7.86(s, 1H), 8.19(d, 2H), 8.52(d, 2H), 8.89(t, 1H), 10.85 (s, 1H), | 60~ 70 | 73 | 419.1 |
| 140 | t-butylamine (excess) | X | 2-[4-(2-methyl-thiazol-4-yl)phenyl-amino]-6-(t-butyl-mino)-3-nitropyridine | 1.31(s, 9H), 2.72(s, 3H), 6.17(d, 1H), 7,64(d, 2H), 7.93(m, 4H), 8.03(d, 1H), 10.83 (s, 1H) | 20 ~ 30 | 77 | 384.2 |

(continued)

| Compounds according to Examples 133 to 145, Solvent S = $CH_3CN$ (all examples) Starting material 2-[4-(2-methylthiazol-4-yl)phenylamino]-6-chloro-3-nitropyridine compound obtained in Preparation Example 2-1-4 | | | | | | | |
|---|---|---|---|---|---|---|---|
| Ex | Amine | A | Compound | NMR (DMSO -d6) | T [°C] | Y [%] | M(+) |
| 141 | 2-ethylamino-ethanol (2 equivalents) | 2 # | 2-[4-(2-methyl-thiazol-4-yl)phenyl-amino]-6-[(N-ethyl-2-6.43(m, hydroxyethyl) amino]-3-nitropyridine | 1.16(t, 3H), 2.72(s, 3H), 3.62 (brm, 6H), 4.90(d, 1H), 1H), 7.77 (brm, 2H), 7.88(s, 1H), 7.94(d, 2H), 8.19(t, 1H), 7.94(d, 2 H), 8.19(t, 1H), 10.81 (s, 1H) | 60~ 70 | 51 | 400.2 |
| 142 | 1-methyl-piperazine (1.5 equivalents) | 2 # | 2-[4-(2-methylthiazol-4-yl)phenylamino]-6-[(4-methyl)-piperazin-1-yl]-3-nitropyridine | 2.20(s, 3H), 2.40(m, 4H), 2.71(s, 3H), 3.73 (brm, 4H), 6.54(d, 1H), 7.70(d, 2H), 7.89(s, 1H), 7.94(d, 2H), 8.21(d, 1H), 10.69 (s, 1H) | 20~ 30 | 64 | 411.2 |

(continued)

| Compounds according to Examples 133 to 145, Solvent S = $CH_3CN$ (all examples) Starting material 2-[4-(2-methylthiazol-4-yl)phenylamino]-6-chloro-3-nitropyridine compound obtained in Preparation Example 2-1-4 | | | | | | | |
|---|---|---|---|---|---|---|---|
| Ex | Amine | A | Compound | NMR (DMSO -d6) | T [°C] | Y [%] | M(+) |
| 143 | Piperazine (5 equivalents) | X | 2-[4-(2-methylthiazol-4-yl)phenyl-amino]-6-(piperazin-1-yl)-3-nitropyridine | 2.72(s, 3H), 2.86(t, 4H), 3.67(m, 4H), 6.52(d, 1H), 7.71(d, 2H), 7.89(s, 3H), 7.71(d, 2H), 7.89(s, 3H), 7.94(d, 2H), 8.19(d, 1H), 10.37 (s, 1H) | 20~ 30 | 78 | 397.2 |
| 144 | 4-amino-piperidine (2 equivalents) | 2 # | 2-[4-(2-methylthiazol-4-yl)phenylamino]-6-(4-amino-piperidino)-3-nitropyridine | 1.23(m, 2H), 1.56(m, 2H), 1.79(m, 2H), 2.72(s, 3H), 2.88(m, 1H), 3.19(t, 2H), 4.29 (brm, 2H), 6.55(d, 1H), 7.72(d, 2H), 7.90(s, 1H), 7.94(d, 2H), 8.21(d, 1H), 10.72 (s, 1H) | 20 ~ 30 | 57 | 411.2 |

(continued)

| Compounds according to Examples 133 to 145, Solvent S = $CH_3CN$ (all examples) Starting material 2-[4-(2-methylthiazol-4-yl)phenylamino]-6-chloro-3-nitropyridine compound obtained in Preparation Example 2-1-4 | | | | | | | |
|---|---|---|---|---|---|---|---|
| Ex | Amine | A | Compound | NMR (DMSO -d6) | T [°C] | Y [%] | M(+) |
| 145 | Morpholine (3 equivalents) | X | 2-[4-(2-methylthiazol-4-yl)phenyl-amino]-6-nitropyridine | 2.71(s, 3H), 3.71 (brm, 8H), 654(d, 1H), 7.71(d, 2H) 7.89(s, 1H), 7.95(d, 2H), 8.25(d, 1H), 10.69 (s, 1H) | 20 ~ 30 | 70 | 398.2 |

**Example 146**

**Preparation of 2-[4-(2-isopropylthiazol-4-yl)phenylamino]-6-(isobutylamino)-3-nitropyridine**

To 10ml of acetonitrile were added 250mg (0.67 mmol) of the 2-[4-(2-isopropylthiazol-4-yl)phenylamino]-6-chloro-3-nitropyridine compound obtained in Preparation Example 2-2-3 and 3ml of isobutylamine, followed by reaction at room temperature for 4 hours. After the reaction was complete, the solvent was distilled under reduced pressure, followed by stirring in 10ml of acetonitrile for 1 hour at room temperature. The resulting solid was filtered, washed with 5ml of methanol and then dried under vacuum at about 40°C to afford 150mg (yield: 54%) of the desired compound.

Mass (M+): 412.2

$^1$H-NMR(DMSO-$d_6$) (ppm) 0.92(d, 6H), 1.37(d, 6H), 1.91(m, 1H), 3.21(t, 2H), 3.34(m, 1H), 6.17(d, 1H), 7.85 (d, 2H), 7.94(m, 3H), 8.10(d, 1H), 8.47(t, 1H), 11.00(s, 1H).

**Examples 147 to 150**

In the same manner as in Example 146 and using amine compounds described in the following Table 12 in place of "isobutylamine", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 12 were obtained.

The following Table 12 shows the name of compounds prepared in Examples 147 to 150, the name and equivalents of amine compounds used in the reaction, use/nonuse and equivalents of triethylamine in the reaction, the reaction temperature, the reaction solvent, yield, Mass analysis results and NMR analysis results.

**Table 12**

| Compounds according to Examples 147 to 150, Solvent S = $CH_3CN$ (all examples) Starting material 2-[4-(2-isopropylthiazol-4-yl)phenylamino]-6-chloro-3-nitropyridine compound obtained in Preparation Example 2-2-3 | | | | | | | |
|---|---|---|---|---|---|---|---|
| Ex | Amine | A | Compound | NMR (DMSO -d6) | T [°C] | Y [%] | M(+) |
| 147 | 4-hydroxypiperidine (2 equivalents) | 2 # | 2-[4-(2-isopropylthia- 1.84 (d, zol-4-yl) phenylamino]-6-(4-hydroxypiperidino) -3-nitropyridine | 1.37(d, 6H), 1.42(m, 2H), . 32(m, 2H), 3.44(m, 2H), 3.81(m, 1H), 4.10 (brm, 1H), 4.84(d, 1H), 6.57(d, 1H), 7.73(d, 2H), 7.96(m, 3H), 8.20(d, 1H), 10.73 (s, 1H) | 20~ 30 | 64 | 440.2 |
| 148 | 2-(ethylamino)-ethanol (1.5 equivalents) | # | 2-[4-(2-isopropyl-thiazol-4-yl) phenyl-1.5 amino]-6-[(N-ethyl-2-hydroxyethyl) amino]-3-nitropyridine | 1.15(t, 3H), 1.39(d, 6H), 3.16(t, 2H), 3.32(m, 1H), 3.61(m, 5H), 4.90(m, 1H), 6.43(s, 1H), 7.78(d, 2H), 7.94(m, 3H), 8.18(d, 1H), 10.82 (s, 1H) | 60 ~ 70 | 73 | 428.2 |

(continued)

| Compounds according to Examples 147 to 150, Solvent S = $CH_3CN$ (all examples) Starting material 2-[4-(2-isopropylthiazol-4-yl)phenylamino]-6-chloro-3-nitropyridine compound obtained in Preparation Example 2-2-3 | | | | | | | |
|---|---|---|---|---|---|---|---|
| Ex | Amine | A | Compound | NMR (DMSO -d6) | T [°C] | Y [%] | M(+) |
| 149 | 1-methyl-piperazine (1.5 equivalents) | 1.5 # | 2-[4-(2-isopropylthiazol-4-yl)phenyl-amino]-6-(4-methyl-piperazin-1-yl)-3-nitropyridine | 1.41(d, 6H), 2.23(s, 3H), 2.45 (brm, 4H), 3.72 (brm, 4H), 6.55(d, 1H), 7.72(d, 2H), 7.95(t, 3H), 8.21(d, 1H), 10.72 (s, 1H) | 20~ 30 | 83 | 439.2 |
| 150 | 4-amino-piperidine (2 equivalents) | 2 # | 2-[4-(2-isopropylthiazol-4-yl)phenylamino]-6-(4-amino-pipendino)-3-nitropyridine | 1.23(m, 2H), 1.39(d, 6H), 1.58(m, 2H), 1,85(m, 2H), 2.89(m, 1H), 3.17(m, 2H), 3.35(m, 1H), 6.57(d, 1H), 7.71(d, 2H), 7.93(d, 3H), 8.20(s, 1H), 10.72 (s, 1H) | 20~ 30 | 52 | 394.2 |

**Example 151**

**Preparation of 2-[4-(2-cyclohexylthiazol-4-yl)phenylamino]-6-(methylamino)-3-nitropyridine**

To 10ml of acetonitrile were added 200mg (0.48 mmol) of the 2-[4-(2-cyclohexylthiazol-4-yl)phenylamino]-6-chloro-3-nitropyridine compound obtained in Preparation Example 2-3-3 and 5ml of a 40% (wt/v) methylamine-methanol solution, followed by reaction at room temperature for 3 hours. After the reaction was complete, the solvent was distilled under reduced pressure, followed by stirring in 5ml of methanol for 1 hour at room temperature. The resulting solid was filtered, washed with 5ml of methanol and then dried under vacuum at about 40°C to afford 162mg (yield: 83%) of the desired compound.

Mass (M+): 410.2

$^1$H-NMR(DMSO-$d_6$) (ppm) 1.23(m, 1H), 1.43(m, 2H), 1.52(m, 2H), 1.78(m, 1H), 1.82(m, 2H), 2.10(m, 2H), 2.94 (d, 3H), 3.04(m, 1H), 6.15(d, 1H), 7.89(d, 2H), 7.93(m, 3H), 8.10(d, 1H), 8.35(m, 1H), 11.04(s, 1H).

**Examples 152 to 165**

In the same manner as in Example 151 and using amine compounds described in the following Table 13 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 13 were obtained.

The following Table 13 shows the name of compounds prepared in Examples 152 to 165, the name and equivalents of amine compounds used in the reaction, use/nonuse and equivalents of triethylamine in the reaction, the reaction temperature, the reaction solvent, yield, Mass analysis results and NMR analysis results.

**Table 13**

| Compounds according to Examples 152 to 165, Solvent S = $CH_3CN$ (all examples) Starting material 2-[4-(2-cyclohexylthiazol-4-yl)phenylamino]-6-chloro-3-nitropyridine compound obtained in Preparation Example 2-3-3 | | | | | | | |
|---|---|---|---|---|---|---|---|
| Ex | Amine | A | Compound | NMR (DMSO -d6) | T [°C] | Y [%] | M(+) |
| 152 | Isopropylamine (excess) | X | 2-[4-(2-cyclohexylthiazol-4-yl)phenyl-amino]-6-(isopropyl-amino)-3-nitro-pyridine | 0.93(d, 6H), 1.28(m, 1H), 1.43(m, 2H), 1.50(m, 2H), 1.68(m, 1H), 1.80(m, 2H), 1.91(m, 1H), 2.10(m, 2H), 3.04(m, 1H), 3.22(m, 2H), 6.18(d, 1H), 7.85(d, 2H), 7.93(m, 3H), 8.09(d, 1H), 8.46(t, 1H), 11.00 (s, 1H) | 20~ 30 | 66 | 452.3 |

(continued)

| Compounds according to Examples 152 to 165, Solvent S = $CH_3CN$ (all examples) Starting material 2-[4-(2-cyclohexylthiazol-4-yl)phenylamino]-6-chloro-3-nitropyridine compound obtained in Preparation Example 2-3-3 | | | | | | | |
|---|---|---|---|---|---|---|---|
| Ex | Amine | A | Compound | NMR (DMSO -d6) | T [°C] | Y [%] | M(+) |
| 153 | Isobutylamine (excess) | X | 2-[4-(2-cyclohexyl-thiazol-4-yl)phenyl-amino]-6-(isobutyl-amino)-3-nitro-pyridine | 1.22(d, 6H), 1.25(m, 1H), 1.40(m, 2H), 1.52(m, 2H), 1.72(m, 1H), 1,80(m, 2H), 2.10(m, 2H), 3.03(m, 1H), 4.16(m, 1H), 6.12(d, 1H), 7.84(d, 2H), 7.93(m, 3H), 8.10(d, 1H), 8.26(d, 1H), 11.03 (s, 1H) | 60~ 70 | 76 | 438.2 |

(continued)

| Compounds according to Examples 152 to 165, Solvent S = $CH_3CN$ (all examples) Starting material 2-[4-(2-cyclohexylthiazol-4-yl)phenylamino]-6-chloro-3-nitropyridine compound obtained in Preparation Example 2-3-3 | | | | | | | |
|---|---|---|---|---|---|---|---|
| Ex | Amine | A | Compound | NMR (DMSO -d6) | T [°C] | Y [%] | M(+) |
| 154 | t-butylamine (excess) | X | 2-[4-(2-cyclohexyl-thiazol-4-yl) phenylamino]-6-(t-butylamino)-3-nitropyridine | 1.28 (s+m, 10H), 1.43(m, 2H), 1.50(m, 2H), 1.70(m, 1H), 1.80(m, 2H), 2.11(m, 2H), 3.02(tt, 1H), 6.17(d, 1H), 7.64(d, 2H), 7.91(d, 1H), 7.94(m, 2H), 8.02(d, 1H), 10.84 (s, 1H) | 20~ 30 | 78 | 452.2 |

(continued)

| Compounds according to Examples 152 to 165, Solvent S = $CH_3CN$ (all examples) Starting material 2-[4-(2-cyclohexylthiazol-4-yl)phenylamino]-6-chloro-3-nitropyridine compound obtained in Preparation Example 2-3-3 | | | | | | | |
|---|---|---|---|---|---|---|---|
| Ex | Amine | A | Compound | NMR (DMSO -d6) | T [°C] | Y [%] | M(+) |
| 155 | 4-hydroxypiperidine (2 equivalents) | 2 # | 2-[4-(2-cyclohexylthiazol-4-yl)phenylamino]-6-(4-hydroxypiperidino)-3-nitropyridine | 1.29(tt, 1H), 1.42(m, 4H), 1.53(m, 2H), 1.70(dt, 1H), 1.82 (brm, 4H), 2.12(dt, 2H), 3.03(tt, 1H), 3.44(m, 2H), 3.81(m, 1H), 4.08(m, 2H), 4.83(d, 1H), 6.56(d, 1H), 7.72(d, 2H), 7.94(m, 3H), 8.18(d, 1H), 10.72 (s, 1H) | 20~ 30 | 66 | 480.3 |

(continued)

| Compounds according to Examples 152 to 165, Solvent S = $CH_3CN$ (all examples) Starting material 2-[4-(2-cyclohexylthiazol-4-yl)phenylamino]-6-chloro-3-nitropyridine compound obtained in Preparation Example 2-3-3 | | | | | | | |
|---|---|---|---|---|---|---|---|
| Ex | Amine | A | Compound | NMR (DMSO -d6) | T [°C] | Y [%] | M(+) |
| 156 | 2-ethylamino)-ethanol (2 equivalents) | 2 # | 2-[4-(2-cyclohexyl-thiazol-4-yl) phenylamino]-6-[(N-ethyl-2- hydroxyethyl) amino]-3-nitropyridine | 1.16(t, 3H), 1.28(tt, 1H), 1.44(m, 2H), 1.53(m, 2H), 1.44(m, 2H), 1.53(m, 2H), 1.70(m, 1H), 1.79(dt, 2H), 2.12(m, 2H), 3.03(tt, 1H), 3.62(m, 6H), 4.90(d, 1H), 6.42(d, 1H), 7.75(d, 2H), 7.93(m, 3H), 8.19(d, 1H), 10.82 (s, 1H) | 20~ 30 | 65 | 468.2 |

(continued)

| Compounds according to Examples 152 to 165, Solvent S = $CH_3CN$ (all examples) Starting material 2-[4-(2-cyclohexylthiazol-4-yl)phenylamino]-6-chloro-3-nitropyridine compound obtained in Preparation Example 2-3-3 | | | | | | | |
|---|---|---|---|---|---|---|---|
| Ex | Amine | A | Compound | NMR (DMSO -d6) | T [°C] | Y [%] | M(+) |
| 157 | 2-isopropyl-imidazole (1.5 equivalents) | 1.5 # | 2-[4-(2-cyclohexyl-thiazol-4-yl)phenyl-amino]-6-[(2-amino]-6-[(2-isopropyl) imidazol-1-yl]-3-nitropyridine | 0.93(d, 6H), 1.28(m, 1H), 1.43(m, 2H), 1.52(m, 2H), 1.70(dt, 1H), 1.80(dt, 2H), 2.11(m 2H) 3.05(tt, 1H), 3.45(p, 1H), 6.92(s, 1H), 3.45(p, 1H), 6.92(s, 1H), 7.11(s, 1H), 7.54(d, 1H), 7.61(s, 1H), 7.96(m, 3H), 8.68(d, 1H), 10.21 (s, 1H) | 60~ 70 | 52 70 | 478.2 |

(continued)

| Compounds according to Examples 152 to 165, Solvent S = $CH_3CN$ (all examples) Starting material 2-[4-(2-cyclohexylthiazol-4-yl)phenylamino]-6-chloro-3-nitropyridine compound obtained in Preparation Example 2-3-3 | | | | | | | |
|---|---|---|---|---|---|---|---|
| Ex | Amine | A | Compound | NMR (DMSO -d6) | T [°C] | Y [%] | M(+) |
| 158 | Piperazine (5 equivalents) | X | 2-[4-(2-cyclohexyl-thiazol-4-yl) phenylamino]-6-(piperazin-1-yl)-3-nitropyridine | 1.28(m, 1H), 1.43(m, 2H), 1.50(m, 2H), 1.70(m, 1H), 1.80(m, 2H), 2.10(m, 2H), 2.77(m, 5H), 3.04(m, 1H), 3.67 (brm, 4H), 6.52(d, 1H), 7.01(d, 2H), 7.93(m, 3H), 8.22(d, 1H), 10.74 (s, 1H) | 20~ 30 | 90 | 465.3 |

(continued)

| Compounds according to Examples 152 to 165, Solvent S = $CH_3CN$ (all examples) Starting material 2-[4-(2-cyclohexylthiazol-4-yl)phenylamino]-6-chloro-3-nitropyridine compound obtained in Preparation Example 2-3-3 | | | | | | | |
|---|---|---|---|---|---|---|---|
| Ex | Amine | A | Compound | NMR (DMSO -d6) | T [°C] | Y [%] | M(+) |
| 159 | 1-methyl-piperazine (2 equivalents) | 2 # | 2-[4-(2-cyclohexyl-thiazol-4-yl) phenylamino]-6-(4-methyl-piperazin-1-yl)-3-nitropyridine | 1.28(m, 1H), 1.42(m, 2H), 1.51(m, 2H), 1.70(m, 1H), 1.78(m, 2H), 2.08(m, 2H), 2.20(s, 3H), 2.39(t, 4H), 3.03((tt, 1H), 3.74 (brm, 4H), 6.54(d, 1H), 7.71(d, 2H), 7.92(s, 1H), 7.95(d, 1H), 8.22(d, 1H), 10.70 (s, 1H) | 20~ 30 | 83 | 479.2 |

(continued)

| Compounds according to Examples 152 to 165, Solvent S = $CH_3CN$ (all examples) Starting material 2-[4-(2-cyclohexylthiazol-4-yl)phenylamino]-6-chloro-3-nitropyridine compound obtained in Preparation Example 2-3-3 | | | | | | | |
|---|---|---|---|---|---|---|---|
| Ex | Amine | A | Compound | NMR (DMSO -d6) | T [°C] | Y [%] | M(+) |
| 160 | Morpholine (3 equivalents) | X | 2-[4-(2-cyclohexyl-thiazol-4-yl) phenylamino]-6-morpholino-3-nitropyridine | 1.28(m, 1H), 1.43(m, 2H), 1.50(m, 2H), 1.60(m, 1H), 1.80(m, 2H), 2.10(m, 2H), 2.10(m, 2H), 1.80(m, 2H), 2.10(m, 2H), 3.04(tt, 1H), 3.70 (brm, 8H), 6.53(d, 1H), 7.01(d, 1H), 7.91(s, 1H), 7.95(m, 2H), 8.23(d, 1H), 10.70 (s, 1H) | 20~ 30 | 94 | 466.2 |

(continued)

| Compounds according to Examples 152 to 165, Solvent S = $CH_3CN$ (all examples) Starting material 2-[4-(2-cyclohexylthiazol-4-yl)phenylamino]-6-chloro-3-nitropyridine compound obtained in Preparation Example 2-3-3 | | | | | | | |
|---|---|---|---|---|---|---|---|
| Ex | Amine | A | Compound | NMR (DMSO -d6) | T [°C] | Y [%] | M(+) |
| 161 | 4-amino-piperidine (1.5 equivalents) | 1.5 | 2-[4-(2-cyclohexyl-thiazol-4-yl)phenyl-piperidino-3-nitropyridine | 1.24(m, 3H), 1.40(m, 2H), 1.50(m, 2H), 1.68(m, 3H), 2.89(m, 1H), 3.01(tt, 1H), 6.56(d, 1H), 7.73(d, 2H), 7.93(m, 3H), 8.19(d, 1H), 10.73 (s, 1H) | 20~ | 77 | 479.3 |

(continued)

| Compounds according to Examples 152 to 165, Solvent S = $CH_3CN$ (all examples) Starting material 2-[4-(2-cyclohexylthiazol-4-yl)phenylamino]-6-chloro-3-nitropyridine compound obtained in Preparation Example 2-3-3 | | | | | | | |
|---|---|---|---|---|---|---|---|
| Ex | Amine | A | Compound | NMR (DMSO -d6) | T [°C] | Y [%] | M(+) |
| 162 | 3-amino-methylpyridine (1.5 equivalents) | 1.5 # | 2-[4-(2-cyclohexylthiazol-4-yl)phenylamino]-6-[(3-pyridyl)-methylamino] -3-nitropyridine | 1.27(m, 1H), 1.39(m, 2H), 1.53(m, 2H), 1.69(m, 1H), 1.80(m, 2H), 2.08(m, 2H), 3.03(tt, 1H), 4.62(d, 2H), 6.23(d, 1H), 7.35(t, 1H), 7.69(d, 3H), 7.87(m, 2H), 7.89(s, 1H), 8.16(d, 1H), 8.46(d, 2H), 8.53(s, 1H), 8.84(t, 1H), 10.90 (s, 1H) | 60~ 70 | 70 | 487.2 |

(continued)

| Compounds according to Examples 152 to 165, Solvent S = $CH_3CN$ (all examples) Starting material 2-[4-(2-cyclohexylthiazol-4-yl)phenylamino]-6-chloro-3-nitropyridine compound obtained in Preparation Example 2-3-3 | | | | | | | |
|---|---|---|---|---|---|---|---|
| Ex | Amine | A | Compound | NMR (DMSO -d6) | T [°C] | Y [%] | M(+) |
| 163 | 4-amino-methylpyridine (1.5 equivalents) | 1.5 # | 2-[4-(2-cyclohexyl-thiazol-4-yl) phenylamino]-6-[(4-pyridyl)-methylamino] -3-nitropyridine | 1.28(m, 1H), 1.43(m, 2H), 1.51(m, 2H), 1.70(m, 1H), 1.80(m, 2H), 2.10(m, 2H), 3.06(tt, 1H), 4.62(d, 2H), 6.28(d, 1H), 7.29(d, 2H), 7.55(d, 2H), 7.79(d, 2H), 7.90(s, 1H), 8.16(d, 1H), 8.51(d, 2H), 8.90(t, 1H), 10.85 (s, 1H) | 60~ 70 | 60 | 487.2 |

(continued)

| Compounds according to Examples 152 to 165, Solvent S = $CH_3CN$ (all examples) Starting material 2-[4-(2-cyclohexylthiazol-4-yl)phenylamino]-6-chloro-3-nitropyridine compound obtained in Preparation Example 2-3-3 | | | | | | | |
|---|---|---|---|---|---|---|---|
| Ex | Amine | A | Compound | NMR (DMSO -d6) | T [°C] | Y [%] | M(+) |
| 164 | 2-(2-aminoethyl)-pyridine (2 equivalents) | 2 # | thiazol-4-yl)phenyl-amino]-6-[2-(2-pyridyl)ethylamino]-3-nitropyridine | 1.28(m, 1H), 1.43(m, 2H), 3.05(m, 3H), 3.77(m, 2H), 6.14(d, 1H), 7.21(d, 2H), 7.64(t, 1H), 7.90(m, 5H), 8.10(d, 1H), 8.51(t, 1H), 8.56(d, 1H), 10.99 (s, 1H) | 60~ 70 | 88 | 501.2 |

(continued)

| Compounds according to Examples 152 to 165, Solvent S = $CH_3CN$ (all examples) Starting material 2-[4-(2-cyclohexylthiazol-4-yl)phenylamino]-6-chloro-3-nitropyridine compound obtained in Preparation Example 2-3-3 | | | | | | | |
|---|---|---|---|---|---|---|---|
| Ex | Amine | A | Compound | NMR (DMSO -d6) | T [°C] | Y [%] | M(+) |
| 165 | n-butylamine (2 equivalents) | 2 # | 2-[4-(2-cyclohexyl-thiazol-4-yl) phenylamino]-6-(n-butyl-amino)-3-nitro-pyridine | 0.89(t, 3H), 1.33(m, 1H), 1.37(m, 4H), 1.53(m, 4H), 1.69(m, 1H), 1.80(m, 2H) 1.69(m, 1H), 1.80(m, 2H) 2.11(m 2H), 3.03(tt, 1H), 3.39(m, 2H), 6.12(d, 1H), 7.84(d, 2H), 7.91(s, 1H), 7.93(d, 2H), 8.08(d, 1H), 8.39(t, 1H), 11.01 (s, 1H) | 60 ~ 70 | 89 | 452.2 |

**Example 166**

**Preparation of 2-[4-(2-dipropylaminothiazol-4-yl)phenylamino]-6-(methylamino)-3-nitropyridine**

To 10ml of acetonitrile were added 200mg (0.43 mmol) of the 2-[4-(2-dipropylaminothiazol-4-yl)phenylamino]-6-chloro-3-nitropyridine compound obtained in Preparation Example 2-4-3 and 5ml of a 40% (wt/v) methylamine-methanol solution, followed by reaction at room temperature for 5 hours. After the reaction was complete, the solvent was distilled under reduced pressure, followed by stirring in 5ml of methanol for 1 hour at room temperature. The resulting solid was filtered, washed with 5ml of methanol and then dried under vacuum at about 40°C to afford 165mg (yield: 84%) of the desired compound.

Mass (M+): 427.2

$^1$H-NMR(DMSO-$d_6$) (ppm) 0.91(t, 6H), 1.65(m, 4H), 2.95(d, 3H), 3.39(t, 4H), 6.14(d, 1H), 7.08(s, 1H), 7.80(m, 4H), 8.09(d, 1H), 8.35(m, 1H), 11.03(s, 1H).

**Examples 167 to 174**

In the same manner as in Example 166 and using amine compounds described in the following Table 14 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 14 were obtained.

The following Table 14 shows the name of compounds prepared in Examples 167 to 174, the name and equivalents of amine compounds used in the reaction, use/nonuse and equivalents of triethylamine in the reaction, the reaction temperature, the reaction solvent, yield, Mass analysis results and NMR analysis results.

**Table 14**

| Compounds according to Examples 167 to 174, Solvent S = $CH_3CN$ (all examples) Starting material 2-[4-(2-dipropylaminothiazol-4-yl)phenylamino]-6-chloro-3-nitropyridine compound obtained in Preparation Example 2-4-3 | | | | | | | |
|---|---|---|---|---|---|---|---|
| Ex | Amine | A | Compound | NMR (DMSO-d6) | T [°C] | Y [%] | M(+) |
| 167 | Isopropylamine (excess) | X | 2-[4-(2-dipropylyl) phenylamino]-6-yl) phenylamino]-6-(isopropylamino)-3-nitropyridine | 0.93(t, 6H), 1.21(d, 6H), 1.65(m, 4H), 3.40(t, 4H), 4.14(m, 1H), 6.11(d, 1H), 7.09(s, 1H), 7.76(d, 2H), 7.83(d, 2H), 8.08(d, 1H), 8.28(d, 1H), 10.99(s, 1H) | 20~3 0 | 87 | 455.3 |

(continued)

| Compounds according to Examples 167 to 174, Solvent S = $CH_3CN$ (all examples) Starting material 2-[4-(2-dipropylaminothiazol-4-yl)phenylamino]-6-chloro-3-nitropyridine compound obtained in Preparation Example 2-4-3 | | | | | | | |
|---|---|---|---|---|---|---|---|
| Ex | Amine | A | Compound | NMR (DMSO-d6) | T [°C] | Y [%] | M(+) |
| 168 | Isobutylamine (excess) | X | 2-[4-(2-dipropylamino-thiazol-4-yl)phenylamino]-6-(isobutylamino)-3-nitropyridine | 0.90(m, 12H), 1.63(m, 4H), 1.88(m, 1H), 3.08(m, 2H), 3.39(t, 4H), 6.00(d, 1H), 6.99(s, 1H), 7.51(s, 1H), 7.75(m, 4H), 9.49(s, 1H), | 60~ 70 | 83 | 460.2 |
| 169 | 4-hydroxy-piperidine (2 equivalents) | 2 # | 2-[4-(2-dipropylaminothiazol-4-yl) phenylamino]-6-(4-hydroxy-piperidino)-3-nitro-pyridine | 0.91(t, 6H), 1,41(m, 2H), 1.65(m, 4H), 1.80(m, 2H), 3.41 (t+m, 6H), 3.80(m, 1H), 4.03 (brm, 2H), 4.83(d, 1H), 6.55(d, 1H), 7.09(s, 1H), 7.66(d, 2H), 7.83(d, 2H) 8.19 (d, 1H), 10.69(s, 1H) | 20~ 30 | 96 | 497.1 |

(continued)

| Compounds according to Examples 167 to 174, Solvent S = $CH_3CN$ (all examples) Starting material 2-[4-(2-dipropylaminothiazol-4-yl)phenylamino]-6-chloro-3-nitropyridine compound obtained in Preparation Example 2-4-3 | | | | | | | |
|---|---|---|---|---|---|---|---|
| Ex | Amine | A | Compound | NMR (DMSO-d6) | T [°C] | Y [%] | M(+) |
| 170 | 2-(ethylamino)-ethanol (2 equivalents) | 2 # | 2-[4-(2-dipropyl-aminothiazol-4-yl) phenylamino]-6-[(N-ethyl-2-hydroxy-ethyl)amino]-3-nitro-pyridine | 0.91(t, 6H), 1.15(t, 3H), 1.65(m, 4H), 3.41(t, 4H), 3.70(m, 6H), 4.90(m, 1H), 6.42(m, 1H), 7.08(s, 1H), 7.70(m, 2H), 7.82(d, 2H), 8.18(m, 1H), 10.78(s, 1H) | 20~ 30 | 85 | 485.1 |
| 171 | Piperazine (5 equivalents) | X | 2-[4-(2-dipropyl-aminothiazol-4-yl) phenylamino]-6-(piperazin-1-yl)-3-nitropyridine | 0.91(t, 6H), 1.65(m, 4H), 2.48 (brm, 1H), 2.75(m, 4H), 3.40(t, 4H), 3.66 (brm, 4H), 6.51(d, 1H), 7.08(s, 1H), 7.64(d, 2H), 7.81(d, 2H),8.19 (d, 1H), 10.70(s, 1H) | 20~ 30 | 78 | 482.3 |

(continued)

| Compounds according to Examples 167 to 174, Solvent S = $CH_3CN$ (all examples) Starting material 2-[4-(2-dipropylaminothiazol-4-yl)phenylamino]-6-chloro-3-nitropyridine compound obtained in Preparation Example 2-4-3 | | | | | | | |
|---|---|---|---|---|---|---|---|
| Ex | Amine | A | Compound | NMR (DMSO-d6) | T [°C] | Y [%] | M(+) |
| 172 | 1-methyl-piperazine (2 equivalents) | 2 # | 2-[4-(2-dipropylaminothiazol-4-yl) phenylamino]-6-(4-methylpiperazin-1-yl)-3-nitropyridine | 0.91(t, 6H), 1.67(m, 4H), 2.22(s, 3H), 2.38 (brm, 4H), 3.41(t, 4H), 3.72 (brm, 4H), 6.53(d, 1H), 7.08(s, 1H), 7.64(d, 2H), 7.83(d, 2H), 8.21(d, 1H), 10.67(s, 1H) | 20~ 30 | 86 | 496.3 |

(continued)

| Compounds according to Examples 167 to 174, Solvent S = $CH_3CN$ (all examples) Starting material 2-[4-(2-dipropylaminothiazol-4-yl)phenylamino]-6-chloro-3-nitropyridine compound obtained in Preparation Example 2-4-3 | | | | | | | |
|---|---|---|---|---|---|---|---|
| Ex | Amine | A | Compound | NMR (DMSO-d6) | T [°C] | Y [%] | M(+) |
| 173 | 4-amino-piperidine (1.5 equivalents) | 1.5 # | 2-[4-(2-dipropylaminopropylthiazol-4-yl)phenylamino]-6-(4-aminopiperidino)-3-nitropyridine | 0.91(t, 6H), 1.21(m, 2H), 1.65(m, 4H), 1.79(m, 2H), 2.14 (brm, 2H), 2.91(m, 1H), 3.81(t, 2H), 3.41(t, 4H), 6.55(d, 1H), 7.09((s, 1H), 7.67(d, 2H), 7.83(d, 2H), 8.19(d, 1H), 10.70(s, 1H) | 20~ 30 | 97 | 496.3 |

(continued)

| Compounds according to Examples 167 to 174, Solvent S = $CH_3CN$ (all examples) Starting material 2-[4-(2-dipropylaminothiazol-4-yl)phenylamino]-6-chloro-3-nitropyridine compound obtained in Preparation Example 2-4-3 | | | | | | | |
|---|---|---|---|---|---|---|---|
| Ex | Amine | A | Compound | NMR (DMSO-d6) | T [°C] | Y [%] | M(+) |
| 174 | 3-amino-methylpyridine (1.5 equivalents) | 1.5 # | 2-[4-(2-dipropylaminothiazol-4-yl) phenylamino]-6-[(3-pyridyl) methyl-amino]-3-nitro-pyridine | 0.91(t, 6H), 1.65(m, 4H), 3.40(t, 4H), 4.61(d, 2H) 6.23 (d 2H), 7.05(s, 1H), 7.35(dd, 1H), 7.58(d, 2H), 7.66(d, 1H), 7.74(d, 2H), 8.16(d, 1H), 8.47(d, 1H), 8.51(s, 1H), 8.83(t, 1H), 10.86(s, 1H) | 60~ 70 | 83 | 504.3 |

**Example 175**

**Preparation of 2-[(3-fluoro-4-diethylamino)phenylamino]-6-(methylamino)-3-nitropyridine**

To 10ml of acetonitrile were added 250mg (0.74 mmol) of the 2-[(3-fluoro-4-diethylamino)-phenylamino]-6-chloro-3-nitropyridine compound obtained in Preparation Example 3-1-3 and 5ml of a 40% (wt/v) methylamine-methanol solution, followed by reaction at room temperature for 4 hours. After the reaction was complete, the solvent was distilled under reduced pressure, followed by column chromatography purification with a 3:1 (v/v) solution of n-hexane and ethyl acetate as a developing solvent and vacuum drying at about 40°C to afford 174mg (yield: 71%) of the desired compound.

Mass (M+): 334.2

$^1$H-NMR(DMSO-$d_6$) (ppm) 0.92(m, 6H), 2.90(s, 3H), 3.01(m, 4H), 6.03(d, 1H), 6.91(d, 1H), 7.26(d, 1H), 7.78 (d, 1H), 7.98(d, 1H), 8.24(s, 1H), 10.84(s, 1H).

**Examples 176 to 190**

In the same manner as in Example 175 and using amine compounds described in the following Table 15 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 15 were obtained. The following Table 15 shows the name of compounds prepared in Examples 176 to 190, the name and equivalents of amine compounds used in the reaction, use/nonuse and equivalents of triethylamine in the reaction, the reaction temperature, the reaction solvent, yield, Mass analysis results and NMR analysis results.

**Table 15**

| Compounds according to Examples 176 to 190, Solvent S = $CH_3CN$ (all examples) Starting material 2-[(3-fluoro-4-diethylamino)phenylamino}-6-chloro-3-nitropyridine compound obtained in Preparation Example 3-1-3 | | | | | | | |
|---|---|---|---|---|---|---|---|
| Ex | Amine | A | Compound | NMR (DMSO -d6) | T [°C] | Y [%] | M(+) |
| 176 | Isopropylamine (excess) | X | 2-[(3-fluoro-4-diethyl-amino)-phenyl-amino]-6-(isopropylamino)-3-nitropyridine | 0.91(m, 6H), 1.10(d, 6H), 3.04(m, 4H), 4.03(m, 1H), 6.02(d, 1H), 6.90(d, 1H), 7.17(d, 1H), 7.85(s, 1H), 7.98(d, 1H), 8.14(s, 1H), 10.78 (s, 1H) | 20~ 30 | 97 | 362.2 |
| 177 | Isobutylamine (excess) | X | 2-[(3-fluoro-4-diethylamino) phenyl amino]-6-(isobutylamino)-3-nitro-pyridine | 0.87(m, 8H), 0.98(d, 6H), 1.87(m, 1H), 3.11(m, 4H), 6.11(d, 1H), 6.97(d, 1H), 7.20(d, 1H), 7.80(d, 1H) 8.05(d, 1H), 8.41(s, 1H), 10.80 (s, 1H) | 20~ 30 | 94 | 376.2 |

(continued)

| Compounds according to Examples 176 to 190, Solvent S = $CH_3CN$ (all examples) Starting material 2-[(3-fluoro-4-diethylamino)phenylamino}-6-chloro-3-nitropyridine compound obtained in Preparation Example 3-1-3 | | | | | | | |
|---|---|---|---|---|---|---|---|
| Ex | Amine | A | Compound | NMR (DMSO -d6) | T [°C] | Y [%] | M(+) |
| 178 | t-butylamine (excess) | X | 2-[(3-fluoro-4-diethylamino) phenyl amino]-6-(t-butyl-amino)-3-nitro-pyridine | 0.95(m, 6H), 1.20(s, 9H), 3.09(m, 4H), 6.07(d, 1H), 6.97(d, 1H), 7.05(d, 1H), 7.35(d, 1H), 7.77(s, 1H), 7.97(d, 1H), 10.50 (s, 1H) | 20~ 30 | 89 | 376.2 |
| 179 | 4-hydroxypiperidine (1.5 equivalents) | 1.5 # | 2-[(3-fluoro-4-diethylamino) phenyl amino]-6-(4-hydroxy-piperidino)-3-nitropyridine | 0.98(m, 6H), 1.38(m, 2H), 1.78(m, 2H), 3.11(m, 4H), 3.33(m, 2H), 3.78(m, 1H), 4.00 (brm, 2H), 4.80(d, 1H), 6.49(d, 1H), 6.38(d, 1H), 7.20(d, 1H), 7.55(d, 1H), 8.13(d, 1H), 10.53 (s, 1H) | 20~ 30 | 59 | 404.2 |

(continued)

| Compounds according to Examples 176 to 190, Solvent S = $CH_3CN$ (all examples) Starting material 2-[(3-fluoro-4-diethylamino)phenylamino}-6-chloro-3-nitropyridine compound obtained in Preparation Example 3-1-3 | | | | | | | |
|---|---|---|---|---|---|---|---|
| Ex | Amine | A | Compound | NMR (DMSO -d6) | T [°C] | Y [%] | M(+) |
| 180 | 2-isopropylimidazole (5 equivalents) | 5 # | 2-[(3-fluoro-4-diethylamino) phenyl amino]-6-[(2-isopropyl)imidazol-1-yl]-3-nitropyridine | 0.93(m, 6H), 1.03(m, 6H), 3.16(, m, 4H), 3.34(m, 1H), 6.91(d, 1H), 7.01(d, 1H), 7.06(d, 1H), 7.11(d, 1H), 7.26(d, 1H), 7.62(d, 1H), 8.64(d, 1H), 10.05 (s, 1H) | 60~ 70 | 85 | 413.2 |
| 181 | 2-methyl-2-imidazoline (2 equivalents) | 2 # | 2-[(3-fluoro-4-diethylamino) phenyl amino]-6-[(2-methyl-4,5-dihydro)imidazol-1-yl]-3-nitropyridine | 0.98(m, 6H), 1.79(s, 3H), 3.12(m, 4H), 3.25(m, 2H), 3.34(m, 2H), 6.10(d, 1H), 7.01(d, 1H), 7.95(m, 1H), 8.05(d, 1H), 8.34(m, 1H), 10.83 (s, 1H) | 60~ 70 | 76 | 387.2 |

(continued)

| Compounds according to Examples 176 to 190, Solvent S = $CH_3CN$ (all examples) Starting material 2-[(3-fluoro-4-diethylamino)phenylamino}-6-chloro-3-nitropyridine compound obtained in Preparation Example 3-1-3 | | | | | | | |
|---|---|---|---|---|---|---|---|
| Ex | Amine | A | Compound | NMR (DMSO -d6) | T [°C] | Y [%] | M(+) |
| 182 | Piperazine (5 equivalents) | X | 2-[(3-fluoro-4-diethylamino) phenyl amino]-6-(piperazin-1-yl)-3-nitropyridine | 0.99(m, 6H), 2.74(m, 4H), 3.12(m, 4H), 3.63(m, 4H), 3.87(s, 1H), 6.48(d, 1H), 6.98(d, 1H), 7.24(d, 1H), 7.52(d, 1H), 8.17(d, 1H), 10.56 (s, 1H), | 20~ 30 | 46 | 389.2 |
| 183 | Amine 1-methyl-piperazine (3 equivalents) | X | 2-[(3-fluoro-4-diethylamino) phenyl amino]-6-(4-methylpiperazin-1-yl)-3-nitropyridine | 0.99(m, 6H), 2.19(m, 4H), 2.35(s, 3H), 3.12(m, 4H), 3.68(m, 4H), 6.40(d, 1H), 6.96(d, 1H), 7.22(d, 1H) 7.53(d, 1H), 8.15(d, 1H), 10,52 (s, 1H) | 20~ 30 | 85 | 403.2 |

(continued)

| Compounds according to Examples 176 to 190, Solvent S = $CH_3CN$ (all examples) Starting material 2-[(3-fluoro-4-diethylamino)phenylamino}-6-chloro-3-nitropyridine compound obtained in Preparation Example 3-1-3 | | | | | | | |
|---|---|---|---|---|---|---|---|
| Ex | Amine | A | Compound | NMR (DMSO -d6) | T [°C] | Y [%] | M(+) |
| 184 | Morpholine (3 equivalents) | X | 2-[(3-fluoro-4-diethylamino) phenyl amino]-6-morpholino-3-nitropyridine | 0.99(m, 6H), 3.12(m, 4H), 3.07 (brm, 8H), 6.48(d, 1H), 6.97(d, 1H), 7.28(d, 1H), 7.52(d, 1H), 8.20(d, 1H), 10.53 (s, 1H) | 20~ 30 | 51 | 390.2 |
| 185 | 3-aminomethylpyridine (1.5 equivalents) | 1.5 # | 2-[(3-fluoro-4-diethylamino) phenyl amino]-6-[(3-pyridyl)-methylamino]-3-nitropyridine | 0.98(m, 6H), 3.10(m, 4H), 4.56(d, 2H), 6.18(d, 1H), 6.98(t, 1H), 7.17(m, 1H), 7.30(m, 2H), 7.60(m, 1H), 8.10(d, 1H), 8.44(m, 2H), 8.80(m, 1H), 10.71 (s, 1H) | 60~ 70 | 84 | 411.2 |

(continued)

| Compounds according to Examples 176 to 190, Solvent S = $CH_3CN$ (all examples) Starting material 2-[(3-fluoro-4-diethylamino)phenylamino}-6-chloro-3-nitropyridine compound obtained in Preparation Example 3-1-3 | | | | | | | |
|---|---|---|---|---|---|---|---|
| Ex | Amine | A | Compound | NMR (DMSO -d6) | T [°C] | Y [%] | M(+) |
| 186 | 4-amino-methylpyridine (1.5 equivalents) | 1.5 # | 2-[(3-fluoro-4-diethylamino)-phenylamino] -6-[(4-pyridyl)methylamino] -3-nitropyridine | 0.97(m, 6H), 3.10(m, 4H), 4.56(d, 2H), 6.23(d, 1H), 6.82(t, 1H), 7.09(d, 1H), 7.21(m, 2H), 7.40(d, 1H), 8.14(d, 1H), 8.47(m, 2H), 8.85(m, 1H), 10.66 (s, 1H) | 60~ 70 | 32 | 411.2 |
| 187 | 4-amino-piperidine (2 equivalents) | 2 # | 2-[(3-fluoro-4-diethylamino) phenyl amino]-6-(4-aminopiperidino)-3-nitropyridine | 1.00(m, 6H), 1.47(m, 2H), 1.95(m, 1H), 2.64(m, 1H), 3.15(m, 8H), 4.35 (brm, 2H), 6.53(d, 1H), 7.00(t, 1H), 7.23(d, 1H), 7.54(d, 1H), 8.20(d, 1H), 10.51 (s, 1H) | 20~ 30 | 98 | 403.3 |

(continued)

| Compounds according to Examples 176 to 190, Solvent S = $CH_3CN$ (all examples) Starting material 2-[(3-fluoro-4-diethylamino)phenylamino}-6-chloro-3-nitropyridine compound obtained in Preparation Example 3-1-3 | | | | | | | |
|---|---|---|---|---|---|---|---|
| Ex | Amine | A | Compound | NMR (DMSO -d6) | T [°C] | Y [%] | M(+) |
| 188 | 4-(2-aminoethyl)-morpholine (1.5 equivalents) | 1.5 # | 2-[(3-fluoro-4-diethylamino) phenyl amino]-6-[2-(morpholin-l-yl) ethylamino]-3-nitropyridine | 0.98(m, 6H), 2.33(m, 4H), 2.43(m, 2H), 3.01(m, 4H), 3.51(m, 2H), 3.53(m, 4H), 6.10(m, 1H), 6.93(t, 1H), 7.24(m, 1H), 7.64(d, 1H), 8.03(d, 1H), 8.28(d, 1H), 10.78 (s, 1H) | 60~ 70 | 74 | 433.3 |

(continued)

| Compounds according to Examples 176 to 190, Solvent S = $CH_3CN$ (all examples) Starting material 2-[(3-fluoro-4-diethylamino)phenylamino}-6-chloro-3-nitropyridine compound obtained in Preparation Example 3-1-3 | | | | | | | |
|---|---|---|---|---|---|---|---|
| Ex | Amine | A | Compound | NMR (DMSO -d6) | T [°C] | Y [%] | M(+) |
| 189 | 1-(3-aminopropyl)-imidazole (1.5 equivalents) | 1.5 # | 2-[(3-fluoro-4-diethylamino) phenyl amino]-6-[(3-imidazol-1-yl)propylamino]-3-nitropyridine | 0.99(m, 6H), 1.99(m, 2H), 3.11(m, 4H), 3.29(m 2H), 4.01(m, 2H), 6.10(d, 1H), 6.87(d, 1H), 6.97(t, 1H), 7.14(d, 1H), 7.30(m, 1H), 7.60(d, 1H), 7.70(m, 1H), 8.06(m, 1H), 8.37(m, 1H), 10.82 (s, 1H) | 60~ 70 | 96 | 428.3 |

(continued)

| Compounds according to Examples 176 to 190, Solvent S = $CH_3CN$ (all examples) Starting material 2-[(3-fluoro-4-diethylamino)phenylamino}-6-chloro-3-nitropyridine compound obtained in Preparation Example 3-1-3 | | | | | | | |
|---|---|---|---|---|---|---|---|
| Ex | Amine | A | Compound | NMR (DMSO -d6) | T [°C] | Y [%] | M(+) |
| 190 | 4-(3-aminopropyl)-morpholine (1.5 equivalents) | 1.5 # | 2-[(3-fluoro-4-diethylamino) phenyl amino]-6-[(3-morpholin-1-yl) propylamino]-3-nitropyridine | 0.98(m, 6H), 1.68(m, 2H), 2.28 (brm, 6H), 3.11(m, 4H), 3.35(m, 2H), 3.53(m, 4H), 6.08(d, 1H), 6.95(t, 1H), 7.24(m, 1H) 7.73(d, 1H), 8.04(d, 1H), 8.35(m, 1H), 10.85 (s, 1H) | 60~ 70 | 93 | 447.3 |

**Example 191**

**Preparation of 2-[(3-fluoro-4-morpholino)phenylamino-6-(methylamino)-3-nitropyridine**

To 10ml of acetonitrile were added 200mg (0.57 mmol) of the 2-[(3-fluoro-4-morpholino)-phenylamino]-6-chloro-3-nitropyridine compound obtained in Preparation Example 3-2-3 and 10ml of a 40% (wt/v) methylamine-methanol solution, followed by reaction at room temperature for 4 hours. After the reaction was complete, the solvent was distilled under reduced pressure, followed by stirring in 10ml of methanol for 1 hour at room temperature. The resulting solid was filtered, washed with 10ml of methanol and then dried under vacuum at about 40°C to afford 181mg (yield: 92%) of the desired compound.

Mass (M+): 348.1

$^1$H-NMR(DMSO-$d_6$) (ppm) 2.91(d, 3H), 2.98(t, 4H), 3.74(t, 4H), 6.12(d, 1H), 7.02(t, 1H), 7.44(d, 1H), 7.88(d, 1H), 8.07(d, 1H), 8.34(m, 1H), 10.91(s, 1H).

**Examples 192 to 202**

In the same manner as in Example 191 and using amine compounds described in the following Table 16 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 16 were obtained.

The following Table 16 shows the name of compounds prepared in Examples 192 to 202, the name and equivalents of amine compounds used in the reaction, use/nonuse and equivalents of triethylamine in the reaction, the reaction temperature, the reaction solvent, yield, Mass analysis results and NMR analysis results.

**Table 16**

| Compounds according to Examples 192 to 202, Solvent S = $CH_3CN$ (all examples) Starting material 2-[(3-fluoro-4-morpholino)phenylamino]-6-chloro-3-nitropyridine compound obtained in Preparation Example 3-2-3 | | | | | | | |
|---|---|---|---|---|---|---|---|
| Ex | Amine | A | Compound | NMR (DMSO-d6) | T [°C] | Y [%] | M(+) |
| 192 | Isopropylamine (excess) | X | 2-[(3-fluoro-4-morpholino) phenyl-amino]-6-(isopropylamino)-3-nitro-pyridine | 1,20(d, 6H), 2.98(t, 4H), 3.74(t, 4H), 4.08(m, 1H), 6.09(d, 1H), 7.01(t, 1H), 7.35(d, 1H), 7.84(d, 1H), 8.06(d, 1H), 8.24(d, 1H), 10.87(s, 1H) | 20~ 30 | 63 | 376.1 |
| 193 | Isobutylamine (excess) | X | 2-[(3-fluoro-4-morpholino) phenyl-amino]-6-(isobutyl-amino)-3-nitro-pyridine | 0.90(d, 6H), 1.87(m, 1H), 2.98(t, 4H), 3.17(t, 2H), 3.74(t, 4H), 6.14(d, 1H), 7.00(t, 1H), 7.28(d, 1H), 7.89(d, 1H), 8.07(d, 1H), 8.46(t, 1H), 10.86(s, 1H) | 20~ 30 | 63 | 390.2 |

(continued)

| Compounds according to Examples 192 to 202, Solvent S = $CH_3CN$ (all examples) Starting material 2-[(3-fluoro-4-morpholino)phenylamino]-6-chloro-3-nitropyridine compound obtained in Preparation Example 3-2-3 | | | | | | | |
|---|---|---|---|---|---|---|---|
| Ex | Amine | A | Compound | NMR (DMSO-d6) | T [°C] | Y [%] | M(+) |
| 194 | 4-hydroxy-piperidine (1.5 equivalents) | 1.5 # | 2-[(3-fluoro-4-morpholino) phenylamino]-6-(4-hydroxy-piperidino)-3-nitro-pyridine | 1.40(m, 2H), 1.83(m, 2H), 2.99 (brm, 4H), 3.43(t, 2H), 3.74(t, 2H), 4.04(m, 1H), 4.82(d, 1H), 6.54(d, 1H), 7.03(t, 1H), 7.31(d, 1H), 7.62(d, 1H), 8.17(d, 1H), 10.56 (S, 1H) | 20~ 30 | 67 | 418.1 |
| 195 | 2-methyl-2-imidazoline (2 equivalents) | 2 # | 2-[(3-fluoro-4-morpholino)-phenylamino]-6-[(2-methyl-4,5-dihydro)-imidazol-1-yl]-3-nitropyridine | 1.99(s, 3H), 2.99(t, 4H), 3.70(m, 2H), 3.74(t, 4H), 3.88(t, 2H), 6.41(d, 1H), 7.05(t, 1H), 7.19(d, 1H), 7.35(d, 1H), 8.37(d, 1H), 10.17(s, 1H) | 60~ 70 | 55 | 401.1 |

(continued)

| Compounds according to Examples 192 to 202, Solvent S = $CH_3CN$ (all examples) Starting material 2-[(3-fluoro-4-morpholino)phenylamino]-6-chloro-3-nitropyridine compound obtained in Preparation Example 3-2-3 | | | | | | | |
|---|---|---|---|---|---|---|---|
| Ex | Amine | A | Compound | NMR (DMSO-d6) | T [°C] | Y [%] | M(+) |
| 196 | 2-isopropyl-imidazole (5 equivalents) | 5 # | 2-[(3-fluoro-4-morpholino) phenyl-amino]-6-[(2-isopropyl)imidazol-1-yl]-3-nitropyridine | 0.93(d, 6H), 3.01(t, 4H), 3.38(m, 1H), 3.75(t, 4H), 6.92(s, 1H), 7.07(m, 2H), 7.18(d, 1H), 7.33(d, 1H), 7.62(s, 1H), 8.65(d, 1H), 10.08(s, 1H) | 60~ 70 | 49 | 427.1 |
| 197 | 3-aminomethylpyridine (1.5 equivalents) | 1.5 # | 2-[(3-fluoro-4-morpholino) phenyl-amino]-6-[(3-pyridyl-)methylamino]-3-nitropyridine | 2.96(t, 4H), 3.73(t, 4H), 4.58(d, 2H), 6.21(d, 1H), 6.94(t, 1H), 7.23(d, 1H), 7.33(m, 1H), 7.60(m, 2H), 8.13(d, 1H), 8.46(s, 2H), 8.83(t, 1H), 10.71(s, 1H) | 60~ 70 | 76 | 425.1 |

(continued)

| Compounds according to Examples 192 to 202, Solvent S = $CH_3CN$ (all examples) Starting material 2-[(3-fluoro-4-morpholino)phenylamino]-6-chloro-3-nitropyridine compound obtained in Preparation Example 3-2-3 | | | | | | | |
|---|---|---|---|---|---|---|---|
| Ex | Amine | A | Compound | NMR (DMSO-d6) | T [°C] | Y [%] | M(+) |
| 198 | 4-amino-methylpyridine (1.5 equivalents) | 1.5 # | 2-[(3-fluoro-4-morpholino) phenyl-amino]-6-[(4-pyridyl)-methylamino]-3-nitropyridine | 2.95 (brm, 4H), 3.73 (brm, 4H), 4.58(d, 2H), 6.26(d, 1H), 6.86(t, 1H), 7.13(d, 1H), 7.22(d, 2H), 7.44(d, 1H), 8.16(d, 1H), 8.48(d, 2H), 8.88(t, 1H), 10.68(s, 1H) | 60~ 70 | 79 | 425.1 |
| 199 | t-butylamine (excess) | X | 2-[(3-fluoro-4-morpholino) phenyl-amino]-6-(t-butyl-amino)-3-nitro-pyridine | 1.27(s, 9H), 2.98(t, 4H), 3.73(t, 4H), 6.13(d, 1H), 7.01(t, 1H), 7.18(d, 1H), 7.52(d, 1H), 7.85(s, 1H), 8.01(d, 1H), 10.63(s, 1H) | 20~ 30 | 45 | 390.2 |

(continued)

| Compounds according to Examples 192 to 202, Solvent S = $CH_3CN$ (all examples) Starting material 2-[(3-fluoro-4-morpholino)phenylamino]-6-chloro-3-nitropyridine compound obtained in Preparation Example 3-2-3 | | | | | | | |
|---|---|---|---|---|---|---|---|
| Ex | Amine | A | Compound | NMR (DMSO-d6) | T [°C] | Y [%] | M(+) |
| 200 | 1-methyl-piperazine (3 equivalents) | X | 2-[(3-fluoro-4-morpholino) phenyl-amino]-6-(4-methyl-piperazin-l-yl)-3-nitropyridine | 2.20(s, 3H), 2.38 (brm, 4H), 2.98 (brm, 4H), 3.73 (brm, 8H), 6.52(d, 1H), 7.02(t, 1H), 7.32(d, 1H), 7.60(d, 1H), 8.08(d, 1H), 10.53(s, 1H) | 20~ 30 | 72 | 417.1 |
| 201 | Piperazine (5 equivalents) | X | 2-[(3-fluoro-4-morpholino) phenyl-amino]-6-(piperazin-1-yl)-3-nitropyridine | 2.75 (brm, 4H), 2.98 (brm, 4H), 3.65 (brm, 4H), 3.75 (brm, 4H), 6.49(d, 1H), 7.02(t 1H), 7.32(d, 2H), 7.60(dd, 1H), 8.17(d, 1H), 10.57(s, 1H) | 20~ 30 | 55 | 403.2 |

(continued)

| Compounds according to Examples 192 to 202, Solvent S = $CH_3CN$ (all examples) Starting material 2-[(3-fluoro-4-morpholino)phenylamino]-6-chloro-3-nitropyridine compound obtained in Preparation Example 3-2-3 | | | | | | | |
|---|---|---|---|---|---|---|---|
| Ex | Amine | A | Compound | NMR (DMSO-d6) | T [°C] | Y [%] | M(+) |
| 202 | 4-amino-piperidine (2 equivalents) | 2 # | 2-[(3-fluoro-4-morpholino) phenyl-amino]-6-(4-amino-piperidino)-3-nitro-pyridine | 1.25(m, 2H), 1.83(m, 2H), 2.99(m, 5H), 3.17(t, 2H), 3.74 (brm, 4H), 4.31 (brm, 2H), 6.54(d, 1H), 7.03(t, 1H), 7.31(d, 1H), 7.63(d, 1H), 8.18(d, 1H), 10.56(s, 1H) | 20~ 30 | 55 | 417.2 |

**Example 203**

**Preparation of 2-[(3-fluoro-4-thiomorpholino)phenylamino]-6-(methylamino)-3-nitropyridine**

To 10ml of acetonitrile were added 200mg (0.54 mmol) of the 2-[(3-fluoro-4-thio-morpholino)phenylamino]-6-chloro-3-nitropyridine compound obtained in Preparation Example 3-3-3 and 10ml of a 40% (wt/v) methylamine-methanol solution, followed by reaction at room temperature for 4 hours. After the reaction was complete, the solvent was distilled under reduced pressure, followed by stirring in 10ml of acetonitrile for 1 hour at room temperature. The resulting solid was filtered, washed with 10ml of acetonitrile and then dried under vacuum at about 40°C to afford 108mg (yield: 55%) of the desired compound.

Mass (M+): 364.1

$^1$H-NMR(DMSO-$d_6$) (ppm) 2.73(t, 4H), 2.91(s, 3H), 3.23(t, 4H), 6.12(d, 1H), 7.08(t, 1H), 7.43(d, 1H), 7.88(d, 1H), 8.07(d, 1H), 8.35(m, 1H), 10.90(s, 1H).

**Examples 204 to 214**

In the same manner as in Example 203 and using amine compounds described in the following Table 17 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 17 were obtained.

The following Table 17 shows the name of compounds prepared in Examples 204 to 214, the name and

equivalents of amine compounds used in the reaction, use/nonuse and equivalents of triethylamine in the reaction, the reaction temperature, the reaction solvent, yield, Mass analysis results and NMR analysis results.

**Table 17**

| Compounds according to Examples 204 to 214, Solvent S = $CH_3CN$ (all examples) Starting material 2-[(3-fluoro-4-thiomorpholino)phenylamino]-6-chloro-3-nitropyridine compound obtained in Preparation Example 3-3-3 | | | | | | | |
|---|---|---|---|---|---|---|---|
| Ex | Amine | A | Compound | NMR (DMSO -d6) | T [°C] | Y [%] | M(+) |
| 204 | Isopropylamine (excess) | X | 2-[(3-fluoro-4-thiomorpholino) phenylamino]-6-(iso-ylamino]-6-(isopropylamino)-3-nitropyridine | 1.20(d, 6H), 2.76 (brm, 4H), 3.22 (brm, 4H), 4.10(m, 1H), 6.10(d, 1H), 7.04(t, 1H), 7.34(d, 1H), 7.83(m, 2H), 8.06(d, 1H), 8.31(d, 1H), 10.87 (s, 1H) | 20~ 30 | 67 | 392.1 |

(continued)

| Compounds according to Examples 204 to 214, Solvent S = $CH_3CN$ (all examples) Starting material 2-[(3-fluoro-4-thiomorpholino)phenylamino]-6-chloro-3-nitropyridine compound obtained in Preparation Example 3-3-3 | | | | | | | |
|---|---|---|---|---|---|---|---|
| Ex | Amine | A | Compound | NMR (DMSO -d6) | T [°C] | Y [%] | M(+) |
| 205 | Isobutylamine (excess) | X | 2-[(3-fluoro-4-thiomorpholino) phenyla mino]-6-(isobutylamino)-3-nitropyridine | 0.90(d, 6H), 1.87(m, 1H), 2,76 (brm, 4H), 3.18 (brm, 4H), 3.21(m, 1H)m 6.14(d, 1H), 7.04(t, 1H), 7.25(d, 1H), 7.89 (dd, 1H), 8.06(d, 1H), 8.47(t, 1H), 10.86 (s, 1H) | 20~ 30 | 54 | 406.1 |

(continued)

| Compounds according to Examples 204 to 214, Solvent S = $CH_3CN$ (all examples) Starting material 2-[(3-fluoro-4-thiomorpholino)phenylamino]-6-chloro-3-nitropyridine compound obtained in Preparation Example 3-3-3 | | | | | | | |
|---|---|---|---|---|---|---|---|
| Ex | Amine | A | Compound | NMR (DMSO -d6) | T [°C] | Y [%] | M(+) |
| 206 | 4-hydroxy-piperidine (1.5 equivalents) | 1.5 # | 2-[(3-fluoro-4-thiomorpholino) phenyla mino]-6-(4-hydroxypiperidirio)-3-nitropyridine | 1.40(m, 2H), 1.83(m, 2H), 2.75(t, 4H), 3.22(t, 4H), 3.40(m, 2H), 3.81(m, 1H), 4.03 (brm, 2H), 4.83(s, 1H), 6.54(d, 1H), 7.05(t, 1H), 7.29(d, 1H), 7.62(d, 1H), 8.17(d, 1H), 10.56 (s, 1H) | 20~ 30 | 43 | 434.1 |

(continued)

| Compounds according to Examples 204 to 214, Solvent S = $CH_3CN$ (all examples) Starting material 2-[(3-fluoro-4-thiomorpholino)phenylamino]-6-chloro-3-nitropyridine compound obtained in Preparation Example 3-3-3 | | | | | | | |
|---|---|---|---|---|---|---|---|
| Ex | Amine | A | Compound | NMR (DMSO -d6) | T [°C] | Y [%] | M(+) |
| 207 | 2-methyl-2-imidazoline | X | 2-[(3-fluoro-4-thiomorpholino) phenyla mino]-6-[(2-methyl-4,5-dihydro)imidazol-1-yl]-3-nitropyridine | 2.00(s, 3H), 2.77(t, 4H), 3.22(t, 4H), 3.71(t, 2H), 3.85(t, 2H), 6.41(d, 1H), 7.09(t, 1H), 7.18(d, 1H), 7.34(d, 1H), 8.37(d, 1H), 10.16 (s, 1H) | 60~ 70 | 60 | 417.1 |
| 208 | 2-isopropyl-imidazole (5 equivalents | 5 # | 2-[(3-fluoro-4-thiomorpholino) phenyla mino]-6-[(2-isopropyl)-imidazol-1-yl]-3-nitropyridine | 0.93(d, 6H), 2.76(t, 4H), 3.25(t, 4H), 3.42(m, 1H), 6.92(s, 1H), 7.09(m, 2H), 7.14(d, 1H), 7.29(d, 1H), 7.62(s, 1H), 8.65(d, 1H), 10.08 (s, 1H) | 60~ 70 | 57 | 443.1 |

(continued)

| Compounds according to Examples 204 to 214, Solvent S = $CH_3CN$ (all examples) Starting material 2-[(3-fluoro-4-thiomorpholino)phenylamino]-6-chloro-3-nitropyridine compound obtained in Preparation Example 3-3-3 | | | | | | | |
|---|---|---|---|---|---|---|---|
| Ex | Amine | A | Compound | NMR (DMSO -d6) | T [°C] | Y [%] | M(+) |
| 209 | 3-amino-methylpyridine (1.5 equivalents) | 1.5 # | 2-[(3-fluoro-4-thio-morpholino)phenyl-amino]-6-[(3-pyridyl)-methylamino]-3-nitropyridine | 2.75 (brm, 4H), 3.20 (brm, 4H), 4.58(d, 2H), 6.21(d, 1H), 6.98(t, 1H), 7.12(d, 1H), 7.34(d, 1H), 7.59(m, 2H), 8.13(d, 1H), 8.46(s, 1H), 8.81(t, 1H), 10.71 (s, 1H) | 60~ 70 | 66 | 441.1 |

(continued)

| Compounds according to Examples 204 to 214, Solvent S = $CH_3CN$ (all examples) Starting material 2-[(3-fluoro-4-thiomorpholino)phenylamino]-6-chloro-3-nitropyridine compound obtained in Preparation Example 3-3-3 | | | | | | | |
|---|---|---|---|---|---|---|---|
| Ex | Amine | A | Compound | NMR (DMSO -d6) | T [°C] | Y [%] | M(+) |
| 210 | 4-amino-methylpyridine (1.5 equivalents) | 1.5 # | 2-[(3-fluoro-4-thio-morpholino)phenyla mino]-6-[(4-pyridyl)-methylamino]-3-nitropyridine | 2.74 (brm, 4H), 3.19 (brm, 4H), 4.58(d, 2H), 6.25(d, 1H), 6.90(t, 1H), 7.14(d, 1H), 7.22(d, 2H), 7.45(d, 1H), 8.16(d, 1H), 8.49(d, 2H), 8.87(t, 1H), 10.68 (s, 1H) | 60~ 70 | 73 | 441.1 |
| 211 | t-butylamine (excess) | X | 2-[(3-fluoro-4-thiomorpholino)-phenylamino] -6-(t-butylamino)-3-nitropyridine | 1.27(s, 9H), 2.75(t, 4H), 3.22(t, 4H), 6.13(d, 1H), 7.04(t, 1H), 7.15(d, 1H), 7.52(d, 1H), 7.85(s, 1H), 8.01(d, 1H), 10.63 (s, 1H) | 20~ 30 | 62 | 406.1 |

(continued)

| Compounds according to Examples 204 to 214, Solvent S = $CH_3CN$ (all examples) Starting material 2-[(3-fluoro-4-thiomorpholino)phenylamino]-6-chloro-3-nitropyridine compound obtained in Preparation Example 3-3-3 | | | | | | | |
|---|---|---|---|---|---|---|---|
| Ex | Amine | A | Compound | NMR (DMSO -d6) | T [°C] | Y [%] | M(+) |
| 212 | 1-methyl-piperazine (3 equivalents) | X | 2-[(3-fluoro-4-thiomorpholino) phenylamino]-6-(4-methyl-piperazin-1-yl)-3-nitropyridine | 2.20(s, 3H), 2.38 (brm, 4H), 3.75 (brm, 4H), 3.22 (brm, 4H), 3.70 (brm, 4H), 6.52(d, 1H), 7.05(t, 1H), 7,31(d, 1H), 7.58(d, 1H), 8.19(d, 1H), 10.53 (s, 1H) | 20~ 30 | 53 | 433.1 |
| 213 | Piperazine (5 equivalents) | X | 2-[(3-fluoro-4-thio-morpholino)phenyl-amino]-6-(piperazin-1-yl)-3-nitropyridine | 2.75 (brm, 8H), 3.22 (brm, 4H), 3.64 (brm, 4H), 6.50(d, 1H), 7.06(t, 1H), 7.32(d, 1H), 7.60 (dd, 1H), 8.17(d, 1H), 10.57 (s, 1H) | 20~ 30 | 70 | 419.2 |

(continued)

| Compounds according to Examples 204 to 214, Solvent S = $CH_3CN$ (all examples) Starting material 2-[(3-fluoro-4-thiomorpholino)phenylamino]-6-chloro-3-nitropyridine compound obtained in Preparation Example 3-3-3 | | | | | | | |
|---|---|---|---|---|---|---|---|
| Ex | Amine | A | Compound | NMR (DMSO -d6) | T [°C] | Y [%] | M(+) |
| 214 | 4-amino-piperidine (1.5 equivalents) | 1,5 # | 2-[(3-fluoro-4-thiomorpholino) phenyl-amino]-6-(4-amino-piperidino)-3-nitro-pyridine | 1.19(m, 2H), 1.58(m, 2H), 1.77(m, 2H), 2.75(m, 4H), 2.91(m, 1H), 3.22(m, 6H), 4.26 (brm, 2H), 6.53(d, 1H), 7.06(t, 1H), 7.30(d, 1H), 7.63(d, 1H), 8.16(d, 1H), 10.57 (s, 1H) | 20~ 30 | 63 | 433.2 |

**Example 215**

**Preparation of 2-[(3-fluoro-4-piperazino)phenylamino]-6-(methylamino)-3-nitropyridine**

To 10ml of acetonitrile were added 500mg (1.1 mmol) of the 2-[3-fluoro-4-(BOC-piperazino)phenylamino]-6-chloro-3-nitropyridine compound obtained in Preparation Example 3-4-3 and 10ml of a 40% (wt/v) methylamine-methanol solution, followed by reaction at room temperature for 4 hours. After the reaction was complete, the solvent was distilled under reduced pressure, followed by column chromatography purification with a 3:1 (v/v) solution of n-hexane and ethyl acetate as a developing solvent, recrystallization from ethyl acetate and hexane, and vacuum drying at about 40°C to afford 214mg (yield: 44%) of the desired compound.

Mass (M+): 447.2

$^1$H-NMR(DMSO-$d_6$) (ppm): 1.42(s, 9H), 2.91(m, 7H), 3.47(m, 4H), 6.11(d, 1H), 7.04(d, 2H), 7.41(t, 1H), 7.88 (d, 1H), 8.06(d, 1H), 8.34(d, 1H), 10.90(s, 1H).

180mg (0.4mmol) of the above-obtained 2-[(3-fluoro-4-BOC-piperazino)phenylamino]-6-(methylamino)-3-nitropyridine was dissolved in 10ml of dichloromethane and 0.3ml (4mmol) of trifluoroacetic acid was added thereto, followed by reaction at room temperature for 5 hours. After the reaction was complete, the solvent was distilled under reduced pressure. The resulting residue was dissolved in 10ml of methanol and pH thereof was adjusted to a value of 7 to 8 by dropwise addition of a sodium bicarbonate solution at a temperature of 0 to 5°C, followed by stirring for about 1 hour. The resulting solid was filtered, washed with a 1:1 (v/v) solution of water and methanol, and then dried under vacuum at about 40°C to afford 59mg (yield: 43%) of the desired compound.

Mass: 347.0

$^1$H-NMR(DMSO-$d_6$) (ppm) 2.90(s, 3H), 3.22(m, 8H), 6.16(d, 1H), 7.08(t, 1H), 7.46(d, 1H), 7.92(d, 1H), 8.06(d,

1H), 8.49(brm, 1H), 9.37(brm, 2H), 10.90(s, 1H).

**Examples 216 to 222**

In the same manner as in Example 215 and using amine compounds described in the following Table 18 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 18 were obtained.

The following Table 18 shows the name of compounds prepared in Examples 216 to 222, the name and equivalents of amine compounds used in the reaction, use/nonuse and equivalents of triethylamine in the reaction, the reaction temperature, the reaction solvent, yield, Mass analysis results and NMR analysis results.

**Table 18**

| Compounds according to Examples 216 to 222, Solvent S = $CH_3CN$ (all examples) Starting material 2-[3-fluoro-4-(BOC-piperazino)phenylamino]-6-chloro-3-nitropyridine compound obtained in Preparation Example 3-4-3 | | | | | | | |
|---|---|---|---|---|---|---|---|
| Ex | Amine | A | Compound | NMR (DMSO-d6) | T [°C] | Y [%] | M(+) |
| 216 | Isopropylamine hydrochloride (excess) | X | 2-[(3-fluoro-4-piperazino) phenyl-amino]-6-(isopropyl-amino)-3-nitro-pyridine hydrochloride | 1.20(d, 6H), 3.22(m, 8H), 4.08(m, 1H), 6.13(d, 1H), 7.08(t, 1H), 7.38(d, 1H), 7.87(d, 1H), 8.06(d, 1H), 8.34(d, 1H), 9.29(m, 2H), 10.88(s, 1H) | 20~ 30 | 55 | 375.2 |

(continued)

| Compounds according to Examples 216 to 222, Solvent S = $CH_3CN$ (all examples) Starting material 2-[3-fluoro-4-(BOC-piperazino)phenylamino]-6-chloro-3-nitropyridine compound obtained in Preparation Example 3-4-3 | | | | | | | |
|---|---|---|---|---|---|---|---|
| Ex | Amine | A | Compound | NMR (DMSO-d6) | T [°C] | Y [%] | M(+) |
| 217 | Isobutylamine (excess) | X | 2-[(3-fluoro-4-piperazino) phenyl-amino]-6-(isobutyl-amino)-3-nitro-pyridine | 0.90(d, 6H), 1.88(m, 1H), 3.17(m, 2H), 3.23(m, 8H), 6.17(d, 1H), 7.08(t, 1H), 7.32(d, 1H), 7.95(d, 1H), 8.07(d, 1H), 8.56(t, 1H), 9.21 (brm, 2H), 10.88(s, 1H) | 20~ 30 | 65 | 389.2 |

(continued)

| Compounds according to Examples 216 to 222, Solvent S = $CH_3CN$ (all examples) Starting material 2-[3-fluoro-4-(BOC-piperazino)phenylamino]-6-chloro-3-nitropyridine compound obtained in Preparation Example 3-4-3 | | | | | | | |
|---|---|---|---|---|---|---|---|
| Ex | Amine | A | Compound | NMR (DMSO-d6) | T [°C] | Y [%] | M(+) |
| 218 | 4-hydroxy-piperidine (1.5 equivalents) | 1.5 # | 2-[(3-fluoro-4-piperazino) phenyl-amino]-6-[(4-hydroxy)-piperidino]-3-nitropyridine | 1.39(m, 2H), 1.79(m, 2H), 2.84(m, 4H), 2.90(m, 4H), 3.43(m, 2H), 3.80(m, 1H), 4.03 (brm, 2H), 4.83(s, 1H), 6.53(d, 1H), 6.98(t, 1H), 7.29(d, 1H), 8.16(d, 1H), 10.56(s, 1H) | 20~ 30 | 85 | 417.2 |
| 219 | 2-isopropyl-imidazole (5 equivalents) | 5 # | 2-[(3-fluoro-4-piperazino)-phenylamino]-6-[(2-isopropyl)imidazol-1-yl]-3-nitropyridine | 0.93(d, 6H), 2.89(m, 4H), 2.93(m, 4H), 3.41(m, 1H), 6.92(d, 1H), 7.06(m, 2H), 7.17(dd, 1H), 7.38(dd, 1H), 7.63(d, 1H), 8.65(d, 1H), 10.07(s, 1H) | 60~ 70 | 92 | 426.2 |

(continued)

| Compounds according to Examples 216 to 222, Solvent S = $CH_3CN$ (all examples) Starting material 2-[3-fluoro-4-(BOC-piperazino)phenylamino]-6-chloro-3-nitropyridine compound obtained in Preparation Example 3-4-3 | | | | | | | |
|---|---|---|---|---|---|---|---|
| Ex | Amine | A | Compound | NMR (DMSO-d6) | T [°C] | Y [%] | M(+) |
| 220 | 3-amino-methylpyridine (1.5 equivalents) | 1.5 # | 2-[(3-fluoro-4-piperazino) phenyl-amino]-6-[(3-pyridyl)-methylamino]-3-nitropyridine | 3.03 (brm, 8H), 4.58(d, 2H), 6.22(d, 1H), 6.96((t, 1H), 7.26(d, 1H), 7.34(m, 1H), 7.60(m, 2H), 8.13(d, 1H), 8.46(m, 2H), 8.89(t, 1H), 10.71(s, 1H), | 60~ 70 | 88 | 424.2 |
| 221 | 4-amino-methylpyridine (1.5 equivalents) | 1.5 # | 2-[(3-fluoro-4-piperazino)-phenyl-amino]-6-[(4-pyridyl)-methylamino]-3-nitropyridine | 2.84(m, 8H), 4.58(d, 2H), 6.25(d, 1H), 6.85(t, 1H), 7.11(d, 1H), 7.22(m, 2H), 7.44(d, 1H), 8.16(d, 1H), 8.48(d, 2H), 8.86 (brm, 1H), 10.67(s, 1H) | 60~ 70 | 74 | 424.1 |

(continued)

| Compounds according to Examples 216 to 222, Solvent S = $CH_3CN$ (all examples) Starting material 2-[3-fluoro-4-(BOC-piperazino)phenylamino]-6-chloro-3-nitropyridine compound obtained in Preparation Example 3-4-3 | | | | | | | |
|---|---|---|---|---|---|---|---|
| Ex | Amine | A | Compound | NMR (DMSO-d6) | T [°C] | Y [%] | M(+) |
| 222 | t-butylamine (excess) | X | 2-[(3-fluoro-4-piperazino)-phenyl-amino]-6-(t-butyl-amino)-3-nitro-pyridine | 1.27(s, 9H), 2.93(m, 8H), 6.13(d, 1H), 7.02(t, 1H), 7.16(d, 1H), 7.50(d, 1H), 7.86(s, 1H), 8.00(d, 1H), 10.62(s, 1H) | 20~ 30 | 92 | 389.1 |

**Example 223**

**Preparation of 2-[(3-fluoro-4-piperidino)phenylamino]-6-(methylamino)-3-nitropyridine**

To 10ml of acetonitrile were added 200mg (0.57 mmol) of the 2-[(3-fluoro-4-pipe-ridino)phenylamino]-6-chloro-3-nitropyridine compound obtained in Preparation Example 3-5-3 and 10ml of a 40% (wt/v) methylamine-methanol solution, followed by reaction at room temperature for 4 hours. After the reaction was complete, the solvent was distilled under reduced pressure, followed by column chromatography purification with a 4:1 (v/v) solution of n-hexane and ethyl acetate as a developing solvent, recrystallization from ethyl acetate and n-hexane, and vacuum drying at about 40°C to afford 161mg (yield: 82%) of the desired compound.

Mass (M+): 346.2

$^1$H-NMR(DMSO-$d_6$) (ppm) 1.52(m, 2H), 1.65(m, 4H), 2.91(d+m, 7H), 6.11(d, 1H), 7.02(t, 1H), 7.38(d, 1H), 7.84 (dd, 1H), 8.06(d, 1H), 8.33(m, 1H), 10.89(s, 1H).

**Examples 224 to 235**

In the same manner as in Example 223 and using amine compounds described in the following Table 19 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 19 were obtained.

The following Table 19 shows the name of compounds prepared in Examples 224 to 235, the name and equivalents of amine compounds used in the reaction, use/nonuse and equivalents of triethylamine in the reaction, the reaction temperature, the reaction solvent, yield, Mass analysis results and NMR analysis results.

**Table 19** Compounds according to Examples 224 to 235, Solvent S = $CH_3CN$ (all examples) Starting material 2-[(3-fluoro-4-piperidino)phenylamino]-6-chloro-3-nitropyridine compound obtained in Preparation Example 3-5-3

| Ex | Amine | A | Compound | NMR (DMSO-d6) | T [°C] | Y [%] | M(+) |
|---|---|---|---|---|---|---|---|
| 224 | Isopropylamine (excess) | X | 2-[(3-fluoro-4-piperidino)-phenylamino]-6-(isopropylamino)-3-nitropyridine | 1.20(d, 6H), 1.52(m, 2H), 1.65(m, 4H), 2.93(t, 4H), 4.08(m, 1H), 6.09(d, 1H), 7.02(t, 1H), 7.30(dd, 1H), 7.81(d, 1H), 8.06(d, 1H), 8.23(m, 1H), 10.86(s, 1H) | 20~ 30 | 51 | 374.2 |
| 225 | Isobutylamine (excess) | X | 2-[(3-fluoro-4-piperidino)-phenyl-amino]-6-(isobutyl-amino)-3-nitro-pyridine | 0.90(d, 6H), 1.52(m, 2H), 1.65(m, 4H), 1.89(m, 1H), 2.93(t, 4H), 3.17(t, 2H), 6.14(d, 1H), 7.00(t, 1H), 7.25(dd, 1H), 7.87(d, 1H), 8.06(d, 1H), 8.47(t, 1H), 10.85(s, 1H) | 20~ 30 | 54 | 388.2 |

(continued)

| Ex | Amine | A | Compound | NMR (DMSO-d6) | T [°C] | Y [%] | M(+) |
|---|---|---|---|---|---|---|---|
| 226 | 4-hydroxy-piperidine (1.5 equivalents) | 1.5 # | 2-[(3-fluoro-4-piperidino) phenyl-amino]-6-(4-hydroxypiperidino)-3-nitropyridine | 1.40(m, 2H), 1.53(m, 2H), 1.64(m, 4H), 1.79(m, 2H) 2.93 (brm, 4H), 3.43(t, 2H), 3.80(m, 1H) 4.05 (brm, 2H), 4.82(d, 1H), 6.53(d, 1H), 7.01(t, 1H), 7.26(d, 1H), 7.58(d, 1H), 8.16(d, 1H), 10.55(s, 1H) | 20~ 30 | 59 | 416.2 |

(continued)

| Ex | Amine | A | Compound | NMR (DMSO-d6) | T [°C] | Y [%] | M(+) |
|---|---|---|---|---|---|---|---|
| 227 | 2-m ethyl-2-imidazoline (2 equivalents) | 2 # | 2-[(3-fluoro-4-piperidino) phenyl-amino]-6-[(2-methyl-4,5-dihydro) imidazol-1-yl]-3-nitropyridine | 1.52(m, 2H), 1.65(m, 4H), 1.85(s, 3H), 2.94 (brm, 4H), 3.25(m, 2H), 3.41(m, 2H), 6.12(d, 1H), 7.02(t, 1H), 7.41(d, 1H), 7.69(d, 1H), 7.96(t, 1H), 8.09(d, 1H), 8.38(t, 1H), 10.83(s, 1H) | 60~ 70 | 55 | 399.2 |

(continued)

| Ex | Amine | A | Compound | NMR (DMSO-d6) | T [°C] | Y [%] | M(+) |
|---|---|---|---|---|---|---|---|
| 228 | 2-isopropyl-imidazole (1.5 equivalents) | 1.5 # | 2-[(3-fluoro-4-piperidino) phenyl-amino]-6-[(2-iso-propyl)imidazol-1-yl]-3-nitropyridine | 0.93(d, 6H), 1.54(m, 2H), 1.66(m, 4H), 2.97(m, 4H), 3.40(m, 1H), 6.92(s, 1H), 7.70(t, 2H), 7.13(d, 1H), 7.29(d, 1H), 7.63(s, 1H), 8.65(d, 1H), 10.07(s, 1H), | 60~ 70 | 46 | 425.2 |
| 229 | 3-amino-methylpyridine (1.5 equivalents) | 1.5 # | 2-[(3-fluoro-4-piperidino)-phenyl-amino]-6-[(3-pyridyl)-methylamino]-3-nitropyridine | 1.50(m, 2H), 1.64(m, 4H), 2.91 (brm, 4H), 4.58(d, 2H), 6.20(d, 1H), 6.93(t, 1H), 7.20(d, 1H),7.34 (m, 1H), 7.54(dd, 1H), 7.60(dd, 1H), 8.13(d, 1H), 8.45(m, 1H), 8.81(t, 1H), 10.70(s, 1H), | 60~ 70 | 70 | 423.2 |

(continued)

| Ex | Amine | A | Compound | NMR (DMSO-d6) | T [°C] | Y [%] | M(+) |
|---|---|---|---|---|---|---|---|
| 230 | 4-amino-methylpyridine (1.5 equivalents) | 1.5 # | 2-[(3-fluoro-4-piperidino) phenyl-amino]-6-[(4-pyridyl)-methylamino]-3-nitropyridine | 1.53(m, 2H), 1.83(m, 2H), 2.71(t, 2H), 3.18(m, 2H), 3.60(m, 1H), 4.56(m, 2H), 4.70(d, 1H), 6.24(d, 1H), 6.85(t, 1H), 7.08(t, 1H), 7.22(m, 2H), 7.42(d, 1H), 8.15(d, 1H), 8.48(d, 1H), 8.65(t, 1H), 10.67(s, 1H), | 60~ 70 | 73 | 439.3 |

(continued)

| Ex | Amine | A | Compound | NMR (DMSO-d6) | T [°C] | Y [%] | M(+) |
|---|---|---|---|---|---|---|---|
| 231 | t-butylamine (excess) | X | 2-[(3-fluoro-4-piperidino) phenyl-amino]-6-(t-butyl-amino)-3-nitro-pyridine | 1.26(s, 9H), 1.52(m, 2H), 1.65(m, 4H), 2.94(t, 4H), 6.13(d, 1H), 7.00(t, 1H), 7.13(dd, 1H), 7.47(d, 1H), 7.85(t, 1H), 8.00(d, 1H), 10.62(s, 1H) | 20~ 30 | 43 | 388.2 |
| 232 | 1-methyl-piperazine (3 equivalents) | X | 2-[(3-fluoro-4-piperidino) phenyl-amino]-6-(4-methyl-piperazin-1-yl)-3-nitropyridine | 1.53(m. 2H), 1.65(m, 4H), 2.20(s, 3H), 2.38(t, 4H), 2.93(t, 4H), 3.70(m, 4H), 6.51(d, 1H), 7.02(t, 1H), 7.28(dd, 1H), 7.54(dd, 1H), 8.18(d, 1H), 10.52(s, 1H) | 20~ 30 | 49 | 415.3 |

(continued)

| Ex | Amine | A | Compound | NMR (DMSO-d6) | T [°C] | Y [%] | M(+) |
|---|---|---|---|---|---|---|---|
| 233 | Piperazine (5 equivalents) | X | 2-[(3-fluoro-4-piperidino) phenyl-amino]-6-(piperazin-1-yl)-3-nitropyridine | 1.54 (brm, 2H), 1.65(m, 4H), 2.75 (brm, 4H), 2.93(t, 4H). 3.64 (brm, 4H), 6.48(d, 1H), 7.01(t, 1H), 7.28(d, 1H), 7.55(dd, 1H), 8.16(d, 1H), 10.56(s, 1H) | 20~ 30 | 44 | 401.2 |
| 234 | 4-amino-piperidine (1.5 equivalents) | 1.5 # | 2-[(3-fluoro-4-piperidino)-phenylamino]-6-[(4-amino)piperidino]-3-nitropyridine | 1.22(m, 2H), 1.51(m, 2H), 1.64(m, 4H), 1.76(m, 4H), 2.93(m, 5H), 3.17(t, 2H), 4.29 (brm, 2H), 6.52(d, 1H), 7.00(t, 1H), 7.26(d, 1H), 7.59(d, 1H), 8.16(d, 1H), 10.56(s, 1H) | 20~ 30 | 94 | 415.2 |

(continued)

| Ex | Amine | A | Compound | NMR (DMSO-d6) | T [°C] | Y [%] | M(+) |
|---|---|---|---|---|---|---|---|
| 235 | Morpholine (3 equivalents)) | X | 2-[(3-fLuoro-4-piperidino) phenylami no]-6-morpholino-3-nitropyridine | 4H),2.95 (brm, 4H), 1.52(m, 2H), 3.68 (brm, 8H), 6.50(d, 1H), 7.00(t, 1H), 7.31(d, 1H), 7.52(dd, 1H), 8.22(d, 1H), 10.52(s, 1H) | 20~ 30 | | 402.2 58 |

**Example 236**

**Preparation of 2-{[3-fluoro-4-(4-hydroxypiperidino)]phenylamino}-6-(methylamino)-3-nitropyridine**

To 10ml of acetonitrile were added 200mg (0.55 mmol) of the 2-{[3-fluoro-4-(4-hydroxypiperidino)]phenylamino}-6-chloro-3-nitropyridine compound obtained in Preparation Example 3-6-3 and 5ml of a 40% methylamine-methanol solution, followed by reaction at room temperature for 4 hours. After the reaction was complete, the solvent was distilled under reduced pressure, followed by column chromatography purification with a 10:5:1 (v/v/v) solution of n-hexane, ethyl acetate and methanol as a developing solvent, recrystallization from ethyl acetate and n-hexane, and vacuum drying at about 40°C to afford 145mg (yield: 73%) of the desired compound.

Mass (M+): 362.2

$^1$H-NMR(DMSO-$d_6$) (ppm) 1.55(m, 2H), 1.84(m, 2H), 2.74(dt, 2H), 2.91(d, 3H), 3.22(m, 2H), 3.60(m, 1H), 4.70 (d, 1H), 6.11(d, 1H), 7.03(t, 1H), 7.38 (dd,1H), 7.85(dd, 1H), 8.06(d, 1H), 8.34(m, 1H), 10.89(s, 1H).

**Examples 237 to 247**

In the same manner as in Example 236 and using amine compounds described in the following Table 20 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 20 were obtained.

The following Table 20 shows the name of compounds prepared in Examples 237 to 247, the name and equivalents of amine compounds used in the reaction, use/nonuse and equivalents of triethylamine in the reaction, the reaction temperature, the reaction solvent, yield, Mass analysis results and NMR analysis results.

**Table 20**

| Compounds according to Examples 237 to 247, Solvent S = $CH_3CN$ (all examples) Starting material 2-{[3-fluoro-4-(4-hydroxypiperidino)]phenylamino}-6-chloro-3-nitropyridine compound obtained in Preparation Example 3-6-3 | | | | | | | |
|---|---|---|---|---|---|---|---|
| Ex | Amine | A | Compound | NMR (DMSO -d6) | T [°C] | Y [%] | M(+) |
| 237 | Isopropylamine (excess) | X | 2-{[3-fLuoro-4-(4-hydroxypiperidino)]p henylamino}-6-(isopropylamino)-3-nitropyridine | 1.20(d, 6H), 1.53(m, 2H), 1.86(m, 2H), 2.73(t, 2H), 3.23(m, 2H), 3.60(m, 1H), 4.08(m, 1H), 4.71(d, 1H), 6.09(d, 1H), 7.02(t, 1H), 7.29 (dd, 1H), 7.82(d, 1H), 8.06(d, 1H), 8.23(d, 1H), 10.86 (s, 1H) | 20~ 30 | 56 | 389.3 |

(continued)

| Compounds according to Examples 237 to 247, Solvent S = $CH_3CN$ (all examples) Starting material 2-{[3-fluoro-4-(4-hydroxypiperidino)]phenylamino}-6-chloro-3-nitropyridine compound obtained in Preparation Example 3-6-3 | | | | | | | |
|---|---|---|---|---|---|---|---|
| Ex | Amine | A | Compound | NMR (DMSO -d6) | T [°C] | Y [%] | M(+) |
| 238 | Isobutylamine (excess) | X | 2-{[3-fluoro-4-(4-hydroxypiperidino)]-phenylamino}-6-(isobutylamino)-3-nitropyridine | 0.91(d, 6H), 1.54(m, 2H), 1.87(m, 3H), 2.74(t, 2H), 3.19(m, 4H), 3.61(m, 4H), 4.71(d, 1H), 6.14(d, 1H), 7.01(t, 1H), 7.24 (dd, 1H), 7.87 (dd, 1H), 8.05(d, 1H), 8.46(t, 1H), 10.85 (s, 1H) | 20~ 30 | 68 | 404.2 |

(continued)

| Compounds according to Examples 237 to 247, Solvent S = $CH_3CN$ (all examples) Starting material 2-{[3-fluoro-4-(4-hydroxypiperidino)]phenylamino}-6-chloro-3-nitropyridine compound obtained in Preparation Example 3-6-3 | | | | | | | |
|---|---|---|---|---|---|---|---|
| Ex | Amine | A | Compound | NMR (DMSO-d6) | T [°C] | Y [%] | M(+) |
| 239 | 4-hydroxy-piperidine (1.5 equivalents) | 1.5 # | 2-{[3-fluoro-4-(4-hydroxypiperidino)]-phenylamino}-6-(4-hydroxypiperidino)-3-nitropyridine | 1.39(m, 2H), 1.53(m, 2H), 1.80 (brm, 4H), 2.74(t, 2H), 3.22(m, 2H), 3.41(m, 2H), 3.61(m, 1H), 3.81(m, 1H), 4.03 (brm, 2H), 4.70(d, 1H), 4.81(d, 1H), 6.52(d, 1H), 7.03(t, 1H), 7.26 (dd, 1H), 7.61 (dd, 1H), 8.16(d, 1H), 10.55 (s, 1H) | 20~ 30 | 53 | 432.1 |

(continued)

| Compounds according to Examples 237 to 247, Solvent S = $CH_3CN$ (all examples) Starting material 2-{[3-fluoro-4-(4-hydroxypiperidino)]phenylamino}-6-chloro-3-nitropyridine compound obtained in Preparation Example 3-6-3 | | | | | | | |
|---|---|---|---|---|---|---|---|
| Ex | Amine | A | Compound | NMR (DMSO -d6) | T [°C] | Y [%] | M(+) |
| 240 | 2-methyl-2-imidazoline (2equiva!ents) | 2 # | 2-{[3-fluoro-4-(4-hydroxypiperidino)]-phenylamino}-6-[(2-methyl-4,5-dihydro)-imidazol-1-yl]-3-nitropyridine | 1.54(t, 2H), 1.83(t, 2H), 1.98(s, 3H), 2.76(t, 2H), 3.22(m, 2H), 3.65(m, 1H), 3.71(m, 2H), 3.85(t, 2H), 4.72(d, 1H), 6.40(d, 1H), 7.05(t, 1H), 7.15(d, 1H), 7.33(d, 1H), 8.38(d, 1H), 10.15 (s, 1H) | 20~ 30 | 46 | 415.2 |

(continued)

| Compounds according to Examples 237 to 247, Solvent S = $CH_3CN$ (all examples) Starting material 2-{[3-fluoro-4-(4-hydroxypiperidino)]phenylamino}-6-chloro-3-nitropyridine compound obtained in Preparation Example 3-6-3 | | | | | | | |
|---|---|---|---|---|---|---|---|
| Ex | Amine | A | Compound | NMR (DMSO -d6) | T [°C] | Y [%] | M(+) |
| 241 | 3-amino-methylpyridine (1.5 equivalents) | 1.5 # | 2-{[3-fluoro-4-(4-hydroxypiperidino)]-phenylamino}-6-[(3-pyridyl)methylamino] -3-nitropyridine | 1.52(m, 2H), 1.82(m, 2H), 2.71(t, 2H), 3.18(m, 2H), 3.60(m, 1H), 4.56(d, 2H), 4.70(d, 1H), 6.20(d, 1H), 6.93(t, 1H), 7.20(d, 1H), 7.34(m, 1H), 7.55 (dd, 1H), 7.60 (dd, 1H), 8.13(d, 1H), 8.45(d, 2H), 8.80(t, 1H), 10.70 (s, 1H) | 20~ 30 | 64 | 439.1 |

(continued)

| Compounds according to Examples 237 to 247, Solvent S = $CH_3CN$ (all examples) Starting material 2-{[3-fluoro-4-(4-hydroxypiperidino)]phenylamino}-6-chloro-3-nitropyridine compound obtained in Preparation Example 3-6-3 | | | | | | | |
|---|---|---|---|---|---|---|---|
| Ex | Amine | A | Compound | NMR (DMSO -d6) | T [°C] | Y [%] | M(+) |
| 242 | 4-amino-methylpyridine (1.5 equivalents) | 1.5 # | 2-{[3-fluoro-4-(4-hydroxypiperidino)]-phenylamino}-6-[(4-pyridyl)methylamino] -3-nitropyridine | 1.53(m, 2H), 1.83(m, 2H), 2.71(t, 2H), 3.18(m, 2H), 3.60(m, 1H), 4.56(m, 2H), 4.70(d, 1H), 6.24(d, 1H), 6.85(t, 1H), 7.08(d, 1H), 7.22(m, 2H), 7.42(d, 1H), 8.15(d, 1H), 8.48(d, 1H), 8.65(t, 1H), 10.67 (s, 1H) | 20~ 30 | 73 | 439.3 |

(continued)

| Compounds according to Examples 237 to 247, Solvent S = $CH_3CN$ (all examples) Starting material 2-{[3-fluoro-4-(4-hydroxypiperidino)]phenylamino}-6-chloro-3-nitropyridine compound obtained in Preparation Example 3-6-3 | | | | | | | |
|---|---|---|---|---|---|---|---|
| Ex | Amine | A | Compound | NMR (DMSO -d6) | T [°C] | Y [%] | M(+) |
| 243 | t-butylamine (excess) | X | 2-{[3-fluoro-4-(4-hydroxypiperidino)]-phenylamino}-6-(t-butylamino)-3-nitropyridine | 1.26(s, 9H), 1.54(m, 2H), 1.83(m, 2H), 2.74(t, 2H), 3.23(m, 2H), 3.61(m, 1H), 4.71(d, 1H), 6.13(d, 1H), 7.03(t, 1H), 7.12(d, 1H), 7.41(d, 1H), 7.84(s, 1H), 8.01(d, 1H), 10.61 (s, 1H) | 20~ 30 | 59 | 404.3 |

(continued)

| Compounds according to Examples 237 to 247, Solvent S = $CH_3CN$ (all examples) Starting material 2-{[3-fluoro-4-(4-hydroxypiperidino)]phenylamino}-6-chloro-3-nitropyridine compound obtained in Preparation Example 3-6-3 | | | | | | | |
|---|---|---|---|---|---|---|---|
| Ex | Amine | A | Compound | NMR (DMSO -d6) | T [°C] | Y [%] | M(+) |
| 244 | 1-methyl-piperazine (1.5 equivalents) | 1.5 # | 2-{[3-fluoro-4-(4-hydroxypiperidino)]-phenylaminol-(4 -methylpiperazin-1-yl)-3-nitropyridine | 1.54(m, 2H), 1.86(m, 2H), 2.21(s, 3H), 2.48(m, 4H), 2.77(m, 2H), 3.22(m, 2H), 3.61(m, 1H), 3.71(m, 4H), 4.71(d, 1H), 6.51(d, 1H), 7.03(t, 1H), 7.28(d, 1H), 7.54 (dd, 1H), 8.19(d, 1H), 10.52 (s, 1H) | 20~ 30 | 52 | 431.3 |

(continued)

| Compounds according to Examples 237 to 247, Solvent S = $CH_3CN$ (all examples) Starting material 2-{[3-fluoro-4-(4-hydroxypiperidino)]phenylamino}-6-chloro-3-nitropyridine compound obtained in Preparation Example 3-6-3 | | | | | | | |
|---|---|---|---|---|---|---|---|
| Ex | Amine | A | Compound | NMR (DMSO -d6) | T [°C] | Y [%] | M(+) |
| 245 | Piperazine (5 equivalents) | X | 2-{[3-fluoro-4-(4-hydroxypiperidino)] p henylaminol-6-(piperazin-1-yl)-3-nitropyridine | 1.54 (brm, 2H), 1.65(m, 4H), 2.75 (brm, 4H), 2.93(t, 4H), 3.64 (brm, 4H), 6.48(d, 1H), 7.01(t, 1H), 7.28(d, 1H), 7.55 (dd, 1H), 8.16(d, 1H), 10.56 (s, 1H) | 20~ 30 | 59 | 417.2 |

(continued)

| Compounds according to Examples 237 to 247, Solvent S = $CH_3CN$ (all examples) Starting material 2-{[3-fluoro-4-(4-hydroxypiperidino)]phenylamino}-6-chloro-3-nitropyridine compound obtained in Preparation Example 3-6-3 | | | | | | | |
|---|---|---|---|---|---|---|---|
| Ex | Amine | A | Compound | NMR (DMSO -d6) | T [°C] | Y [%] | M(+) |
| 246 | 4-amino-piperidine (1.5 equivalents) | 1.5 # | 2-{[3-fluoro-4-(4-hydroxypiperidino)]-phenylamino}-6-(4-aminopiperidino)-3-nitropyridine | 1.22(m, 2H), 1.51(m, 2H), 1.64(m, 4H), 1.76(m, 4H), 2.93(m, 5H), 3.17(t, 2H), 4.29 (brm, 2H), 6.52(d, 1H), 7.00(t, 1H), 7.26(d, 1H), 7.59(d, 1H), 8.16(d, 1H), 10.56 (s, 1H) | 20~ 30 | 44 | 431.3 |

(continued)

| Compounds according to Examples 237 to 247, Solvent S = $CH_3CN$ (all examples) Starting material 2-{[3-fluoro-4-(4-hydroxypiperidino)]phenylamino}-6-chloro-3-nitropyridine compound obtained in Preparation Example 3-6-3 | | | | | | | |
|---|---|---|---|---|---|---|---|
| Ex | Amine | A | Compound | NMR (DMSO -d6) | T [°C] | Y [%] | M(+) |
| 247 | Morpholine (3 equivalents) | X | 2-{[3-fluoro-4-(4-hydroxypiperidino)]-phenylaminol-6-morpholino-3-nitropyridine | 1.52(m, 2H), 1.64 (brm, 4H), 2.95 (brm, 4H), 3.68 (brm, 8H), 6.50(d, 1H), 7.00(t, 1H), 7.31(d, 1H), 7.52 (dd, 1H), 8.22(d, 1H), 10.52 (s, 1H) | 20~ 30 | 66 | 418.2 |

**Example 248**

**Preparation of 2-{[3-fluoro-4-(4-aminopiperidino)]phenylamino}-6-(methylamino)-3-nitropyridine**

To 10ml of acetonitrile were added 300mg (0.64mmol) of the 2-{[3-fluoro-4-(4-BOC-aminopiperidino)]phenylamino}-6-chloro-3-nitropyridine compound obtained in Preparation Example 3-7-4 and 5ml of a 40% (wt/v) methylamine-methanol solution, followed by reaction at room temperature for 4 hours. After the reaction was complete, the solvent was distilled under reduced pressure, followed by recrystallization from 5ml of methanol and vacuum drying at about 40°C to afford 255mg (yield: 87%) of 2-{[3-fluoro-4-(4-BOC-amino)-piperidino]phenylamino}-6-(methylamino)-3-nitropyridine.

Mass (M+): 461.3

$^1$H-NMR(DMSO-$d_6$) (ppm): 1.39(s. 9H), 1.53(m, 2H), 1.80(m, 2H), 2.63(t, 2H), 2.90(s, 3H), 3.26(m, 2H), 3.34 (m, 1H), 6.12(d, 1H), 6.90(d, 1H), 7.03(t, 1H), 7.41(d, 1H), 7.85(d, 1H), 8.07(d, 1H), 8.54(d, 1H), 10.89(s, 1H).

200mg (0.43mmol) of the above-obtained 2-{[3-fluoro-4-(4-BOC-amino)piperidino]-phenylaminol-6-(methylamino)-3-nitropyridine was dissolved in 10ml of dichloromethane and 0.64ml (8.6mmol) of trifluoroacetic acid was added thereto, followed by reaction at room temperature (20 to 30°C) for 24 hours. After the reaction was complete, the solvent was distilled under reduced pressure. The residue was dissolved in 10ml of methanol and pH thereof was adjusted to a value of 7 to 8 by dropwise addition of a sodium bicarbonate solution at a temperature of 0 to 5°C, followed by stirring for about 1 hour. The resulting solid was filtered, washed with a 1:1 (v/v) solution of water and methanol, and then dried under vacuum at about 40°C to afford 128mg (yield: 83%) of the desired compound.

Mass: 361.2

$^1$H-NMR(DMSO-$d_6$) (ppm) 1.41(m, 2H), 1.78(m, 2H), 2.66(m, 2H), 2.90(d+m, 3H), 3.20(m, 2H), 3.28(brm, 2H), 6.11(d, 1H), 7.01(t, 1H), 7.38(d, 1H), 7.86(d, 1H), 8.06(d, 1H), 8.34(s, 1H), 10.89(s, 1H).

**Examples 249 to 260**

In the same manner as in Example 248 and using amine compounds described in the following Table 21 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 21 were obtained.

The following Table 21 shows the name of compounds prepared in Examples 249 to 260, the name and equivalents of amine compounds used in the reaction, use/nonuse and equivalents of triethylamine in the reaction, the reaction temperature, the reaction solvent, yield, Mass analysis results and NMR analysis results.

**Table 21**

| Compounds according to Examples 249 to 260, Solvent S = $CH_3CN$ (all examples) Starting material 2-{[3-fluoro-4-(4-BOC-aminopiperidino)]phenylamino}-6-chloro-3-nitropyridine compound obtained in Preparation Example 3-7-4 | | | | | | | |
|---|---|---|---|---|---|---|---|
| Ex | Amine | A | Compound | NMR (DMSO -d6) | T [°C] | Y [%] | M(+) |
| 249 | Isopropylamine (excess) | X | 2-{[3-fluoro-4-(4-aminopiperidino)]-phenylamino} -6-(isopropylamino)-3-nitropyridine | 1.20(d, 6H), 1.47(m, 2H), 1.84(m, 2H), 2.67(t, 2H), 2.92(m, 1H) 3.27(m, 2H), 4.08(m, 3H), 6.09(d, 1H), 7.01(t, 1H), 7.32(d, 1H), 7.81(d, 1H), 8.06(d, 1H), 8.26(d, 1H), 10.87 (s, 1H) | 20~ 30 | 91 | 389.3 |

(continued)

| Compounds according to Examples 249 to 260, Solvent S = $CH_3CN$ (all examples) Starting material 2-{[3-fluoro-4-(4-BOC-aminopiperidino)]phenylamino}-6-chloro-3-nitropyridine compound obtained in Preparation Example 3-7-4 | | | | | | | |
|---|---|---|---|---|---|---|---|
| Ex | Amine | A | Compound | NMR (DMSO -d6) | T [°C] | Y [%] | M(+) |
| 250 | Isobutylamine (excess) | X | 2-{[3-fluoro-4-(4-aminopiperidino)]-phenylamino}-6-(isobutylamino)-3-nitropyridine)-3 | 0.90(d, 6H), 1.68(m, 2H), 1.89(m, 1H), 1.98(m, 2H), 2.73(t, 2H), 3.17(m, 3H), 3.33(m, 2H), 6.15(d, 1H), 7.03(d, 1H), 7.28(d, 1H), 7.90(m, 3H), 8.07(d, 1H), 8.48(t, 1H), 10.87 (s, 1H) | 20~ 30 | 65 | 403.2 |

(continued)

| Compounds according to Examples 249 to 260, Solvent S = $CH_3CN$ (all examples) Starting material 2-{[3-fluoro-4-(4-BOC-aminopiperidino)]phenylamino}-6-chloro-3-nitropyridine compound obtained in Preparation Example 3-7-4 | | | | | | | |
|---|---|---|---|---|---|---|---|
| Ex | Amine | A | Compound | NMR (DMSO -d6) | T [°C] | Y [%] | M(+) |
| 251 | 4-hydroxy-piperidine (1.5 equivalents) | 1.5 # | 2-{[3-fluoro-4-(4-aminopiperidino)]-phenylamino}-6-(4-hydroxypiperidino)-3-nitropyridine | 1.40(m, 2H), 1.65(m, 2H), 1.79(m, 2H), 1.96(m, 2H), 2.72(t, 2H), 3.09(m, 1H), 3.36(m, 2H), 3.40(m, 2H), 3.81(m, 1H), 4.03 (brm, 2H), 4.81 (brm, 1H), 6.54(d, 1H), 7.03(t, 1H), 7.30(d, 1H), 7.50 (brm, 2H), 7.62 (dd, 1H), 8.17(d, 1H), 10.56 (s, 1H) | 20~ 30 | 99 | 431.3 |

(continued)

| Compounds according to Examples 249 to 260, Solvent S = $CH_3CN$ (all examples) Starting material 2-{[3-fluoro-4-(4-BOC-aminopiperidino)]phenylamino}-6-chloro-3-nitropyridine compound obtained in Preparation Example 3-7-4 | | | | | | | |
|---|---|---|---|---|---|---|---|
| Ex | Amine | A | Compound | NMR (DMSO -d6) | T [°C] | Y [%] | M(+) |
| 252 | 2-methyl-2-imidazoline (2 equivalents) | 2 # | 2-{[3-fluoro-4-(4-aminopiperidino)]-methyl-4,5-dihydro)imidazol-1-yl]-3-nitropyridine | 1.71(m 2H), 1.80(s, 3H), 2.00(m, 2H), 2.75(t, 2H), 3.15(m, 1H), 3.25(m, 2H), 3.40(m, 4H), 6.15(d, 1H), 7.06(d, 1H), 7.41(d, 1H), 7.70(d, 1H), 8.51(t, 1H), 10.83 (s, 1H) | 60~ 70 | 35 | 414.1 |

(continued)

| Compounds according to Examples 249 to 260, Solvent S = $CH_3CN$ (all examples) Starting material 2-{[3-fluoro-4-(4-BOC-aminopiperidino)]phenylamino}-6-chloro-3-nitropyridine compound obtained in Preparation Example 3-7-4 | | | | | | | |
|---|---|---|---|---|---|---|---|
| Ex | Amine | A | Compound | NMR (DMSO -d6) | T [°C] | Y [%] | M(+) |
| 253 | Piperazine (5 equivalents) | | 2-{[3-fluoro-4-(4-aminopiperidino)]-phenylamino}-6-(piperazin-1-yl)-3-nitropyridine | 1.48(m, 2H), 1.85(m, 2H), 2.66(m, 4H), 2.73 (brm, 4H), 2,82(m, 1H), 3.17(s, 1H), 3.28(d, 2H), 3.64 (brm, 4H), 6.49(d, 1H), 7.01(t, 1H), 7.29(d, 1H), 7.57(d, 1H), 8.17(d, 1H), 10.56 (s, 1H) | 20~ 30 | 89 | 416.3 |

(continued)

| Compounds according to Examples 249 to 260, Solvent S = $CH_3CN$ (all examples) Starting material 2-{[3-fluoro-4-(4-BOC-aminopiperidino)]phenylamino}-6-chloro-3-nitropyridine compound obtained in Preparation Example 3-7-4 | | | | | | | |
|---|---|---|---|---|---|---|---|
| Ex | Amine | A | Compound | NMR (DMSO -d6) | T [°C] | Y [%] | M(+) |
| 254 | Amine Methyl-piperazine (3 equivalents) | | 2-{[3-fluoro-4-(4-aminopiperidino)]-phenylamino}-6-(4-methylpiperazin-1-yl)-3-nitropyridine | 1.55(m, 2H), 1.90(m, 2H), 2.20(s, 3H), 2.37(m, 4H), 2.69(m, 2H), 2.90(m, 1H), 3.30(d, 2H), 3.70(m, 4H), 6.50(d, 1H), 7.01(t, 1H), 7.27 (dd, 1H), 7.54 (dd, 1H), 8.17(d, 1H), 10.52 (s, 1H) | 20~ 30 | 85 | 430.2 |

(continued)

| Compounds according to Examples 249 to 260, Solvent S = $CH_3CN$ (all examples) Starting material 2-{[3-fluoro-4-(4-BOC-aminopiperidino)]phenylamino}-6-chloro-3-nitropyridine compound obtained in Preparation Example 3-7-4 | | | | | | | |
|---|---|---|---|---|---|---|---|
| Ex | Amine | A | Compound | NMR (DMSO -d6) | T [°C] | Y [%] | M(+) |
| 255 | Morpholine (3 equivalents | | 2-{[3-fluoro-4-(4-aminopiperidino)]-phenylaminol -6-morpholino-3-nitropyridine | 1.68(m, 2H), 1.97(m, 2H), 2.72(t, 2H), 3.15(m, 1H), 3.35(m, 2H), 3.69 (brm, 8H), 6.51( (d, 1H), 7.05(t, 1H), 7.33(d, 1H), 7.56 (dd, 1H), 7.91 (brm, 3H), 8.22(d, 1H), 10.52 (s, 1H) | 20~ 30 | 83 | 417.2 |

(continued)

| Compounds according to Examples 249 to 260, Solvent S = $CH_3CN$ (all examples) Starting material 2-{[3-fluoro-4-(4-BOC-aminopiperidino)]phenylamino}-6-chloro-3-nitropyridine compound obtained in Preparation Example 3-7-4 | | | | | | | |
|---|---|---|---|---|---|---|---|
| Ex | Amine | A | Compound | NMR (DMSO -d6) | T [°C] | Y [%] | M(+) |
| 256 | Amino-piperidine (1.5 equivalents) | 1.5 # | 2-{[3-fluoro-4-(4-aminopiperidino)]-phenylamino}-6-(4-aminopiperidino)-3-nitropyridine | 1.20(m, 4H), 1.40(m, 2H), 1.78(m, 4H), 2.66(m, 3H), 2.89(m, 1H), 3.18(m, 2H), 3.26(m, 2H), 4.29 (brm, 2H), 6.52(d, 1H), 7.02(t, 1H), 7.27 (dd, 1H), 7.59 (dd, 1H), 8.15(d, 1H), 10.56 (s, 1H) | 20~ 30 | 52 | 430.1 |

(continued)

| Compounds according to Examples 249 to 260, Solvent S = $CH_3CN$ (all examples) Starting material 2-{[3-fluoro-4-(4-BOC-aminopiperidino)]phenylamino}-6-chloro-3-nitropyridine compound obtained in Preparation Example 3-7-4 | | | | | | | |
|---|---|---|---|---|---|---|---|
| Ex | Amine | A | Compound | NMR (DMSO -d6) | T [°C] | Y [%] | M(+) |
| 257 | 3-amino-methylpyridine (1.5 equivalents) | 1.5 # | 2-{[3-fluoro-4-(4-aminopiperidino)]phenylamino}-6-[(3-pyridyl) methylamino] -3-nitropyridine | 1.67(m, 2H), 1.97(m, 2H), 3.69(m, 2H), 3.17(m, 1H), 3.32(m, 2H), 4.62(d, 2H), 6.23(d, 1H), 6.94(t, 1H). 7.21(d, 1H), 7.51(m, 1H), 7.80(d, 1H), 7.93(m, 2H), 8.15(d, 1H), 8.54(s, 1H), 8.86(t, 1H), 10.69 (s, 1H) | 60~70 | 74 | 424.1 |

(continued)

| Compounds according to Examples 249 to 260, Solvent S = $CH_3CN$ (all examples) Starting material 2-{[3-fluoro-4-(4-BOC-aminopiperidino)]phenylamino}-6-chloro-3-nitropyridine compound obtained in Preparation Example 3-7-4 | | | | | | | |
|---|---|---|---|---|---|---|---|
| Ex | Amine | A | Compound | NMR (DMSO -d6) | T [°C] | Y [%] | M(+) |
| 258 | 4-amino-methylpyridine (1.5 equivalents) | 1.5 # | 2-{[3-fluoro-4-(4-aminopiperidino)]-phenylamino} -6-[(4-pyridyl)methylamino] -3-nitropyridine | 1.39(m, 2H), 1.80(m, 2H), 2.63(m, 2H), 3.21(m, 3H), 4.57(d, 2H), 6.25(d, 1H), 6.85(t, 1H), 7.07(d, 1H). 7.21(d, 2H), 7.42 (dd, 1H), 8.15(d, 1H), 8.47(d, 2H), 8.94 (brs, 1H), 10.67 (s, 1H) | 60~ 70 | 71 | 438.1 |

(continued)

| Compounds according to Examples 249 to 260, Solvent S = $CH_3CN$ (all examples) Starting material 2-{[3-fluoro-4-(4-BOC-aminopiperidino)]phenylamino}-6-chloro-3-nitropyridine compound obtained in Preparation Example 3-7-4 | | | | | | | |
|---|---|---|---|---|---|---|---|
| Ex | Amine | A | Compound | NMR (DMSO -d6) | T [°C] | Y [%] | M(+) |
| 259 | 4-(2-aminoethyl)-morpholine (1.5 equivalents) | 1.5 # | 2-{[3-fluoro-4-(4-aminopiperidino)]-phenylamino}-6-[2-(morpholin-1-yl)-ethylamino]-3-nitropyridine | 1.39(m, 2H), 1.80(m, 2H), 2.33 (brm, 4H), 2.43(t, 2H), 2.66(t, 2H), 3.24(m, 3H), 3.46(m, 2H), 3.53(m, 4H). 6.14(d, 1H), 7.01(t, 1H), 7.31(d, 1H), 7.67(d, 1H), 8.06(d, 1H), 8.32(t, 1H), 10.76 (s, 1H) | 60~ 70 | 64 | 460.2 |

(continued)

| Compounds according to Examples 249 to 260, Solvent S = $CH_3CN$ (all examples) Starting material 2-{[3-fluoro-4-(4-BOC-aminopiperidino)]phenylamino}-6-chloro-3-nitropyridine compound obtained in Preparation Example 3-7-4 | | | | | | | |
|---|---|---|---|---|---|---|---|
| Ex | Amine | A | Compound | NMR (DMSO -d6) | T [°C] | Y [%] | M(+) |
| 260 | 4-(3-morpholine (1.5 equivalents) | 1.5 # | 2-{[3-fluoro-4-(4-aminopiperidino)]-phenylaminol -6-[(3-morpholin-1-yl)-propylamino]-3-nitropyridine | 1.39(m, 2H), 1.67(m, 2H), 1.79(m, 2H), 2.27 (brm, 6H), 2.66(m, 2H), 3.23(m, 3H), 3.35(m, 2H), 3.52 (brm, 4H). 6.11(d, 1H), 6.99(t, 1H), 7.30(d, 1H), 7.80(d, 1H), 8.05(d, 1H), 8.46(t, 1H), 10.84 (s, 1H) | 60~ 70 | 60 | 415.1 |

**Example 261**

**Preparation of 2-{[3-fluoro-4-(2-methylpiperidino)]phenylamino}-6-(methylamino)-3-nitropyridine**

To 10ml of acetonitrile were added 300mg (0.82 mmol) of the 2-{[3-fluoro-4-(2-methyl-piperidino)]phenylamino}-6-chloro-3-nitropyridine compound obtained in Preparation Example 3-8-3 and 5ml of a 40% methylamine-methanol solution, followed by reaction at room temperature for 4 hours. After the reaction was complete, the solvent was distilled under reduced pressure, followed by recrystallization from 5ml of methanol. The resulting solid was filtered and dried under vacuum at about 40°C to afford 270mg (yield: 92%) of the desired compound.

Mass (M+): 350.1

$^1$H-NMR(DMSO-$d_6$) (ppm): 0.85(d, 3H), 1.39(m, 2H), 1.60(m, 3H), 1.76(m, 1H), 2.73(m, 1H), 2.90(m, 3H), 3.01 (m, 1H), 3.26(m, 2H), 6.09(d, 1H), 7.07(m, 1H), 7.36(m, 1H), 7.85(m, 1H), 8.04(d, 1H), 8.33(m, 1H), 10.91(s, 1H).

**Examples 262 to 274**

In the same manner as in Example 261 and using amine compounds described in the following Table 22 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 22 were obtained.

The following Table 22 shows the name of compounds prepared in Examples 262 to 274, the name and equivalents of amine compounds used in the reaction, use/nonuse and equivalents of triethylamine in the reaction, the reaction temperature, the reaction solvent, yield, Mass analysis results and NMR analysis results.

**Table 22**

| Compounds according to Examples 262 to 274, Solvent S = $CH_3CN$ (all examples) Starting material 2-{[3-fluoro-4-(2-methylpiperidino)]phenylamino}-6-chloro-3-nitropyridine compound obtained in Preparation Example 3-8-3 | | | | | | | |
|---|---|---|---|---|---|---|---|
| Ex | Amine | A | Compound | NMR (DMSO-d6) | T [°C] | Y [%] | M(+) |
| 262 | Isopropylamine (excess) | X | 2-{[3-fluoro-4-(2-methylpiperidino)]-phenylamino}-6-(isopropylamino)-3-nitropyridine | 0.83(d, 3H), 1.17(d, 6H), 1.36(m, 2H), 1.57(m, 3H), 1.73(m, 1H), 2.71(m, 1H), 2.99(m, 1H), 3.24(m, 1H), 4.06(m, 1H), 6.08(m, 1H), 7.04(m, 1H), 7.25(m, 1H), 7.78(m, 1H), 8.03(m, 1H), 8.21(s, 1H), 10.86(s, 1H) | 20~ 30 | 97 | 389.1 |

(continued)

| Compounds according to Examples 262 to 274, Solvent S = $CH_3CN$ (all examples) Starting material 2-{[3-fluoro-4-(2-methylpiperidino)]phenylamino}-6-chloro-3-nitropyridine compound obtained in Preparation Example 3-8-3 | | | | | | | |
|---|---|---|---|---|---|---|---|
| Ex | Amine | A | Compound | NMR (DMSO-d6) | T [°C] | Y [%] | M(+) |
| 263 | Isobutylamine (excess) | X | 2-{[3-fluoro-4-(2-methylpiperidino)]-phenylamino}-6-(isobutylamino)-3-nitropyridine | 0.88(m, 9H), 1.40(m, 2H), 1.63(m, 3H), 1.77(m, 1H), 1.85(m, 1H), 2.74(m, 1H), 3.03(m, 1H), 3.16(m, 2H), 3.28(m, 1H), 6.14(d, 1H), 7.09(t, 1H), 7.25(m, 1H), 7.82(dd, 1H), 8.06(d, 1H), 8.44(t, 1H), 10.84(s, 1H) | 20~ 30 | 97 | 402.1 |

(continued)

| Compounds according to Examples 262 to 274, Solvent S = $CH_3CN$ (all examples) Starting material 2-{[3-fluoro-4-(2-methylpiperidino)]phenylamino}-6-chloro-3-nitropyridine compound obtained in Preparation Example 3-8-3 | | | | | | | |
|---|---|---|---|---|---|---|---|
| Ex | Amine | A | Compound | NMR (DMSO-d6) | T [°C] | Y [%] | M(+) |
| 264 | t-butylamine (excess) | X | 2-{[3-fluoro-4-(2-methylpiperidino)]-phenylamino}-6-(t-butylamino)-3-nitropyridine | 0.83(d, 3H), 1.22(s, 9H), 1.38(m, 2H), 1.58(m, 3H), 1.73(m, 1H), 2.71(m, 1H), 2.98(m, 1H), 3.26(m, 1H), 6.11(d, 1H), 7.07(m, 2H), 7.38(d, 1H), 7.80(m, 1H), 7.97(d, 1H), 10.56(s, 1H) | 20~ 30 | 88 | 402.1 |

(continued)

| Compounds according to Examples 262 to 274, Solvent S = $CH_3CN$ (all examples) Starting material 2-{[3-fluoro-4-(2-methylpiperidino)]phenylamino}-6-chloro-3-nitropyridine compound obtained in Preparation Example 3-8-3 | | | | | | | |
|---|---|---|---|---|---|---|---|
| Ex | Amine | A | Compound | NMR (DMSO-d6) | T [°C] | Y [%] | M(+) |
| 265 | 4-hydroxy-piperidine (1.5 equivalents) | 1.5 # | 2-{[3-fluoro-4-(2-methylpiperidino)]-phenylamino}-6-(4-hydroxypiperidino)-3-nitropyridine | 0.86(d, 3H), 1.38(m, 4H), 1.60(m, 2H), 1.65(m, 1H), 1.78(m, 3H), 2.75(m, 1H), 3.02(m, 1H), 3.28(m, 1H), 3.41(m, 2H), 3.80(m, 1H), 4.02(m, 2H), 4.79(d, 1H), 6.51(d, 1H), 7.09(t, 1H), 7.26(m, 1H), 7.57(dd, 1H), 8.15(d, 1H), 10.55(s, 1H) | 20~ 30 | 83 | 430.1 |

(continued)

| Compounds according to Examples 262 to 274, Solvent S = $CH_3CN$ (all examples) Starting material 2-{[3-fluoro-4-(2-methylpiperidino)]phenylamino}-6-chloro-3-nitropyridine compound obtained in Preparation Example 3-8-3 | | | | | | | |
|---|---|---|---|---|---|---|---|
| Ex | Amine | A | Compound | NMR (DMSO-d6) | T [°C] | Y [%] | M(+) |
| 266 | 2-methyl-2-imidazoline (2 equivalents) | 2 # | 2-{[3-fluoro-4-(2-methylpiperidino)]-phenylamino}-6-[(2-methyl-4,5-dihydro) imidazol-1-yl]-3-nitropyridine | 0.82(d, 3H), 1.41(m, 3H), 1.66(m, 2H), 1.78(m, 4H), 2.75(m, 1H) 3.06 (m, 1H), 3.23(m, 1H), 3.25(m, 1H), 6.14(d, 1H), 7.12(t, 1H), 7.41(m, 1H), 7.70(d, 1H), 7.98(m, 1H), 8.09(d, 1H), 8.42(m, 1H), 10.85(s, 1H) | 20~ 30 | 88 | 413.2 |

(continued)

| Compounds according to Examples 262 to 274, Solvent S = $CH_3CN$ (all examples) Starting material 2-{[3-fluoro-4-(2-methylpiperidino)]phenylamino}-6-chloro-3-nitropyridine compound obtained in Preparation Example 3-8-3 | | | | | | | |
|---|---|---|---|---|---|---|---|
| Ex | Amine | A | Compound | NMR (DMSO-d6) | T [°C] | Y [%] | M(+) |
| 267 | Piperazine (5 equivalents) | X | 2-{[3-fluoro-4-(2-methylpiperidino)]-phenylamino}-6-(piperazin-1-yl)-3-nitropyridine | 0.86(d, 3H), 1.40(m, 2H), 1.62(m, 3H), 1.77(m, 1H), 2.74(m, 5H), 3.02(m, 1H), 3.28(m, 1H), 3.62(m, 3H), 3.71(m, 1H), 6.46(d, 1H), 7.09(t, 1H), 7.26(m, 1H), 7.54(d, 1H), 8.16(d, 1H), 10.56(s, 1H) | 20~ 30 | 91 | 415.1 |

(continued)

| Compounds according to Examples 262 to 274, Solvent S = $CH_3CN$ (all examples) Starting material 2-{[3-fluoro-4-(2-methylpiperidino)]phenylamino}-6-chloro-3-nitropyridine compound obtained in Preparation Example 3-8-3 | | | | | | | |
|---|---|---|---|---|---|---|---|
| Ex | Amine | A | Compound | NMR (DMSO-d6) | T [°C] | Y [%] | M(+) |
| 268 | 1-methyl-piperazine (1.5 equivalents) | 1.5 # | 2-{[3-fluoro-4-(2-methylpiperidino)]-phenylamino}-6-(4-methylpiperazin-1-yl)-3-nitropyridine | 0.86(d, 3H), 1.40(m, 2H), 1.60(m, 2H), 1.78(m, 1H), 2019(s, 3H), 2.36 (brm, 4H), 2.75(m, 1H), 3.03(m, 1H), 3.28(m, 1H), 3.41(m, 1H), 3.67 (brm, 4H), 6.48(d, 1H), 7.09(t, 1H), 7.26(d, 1H), 7.39(m, 1H), 7.53(d, 1H), 8.15(d, 1H), 10.54(s, 1H) | 20~ 30 | 97 | 429.3 |

(continued)

| Compounds according to Examples 262 to 274, Solvent S = $CH_3CN$ (all examples) Starting material 2-{[3-fluoro-4-(2-methylpiperidino)]phenylamino}-6-chloro-3-nitropyridine compound obtained in Preparation Example 3-8-3 | | | | | | | |
|---|---|---|---|---|---|---|---|
| Ex | Amine | A | Compound | NMR (DMSO-d6) | T [°C] | Y [%] | M(+) |
| 269 | Morpholine (3 equivalents) | X | 2-{[3-fluoro-4-(2-methylpiperidino)]-phenylamino}-6-morpholino-3-nitropyridine | 0.87(d, 3H), 1.40(m, 2H), 1.61(m, 3H), 1.78(m, 1H), 2.75(m, 1H), 3.04(m, 1H), 3.28(m, 2H), 3.68 (brm, 8H), 6.49(d, 1H), 7.10(m, 1H), 7.31(m, 1H), 7.54(dd, 1H), 8.21(d, 1H), 10.54(s, 1H) | 20~ 30 | 97 | 416.3 |

(continued)

| Compounds according to Examples 262 to 274, Solvent S = $CH_3CN$ (all examples) Starting material 2-{[3-fluoro-4-(2-methylpiperidino)]phenylamino}-6-chloro-3-nitropyridine compound obtained in Preparation Example 3-8-3 | | | | | | | |
|---|---|---|---|---|---|---|---|
| Ex | Amine | A | Compound | NMR (DMSO-d6) | T [°C] | Y [%] | M(+) |
| 270 | 4-amino-piperidine (1.5 equivalents) | 1.5 # | 2-{[3-fluoro-4-(2-methylpiperidino)]-phenylamino}-6-(4-aminopiperidino)-3-nitropyridine | 0.88(d, 3H), 1.28(m, 3H), 1.39(m, 2H), 1.66(m, 3H), 1.83(m, 4H), 2.75(m, 1H), 3.02(m, 2H), 3.14(m, 2H), 3.29(m, 1H) 4.35 (brm, 2H), 6.53(d, 1H), 7.10(t, 1H), 7.27(m, 1H), 7.56(dd, 1H), 8.17(d, 1H), 10.56(s, 1H) | 20~ 30 | 91 | 429.2 |

(continued)

| Compounds according to Examples 262 to 274, Solvent S = $CH_3CN$ (all examples) Starting material 2-{[3-fluoro-4-(2-methylpiperidino)]phenylamino}-6-chloro-3-nitropyridine compound obtained in Preparation Example 3-8-3 | | | | | | | |
|---|---|---|---|---|---|---|---|
| Ex | Amine | A | Compound | NMR (DMSO-d6) | T [°C] | Y [%] | M(+) |
| 271 | 4-amino-methylpyridine (1.5 equivalents) | 1.5 # | 2-{[3-fluoro-4-(2-methylpiperidino)]-phenylamino}-6-[(4-pyridyl)methylamino] -3-nitropyridine | 0.83(d, 3H), 1.40(m, 2H), 1.63(m, 3H), 1.76(m, 1H), 2.71(m, 1H), 2.99(m, 1H), 3.25(m, 1H), 4.57(d, 2H), 6.25(d, 1H), 6.94(t, 1H), 7.12(m, 1H), 7.20(m, 2H), 7.43(d, 1H), 8.15((d, 1H), 8.46(d, 1H), 8.86(m, 1H), 10.68(s, 1H) | 60~ 70 | 86 | 437.2 |

(continued)

| Compounds according to Examples 262 to 274, Solvent S = $CH_3CN$ (all examples) Starting material 2-{[3-fluoro-4-(2-methylpiperidino)]phenylamino}-6-chloro-3-nitropyridine compound obtained in Preparation Example 3-8-3 | | | | | | | |
|---|---|---|---|---|---|---|---|
| Ex | Amine | A | Compound | NMR (DMSO-d6) | T [°C] | Y [%] | M(+) |
| 272 | 1-(3-amino-propyl)-imidazole (1.5 equivalents) | 1.5 # | 2-{[3-fluoro-4-(2-methylpiperidino)]-phenylaminol-6-[(3-imidazol-1-yl)propylamino]-3-nitropyridine | 0.86(d, 3H), 1.40(m, 2H), 1.63(m, 3H), 1.76(m, 1H), 2.00(t, 2H), 2.75(m, 1H), 3.02(m, 1H), 3.28(m, 3H), 4.00(t, 2H), 6.12(d, 1H), 6.87(m, 1H), 7.12(m, 2H), 7.34(m, 1H), 7.59(m, 1H), 7.71(d, 1H), 8.09(d, 1H), 8.37(m, 1H), 10.82(s, 1H) | 60~ 70 | 96 | 454.2 |

(continued)

| Compounds according to Examples 262 to 274, Solvent S = $CH_3CN$ (all examples) Starting material 2-{[3-fluoro-4-(2-methylpiperidino)]phenylamino}-6-chloro-3-nitropyridine compound obtained in Preparation Example 3-8-3 | | | | | | | |
|---|---|---|---|---|---|---|---|
| Ex | Amine | A | Compound | NMR (DMSO-d6) | T [°C] | Y [%] | M(+) |
| 273 | 4-(2-amino-ethyl)-morpholine (1.5 equivalents) | 1.5 # | 2-{[3-fluoro-4-(2-methylpiperidino)]-phenylamino}-6-[2-(morpholin-1-yl)ethylamino]-3-nitropyridine | 0.87(d, 3H) 1.41 (m, 2H), 1.64(m, 3H), 1.77(m, 1H), 2.34(m, 4H), 2.45(t, 2H), 2.74(m, 1H), 3.03(m, 1H), 3.28(m, 1H), 3.48(m, 3H), 3.53(m, 3H), 6.14(d, 1H), 7.09(t, 1H), 7.32(m, 1H), 7.68(d, 1H), 8.07(d, 1H), 8.29(m, 1H), 10.79(s, 1H) | 60~ 70 | 84 | 459.1 |

(continued)

| Compounds according to Examples 262 to 274, Solvent S = $CH_3CN$ (all examples) Starting material 2-{[3-fluoro-4-(2-methylpiperidino)]phenylamino}-6-chloro-3-nitropyridine compound obtained in Preparation Example 3-8-3 | | | | | | | |
|---|---|---|---|---|---|---|---|
| Ex | Amine | A | Compound | NMR (DMSO-d6) | T [°C] | Y [%] | M(+) |
| 274 | 4-(3-amino-propyl)-morpholine (1.5 equivalents) | 1.5 # | 2-{[3-fluoro-4-(2-methylpiperidino)]- phenylamino}-6-[(3- morpholin-1-yl) propylamino]-3-nitropyridine | 0.86(d, 3H), 1.38(m, 2H), 1.60(m, 2H), 1.68(m, 3H), 1.74(m, 1H), 2.30(m, 6H), 2.75(m, 1H), 3.02(m, 1H), 3.28(m, 1H), 3.38(m, 2H), 3.52(m, 4H), 6.10(d, 1H), 7.09(t, 1H), 7.31(m, 1H), 7.80(m, 1H), 8.06(d, 1H), 8.37(m, 1H), 10.86(s, 1H) | 60~ 70 | 65 | 473.1 |

**Example 275**

**Preparation of 2-{[3-fluoro-4-(3-hydroxymethylpiperidino)phenylamino]-6-(methylamino)-3-nitropyridine**

To 10ml of acetonitrile were added 200mg (0.53 mmol) of the 2-{[3-fluoro-4-(3-hydroxy-methylpiperidino)] phenylaminol-6-chloro-3-nitropyridine compound obtained in Preparation Example 3-9-3 and 5ml of a 40% methylamine-methanol solution, followed by reaction at room temperature for 4 hours. After the reaction was complete, the solvent was distilled under reduced pressure, followed by recrystallization from 5ml of methanol. The resulting solid was filtered and dried under vacuum at about 40°C to afford 136mg (yield: 68%) of the desired compound.

Mass (M+): 376.2

$^1$H-NMR(DMSO-$d_6$) (ppm): 1.05(m, 1H), 1.61(m, 1H), 1.72(m, 3H), 2.36(t, 1H), 2.60(td, 1H), 2.91(d, 3H), 3.25 (m, 2H), 3.37(m, 2H), 4.51(t, 1H), 6.11(d, 1H), 7.00(t, 1H), 7.39(dd, 1H), 7.85(dd, 1H), 8.06(d, 1H) 8.33(m, 1H), 10.89 (s, 1H).

**Examples 276 to 288**

In the same manner as in Example 275 and using amine compounds described in the following Table 23 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 23 were obtained.

The following Table 23 shows the name of compounds prepared in Examples 276 to 288, the name and equivalents of amine compounds used in the reaction, use/nonuse and equivalents of triethylamine in the reaction, the reaction temperature, the reaction solvent, yield, Mass analysis results and NMR analysis results.

**Table 23**

| Compounds according to Examples 276 to 288, Solvent S = $CH_3CN$ (all examples) Starting material 2-{[3-fluoro-4-(3-hydroxymethylpiperidino)]phenylamino}-6-chloro-3-nitropyridine compound obtained in Preparation Example 3-9-3 | | | | | | | |
|---|---|---|---|---|---|---|---|
| Ex | Amine | A | Compound | NMR (DMSO-d6) | T [°C] | Y [%] | M(+) |
| 276 | Isopropylamine (excess) | | 2-{[3-fluoro-4-(3-hydroxymethylpiperidino)]phenylamino}-6-(isopropylamino)-3-nitropyridine | 1.06(m, 1H), 1.21 (d, 6H), 1.60(m, 1H), 1.73 (m, 3H), 2.37(t, 1H), 2.60 (td, 1H), 3.25(m, 2H), 3.38 (m, 2H), 4.10(m, 1H), 4.51 (t, 1H), 6.09(d, 1H), 7.02 (t, 1H) 7.32(d, 1H), 7.85 (d, 1H), 8.06(d, 1H), 8.24 (d, 1H), 10.88(s, 1H) | 20~ 30 | 79 | 404.2 |

(continued)

| Compounds according to Examples 276 to 288, Solvent S = $CH_3CN$ (all examples) Starting material 2-{[3-fluoro-4-(3-hydroxymethylpiperidino)]phenylamino}-6-chloro-3-nitropyridine compound obtained in Preparation Example 3-9-3 | | | | | | | |
|---|---|---|---|---|---|---|---|
| Ex | Amine | A | Compound | NMR (DMSO-d6) | T [°C] | Y [%] | M(+) |
| 277 | Isobutylamine (excess) | X | 2-{[3-fluoro-4-(3-hydroxymethyl-hydroxymethyl-piperidino)]phenyl-amino}-6-(isobutyl-amino)-3-nitro-pyridine | 0.90(d, 6H), 1.06 (m, 1H), 1.62(m, 1H), 1.73 (m, 3H), 1.88(m, 4H), 2.36 (t, 1H), 2.59(td, 1H), 3.18 (t, 1H), 3.25(m, 2H), 3.36 (t, 1H), 4.51(t, 1H), 6.14 (d, 1H), 6.99(t, 1H), 7.26 (d, 1H), 7.86(d, 1H), 8.07 (d, 1H), 8.46(t, 1H), 10.86 (s, 1H) | 20~ 30 | 72 | 418.2 |

(continued)

| Compounds according to Examples 276 to 288, Solvent S = $CH_3CN$ (all examples) Starting material 2-{[3-fluoro-4-(3-hydroxymethylpiperidino)]phenylamino}-6-chloro-3-nitropyridine compound obtained in Preparation Example 3-9-3 | | | | | | | |
|---|---|---|---|---|---|---|---|
| Ex | Amine | A | Compound | NMR (DMSO-d6) | T [°C] | Y [%] | M(+) |
| 278 | t-butylamine (excess) | X | 2-{[3-fluoro-4-(3-hydroxymethyl-piperidino)]phenylamino}-6-(t-butyl-amino)-3-nitro-pyridine | 1.03(m, 1H), 1.28 (s, 9H), 1.63(m, 1H), 1.73 (m, 3H), 2.36(t, 1H), 2.59 (td, 1H), 3.27(m, 2H), 3.38 (m, 2H), 4.52(t, 1H), 6.13 (d, 1H), 7.00(t, 1H), 7.15 (d, 1H) 7.50(d, 1H), 7.85 (s, 1H), 8.02(d, 1H) 10.64 (s, 1H) | 20~ 30 | 81 | 418.3 |

(continued)

| Compounds according to Examples 276 to 288, Solvent S = $CH_3CN$ (all examples) Starting material 2-{[3-fluoro-4-(3-hydroxymethylpiperidino)]phenylamino}-6-chloro-3-nitropyridine compound obtained in Preparation Example 3-9-3 | | | | | | | |
|---|---|---|---|---|---|---|---|
| Ex | Amine | A | Compound | NMR (DMSO-d6) | T [°C] | Y [%] | M(+) |
| 279 | 4-hydroxy-piperidine (1.5 equivalents) | 1.5 # | 2-{[3-fluoro-4-(3-hydroxymethyl-piperidino)]phenyl-amino}-6-(4-hydroxy-piperidino}-3-nitropyridine | 1.06(m, 1H), 1.42 (m, 2H), 1.62(m, 1H), 1.73 (m, 2H), 1.79(m, 3H), 2.37 (t, 1H), 2.60(td, 1H), 3.30 (m, 2H), 3.39(m, 4H), 3.81 (m, 1H),) 4.05(brm, 2H), 4.51 (t, 1H), 4.82(dd, 1H) 6.53 (d, 1H), 7.02(t, 1H), 7.28 (d, 1H), 7.60(d, 1H), 8.17 (d, 1H), 10.57(s, 1H) | 20~ 30 | 74 | 446.3 |

(continued)

| Compounds according to Examples 276 to 288, Solvent S = $CH_3CN$ (all examples) Starting material 2-{[3-fluoro-4-(3-hydroxymethylpiperidino)]phenylamino}-6-chloro-3-nitropyridine compound obtained in Preparation Example 3-9-3 | | | | | | | |
|---|---|---|---|---|---|---|---|
| Ex | Amine | A | Compound | NMR (DMSO-d6) | T [°C] | Y [%] | M(+) |
| 280 | 2-methyl-2-imidazoline (2 equivalents) | 2 # | 2-{[3-fluoro-4-(3-hydroxymethyl-piperidino)]phenyl-amino}-6-[(2-methyl-4,5-dihydro)imidazol-1-yl]-3-nitropyridine | 1.04(m, 1H), 1.63 (m, 1H), 1.74(m, 3H), 1.80 (s, 3H), 2.37(t, 1H), 2.60 (td, 1H), 3.26(m, 4H), 3.42 (m, 4H), 4,51(t, 1H),6.12 (d,1H), 7.02(t, 1H), 7.42 (d, 1H), 7.68(d, 1H), 7.93 (t, 1H), 8.09(d, 1H), 8.38 (t, 1H), 10.84(s, 1H) | 60~70 | 76 | 429.3 |

(continued)

| Compounds according to Examples 276 to 288, Solvent S = $CH_3CN$ (all examples) Starting material 2-{[3-fluoro-4-(3-hydroxymethylpiperidino)]phenylamino}-6-chloro-3-nitropyridine compound obtained in Preparation Example 3-9-3 | | | | | | | |
|---|---|---|---|---|---|---|---|
| Ex | Amine | A | Compound | NMR (DMSO-d6) | T [°C] | Y [%] | M(+) |
| 281 | Piperazine (5 equivalents) | X | 2-{[3-fluoro-4-(3-hydroxymethyl-piperidino)]phenyl-amino}-6-(piperazin-1-yl)-3-nitropyridine | 1.05(m, 1H), 1.60 (m, 1H), 1.73(m, 3H), 2.36 (t, 1H), 2.59(td, 1H), 2.75 (t, 4H), 3.24(m, 2H), 3.36 (m, 2H), 3.64(brm, 4H), 4.51 (t, 1H), 6.48(d, 1H). 6.99 (t, 1H), 7.28(d, 1H), 7.56 (dd, 1H), 8.16(d, 1H), 10.57 (s, 1H) | 20~30 77 | | 431.3 |

(continued)

| Compounds according to Examples 276 to 288, Solvent S = $CH_3CN$ (all examples) Starting material 2-{[3-fluoro-4-(3-hydroxymethylpiperidino)]phenylamino}-6-chloro-3-nitropyridine compound obtained in Preparation Example 3-9-3 | | | | | | | |
|---|---|---|---|---|---|---|---|
| Ex | Amine | A | Compound | NMR (DMSO-d6) | T [°C] | Y [%] | M(+) |
| 282 | 1-methyl-piperazine (1.5 equivalents) | 1.5 # | 2-{[3-fluoro-4-(3-hydroxymethyl-piperidino)]phenyl-amino}-6-(4-methyl-piperazin-1-yl)-3-nitropyridine | 1.04(m, 1H), 1.61 (m, 1H), 1.74(m, 3H), 2.20 (s, 3H), 2.38(t+m, 5H), 2.59 (td, 1H), 3.23(m, 2H), 3.37 (m, 2H), 3.71(brm, 4H), 4.51 (t, 1H), 6.51(d, 1H), 7.02 (t, 1H), 7.28(d, 1H), 7.54 (dd, 1H), 8.18(d, 1H), 10.53 (s, 1H) | 20~ 30 | 63 | 445.3 |

(continued)

| Compounds according to Examples 276 to 288, Solvent S = $CH_3CN$ (all examples) Starting material 2-{[3-fluoro-4-(3-hydroxymethylpiperidino)]phenylamino}-6-chloro-3-nitropyridine compound obtained in Preparation Example 3-9-3 | | | | | | | |
|---|---|---|---|---|---|---|---|
| Ex | Amine | A | Compound | NMR (DMSO-d6) | T [°C] | Y [%] | M(+) |
| 283 | Morpholine (3 equivalents) | X | 2-{[3-fluoro-4-(3-piperidino)] phenylamino}-6-morpho-amino}-6-morpholino-3-nitropyridine | 1.06(m, 1H), 1.61 (m, 1H), 1.74(m, 3H), 2.36 (t, 1H), 2.59(td, 1H) 3.24 (m 2H), 3.36(m, 2H), 3.68 (brm, 8H), 4.51(t, 1H), 6.50 (d, 1H), 7.01(t, 1H), 7.32 (dd, 1H), 7.53(dd, 1H), 8.21 (d, 1H), 10.53(s, 1H) | 20~30 | 80 | 432.3 |

(continued)

| Compounds according to Examples 276 to 288, Solvent S = $CH_3CN$ (all examples) Starting material 2-{[3-fluoro-4-(3-hydroxymethylpiperidino)]phenylamino}-6-chloro-3-nitropyridine compound obtained in Preparation Example 3-9-3 | | | | | | | |
|---|---|---|---|---|---|---|---|
| Ex | Amine | A | Compound | NMR (DMSO-d6) | T [°C] | Y [%] | M(+) |
| 284 | 4-amino-piperidine equivalents) | 1.5 # | 2-{[3-fluoro-4-(3-hydroxymethyl-piperidino)]phenyl-amino}-6-(4-amino-piperidino-3-nitro-pyridine | 1.07(m, 1H), 1.25 (m, 3H), 1.60(m, 1H), 1.73 (m, 2H), 1.79(m, 3H), 2.37 (t, 1H), 2.40(m, 1H), 2.61 (td, 1H), 2.94(m, 1H) 3.21 (t, 2H), 3.25(m, 2H), 3.36 (m, 2H), 4.28(brm, 2H), 4.52 (t, 1H), 6.53(d, 1H), 7.02 (t, 1H), 7.28(d, 1H), 7.60 (dd, 1H), 8.17(d, 1H), 10.57 (s, 1H) | 20~30 | 64 | 445.3 |

(continued)

| Compounds according to Examples 276 to 288, Solvent S = $CH_3CN$ (all examples) Starting material 2-{[3-fluoro-4-(3-hydroxymethylpiperidino)]phenylamino}-6-chloro-3-nitropyridine compound obtained in Preparation Example 3-9-3 | | | | | | | |
|---|---|---|---|---|---|---|---|
| Ex | Amine | A | Compound | NMR (DMSO-d6) | T [°C] | Y [%] | M(+) |
| 285 | 3-amino-methylpyridine (1.5 equivalents) | 1.5 # | 2-{[3-fluoro-4-(3-hydroxymethyl-piperidino)]phenyl-amino}-6-[(3-pyridyl)-methylamino]-3-nitropyridine | 1.04(m, 1H), 1.60 (m, 1H), 1.73(m, 3H), 2.34 (t, 1H), 2.58(td, 1H), 3.20 (m, 1H), 3.28(m, 1H), 3.36 (m, 2H), 4.51(t, 1H), 4.58 (d, 2H), 6.20(d, 1H), 6.93 (t, 1H), 7.22(dd, 1H), 7.32 (dd, 1H), 7.55(dd, 1H), 6.72 (d, 1H), 8.13(d, 1H), 8.46 (m, 2H), 8.81(t, 1H), 10.72 (s.1H) | 60~70 | 75 | 453.3 |

(continued)

| Compounds according to Examples 276 to 288, Solvent S = $CH_3CN$ (all examples) Starting material 2-{[3-fluoro-4-(3-hydroxymethylpiperidino)]phenylamino}-6-chloro-3-nitropyridine compound obtained in Preparation Example 3-9-3 | | | | | | | |
|---|---|---|---|---|---|---|---|
| Ex | Amine | A | Compound | NMR (DMSO-d6) | T [°C] | Y [%] | M(+) |
| 286 | 4-amino-methylpyridine (1.5 equivalents) | 1.5 # | 2-{[3-fluoro-4-(3-hydroxymethyl-piperidino)]phenyl-amino}-6-[(4-pyridyl)-methylamino]-3-nitropyridine | 1.06(m, 1H), 1.61 (m, 1H), 1.74(m, 3H), 2.34 (t, 1H), 2.51(td, 1H), 3.19 (m, 1H), 3.28(m, 1H), 3.37 (m, 1H), 4.51(t, 1H) 4.58 (t, 1H), 6.25(d, 1H), 6.87 (t, 1H), 7.11(d, 1H), 7.21 (d, 2H), 7.43(d, 1H), 8.16 (d, 1H), 8.47(d, 2H), 8.87 (t, 1H), 10.68(s. 1H) | 60~70 | 55 | 453.2 |

(continued)

| Compounds according to Examples 276 to 288, Solvent S = $CH_3CN$ (all examples) Starting material 2-{[3-fluoro-4-(3-hydroxymethylpiperidino)]phenylamino}-6-chloro-3-nitropyridine compound obtained in Preparation Example 3-9-3 | | | | | | | |
|---|---|---|---|---|---|---|---|
| Ex | Amine | A | Compound | NMR (DMSO-d6) | T [°C] | Y [%] | M(+) |
| 287 | 2-2-amino-ethylpyridine (1.5 equivalents) | 1.5 # | 2-{[3-fluoro-4-(3-hydroxymethyl-piperidino)]phenylamino}-6-[2-(2-pyridyl)-ethylamino]-3-nitro-pyridine | 1.06(m, 1H), 1.62 (m, 1H), 1.74(m, 3H), 2.34 (t, 1H), 2.56(td, 1H), 3.01 (t, 2H), 3.20(m, 1H), 3.24 (m, 1H), 3.20(m, 1H), 3.24 (m, 1H), 3.36(m, 2H), 3.73 (q, 2H), 4.53(t, 1H), 6.10 (d, 1H), 4.53(t, 1H), 6.10 (d, 1H), 6.92(t, 1H), 7.44 (dd, 1H), 7.23 (t, 1H), (dd, 1H), 7.67 (s, 1H), 7.70(d, 1H), 8.07 (d, 1H), 8.43(t, 1H), 8.52 (d, 1H), 10.82(s. 1H) | 60~70 | 81 | 467.3 |

(continued)

| Compounds according to Examples 276 to 288, Solvent S = $CH_3CN$ (all examples) Starting material 2-{[3-fluoro-4-(3-hydroxymethylpiperidino)]phenylamino}-6-chloro-3-nitropyridine compound obtained in Preparation Example 3-9-3 | | | | | | | |
|---|---|---|---|---|---|---|---|
| Ex | Amine | A | Compound | NMR (DMSO-d6) | T [°C] | Y [%] | M(+) |
| 288 | Cyclopropyl-amine (excess) | X | 2-{[3-fluoro-4-(3-hydroxymethyl-piperidino)]phenyl-amino}-6-(cyclo-propylammo)-3-nitropyridine | 0.59(m, 2H), 0.84 (m, 2H), 1.06(m, 1H), 1.61 (m, 1H), 1.73(m, 3H), 2.36 (t, 1H), 2.59(td, 1H), 2.81 (m, 1H), 3.24(m, 2H), 3.38 (m, 2H), 4.51(t, 1H), 6.08 (d, 1H), 4.51(t, 1H), 6.08 (d, 1H), 7.00(t, 1H), 7.43 (d, 1H), 7.00(t, 1H), 7.43 (d, 1H), 8.09(d, 1H), 8.22 (d, 1H), 8.51(s, 1H), 10.91 (s, 1H) | 20~30 | 88 | 402.2 |

**Example 289**

**Preparation of 2-{[3-fluoro-4-(4-carbamoylpiperidino)]phenylamino}-6-(methylamino)-3-nitropyridine**

To 10ml of acetonitrile were added 300mg (0.53 mmol) of the 2-{[3-fluoro-4-(4-carbamoylpiperidino)]phenylamino}-6-chloro-3-nitropyridine compound obtained in Preparation Example 3-10-3 and 5ml of a 40% methylamine-methanol solution, followed by reaction at room temperature for 4 hours. After the reaction was complete, the solvent was distilled under reduced pressure, followed by recrystallization from 5ml of methanol. The resulting solid was filtered and dried under vacuum at about 40°C to afford 270mg (yield: 93%) of the desired compound.

Mass (M+): 389.2

$^1$H-NMR(DMSO-$d_6$) (ppm): 1.73(m, 2H), 1.79(m, 2H), 2.20(m, 1H), 2.64(m, 2H), 2.90(d, 3H), 3.36(m, 2H), 6.10 (d, 1H), 6.80(d, 1H), 7.02(t, 1H), 7.30(s, 1H), 7.37(t, 1H), 7.85(dd, 1H), 8.05(d, 1H), 8.32(d, 1H), 10.90(s, 1H) .

**Examples 290 to 300**

In the same manner as in Example 289 and using amine compounds described in the following Table 24 in

place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 24 were obtained.

The following Table 24 shows the name of compounds prepared in Examples 290 to 300, the name and equivalents of amine compounds used in the reaction, use/nonuse and equivalents of triethylamine in the reaction, the reaction temperature, the reaction solvent, yield, Mass analysis results and NMR analysis results.

**Table 24**

| Compounds according to Examples 290 to 300, Solvent S = $CH_3CN$ (all examples) Starting material 2-{[3-fluoro-4-(4-carbamoylpiperidino)]phenylamino}-6-chloro-3-nitropyridine compound obtained in Preparation Example 3-10-3 | | | | | | | |
|---|---|---|---|---|---|---|---|
| Ex | Amine | A | Compound | NMR (DMSO -d6) | T [°C] | Y [%] | M(+) |
| 290 | Isopropylamine (excess) | X | 2-{[3-fluoro-4-(4-carbamoylpiperidino)] phenylaminol-6-(isopropylamino)-3-nitropyridine | 1.19(d, 6H), 1.75(m, 5H), 2.22(m, 1H), 2.64(t, 2H), 3.32(m, 1H) 4.10(q, 1H), 6.10(d, 1H), 6.79(s, 1H), 7.00(t, 1H), 7.30(s, 2H), 7.84(d, 1H), 8.06(d, 1H), 8.23(d, 1H), 10.87 (s, 1H) | 20~ 30 | 88 | 417.2 |

(continued)

| Compounds according to Examples 290 to 300, Solvent S = $CH_3CN$ (all examples) Starting material 2-{[3-fluoro-4-(4-carbamoylpiperidino)]phenylamino}-6-chloro-3-nitropyridine compound obtained in Preparation Example 3-10-3 | | | | | | | |
|---|---|---|---|---|---|---|---|
| Ex | Amine | A | Compound | NMR (DMSO -d6) | T [°C] | Y [%] | M(+) |
| 291 | Isobutylamine (excess) excess | X | 2-{3-fluoro-4-(4-carbamoylpiperidino)]phenylaminol-6-(isobutylamino)-3-nitropyridine | 0.89 (d+m, 7H), 1.72(m, 2H), 1.73(m, 3H), 1.81(m, 1H), 2.22(m, 1H), 2.62(t, 2H), 3.18(t, 2H), 6.15(d, 1H), 6.79(s, 1H), 7.02(t, 1H), 7.23(d, 1H), 7.29(s, 1H), 7.84(d, 1H) 8.05(d, 1H), 8.44(t, 1H), 10.86 (s, 1H) | 20~ 30 | 87 | 431.3 |

(continued)

| Compounds according to Examples 290 to 300, Solvent S = $CH_3CN$ (all examples) Starting material 2-{[3-fluoro-4-(4-carbamoylpiperidino)]phenylamino}-6-chloro-3-nitropyridine compound obtained in Preparation Example 3-10-3 | | | | | | | |
|---|---|---|---|---|---|---|---|
| Ex | Amine | A | Compound | NMR (DMSO -d6) | T [°C] | Y [%] | M(+) |
| 292 | t-butylamine (excess) | X | 2-{3-fluoro-4-(4-carbamoylpiperidino)] phenylamino}-6-(t-butylamino)-3-nitropyridine | 1.28(s, 9H), 1.47(m, 1H), 1.62(m, 1H), 1.75(m, 1H), 1.85(m, 1H), 2.58(t, 1H), 2.68(t, 1H), 3.24(m, 2H), 3.30(m, 1H), 6.13(d, 1H), 6.88(m, 1H), 7.03(t, 1H), 7.15(m, 1H), 7.37(m, 1H), 7,50(d, 1H), 7.86(m, 1H), 8.00(d, 1H), 10.64 (s, 1H) | 20~ 30 | 81 | 431.2 |

(continued)

| Compounds according to Examples 290 to 300, Solvent S = $CH_3CN$ (all examples) Starting material 2-{[3-fluoro-4-(4-carbamoylpiperidino)]phenylamino}-6-chloro-3-nitropyridine compound obtained in Preparation Example 3-10-3 | | | | | | | |
|---|---|---|---|---|---|---|---|
| Ex | Amine | A | Compound | NMR (DMSO -d6) | T [°C] | Y [%] | M(+) |
| 293 | 4-hydroxy-piperidine (1.5 equivalents) | 15 # | 2-{[3-fluoro-4-(4-carbamoylpiperidino)] phenylamino}-6-(4-hydroxypiperidino)-3-nitropyridine | 1.40(m, 2H), 1.70(m, 2H), 1.81(m, 4H), 2.22(m, 1H), 2.65(m, 2H), 3.35(m, 1H), 3.43(m, 2H), 3.81(m, 1H), 4.03(m, 2H), 4.81(d, 1H), 6.52(d, 1H), 6.80(m, 1H), 7.02 (m, 1H), 7.28(m, 2H), 7,60 (dd, 1H), 8.16(d, 1H), 10.56 (s, 1H) | 20~ 30 | 83 | 459.1 |

(continued)

| Compounds according to Examples 290 to 300, Solvent S = $CH_3CN$ (all examples) Starting material 2-{[3-fluoro-4-(4-carbamoylpiperidino)]phenylamino}-6-chloro-3-nitropyridine compound obtained in Preparation Example 3-10-3 | | | | | | | |
|---|---|---|---|---|---|---|---|
| Ex | Amine | A | Compound | NMR (DMSO -d6) | T [°C] | Y [%] | M(+) |
| 294 | Piperazine (5 equivalents) | X | 2-{[3-fluoro-4-(4-carbamoylpiperidino)] phenylamino}-6-(piperazin-1-yl)-3-nitropyridine | 1.72(m, 2H), 1.79(m, 2H), 2.19(m, 1H), 2.65(m, 3H), 2.76(m, 4H), 3.32(m, 2H), 3.65(m, 4H), 6.48(d, 1H), 6.80(s, 1H), 7.03(t, 1H), 7.30(m, 2H), 7.57(d, 1H), 8.16(d, 1H), 10.57 (s, 1H), | 20~ 30 | 94 | 444.2 |

(continued)

| Compounds according to Examples 290 to 300, Solvent S = $CH_3CN$ (all examples) Starting material 2-{[3-fluoro-4-(4-carbamoylpiperidino)]phenylamino}-6-chloro-3-nitropyridine compound obtained in Preparation Example 3-10-3 | | | | | | | |
|---|---|---|---|---|---|---|---|
| Ex | Amine | A | Compound | NMR (DMSO -d6) | T [°C] | Y [%] | M(+) |
| 295 | 1-methyl-piperazine (1.5 equivalents) | 1.5 # | 2-{[3-fluoro-4-(4-carbamoylpiperidino)]phenylamino}]-6-(4-methylpiperazin-1-yl)-3-nitropyridine | 1.72(m, 2H), 1.79(m, 2H), 2.21(m, 4H), 2.39(m, 4H), 2.65(t, 3H), 3.36(m, 1H), 3.71(m, 4H), 6.51(d, 1H), 6.80(m, 1H), 7.02(m, 1H), 7.30(m, 2H), 7.56 (dd, 1H), 8.18(d, 1H), 10.53 (s, 1H) | 20~ 30 | 88 | 458.1 |

(continued)

| Compounds according to Examples 290 to 300, Solvent S = $CH_3CN$ (all examples) Starting material 2-{[3-fluoro-4-(4-carbamoylpiperidino)]phenylamino}-6-chloro-3-nitropyridine compound obtained in Preparation Example 3-10-3 | | | | | | | |
|---|---|---|---|---|---|---|---|
| Ex | Amine | A | Compound | NMR (DMSO -d6) | T [°C] | Y [%] | M(+) |
| 296 | Morpholine (3 equivalents) (3 equivalents) | X | 2-1[3-fluoro-4-(4-carbamoylpiperidino)] phenylamino}-6-morpholino-3-nitropyridine | 1.72(m, 2H), 1.79(m, 2H), 2.22(m, 1H), 2.25(m, 2H), 2.65(m, 2H), 3.69(m, 8H), 8.49(d, 1H), 6.80(s, 1H), 7.03(t, 1H), 7.29(m, 2H), 7.53(d, 1H), 8.21(d, 1H), 10.53 (s, 1H) | 20~ 30 | 88 | 445.2 |

(continued)

| Compounds according to Examples 290 to 300, Solvent S = $CH_3CN$ (all examples) Starting material 2-{[3-fluoro-4-(4-carbamoylpiperidino)]phenylamino}-6-chloro-3-nitropyridine compound obtained in Preparation Example 3-10-3 | | | | | | | |
|---|---|---|---|---|---|---|---|
| Ex | Amine | A | Compound | NMR (DMSO -d6) | T [°C] | Y [%] | M(+) |
| 297 | 4-amino-piperidine (1.5 equivalents) | 1.5 # | 2-1[3-fluoro-4-(4-carbamoylpiperidino)] phenylamino}-6-(4-aminopiperidino)-3-nitropyridine | 1.21(m, 2H), 1.70(m, 2H), 1.80(m, 6H), 2.22(m, 1H), 2.65(m, 2H), 2.91(m, 1H), 3.18(m, 2H), 3.33(m, 2H), 4.26 (brm, 2H), 6.51(d, 1H), 6.86(m, 1H), 7.02(m, 1H), 7.29(m, 1H), 7.30(m, 1H), 7.59 (dd, 1H), 8.15(d, 1H), 10.57 (s, 1H) | 20~30 | 80 | 458.1 |

(continued)

| Compounds according to Examples 290 to 300, Solvent S = $CH_3CN$ (all examples) Starting material 2-{[3-fluoro-4-(4-carbamoylpiperidino)]phenylamino}-6-chloro-3-nitropyridine compound obtained in Preparation Example 3-10-3 | | | | | | | |
|---|---|---|---|---|---|---|---|
| Ex | Amine | A | Compound | NMR (DMSO -d6) | T [°C] | Y [%] | M(+) |
| 298 | 3-amino-methylpyridine(1.5 quivalents) | 1.5 # | 2-{[3-fluoro-4-(4-carbamoylpiperidino)] phenylamino}-6-[(4-pyridyl)methylamino]-3-nitro-pyridine | 1.72(m, 2H), 1.79(m, 2H), 2.22(m, 1H), 2.63(m, 2H), 3.29(m, 2H 4.57 (m, 2H), 6.25(d, 1H), 6.80(d, 1H), 6.86(m, 1H), 7.11(d, 1H), 6.86(m, 1H), 7.11(d, 1H), 7.22(d, 2H), 7.29(d, 1H), 744(dd, 1H), 8.15(d, 1H), 8.49(d, 2H), 8.86(t, 1H) 10.69 (s, 1H) | 60~70 | 89 | 466.1 |

(continued)

| Compounds according to Examples 290 to 300, Solvent S = $CH_3CN$ (all examples) Starting material 2-{[3-fluoro-4-(4-carbamoylpiperidino)]phenylamino}-6-chloro-3-nitropyridine compound obtained in Preparation Example 3-10-3 | | | | | | | |
|---|---|---|---|---|---|---|---|
| Ex | Amine | A | Compound | NMR (DMSO -d6) | T [°C] | Y [%] | M(+) |
| 299 | 1-(3-amino-propyl) imidazo le (1.5 equivalents) | 1.5 | 2-{[3-fluoro-4-(4-carbamoylpiperidino) ]phenylaminol-6-[(3-imidazol-1-yl)propyl-amino]-3-nitro-pyridine | 1.72(m, 2H), 1.79(m, 2H), 1.99(m, 2H), 2.02(m, 1H), 2.65(m, 3H), 3.27(m, 2H), 3.36(m, 1H), 6.14(d, 1H), 6.79(m, 1H), 6.89(m, 1H), 7.02(m, 1H), 7.16(m, 1H), 7.30(m, 1H), 7.34(m, 1H), 7.61(m, 1H), 7.70(d, 1H), 8.09(d, 1H), 8.38(m, 1H), 10.81 (s, 1H) | 60 ~70 | 92 | 483.2 |

(continued)

| Compounds according to Examples 290 to 300, Solvent S = $CH_3CN$ (all examples) Starting material 2-{[3-fluoro-4-(4-carbamoylpiperidino)]phenylamino}-6-chloro-3-nitropyridine compound obtained in Preparation Example 3-10-3 | | | | | | | |
|---|---|---|---|---|---|---|---|
| Ex | Amine | A | Compound | NMR (DMSO -d6) | T [°C] | Y [%] | M(+) |
| 300 | 4-(2-amino-ethyl)-morpholine (1.5 equivalents) | 1.5 # | 2-{[3-fluoro-4-(4-carbamoylpiperidino)]phenylamino}-6-[2-(morpholin-1-yl)-ethylamino]-3-nitropyridine | 1.70(m, 2H), 1.79(m, 2H), 2.20(m, 1H), 2.34(m, 3H), 2.45(m, 3H), 2.65(m, 2H), 3.34(m, 1H), 3.47(m, 2H), 3.55(m, 4H), 6.13(d, 1H), 6.80(m, 1H), 7.00(m, 1H), 7.31(m, 2H), 7.68 (dd, 1H), 8.06(d, 1H), 8.29(m, 1H), 10.77 (s, 1H) | 60~70 | 84 | 488.2 |

**Example 301**

**Preparation of 2-{[3-fluoro-4-(3-carbamoylpiperidino)]phenylamino}-6-(methylamino)-3-nitropyridine**

To 10ml of acetonitrile were added 200mg (0.51 mmol) of the 2-{[3-fluoro-4-(3-carbamoylpiperidino)]phenylamino}-6-chloro-3-nitropyridine compound obtained in Preparation Example 3-11-3 and 5ml of a 40% methylamine-methanol solution, followed by reaction at room temperature for 4 hours. After the reaction was complete, the solvent was distilled under reduced pressure, followed by recrystallization from 5ml of methanol. The resulting solid was filtered and dried under vacuum at about 40°C to afford 195mg (yield: 98%) of the desired compound.

Mass (M+): 389.2

$^1$H-NMR(DMSO-$d_6$) (ppm): 1.47(m, 1H), 1.60(m, 1H), 1.75(m, 1H), 1.85(m, 1H), 2.48(m, 1H), 2.59(m, 1H), 2.69(m, 1H), 2.90(s, 3H), 3.30(m, 2H), 6.10(d, 1H), 6.86(s, 1H), 7.02(t, 1H), 7.38(m, 2H), 7.85(d, 1H), 8.05(d, 1H), 8.31 (d, 1H), 10.89(s, 1H).

**Examples 302 to 315**

In the same manner as in Example 301 and using amine compounds described in the following Table 25 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 25 were obtained.

The following Table 25 shows the name of compounds prepared in Examples 302 to 315, the name and equivalents of amine compounds used in the reaction, use/nonuse and equivalents of triethylamine in the reaction, the reaction temperature, the reaction solvent, yield, Mass analysis results and NMR analysis results.

**Table 25**

| Compounds according to Examples 302 to 315, Solvent S = $CH_3CN$ (all examples) Starting material 2-[3-fluoro-4-(3-carbamoylpiperidino)phenylamino]-6-chloro-3-nitropyridine compound obtained in Preparation Example 3-11-3 | | | | | | | |
|---|---|---|---|---|---|---|---|
| Ex | Amine | A | Compound | NMR (DMSO -d6) | T [°C] | Y [%] | M(+) |
| 302 | Isopropylamine (excess) | X | 2-{[3-fluoro-4-(3-carbamoylpiperidino) ]phenylamino}-6-(isopropylamino)-3-nitropyridine | 1.22(m, 6H), 1.46(m, 1H), 1.60(m, 1H), 1.73(m, 1H), 1.98(m, 1H), 2.50(m, 1H), 2.71(m, 2H), 3.30(m, 2H), 4.01(m, 1H), 6.10(d, 1H), 6.87(d, 1H), 7.03(m, 1H), 7.30(m, 2H), 7.83(d, 1H), 8.06(d, 1H), 8.26(d, 1H), 10.80 (s, 1H) | 20 ~ 30 | 28 | 417.2 |

(continued)

| Compounds according to Examples 302 to 315, Solvent S = $CH_3CN$ (all examples) Starting material 2-[3-fluoro-4-(3-carbamoylpiperidino)phenylamino]-6-chloro-3-nitropyridine compound obtained in Preparation Example 3-11-3 | | | | | | | |
|---|---|---|---|---|---|---|---|
| Ex | Amine | A | Compound | NMR (DMSO -d6) | T [°C] | Y [%] | M(+) |
| 303 | Isobutylamine (excess) | X | 2-{[3-fluoro-4-(3-carbamoylpiperidino) ]phenylaminol-6-(isobutylamino)-3-nitropyridine | 0.90(m, 6H), 1.44(m, 1H), 1.60(m, 1H), 1.84(m, 1H), 1.87(m, 3H), 2.59(m, 1H), 2.65(m, 1H), 3.16(m, 2H), 3.29(m, 2H), 6.16(d, 1H), 6.87(s, 1H), 7.03(m, 1H), 7.23(m, 1H), 7.83(s, 1H), 7.87(m, 1H), 8.05(s, 1H), 8.56(m, 1H), 10.86 (s, 1H) | 20 ~ 30 | 93 | 431.2 |

(continued)

| Compounds according to Examples 302 to 315, Solvent S = $CH_3CN$ (all examples) Starting material 2-[3-fluoro-4-(3-carbamoylpiperidino)phenylamino]-6-chloro-3-nitropyridine compound obtained in Preparation Example 3-11-3 | | | | | | | |
|---|---|---|---|---|---|---|---|
| Ex | Amine | A | Compound | NMR (DMSO -d6) | T [°C] | Y [%] | M(+) |
| 304 | t-butylamine (excess) | X | 2-{[3-fluoro-4-(3-carbamoylpiperidino) ]phenylaminol-6-(t-butylamino)-3-nitropyridine | 1.27(s, 9H), 1.46(m, 1H), 1.60(m, 1H), 1.75(m, 1H), 1.86(m, 1H), 2.58(m, 1H), 2.68(m, 1H), 3.16(m, 1H), 3.30(m, 2H), 6.13(d, 1H), 6.86(s, 1H), 7.02(t, 1H), 7.15(d, 1H), 7.36(s, 1H), 7.50(d, 1H), 7.85(s, 1H), 8.00(d, 1H), 10.63 (s, 1H) | 20~30 | 68 | 431.3 |

(continued)

| Compounds according to Examples 302 to 315, Solvent S = $CH_3CN$ (all examples) Starting material 2-[3-fluoro-4-(3-carbamoylpiperidino)phenylamino]-6-chloro-3-nitropyridine compound obtained in Preparation Example 3-11-3 | | | | | | | |
|---|---|---|---|---|---|---|---|
| Ex | Amine | A | Compound | NMR (DMSO -d6) | T [°C] | Y [%] | M(+) |
| 305 | 4-hydroxy-piperidine (1.5 equivalents) | 1.5 # | 2-1[3-fluoro-4-(3-carbamoylpiperidino) ]phenylaminol-6-(4-hydroxypiperidino)-3-nitropyridine | 1.43(m, 4H), 1.62(m, 1H), 1.80(m, 4H), 2.58(m, 1H), 2.72(m, 2H), 3.05(m, 1H), 3.30(m, 1H), 3.39(m, 2H), 3.41(m, 1H), 3.64(m, 1H), 3.79(m, 1H), 4.03 (brm, 2H), 6.52(d, 1H), 6.87(m, 1H), 7.03(m, 1H), 7.28(m, 1H), 7.38(s, 1H), 7.61(m, 1H), 8.16(d, 1H), 10.56 (s, 1H) | 20~30 | 73 | 459.2 |

(continued)

| Compounds according to Examples 302 to 315, Solvent S = $CH_3CN$ (all examples) Starting material 2-[3-fluoro-4-(3-carbamoylpiperidino)phenylamino]-6-chloro-3-nitropyridine compound obtained in Preparation Example 3-11-3 | | | | | | | |
|---|---|---|---|---|---|---|---|
| Ex | Amine | A | Compound | NMR (DMSO-d6) | T [°C] | Y [%] | M(+) |
| 306 | Piperazine (5 equivalents) | X | 2-{[3-fluoro-4-(3-carbamoylpiperidino)]phenylamino}-6-(piperazin-1-yl)-3-nitropyridine | 1.46(m, 1H), 1.60(m, 1H), 1.76(m, 1H), 1.87(m, 1H), 2.47(m, 1H) 2.59(m, 1H), 2.68(m, 1H), 2.80(m, 1H), 2.87 (brm 4H), 3.32(m, 2H), 3.68 (brm, 4H), 6.50(d, 1H), 6.87(s, 1H), 7.04(t, 1H), 7.30(m, 1H), 7.37(s, 1H), 7.58(m, 1H), 8.18(d, 1H), 10.56 (s, 1H), | 20~30 | 99 | 444.3 |

(continued)

| Compounds according to Examples 302 to 315, Solvent S = $CH_3CN$ (all examples) Starting material 2-[3-fluoro-4-(3-carbamoylpiperidino)phenylamino]-6-chloro-3-nitropyridine compound obtained in Preparation Example 3-11-3 | | | | | | | |
|---|---|---|---|---|---|---|---|
| Ex | Amine | A | Compound | NMR (DMSO -d6) | T [°C] | Y [%] | M(+) |
| 307 | 1-methyl-piperazine (1.5 equivalents) | 1.5 # | 2-{[3-fluoro-4-(3-carbamoylpiperidino)]phenylamino}-6-(4-methylpiperazin-1-yl)-3-nitropyridine | 1.47(m, 1H), 1.60(m, 1H), 1.76(m, 1H), 1.86(m, 1H), 2.20(s, 3H), 2.38 (brm, 4H), 2.50(m, 1H), 2.58(m, 1H), 2.70(m, 1H), 3.25(m, 1H) 2.70(m, 1H), 3.25(m, 1H), 3.70 (brm, 4H), 6.52(s, 1H), 6.87(s, 1H), 7.03(m, 1H), 7.30(m, 1H), 7.36(m, 1H), 7.54(d, 1H), 8.18(d, 1H), 10.53 (s, 1H) | 20~30 | 75 | 458.3 |

(continued)

| Compounds according to Examples 302 to 315, Solvent S = $CH_3CN$ (all examples) Starting material 2-[3-fluoro-4-(3-carbamoylpiperidino)phenylamino]-6-chloro-3-nitropyridine compound obtained in Preparation Example 3-11-3 | | | | | | | |
|---|---|---|---|---|---|---|---|
| Ex | Amine | A | Compound | NMR (DMSO-d6) | T [°C] | Y [%] | M(+) |
| 308 | Morpholine (3 equivalents) | X | 2-{[3-fluoro-4-(3-carbamoylpiperidino)]phenylaminol-6-morpholino-3-nitropyridine | 1.45(m, 1H), 1.61(m, 1H), 1.76(m, 1H), 1.84(m, 1H), 2.49 (brm, 4H), 2.59(m, 1H), 2.68(m, 1H), 3.25(m, 2H), 3.68 (brm, 8H), 6.50(d, 1H), 6.87(s, 1H) 7.03(t, 1H), 7.33(m, 2H), 7.52(q, 1H), 8.21(d, 1H), 1-.53(s, 1H) | 20~30 | 63 | 445.2 |

(continued)

| Compounds according to Examples 302 to 315, Solvent S = $CH_3CN$ (all examples) Starting material 2-[3-fluoro-4-(3-carbamoylpiperidino)phenylamino]-6-chloro-3-nitropyridine compound obtained in Preparation Example 3-11-3 | | | | | | | |
|---|---|---|---|---|---|---|---|
| Ex | Amine | A | Compound | NMR (DMSO -d6) | T [°C] | Y [%] | M(+) |
| 309 | 4-amino-piperidine (1.5 equivalents) | 1,5 # | 2-{[3-fluoro-4-(3-carbamoylpiperidino)]phenylamino}-6-(4-aminopiperidino)-3-nitropyridine | 1.26(m, 3H), 1.46(m, 1H), 1.47(m, 1H), 1.76(d, 3H), 1.84(d, 3H), 2.59(m, 1H), 2.70(m, 1H), 2.84(m, 1H), 3.00(m, 3H), 3.16(m 1H) 3.25(m, 2H), 6.53(d, 1H), 6.87(s, 1H), 7.03(t, 1H), 7.29(d, 1H), 7.38(s, 1H), 7.58(m, 1H), 8.16(d, 1H), 10.55 (s, 1H) | 20~30 | 91 | 458.4 |

(continued)

| Compounds according to Examples 302 to 315, Solvent S = $CH_3CN$ (all examples) Starting material 2-[3-fluoro-4-(3-carbamoylpiperidino)phenylamino]-6-chloro-3-nitropyridine compound obtained in Preparation Example 3-11-3 | | | | | | | |
|---|---|---|---|---|---|---|---|
| Ex | Amine | A | Compound | NMR (DMSO -d6) | T [°C] | Y [%] | M(+) |
| 310 | 3-amino-methylpyridine (1.5 equivalents) | 1.5 | 2-{[3-fluoro-4-(3-carbamoylpiperidino) ]phenylamino}-6-[(3-pyridyl)methylamino] -3-nitropyridine | 1.44(m, 1H), 1.60(m, 1H), 1.79(m, 1H), 1.90(m, 1H), 2.41(m, 1H), 2.53(m, 1H), 2.70(m, 1H), 3.36(m, 2H), 4.56(d, 2H), 6.20(d, 1H), 6.84(s, 1H), 6.98(t, 7.21(m, 1H), 7.40(m, 2H), 7.56(m, 2H), 8.12(d, 1H), 8.44(m, 2H), 8.80(m, 1H), 10.73 (s, 1H) | 60~70 | 51 | 466.2 |

(continued)

| Compounds according to Examples 302 to 315, Solvent S = $CH_3CN$ (all examples) Starting material 2-[3-fluoro-4-(3-carbamoylpiperidino)phenylamino]-6-chloro-3-nitropyridine compound obtained in Preparation Example 3-11-3 | | | | | | | |
|---|---|---|---|---|---|---|---|
| Ex | Amine | A | Compound | NMR (DMSO -d6) | T [°C] | Y [%] | M(+) |
| 311 | 4-amimo-methylpyridine (1.5 equivalents) | 1.5 # | 2-{[3-fluoro-4-(3-carbamoylpiperidino)]phenylamino}-6-[(4-pyridyl)methylamino]-3-nitropyridine | 1.46(m, 1H), 1.62(m, 1)0, 1.77(m, 1H), 1.86(m, 1H), 2.47(m, 1H), 2.57(m, 1H), 2.66(m, 1H), 3.23(m, 2H), 4.58(m, 2H) 6.23(d, 1H), 6.88(d, 2H), 7.12(d, 1H) 7.21(d, 2H) 7.42(d, 1H), 7.46(d, 2H) 8.16(d, 1H), 8.48(d, 2H), 8.86(m, 1H), 10.68 (s, 1H) | 60 ~ 70 | 61 | 466.3 |

(continued)

| Compounds according to Examples 302 to 315, Solvent S = $CH_3CN$ (all examples) Starting material 2-[3-fluoro-4-(3-carbamoylpiperidino)phenylamino]-6-chloro-3-nitropyridine compound obtained in Preparation Example 3-11-3 | | | | | | | |
|---|---|---|---|---|---|---|---|
| Ex | Amine | A | Compound | NMR (DMSO -d6) | T [°C] | Y [%] | M(+) |
| 312 | 1-(3-amino-propyl)-imidazole (1.5 equivalents) | 1.5 # | 2-{[3-fluoro-4-(3-carbamoylpiperidino)]phenylamino}-6-[(3-imidazol-1-yl) propylamino]-3-nitropyridine | 1.48(m, 1H), 1.60(m, 1H), 1.78(m, 1H), 1.89(m, 1H), 2.02(m, 2H), 2.61(m, 1H), 2.73(m, 1H), 3.29(m, 1H), 4.02(m, 2H), 6.14(d, 1H), 6.88(s, 2H), 7.06(t, 1H), 7.16(s, 1H), 7.38(d, 2H), 7.62(s, 1H), 7.72(d, 1H), 8.10(d, 1H), 8.39(s, 1H), 10.83 (s, 1H) | 60~70 | 84 | 483.3 |

(continued)

| Compounds according to Examples 302 to 315, Solvent S = $CH_3CN$ (all examples) Starting material 2-[3-fluoro-4-(3-carbamoylpiperidino)phenylamino]-6-chloro-3-nitropyridine compound obtained in Preparation Example 3-11-3 | | | | | | | |
|---|---|---|---|---|---|---|---|
| Ex | Amine | A | Compound | NMR (DMSO -d6) | T [°C] | Y [%] | M(+) |
| 313 | 4-(2-amino-ethyl)-morpholine (1.5 equivalents) | 1.5 # | 2-{[3-fluoro-4-(3-carbamoylpiperidino)]phenylamino}-6-[2-(morpholin-1-yl) ethylamino]-3-nitropyridine | 1.46(m, 1H), 1.63(m, 1H), 1.76(m, 1H), 1.88(m, 1H), 2.35 (brm, 4H), 2.46(m, 3H), 2.59(m, 1H), 2.69(m, 1H), 3.27(m, 2H), 3.46m, 2H), 3.54 (brm, 4H), 6.15(d, 1H), 6.87(s, 1H), 7.02(t, 1H), 7.35(d, 2H), 7.70(d, 1H), 8.08(d, 1H), 8.30(s, 1H), 10.78 (s, 1H) | 60 ~70 | 80 | 488.3 |

(continued)

| Compounds according to Examples 302 to 315, Solvent S = $CH_3CN$ (all examples) Starting material 2-[3-fluoro-4-(3-carbamoylpiperidino)phenylamino]-6-chloro-3-nitropyridine compound obtained in Preparation Example 3-11-3 | | | | | | | |
|---|---|---|---|---|---|---|---|
| Ex | Amine | A | Compound | NMR (DMSO -d6) | T [°C] | Y [%] | M(+) |
| 314 | 4-(3-amino-propyl)-morpholine (1.5 equivalents) | 1.5 # | 2-{[3-fluoro-4-(3-carbamoylpiperidino)]phenylamino}-6-[(3-morpholin-1-yl) propylamino]-3-nitropyridine | 1.47(m, 1H), 1.68(m, 1H), 1.74(m, 3H), 1.89(m, 1H), 2.30(s, 6H), 2.59(m, 2H), 2.68(m, 1H), 3.25(m, 2H), 3,40(m, 2H), 3.50(m, 4H), 3.40(m, 2H), 3.50(m, 4H), 6.11(d, 1H), 6.87(s, 1H), 7.03(t, 1H), 7.34(d, 1H), 7.80(m, 1H), 8.07(d, 1H), 8.38(m, 1H), 10.84 (s, 1H) | 60~70 | 77 | 502.3 |

(continued)

| Compounds according to Examples 302 to 315, Solvent S = $CH_3CN$ (all examples) Starting material 2-[3-fluoro-4-(3-carbamoylpiperidino)phenylamino]-6-chloro-3-nitropyridine compound obtained in Preparation Example 3-11-3 | | | | | | | |
|---|---|---|---|---|---|---|---|
| Ex | Amine | A | Compound | NMR (DMSO -d6) | T [°C] | Y [%] | M(+) |
| 315 | Diethylamine (excess) | X | 2-{[3-fluoro-4-(3-carbamoylpiperidino)]phenylamino}-6-(diethylamino)-3-nitropyridine | 1.15(m, 6H), 1.46(m, 1H), 1.65(m, 1H), 1.75(m, 1H), 1.86(m, 1H), 2.59(m, 2H), 2.69(m, 2H), 3.27(m, 3H), 3.56 (brm, 4H), 6.34(d, 1H), 6.86(s, 1H), 7.02(m, 1H), 7.27(d, 1H), 7.35(d, 1H), 7.73(d, 1H), 8.16(d, 1H), 10.66 (s, 1H) | 60~70 | 45 | 431.2 |

**Example 316**

**Preparation of 2-{[3-fluoro-4-(4-carboxypiperidino)]phenylamino}-6-(methylamino)-3-nitropyridine**

To 10ml of acetonitrile were added 200mg (0.51 mmol) of the 2-1[3-fluoro-4-(4-carboxy-piperidino)]phenylamino}-6-chloro-3-nitropyridine compound obtained in Preparation Example 3-12-3 and 5ml of a 40% methylamine-methanol solution, followed by reaction at room temperature for 4 hours. After the reaction was complete, the solvent was distilled under reduced pressure, followed by recrystallization from 5ml of methanol. The resulting solid was filtered and dried under vacuum at about 40°C to afford 114mg (yield: 57%) of the desired compound.

Mass (M+): 389.2

$^1$H-NMR(DMSO-$d_6$) (ppm): 1.70(m, 2H), 1.90(m, 2H), 2.30(m, 2H), 2.69(t, 2H), 2.91(s, 3H), 3.25(m, 1H), 6.12 (d, 1H), 7.01(t, 1H), 7.38(d, 1H), 7.85(m, 1H), 8.06(d, 1H), 8.37(s, 1H), 10.89(s, 1H).

**Examples 317 to 325**

In the same manner as in Example 316 and using amine compounds described in the following Table 26 in place of "40% methylamine-methanol solution", the following desired compounds can be synthesized by adjusting equivalents of the to-be-substituted amines depending on the difference in reactivity among the to-be-substituted amines during carrying out the reaction, or by adjusting the reaction temperature, or by adjusting use of a tertiary organic base such as triethylamine upon carrying out the reaction. Taking into consideration these various factors, the desired compounds described in the following Table 26 were obtained.

The following Table 26 shows the name of compounds prepared in Examples 317 to 325, the name and equivalents of amine compounds used in the reaction, use/nonuse and equivalents of triethylamine in the reaction, the reaction temperature, the reaction solvent, yield, Mass analysis results and NMR analysis results.

**Table 26**

| Compounds according to Examples 317 to 325, Solvent S = $CH_3CN$ (all examples) Starting material 2-{[3-fluoro-4-(4-carboxypiperidino)]phenylamino}-6-chloro-3-nitropyridine compound obtained in Preparation Example 3-12-3 | | | | | | | |
|---|---|---|---|---|---|---|---|
| Ex | Amine | A | Compound | NMR (DMSO-d6) | T [°C] | Y [%] | M(+) |
| 317 | Isopropylamine (excess) | X | 2-{[3-fluoro-4-(4-carboxypiperidino)] phenylamino}-6-(isopropylamino)-3-nitropyridine | 1.21(d, 6H), 1.68(m, 2H), 1.84(m, 2H), 1.99(m, 1H), 2.64(m, 2H), 3.25(m, 2H), 3.40 (brm, 1H), 4.11(m, 1H), 6.09(d, 1H), 6.98(t, 1H), 7.30(m, 1H), 7.79(m, 1H), 8.06(d, 1H), 8.33(d, 1H), 10.85(s, 1H) | 20 ~ 30 | 41 | 418.2 |

(continued)

| Compounds according to Examples 317 to 325, Solvent S = $CH_3CN$ (all examples) Starting material 2-{[3-fluoro-4-(4-carboxypiperidino)]phenylamino}-6-chloro-3-nitropyridine compound obtained in Preparation Example 3-12-3 | | | | | | | |
|---|---|---|---|---|---|---|---|
| Ex | Amine | A | Compound | NMR (DMSO-d6) | T [°C] | Y [%] | M(+) |
| 318 | Isobutylamine (excess) | X | 2-{[3-fluoro-4-(4-carboxypiperidino)]phenylamino}-6-(isobutylamino)-3-nitropyridine | 0.89(d, 6H), 1.68(m, 2H), 1.89(m, 3H), 2.24(m, 1H), 2.49(d, 1H), 2.68(m, 2H), 3.17(m, 2H), 3.25(m, 1H), 6.14(d, 1H), 6.99(t, 1H), 7.26(m, 1H), 7.84(m, 1H), 8.06(d, 1H), 8.48(m, 1H), 10.85(s, 1H) | 20 ~ 30 | 46 | 432.3 |

(continued)

| Compounds according to Examples 317 to 325, Solvent S = $CH_3CN$ (all examples) Starting material 2-{[3-fluoro-4-(4-carboxypiperidino)]phenylamino}-6-chloro-3-nitropyridine compound obtained in Preparation Example 3-12-3 | | | | | | | |
|---|---|---|---|---|---|---|---|
| Ex | Amine | A | Compound | NMR (DMSO-d6) | T [°C] | Y [%] | M(+) |
| 319 | 4-hydroxy-piperidine (1.5 equivalents) | 1.5 # | 2-{[3-fluoro-4-(4-carboxypiperidino)] phenylamino}-6-(4-hydroxypiperidino)-3-nitropyridine | 1.37(m, 2H), 1.68(m, 2H), 1.74(m, 2H), 1.90(m, 2H), 2.27(m, 1H), 2.68(m, 2H), 3.16(m, 2H) 3.50 (m, 2H), 3.82(m, 1H), 4.10(t, 2H), 6.52(d, 1H), 6.69(d, 2H), 7.26(d, 1H) 7.62 (d, 1H), 8.15(d, 1H), 10.55(s, 1H) | 20 ~ 30 | 66 | 460.3 |

(continued)

| Compounds according to Examples 317 to 325, Solvent S = $CH_3CN$ (all examples) Starting material 2-{[3-fluoro-4-(4-carboxypiperidino)]phenylamino}-6-chloro-3-nitropyridine compound obtained in Preparation Example 3-12-3 | | | | | | | |
|---|---|---|---|---|---|---|---|
| Ex | Amine | A | Compound | NMR (DMSO-d6) | T [°C] | Y [%] | M(+) |
| 320 | 1-methyl-piperazine (1.5 equivalents) | 1.5 # | 2-{[3-fluoro-4-(4-carboxypiperidino)]phenylamino}-6-(4-methylpiperazin-1-yl)-3-nitropyridine | 1.72(m, 2H), 1.92(m, 2H), 2.21(s, 1H), 2.37(m, 1H), 2.39(m, 2H), 2.51(m, 2H), 2.71(m, 2H), 3.30(m, 2H), 3.71 (brm, 4H), 6.51(d, 1H), 7.01(t, 1H), 7.29(m, 2H), 7.54(m, 1H), 8.18(d, 1H), 10.52(s, 1H) | 20 ~ 30 | 75 | 459.2 |

(continued)

| Compounds according to Examples 317 to 325, Solvent S = $CH_3CN$ (all examples) Starting material 2-{[3-fluoro-4-(4-carboxypiperidino)]phenylamino}-6-chloro-3-nitropyridine compound obtained in Preparation Example 3-12-3 | | | | | | | |
|---|---|---|---|---|---|---|---|
| Ex | Amine | A | Compound | NMR (DMSO-d6) | T [°C] | Y [%] | M(+) |
| 321 | 3-amino-methylpyridine (1.5 equivalents) | 1.5 # | 2-{[3-fluoro-4-(4-carboxypiperidino)] phenylamino}-6-[(3-pyridyl) methyl-amino]-3-nitro-pyridine | 1.70(m, 2H), 1.90(m, 2H), 2.34(m, 1H), 2.69(m, 2H), 3.25(m, 2H), 4.58(d, 2H), 6.20(d, 1H), 6.94(t, 1H), 7.20(d, 1H), 7.33(m, 1H), 7.55(m, 2H), 8.13(d, 1H), 8.45(m, 2H), 8.81(m, 1H), 10.71(s, 1H) | 60 ~ 70 | 29 | 467.3 |

(continued)

| Compounds according to Examples 317 to 325, Solvent S = $CH_3CN$ (all examples) Starting material 2-{[3-fluoro-4-(4-carboxypiperidino)]phenylamino}-6-chloro-3-nitropyridine compound obtained in Preparation Example 3-12-3 | | | | | | | |
|---|---|---|---|---|---|---|---|
| Ex | Amine | A | Compound | NMR (DMSO-d6) | T [°C] | Y [%] | M(+) |
| 322 | 4-aminomethyl-pyridine (1.5 equivalents) | 1.5 # | 2-{[3-fluoro-4-(4-carboxypiperidino)] phenylamino}-6-[(4-pyridyl) methyl-amino]-3-nitro-pyridine | 1.72(m, 2H), 1.89(m, 2H), 2.36(m, 1H), 2.70(m, 2H), 3.18(m, 2H), 4.56(m, 2H), 6.23(d, 1H), 6.87(t, 1H), 7.11(m, 1H), 7.20(m, 2H), 7.43(m, 1H), 8.20(d, 1H), 8.47(m, 2H), 8.82(m, 1H), 10.65(s, 1H) | 60 ~70 | 43 | 467.2 |

(continued)

| Compounds according to Examples 317 to 325, Solvent S = $CH_3CN$ (all examples) Starting material 2-{[3-fluoro-4-(4-carboxypiperidino)]phenylamino}-6-chloro-3-nitropyridine compound obtained in Preparation Example 3-12-3 | | | | | | | |
|---|---|---|---|---|---|---|---|
| Ex | Amine | A | Compound | NMR (DMSO-d6) | T [°C] | Y [%] | M(+) |
| 323 | 1-(3-aminopropyl)-imidazole (1.5 equivalents) | 1.5 # | 2-{[3-fluoro-4-(4-carboxypiperi-dino)] phenylaminol-6-[(3-imidazol-1-7,15(s, 1H), 7.35(m, yl) propylamino]-3-nitropyridine | 1.70(m, 2H), 1.91(m, 2H), 2.00(m, 2H), 2.36(m, 1H), 2.71(m, 2H), 3.27(m, 4H), 4.01(m, 2H), 6.13(d, 1H), 4.01(m, 2H), 6.13(d, 1H), 6.88(s, 1H), 7.02(t, 1H), 1H), 7.60(s, 1H), 7.72(m, 1H), 8.09(m, 1H) 8.37 (m, 1H), 10.80(s, 1H) | 60~70 | 28 | 484.3 |

(continued)

| Compounds according to Examples 317 to 325, Solvent S = $CH_3CN$ (all examples) Starting material 2-{[3-fluoro-4-(4-carboxypiperidino)]phenylamino}-6-chloro-3-nitropyridine compound obtained in Preparation Example 3-12-3 | | | | | | | |
|---|---|---|---|---|---|---|---|
| Ex | Amine | A | Compound | NMR (DMSO-d6) | T [°C] | Y [%] | M(+) |
| 324 | 4-(2-aminoethyl)-morpholine (1.5 equivalents) | 1.5 # | 2-{[3-fluoro-4-(4-carboxypiperidino)] phenylamino}-6-[2-(morpholin-1-yl)ethylamino]-3-nitropyridine | 1.70(m, 2H), 1.92(m, 2H), 2.19(m, 1H), 2.36(m, 4H), 2.37(m, 2H), 2.72(m, 4H), 3.27(m, 1H), 3.43 (brm, 2H), 3.57(m, 4H), 6.12(d, 1H), 7.00(t, 1H), 7.32(m, 1H), 7.68(m, 1H), 8.06(d, 1H), 8.33(s, 1H) 10.75(s, 1H) | 60 ~ 70 | 51 | 489.3 |

(continued)

| Compounds according to Examples 317 to 325, Solvent S = $CH_3CN$ (all examples) Starting material 2-{[3-fluoro-4-(4-carboxypiperidino)]phenylamino}-6-chloro-3-nitropyridine compound obtained in Preparation Example 3-12-3 | | | | | | | |
|---|---|---|---|---|---|---|---|
| Ex | Amine | A | Compound | NMR (DMSO-d6) | T [°C] | Y [%] | M(+) |
| 325 | 4-(3-aminopropyl)-morpholine (1.5 equivalents) | 1.5 # | 2-{[3-fluoro-4-(4-carboxypiperidino)] phenylamino}-6-[(3-morpholin-1-yl)propylamino]-3-nitropyridine | 1.59(m, 2H), 1.70(m, 2H), 1.86(m, 2H), 2.20(m, 1H), 2.30(m, 4H), 2.69(m, 4H), 3.24(d, 2H), 3.52 (brm, 2H), 3.56(m, 4H), 6.11(d, 1H), 6.99(t, 1H), 7.29(m, 1H), 7.78(m, 1H), 8.05(d, 1H), 8.41(s, 1H) 10.84(s, 1H) | 60 ~70 | 25 | 501.3 |

A better understanding of the present invention may be obtained through the following preferable Experimental Examples, which are set forth to illustrate, but are not to be construed as the limit of the present invention.

**Experimental Example 1**

**Osteoclastogenesis inhibitory effects of compounds via co-culture system**

Osteoclastogenesis inhibitory effects of the compounds of the present invention were evaluated via a co-culture system (Reference: Endocrinology 137(1996), 2187 to 2190, E. Jimi et al.). A specific experimental method is as follows.

**1) Preparation of bone marrow cells and osteoblasts**

Femora and tibia were aseptically dissected from 6 to 8-week-old male ddY mice to harvest bone marrow cells by a conventional method using a syringe. In brief, tissues were removed from the dissected bone, the bone ends were cut off with scissors, and the bone marrow was isolated by pushing a medium-containing syringe (23G) against the one end of the cut bone. The isolated bone marrow was subjected to repeated piston movement of a syringe such that single

cells were obtained (Reference: Endocrinology 123(1988), 2600 to 2602, Takahashi et al.). After removal of red blood cells within the bone marrow, the bone marrow cells recovered by centrifugation were placed in an α-MEM supplemented with 10% fetal bovine serum (FBS), followed by counting of nucleated cells and then were immediately used for a

For the preparation of osteoblasts (Calvarial cells), the calvaria were aseptically dissected from 1 to 2-day-old neonatal ICR mice and subjected to continuous reaction with a 0.2% collagenase solution to separate osteoblasts. The cell-suspended supernatant was centrifuged to recover osteoblasts which were grown to full confluence in an α-MEM supplemented with 10% FBS and then diluted to a desired cell density for use in the experiment.

**2) Osteoclastogenesis inhibition experiment via co-culture system**

As the medium used for a co-culture system, a differentiation medium with the addition of differentiation factors 1α,25-dihydroxyvitamin D3 ($10^{-8}$M) and dexamethasone ($10^{-8}$M) to α-MEM supplemented with 10% FBS was used for the induction of osteoclastogenesis. First, the compounds dissolved in dimethyl sulfoxide (DMSO) at a concentration of 1mM were diluted to 2 μM using the above-mentioned differentiation medium. As a vehicle control group, 0.2% (v/v) DMSO was added to the medium. 100μL/well of each medium was added to 96-well plates. In addition, the above prepared bone marrow cells and osteoblasts were plated onto 96-well plates at a density of $1x10^5$ cells/50μL/well and $3x10^3$ cells/50μL/well, respectively. The total volume/well was 200μL and the final compound concentration was 1μM. The control group was 0.1% DMSO. The cells were cultured with exchange of the culture media with fresh media containing differentiation factors and test materials at an interval of 2 to 3 days.

7 days after culturing of cells, the formation of multinucleated osteoclasts was confirmed by microscopic examination, the medium was removed from the wells and then the cells were fixed in a 10% phosphate-buffered formalin solution. The degree of formation of mature osteoclasts was measured taking advantage of the characteristics of osteoclasts showing a positive reaction to a tartrate-resistant acid phosphatase (TRAP) staining solution. The TRAP staining solution was prepared in a manner such that naphthol AS-MS phosphate as a substrate and a coloring agent (Fast Red Violet LB salt) were dissolved in N,N-dimethylformamide, and a 0.1N $NaHCO_3$ buffer solution containing 50 mM of tartaric acid was added thereto. Among the TRAP-positive cells under a light microscope, multinucleated osteoclasts having 6 to 7 nuclei were regarded as mature osteoclasts.

The degree of inhibition of osteoclastogenesis was calculated according to the following equation 1. The results are summarized in Table 27 below (Experiments were carried out for 4 wells/experimental group (n=4), and the results are given in terms of mean ± standard deviation)

$$\text{Inhibition of osteoclastogenesis (\%)} = 1 - \frac{\text{numbers of osteoclasts observed in experimental group}}{\text{numbers of osteoclasts observed in vehicle control group)}} \times 100\ (\%)$$

**Table 27**

| Inhibition of oestoclastogenesis | | | | | |
|---|---|---|---|---|---|
| Example | Oestoclastogenesis inhibition % | Example | Oestoclastogenesis inhibition % | Example | Oestoclastogenesis inhibition % |
| | 1μM | | | | |
| 7 | 100 | 43 | 100 | 93 | 66 |
| 9 | 93 | 50 | 94 | 94 | 93 |
| 10 | 64 | 53 | 97 | 97 | 80 |
| 12 | 63 | 55 | 98 | 103 | 74 |
| 13 | 92 | 56 | 99 | 106 | 87 |
| 25 | 98 | 59 | 81 | 115 | 89 |
| 28 | 88 | 64 | 65 | 120 | 63 |
| 39 | 98 | 66 | 65 | 121 | 89 |

(continued)

| Inhibition of oestoclastogenesis | | | | | |
|---|---|---|---|---|---|
| Example | Oestoclastogenesis inhibition % | Example | Oestoclastogenesis inhibition % | Example | Oestoclastogenesis inhibition % |
| | 1$\mu$M | | | | |
| 40 | 89 | 89 | 73 | 132 | 80 |
| 42 | 96 | 92 | 64 | 134 | 61 |
| 135 | 93 | 199 | 67 | 226 | 66 |
| 138 | 98 | 201 | 86 | 227 | 65 |
| 139 | 99 | 202 | 90 | 228 | 73 |
| 141 | 82 | 209 | 85 | 229 | 97 |
| 143 | 99 | 210 | 70 | 230 | 93 |
| 144 | 100 | 212 | 85 | 231 | 82 |
| 145 | 94 | 213 | 94 | 232 | 86 |
| 151 | 70 | 214 | 98 | 233 | 96 |
| 152 | 85 | 215 | 91 | 234 | 100 |
| 153 | 78 | 216 | 93 | 235 | 86 |
| 154 | 78 | 217 | 94 | 236 | 76 |
| 163 | 67 | 220 | 82 | 238 | 75 |
| 177 | 61 | 220 | 82 | 241 | 87 |
| 193 | 81 | 221 | 89 | 242 | 93 |
| 197 | 81 | 223 | 82 | 244 | 62 |
| 198 | 84 | 225 | 60 | 246 | 80 |

As shown in Table 27 above, it was demonstrated that most of the compounds of the present invention inhibit the formation of osteoclasts.

**Experimental Example 2**

**Evaluation of alkaline phosphatase (ALP) activity**

Differentiation and activity of osteoblasts were indirectly evaluated by measuring an ALP activity having a close relationship with osteogenesis.

Osteoblasts (Calvarial cells) prepared in Experimental Example 1 and MC3T3-E1 cells (available from RIKEN Cell Bank, Japan) were collected in $\alpha$-MEM supplemented with 10% FBS, followed by cell counting. The cells were dispensed into 24-well cultureware at a density of $2 \times 10^4$ cells/well. After culturing of the cells for 24 hours, the culture media were discarded and replaced with fresh media in which test compounds were diluted to a concentration of 1$\mu$M (1mL/well). In addition, the vehicle control group containing 0.1% DMSO was also treated. Under the conditions where the compounds were treated, the cells were cultured in a 5% $CO_2$ inhibitor at 37°C for 3 days. When the experiment was terminated, the supernatant was removed and the cells were washed three times with cold phosphate buffer at 4°C. 0.2% Triton X-100 was added to the washed cells which were then subjected to three cycles of freezing at -70°C and thawing at room temperature for the complete lysis of cells. The cell extracts were pooled and centrifuged to collect the cell supernatant which was used for the measurement of ALP activity and proteins. The protein concentration was measured using a BCA assay kit (manufactured by Sigma-Aldrich). For the measurement of ALP activity, p-nitrophenylphosphate was added to the cell supernatant which was then incubated at 37°C for 30 minutes, and the reaction was terminated with the addition of 50$\mu$L of 0.2N sodium hydroxide. The standard curve was plotted at the absorbance of 405 nm using p-nitrophenol as a standard material and then the absorbance of test materials thus reacted was measured to determine the production amount of p-nitrophenol.

The ALP activity was calculated by dividing the amount of p-nitrophenol produced from each test material by the protein amount and the reaction time. Therefore, the unit of ALP activity was given in terms of p-nitrophenol/μg protein/min. The results are given in Tables 28-1 and 28-2 where the ALP activity unit of each test material was given in terms of % change through the comparison between the individual test materials and the vehicle control group.

**Table 28-1**

| ALP activity (calvarial cells) | | | | | |
|---|---|---|---|---|---|
| Example | ALP activity (1μM, % of Control) | Example | ALP activity (1μM, % of Control) | Example | ALP activity (1μM, % of Control) |
| | Calvarial cell | | Calvarial cell | | Calvarial cell |
| 6 | 116 | 95 | 138 | 193 | 114 |
| 9 | 129 | 97 | 188 | 199 | 118 |
| 13 | 115 | 99 | 189 | 201 | 118 |
| 14 | 135 | 102 | 122 | 203 | 154 |
| 22 | 121 | 104 | 112 | 205 | 132 |
| 25 | 126 | 105 | 121 | 206 | 124 |
| 31 | 134 | 106 | 116 | 213 | 121 |
| 35 | 132 | 107 | 121 | 215 | 125 |
| 40 | 126 | 110 | 143 | 216 | 112 |
| 43 | 121 | 112 | 122 | 217 | 119 |
| 45 | 133 | 115 | 127 | 223 | 247 |
| 47 | 111 | 118 | 111 | 224 | 125 |
| 49 | 112 | 120 | 115 | 225 | 150 |
| 50 | 149 | 121 | 127 | 227 | 122 |
| 51 | 116 | 132 | 198 | 229 | 114 |
| 53 | 134 | 133 | 122 | 230 | 120 |
| 57 | 112 | 135 | 113 | 231 | 121 |
| 58 | 127 | 137 | 122 | 235 | 121 |
| 59 | 115 | 138 | 121 | 236 | 209 |
| 60 | 131 | 139 | 122 | 237 | 117 |
| 79 | 133 | 140 | 118 | 239 | 130 |
| 80 | 116 | 141 | 129 | 244 | 118 |
| 81 | 123 | 145 | 117 | 252 | 112 |
| 86 | 144 | 161 | 121 | 253 | 115 |
| 87 | 116 | 191 | 156 | 257 | 122 |
| 90 | 161 | 192 | 113 | 258 | 115 |
| | | | | Control | 100 |

**Table 28-2**

| ALP activity (MC3t3-E1 cells) | | | | | |
|---|---|---|---|---|---|
| Example | ALP activity (1μM,% of Control) | Example | ALP activity (1μM,% of Control) | Example | ALP activity (1μM,% of Control) |
| 9 | 175 | 45 | 124 | 101 | 123 |
| 16 | 118 | 47 | 117 | 102 | 126 |
| 18 | 113 | 49 | 122 | 103 | 151 |
| 20 | 124 | 50 | 177 | 108 | 111 |
| 21 | 124 | 53 | 121 | 112 | 148 |
| 22 | 123 | 94 | 134 | 115 | 148 |
| 25 | 148 | 95 | 185 | 119 | 167 |
| 39 | 175 | 96 | 137 | | |
| 40 | 210 | 100 | 126 | Control | 100 |

As shown in Table 28-1 and Table 28-2, it was demonstrated that the compounds of the present invention exhibit excellent ALP activity on both Calvarial cells and MC3T3-E1 cells.

**Experimental Example 3**

**Cytotoxicity test**

Cytotoxicity of the compounds of the present invention was evaluated by carrying out the experiment described below.

Drugs of Compound 1 to Compound 325 were diluted to a concentration of 2μM in α-MEM culture media supplemented with 10% FBS. The vehicle control group was established to contain 0.2% DMSO. 100μL/well of the diluted drugs were dispensed into 96-well plates to which osteoblasts (calvarial cells) prepared in Experimental Example 1 were then added at a density of $1.0 \times 10^4$ cells/100μL/well. Here, the final compound concentration in the cell culture was 1μM, and the vehicle control group contained 0.1% DMSO. The cells were cultured in a 5% $CO_2$ inhibitor at 37°C for 72 hours. 25 μL of 3-(4,5-dimethyl-2-thiazolyl)-2,5-diphenyl-2H-tetrazolium bromide (MTT) dissolved in PBS (2 mg/mL) was added to each cell culture 4 hours before the end of culture. After completion of the reaction, the plates were centrifuged, the media were discarded, and 100μL of formazan was added and dissolved in dimethyl sulfoxide (DMSO). Finally, the absorbance of the developed plates was measured at 540 nm. The cell viability was expressed as % concentration in comparison with the vehicle control group. The results are given in Table 29.

**Table 29**

| Cell viability | | | | | |
|---|---|---|---|---|---|
| Example | Cell viability (%) | Example | Cell viability | Example | Cell viability |
| | Calvarial cell | | Calvarial cell | | Calvarial cell |
| 1 | 106 | 27 | 97 | 53 | 115 |
| 2 | 104 | 28 | 89 | 55 | 105 |
| 3 | 102 | 29 | 93 | 56 | 104 |
| 4 | 104 | 30 | 90 | 57 | 103 |
| 5 | 96 | 31 | 108 | 58 | 107 |
| 6 | 94 | 32 | 91 | 59 | 99 |
| 7 | 103 | 33 | 93 | 60 | 119 |
| 8 | 92 | 34 | 97 | 61 | 102 |
| 9 | 121 | 35 | 96 | 62 | 103 |

(continued)

| Cell viability | | | | | |
|---|---|---|---|---|---|
| Example | Cell viability (%) | Example | Cell viability | Example | Cell viability |
| | Calvarial cell | | Calvarial cell | | Calvarial cell |
| 10 | 116 | 36 | 118 | 63 | 104 |
| 11 | 103 | 37 | 104 | 64 | 102 |
| 12 | 103 | 38 | 96 | 65 | 102 |
| 13 | 97 | 39 | 111 | 66 | 107 |
| 14 | 99 | 40 | 82 | 67 | 111 |
| 15 | 100 | 41 | 85 | 68 | 103 |
| 16 | 100 | 42 | 103 | 69 | 110 |
| 17 | 96 | 43 | 114 | 70 | 105 |
| 18 | 91 | 44 | 101 | 71 | 114 |
| 19 | 94 | 45 | 89 | 72 | 106 |
| 21 | 90 | 46 | 100 | 73 | 102 |
| 22 | 92 | 47 | 88 | 74 | 93 |
| 23 | 90 | 48 | 103 | 75 | 103 |
| 24 | 99 | 49 | 96 | 76 | 102 |
| 25 | 101 | 50 | 115 | 77 | 100 |
| 26 | 90 | 51 | 100 | 78 | 93 |
| 79 | 99 | 111 | 95 | 151 | 101 |
| 80 | 102 | 112 | 103 | 152 | 105 |
| 81 | 100 | 114 | 92 | 153 | 107 |
| 82 | 106 | 115 | 987 | 154 | 116 |
| 83 | 99 | 116 | 89 | 155 | 105 |
| 84 | 108 | 117 | 90 | 156 | 108 |
| 85 | 101 | 118 | 102 | 157 | 115 |
| 86 | 111 | 119 | 107 | 158 | 88 |
| 87 | 101 | 120 | 94 | 159 | 100 |
| 88 | 96 | 121 | 100 | 160 | 105 |
| 89 | 89 | 122 | 121 | 161 | 98 |
| 90 | 106 | 123 | 112 | 162 | 67 |
| 92 | 101 | 124 | 106 | 163 | 99 |
| 93 | 98 | 126 | 95 | 164 | 104 |
| 94 | 85 | 127 | 99 | 165 | 102 |
| 95 | 93 | 128 | 87 | 175 | 104 |
| 96 | 90 | 129 | 88 | 176 | 109 |
| 97 | 114 | 130 | 101 | 177 | 107 |
| 98 | 92 | 131 | 100 | 178 | 109 |
| 99 | 104 | 132 | 101 | 179 | 104 |

(continued)

| Cell viability | | | | | |
|---|---|---|---|---|---|
| Example | Cell viability (%) | Example | Cell viability | Example | Cell viability |
| | Calvarial cell | | Calvarial cell | | Calvarial cell |
| 100 | 91 | 133 | 102 | 180 | 100 |
| 101 | 92 | 134 | 93 | 181 | 106 |
| 102 | 92 | 135 | 106 | 182 | 101 |
| 103 | 93 | 137 | 92 | 183 | 113 |
| 104 | 97 | 138 | 94 | 184 | 110 |
| 105 | 90 | 139 | 95 | 185 | 111 |
| 106 | 82 | 140 | 102 | 186 | 113 |
| 107 | 97 | 141 | 102 | 187 | 102 |
| 108 | 100 | 143 | 85 | 188 | 111 |
| 109 | 101 | 144 | 35 | 189 | 129 |
| 110 | 112 | 145 | 96 | 190 | 123 |
| 191 | 97 | 222 | 95 | 253 | 104 |
| 192 | 99 | 223 | 100 | 254 | 101 |
| 193 | 98 | 224 | 101 | 255 | 100 |
| 194 | 107 | 225 | 90 | 256 | 104 |
| 195 | 104 | 226 | 104 | 257 | 101 |
| 196 | 100 | 227 | 103 | 258 | 101 |
| 197 | 95 | 228 | 99 | 260 | 107 |
| 198 | 96 | 229 | 104 | 261 | 114 |
| 199 | 118 | 230 | 101 | 262 | 109 |
| 200 | 107 | 231 | 112 | 263 | 108 |
| 201 | 95 | 232 | 100 | 264 | 117 |
| 202 | 96 | 233 | 102 | 265 | 104 |
| 203 | 97 | 234 | 94 | 266 | 121 |
| 204 | 102 | 235 | 105 | 267 | 107 |
| 205 | 109 | 236 | 91 | 268 | 104 |
| 206 | 102 | 237 | 99 | 269 | 113 |
| 207 | 102 | 238 | 106 | 270 | 95 |
| 208 | 101 | 239 | 98 | 271 | 107 |
| 209 | 99 | 240 | 97 | 272 | 98 |
| 210 | 105 | 241 | 100 | 273 | 115 |
| 211 | 110 | 242 | 114 | 274 | 102 |
| 212 | 100 | 243 | 97 | 275 | 121 |
| 213 | 95 | 244 | 99 | 276 | 102 |
| 214 | 103 | 245 | 101 | 277 | 107 |
| 215 | 104 | 246 | 105 | 278 | 118 |

(continued)

| Cell viability | | | | | |
|---|---|---|---|---|---|
| Example | Cell viability (%) | Example | Cell viability | Example | Cell viability |
| | Calvarial cell | | Calvarial cell | | Calvarial cell |
| 216 | 92 | 247 | 94 | 279 | 102 |
| 217 | 92 | 248 | 95 | 280 | 103 |
| 218 | 96 | 249 | 101 | 281 | 107 |
| 219 | 89 | 250 | 85 | 282 | 107 |
| 220 | 93 | 251 | 97 | 283 | 103 |
| 221 | 91 | 252 | 103 | 284 | 100 |
| 285 | 106 | 229 | 125 | 313 | 106 |
| 286 | 123 | 300 | 118 | 314 | 103 |
| 287 | 103 | 301 | 95 | 315 | 104 |
| 288 | 103 | 302 | 103 | 316 | 95 |
| 289 | 115 | 303 | 102 | 317 | 99 |
| 290 | 119 | 304 | 103 | 318 | 103 |
| 291 | 87 | 305 | 107 | 319 | 112 |
| 292 | 102 | 306 | 122 | 320 | 101 |
| 293 | 104 | 307 | 131 | 321 | 101 |
| 294 | 95 | 308 | 109 | 322 | 106 |
| 295 | 106 | 309 | 110 | 323 | 105 |
| 296 | 97 | 310 | 97 | 324 | 82 |
| 297 | 107 | 311 | 98 | 325 | 106 |
| 298 | 108 | 312 | 98 | | |

As shown in Table 29, it was demonstrated that the compounds of the present invention show substantially no cytotoxicity.

## Claims

**1.** A 2,6-substituted-3-nitropyridine derivative compound represented by formula 1:

[Formula 1]

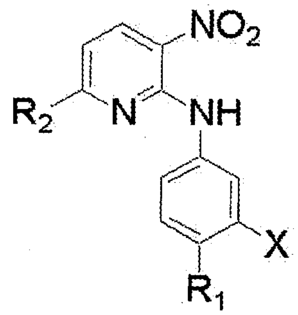

wherein $R_1$ represents hydrogen, fluoro, a $C_1$-$C_6$ linear or branched alkyl group, a methoxy group, a methylsulfanyl group, a nitrile group, a hydroxyl group or $NR_3R_4$ wherein $R_3$ and $R_4$ each independently represent H, a methyl group or an ethyl group, or $R_3$ and $R_4$ taken together form a saturated or unsaturated 5-, 6- or 7-membered heterocyclic amino compound which contains 1 to 3 hetero atoms selected from N, O and S and is unsubstituted or substituted by a $C_1$-$C_3$ alkyl group, a hydroxyl group, a $C_1$-$C_3$ hydroxyalkyl group, an amino group, a carboxyl group or a carbamoyl group; when $R_1$ represents a thiazolyl group

( 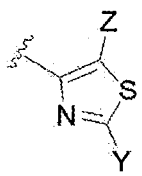 ),

Y is substituted by a $C_1$-$C_5$ linear or branched alkyl group, a $C_1$-$C_3$ alkylamine or dialkylamine group or a $C_5$-$C_6$ saturated or unsaturated cyclic amine group, and Z represents hydrogen or a $C_1$-$C_3$ alkyl group, $R_1$ optionally contains an asymmetric carbon atom,

$R_2$ represents $NR_5(CH_2)_nR_6$ wherein $R_5$ represents H, a $C_1$-$C_6$ linear or branched alkyl group or an unsubstituted or substituted $C_3$-$C_6$ cyclic alkyl group, and $R_6$ represents H, a hydroxyl group, a phenyl group, a $C_1$-$C_2$ alkoxy group, a $C_1$-$C_6$ linear or branched alkylamine group, or a $C_1$-$C_6$ linear or branched alkyl group which is terminally substituted by a saturated or unsaturated 5 to 7-membered heterocyclic compound containing 1 to 3 hetero atoms selected from N, O and S, or $R_5$ and $R_6$ taken together form a saturated or unsaturated 5 to 7-membered heterocyclic amine compound which contains 1 to 3 hetero atoms selected from N, O and S and is unsubstituted or substituted by a $C_1$-$C_3$ alkyl group, an amine group, a hydroxyl group or a $C_1$-$C_2$ hydroxyalkyl group,

n represents an integer of 0 to 3, and

X represents hydrogen, a fluoro group, a hydroxyl group, an amino group, an acetyl group or a nitrile group; or a pharmaceutically acceptable salt thereof.

**2.** The compound according to claim 1, wherein $R_1$ represents hydrogen, fluoro, a methyl group, an n-butyl group, a t-butyl group, a methoxy group, a methylsulfanyl group, a nitrile group, a hydroxyl group or $NR_3R_4$ wherein $R_3$ and $R_4$ each independently represent H, a methyl group or an ethyl group, or $R_3$ and $R_4$ taken together form a heterocyclic compound which is morpholine, thiomorpholine, piperazine, piperidine, methylpiperidine, hydroxypiperidine, hydroxymethylpiperidine, aminopiperidine, 3- or 4-carbamoylpiperidine, carboxylicpiperidine, imidazol-1-yl or thiazol-4-yl derivative

( 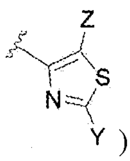 )

wherein Y represents methyl, isopropyl, cyclohexyl or dipropylamino, and Z represents hydrogen or a $C_1$-$C_3$ alkyl group,

$R_2$ represents $NR_5(CH_2)_nR_6$ wherein $R_5$ represents H, methyl, ethyl, isopropyl, cyclopropyl, n-butyl, isobutyl or t-butyl, and $R_6$ represents H, a hydroxyl group, a morpholinyl group, a phenyl group, a pyridin-2-yl, pyridin-3-yl, pyridin-4-yl, imidazol-1-yl or 1,3-dioxolan-2-yl, or $R_5$ and $R_6$ taken together form a heterocyclic compound which is morpholine, piperazine, methylpiperazine, aminopiperidine, 2-methyl-4,5-dihydroimidazol-1-yl, 2-methylimidazol-1-yl or isopropylimidazol-1-yl,

n represents an integer of 0 to 3, and

X represents hydrogen, a fluoro group, an amino group, an acetyl group or a nitrile group.

**3.** The compound according to claim 2, wherein the compound is selected from the group consisting of:

1) 2-(4-methylphenylamino)-6-(methylamino)-3-nitropyridine,
2) 2-(4-methylphenylamino)-6-(isopropylamino)-3-nitropyridine,
3) 2-(4-methylphenylamino)-6-(isobutylamino)-3-nitropyridine,
4) 2-(4-methylphenylamino)-6-[(N-[1,3]-dioxolan-2-ylmethyl)methylamino]-3-nitropyridine,
5) 2-(4-methylphenylamino)-6-(4-hydroxypiperidino)-3-nitropyridine,
6) 2-(4-methylphenylamino)-6-[(2-methyl-4,5-dihydro)imidazol-1-yl]-3-nitropyridine,
7) 2-(4-methylphenylamino)-6-[(2-isopropyl)imidazol-1-yl]-3-nitropyridine,
8) 2-(4-methylphenylamino)-6-[(4-pyridyl)methylamino]-3-nitropyridine,
9) 2-(4-methylphenylamino)-6-[(3-imidazol-1-yl)propylamino]-3-nitropyridine,
10) 2-(4-methylphenylamino)-6-[2-(3-pyridyl)ethylamino]-3-nitropyridine,
11) 2-(4-methylphenylamino)-6-(4-methylpiperazin-1-yl)-3-nitropyridine,
12) 2-(4-methylphenylamino)-6-(piperazin-1-yl)-3-nitropyridine,
13) 2-(4-methylphenylamino)-6-(4-aminopiperidino)-3-nitropyridine,
14) 2-(4-methylphenylamino)-6-morpholino-3-nitropyridine,
15) 2-(4-methoxyphenylamino)-6-(methylamino)-3-nitropyridine,
16) 2-(4-methoxyphenylamino)-6-(isopropylamino)-3-nitropyridine,
17) 2-(4-methoxyphenylamino)-6-(isobutylamino)-3-nitropyridine,
18) 2-(4-methoxyphenylamino)-6-[(N-[1,3]-dioxolan-2-ylmethyl)methylamino]-3-nitropyridine,
19) 2-(4-methoxyphenylamino)-6-(4-hydroxypiperidino)-3-nitropyridine,
20) 2-(4-methoxyphenylamino)-6-[(2-methyl-4,5-dihydro)imidazol-1-yl]-3-nitropyridine,
21) 2-(4-methoxyphenylamino)-6-[(2-isopropyl)imidazol-1-yl]-3-nitropyridine,
22) 2-(4-methoxyphenylamino)-6-[(4-pyridyl)methylamino]-3-nitropyridine,
23) 2-(4-methoxyphenylamino)-6-(t-butylamino)-3-nitropyridine,
24) 2-(4-methoxyphenylamino)-6-[(N-methyl-2-hydroxy)ethylamino]-3-nitropyridine,
25) 2-(4-methoxyphenylamino)-6-[(3-imidazol-1-yl)propylamino]-3-nitropyridine,
26) 2-(4-methoxyphenylamino)-6-(4-methylpiperazin-1-yl)-3-nitropyridine,
27) 2-(4-methoxyphenylamino)-6-(piperazin-1-yl)-3-nitropyridine,
28) 2-(4-methoxyphenylamino)-6-(4-aminopiperidino)-3-nitropyridine,
29) 2-(4-methoxyphenylamino)-6-morpholino-3-nitropyridine,
30) 2-[4-(t-butyl)phenylamino]-6-(methylamino)-3-nitropyridine,
31) 2-[4-(t-butyl)phenylamino]-6-(isopropylamino)-3-nitropyridine,
32) 2-[4-(t-butyl)phenylamino]-6-(isobutylamino)-3-nitropyridine,
33) 2-[4-(t-butyl)phenylamino]-6-[(N-[1,3]-dioxolan-2-ylmethyl)methylamino]-3-nitropyridine,
34) 2-[4-(t-butyl)phenylamino]-6-(4-hydroxypiperidino)-3-nitropyridine,
35) 2-[4-(t-butyl)phenylamino]-6-[(2-methyl-4,5-dihydro)imidazol-1-yl]-3-nitropyridine,
36) 2-[4-(t-butyl)phenylamino]-6-[(2-isopropyl)imidazol-1-yl]-3-nitropyridine,
37) 2-[4-(t-butyl)phenylamino]-6-[(3-pyridyl)methylamino]-3-nitropyridine,
38) 2-[4-(t-butyl)phenylamino]-6-[(4-pyridyl)methylamino]-3-nitropyridine,
39) 2-[4-(t-butyl)phenylamino]-6-[(3-imidazol-1-yl)propylamino]-3-nitropyridine,
40) 2-[4-(t-butyl)phenylamino]-6-[2-(2-pyridyl)ethylamino]-3-nitropyridine,
41) 2-[4-(t-butyl)phenylamino]-6-(4-methylpiperazin-1-yl)-3-nitropyridine,
42) 2-[4-(t-butyl)phenylamino]-6-(piperazin-1-yl)-3-nitropyridine,
43) 2-[4-(t-butyl)phenylamino]-6-(4-aminopiperidino)-3-nitropyridine,
44) 2-[4-(t-butyl)phenylamino]-6-morpholino-3-nitropyridine,
45) 2-(4-cyanophenylamino)-6-(methylamino)-3-nitropyridine,
46) 2-(4-cyanophenylamino)-6-(isobutylamino)-3-nitropyridine,

47) 2-(4-cyanophenylamino)-6-(4-hydroxypiperidino)-3-nitropyridine,
48) 2-(4-cyanophenylamino)-6-[(2-methyl-4,5-dihydro)imidazol-1-yl]-3-nitropyridine,
49) 2-(4-cyanophenylamino)-6-[(2-isopropyl)imidazol-1-yl]-3-nitropyridine,
50) 2-(4-cyanophenylamino)-6-[(4-pyridyl)methylamino]-3-nitropyridine,
51) 2-(4-cyanophenylamino)-6-[(N-ethyl-2-hydroxy)ethylamino]-3-nitropyridine,
52) 2-(4-cyanophenylamino)-6-[(3-imidazol-1-yl)propylamino]-3-nitropyridine,
53) 2-[3-cyanophenylamino]-6-[(3-imidazol-1-yl)propylamino]-3-nitropyridine,
54) 2-(4-hydroxyphenylamino)-6-[(3-imidazol-1-yl)propylamino]-3-nitropyridine,
55) 2-[4-(methylsulfanyl)phenylamino]-6-[(3-imidazol-1-yl)propylamino]-3-nitropyridine,
56) 2-[4-(n-butyl)phenylamino]-6-[(3-imidazol-1-yl)propylamino]-3-nitropyridine,
57) 2-[4-(amino)phenylamino]-6-(methylamino)-3-nitropyridine,
58) 2-[4-(amino)phenylamino]-6-(isopropylamino)-3-nitropyridine,
59) 2-[4-(amino)phenylamino]-6-(isobutylamino)-3-nitropyridine,
60) 2-[4-(amino)phenylamino]-6-(t-butylamino)-3-nitropyridine,
61) 2-[4-(amino)phenylamino]-6-(4-hydroxypiperidino)-3-nitropyridine,
62) 2-[4-(amino)phenylamino]-6-(piperazin-1-yl)-3-nitropyridine,
63) 2-[4-(amino)phenylamino]-6-(4-methylpiperazin-1-yl)-3-nitropyridine,
64) 2-[4-(amino)phenylamino]-6-morpholino-3-nitropyridine,
65) 2-[4-(amino)phenylamino]-6-(4-aminopiperidino)-3-nitropyridine,
66) 2-[4-(amino)phenylamino]-6-[(4-pyridyl)methylamino]-3-nitropyridine,
67) 2-[4-(amino)phenylamino]-6-[(3-imidazol-1-yl)propylamino]-3-nitropyridine,
68) 2-[4-(amino)phenylamino]-6-[2-(morpholin-1-yl)ethylamino]-3-nitropyridine,
69) 2-[4-(amino)phenylamino]-6-[3-(morpholin-1-yl)propylamino]-3-nitropyridine,
70) 2-[3-(amino)phenylamino]-6-(methylamino)-3-nitropyridine,
71) 2-[3-(amino)phenylamino]-6-(isopropylamino)-3-nitropyridine,
72) 2-[3-(amino)phenylamino]-6-(isobutylamino)-3-nitropyridine,
73) 2-[3-(amino)phenylamino]-6-(t-butylamino)-3-nitropyridine,
74) 2-[3-(amino)phenylamino]-6-(4-hydroxypiperidino)-3-nitropyridine,
75) 2-[3-(amino)phenylamino]-6-[(2-isopropyl)imidazol-1-yl]-3-nitropyridine,
76) 2-[3-(amino)phenylamino]-6-(piperazin-1-yl)-3-nitropyridine,
77) 2-[3-(amino)phenylamino]-6-(4-methylpiperazin-1-yl)-3-nitropyridine,
78) 2-[3-(amino)phenylamino]-6-morpholino-3-nitropyridine,
79) 2-[3-(amino)phenylamino]-6-(4-aminopiperidino)-3-nitropyridine,
80) 2-[3-(amino)phenylamino]-6-[(3-pyridyl)methylamino]-3-nitropyridine,
81) 2-[3-(amino)phenylamino]-6-[(4-pyridyl)methylamino]-3-nitropyridine,
82) 2-[3-(amino)phenylamino]-6-[(3-imidazol-1-yl)propylamino]-3-nitropyridine,
83) 2-[3-(amino)phenylamino]-6-[2-(morpholin-1-yl)ethylamino]-3-nitropyridine,
84) 2-[3-(amino)phenylamino]-6-[3-(morpholin-1-yl)propylamino]-3-nitropyridine,
85) 2-[3-(amino)phenylamino]-6-[(2-methyl)imidazol-1-yl]-3-nitropyridine,
86) 2-[4-(imidazol-1-yl)phenylamino]-6-(methylamino)-3-nitropyridine,
87) 2-[4-(imidazol-1-yl)phenylamino]-6-(isopropylamino)-3-nitropyridine,
88) 2-[4-(imidazol-1-yl)phenylamino]-6-(isobutylamino)-3-nitropyridine,
89) 2-[4-(imidazol-1-yl)phenylamino]-6-[(N-[1,3]-dioxolan-2-ylmethyl)methylamino]-3-nitropyridine,
90) 2-[4-(imidazol-1-yl)phenylamino]-6-(4-hydroxypiperidino)-3-nitropyridine,
91) 2-[4-(imidazol-1-yl)phenylamino]-6-[(2-methyl-4,5-dihydro)imidazol-1-yl]-3-nitropyridine,
92) 2-[4-(imidazol-1-yl)phenylamino]-6-[(2-isopropyl)imidazol-1-yl]-3-nitropyridine,
93) 2-[4-(imidazol-1-yl)phenylamino]-6-[(3-pyridyl)methylamino]-3-nitropyridine,
94) 2-[4-(imidazol-1-yl)phenylamino]-6-[(4-pyridyl)methylamino]-3-nitropyridine,
95) 2-[4-(imidazol-1-yl)phenylamino]-6-[(3-imidazol-1-yl)propylamino]-3-nitropyridine,
96) 2-(3-acetylphenylamino)-6-(methylamino)-3-nitropyridine,
97) 2-(3-acetylphenylamino)-6-(isopropylamino)-3-nitropyridine,
98) 2-(3-acetylphenylamino)-6-(isobutylamino)-3-nitropyridine,
99) 2-(3-acetylphenylamino)-6-(4-hydroxypiperidino)-3-nitropyridine,
100) 2-(3-acetylphenylamino)-6-[(2-methyl-4,5-dihydro)imidazol-1-yl]-3-nitropyridine,
101) 2-(3-acetylphenylamino)-6-[(2-isopropyl)imidazol-1-yl]-3-nitropyridine,
102) 2-(3-acetylphenylamino)-6-[(3-pyridyl)methylamino]-3-nitropyridine,
103) 2-(3-acetylphenylamino)-6-[(4-pyridyl)methylamino]-3-nitropyridine,
104) 2-(3-acetylphenylamino)-6-(t-butylamino)-3-nitropyridine,

105) 2-(3-acetylphenylamino)-6-(4-methylpiperazin-1-yl)-3-nitropyridine,
106) 2-(3-acetylphenylamino)-6-(piperazin-1-yl)-3-nitropyridine,
107) 2-(3-acetylphenylamino)-6-morpholino-3-nitropyridine,
108) 2-(4-morpholinophenylamino)-6-(methylamino)-3-nitropyridine,
109) 2-(4-morpholinophenylamino)-6-(isopropylamino)-3-nitropyridine,
110) 2-(4-morpholinophenylamino)-6-(isobutylamino)-3-nitropyridine,
111) 2-(4-morpholinophenylamino)-6-[(N-[1,3]-dioxolan-2-ylmethyl)methylamino]-3-nitropyridine,
112) 2-(4-morpholinophenylamino)-6-(4-hydroxypiperidino)-3-nitropyridine,
113) 2-(4-morpholinophenylamino)-6-[(2-methyl-4,5-dihydro)imidazol-1-yl]-3-nitropyridine,
114) 2-(4-morpholinophenylamino)-6-[(2-isopropyl)imidazol-1-yl]-3-nitropyridine,
115) 2-(4-morpholinophenylamino)-6-[(3-pyridyl)methylamino]-3-nitropyridine,
116) 2-(4-morpholinophenylamino)-6-[(4-pyridyl)methylamino]-3-nitropyridine,
117) 2-(4-morpholinophenylamino)-6-(t-butylamino)-3-nitropyridine,
118) 2-(4-morpholinophenylamino)-6-[(N-ethyl-2-hydroxy)ethylamino]-3-nitropyridine,
119) 2-(4-morpholinophenylamino)-6-[(3-imidazol-1-yl)propylamino]-3-nitropyridine,
120) 2-(4-morpholinophenylamino)-6-(piperazin-1-yl)-3-nitropyridine,
121) 2-(4-morpholinophenylamino)-6-(4-aminopiperidino)-3-nitropyridine,
122) 2-[(3,4-difluoro)phenylamino]-6-(methylamino)-3-nitropyridine,
123) 2-[(3,4-difluoro)phenylamino]-6-(isopropylamino)-3-nitropyridine,
124) 2-[(3,4-difluoro)phenylamino]-6-(isobutylamino)-3-nitropyridine,
125) 2-[(3,4-difluoro)phenylamino]-6-(t-butylamino)-3-nitropyridine,
126) 2-[(3,4-difluoro)phenylamino]-6-(4-hydroxypiperidino)-3-nitropyridine,
127) 2-[(3,4-difluoro)phenylamino]-6-[(N-[1,3]-dioxolan-2-ylmethyl)-methylamino]-3-nitropyridine,
128) 2-[(3,4-difluoro)phenylamino]-6-(4-methylpiperazin-1-yl)-3-nitropyridine,
129) 2-[(3,4-difluoro)phenylamino]-6-morpholino-3-nitropyridine,
130) 2-[(3,4-difluoro)phenylamino]-6-(4-aminopiperidino)-3-nitropyridine,
131) 2-[(3,4-difluoro)phenylamino]-6-[(4-pyridyl)methylamino]-3-nitropyridine,
132) 2-[4-(2-methylthiazol-4-yl)phenylamino]-6-(methylamino)-3-nitropyridine,
133) 2-[4-(2-methylthiazol-4-yl)phenylamino]-6-(isopropylamino)-3-nitropyridine,
134) 2-[4-(2-methylthiazol-4-yl)phenylamino]-6-(isobutylamino)-3-nitropyridine,
135) 2-[4-(2-methylthiazol-4-yl)phenylamino]-6-(4-hydroxypiperidino)-3-nitropyridine,
136) 2-[4-(2-methylthiazol-4-yl)phenylamino]-6-[(2-methyl-4,5-dihydro)imidazol-1-yl]-3-nitropyridine,
137) 2-[4-(2-methylthiazol-4-yl)phenylamino]-6-[(2-isopropyl)imidazol-1-yl]-3-nitropyridine,
138) 2-[4-(2-methylthiazol-4-yl)phenylamino]-6-[(3-pyridyl)methylamino]-3-nitropyridine,
139) 2-[4-(2-methylthiazol-4-yl)phenylamino]-6-[(4-pyridyl)methylamino]-3-nitropyridine,
140) 2-[4-(2-methylthiazol-4-yl)phenylamino]-6-(t-butylamino)-3-nitropyridine,
141) 2-[4-(2-methylthiazol-4-yl)phenylamino]-6-[(N-ethyl-2-hydroxy)ethylamino]-3-nitropyridine,
142) 2-[4-(2-methylthiazol-4-yl)phenylamino]-6-(4-methylpiperazin-1-yl)-3-nitropyridine,
143) 2-[4-(2-methylthiazol-4-yl)phenylamino]-6-(piperazin-1-yl)-3-nitropyridine,
144) 2-[4-(2-methylthiazol-4-yl)phenylamino]-6-(4-aminopiperidino)-3-nitropyridine,
145) 2-[4-(2-methylthiazol-4-yl)phenylamino]-6-morpholino-3-nitropyridine,
146) 2-[4-(2-isopropylthiazol-4-yl)phenylamino]-6-(isobutylamino)-3-nitropyridine,
147) 2-[4-(2-isopropylthiazol-4-yl)phenylamino]-6-(4-hydroxypiperidino)-3-nitropyridine,
148) 2-[4-(2-isopropylthiazol-4-yl)phenylamino]-6-[(N-ethyl-2-hydroxyethyl)amino]-3-nitropyridine,
149) 2-[4-(2-isopropylthiazol-4-yl)phenylamino]-6-(4-methylpiperazin-1-yl)-3-nitropyridine,
150) 2-[4-(2-isopropylthiazol-4-yl)phenylamino]-6-(4-aminopiperidino)-3-nitropyridine,
151) 2-[4-(2-cyclohexylthiazol-4-yl)phenylamino]-6-(methylamino)-3-nitropyridine,
152) 2-[4-(2-cyclohexylthiazol-4-yl)phenylamino]-6-(isopropylamino)-3-nitropyridine,
153) 2-[4-(2-cyclohexylthiazol-4-yl)phenylamino]-6-(isobutylamino)-3-nitropyridine,
154) 2-[4-(2-cyclohexylthiazol-4-yl)phenylamino]-6-(t-butylamino)-3-nitropyridine,
155) 2-[4-(2-cyclohexylthiazol-4-yl)phenylamino]-6-(4-hydroxypiperidino)-3-nitropyridine,
156) 2-[4-(2-cyclohexylthiazol-4-yl)phenylamino]-6-[(N-ethyl-2-hydroxyethyl)amino]-3-nitropyridine,
157) 2-[4-(2-cyclohexylthiazol-4-yl)phenylamino]-6-[(2-isopropyl)imidazol-1-yl]-3-nitropyridine,
158) 2-[4-(2-cyclohexylthiazol-4-yl)phenylamino]-6-(piperazin-1-yl)-3-nitropyridine,
159) 2-[4-(2-cyclohexylthiazol-4-yl)phenylamino]-6-(4-methyl)piperazin-1-yl)-3-nitropyridine,
160) 2-[4-(2-cyclohexylthiazol-4-yl)phenylamino]-6-morpholino-3-nitropyridine,
161) 2-[4-(2-cyclohexylthiazol-4-yl)phenylamino]-6-(4-aminopiperidino)-3-nitropyridine,
162) 2-[4-(2-cyclohexylthiazol-4-yl)phenylamino]-6-[(3-pyridyl)methylamino]-3-nitropyridine,

163) 2-[4-(2-cyclohexylthiazol-4-yl)phenylamino]-6-[(4-pyridyl)methylamino]-3-nitropyridine,
164) 2-[4-(2-cyclohexylthiazol-4-yl)phenylamino]-6-[2-(2-pyridyl)ethylamino]-3-nitropyridine,
165) 2-[4-(2-cyclohexylthiazol-4-yl)phenylamino]-6-(n-butylamino)-3-nitropyridine,
166) 2-[4-(2-dipropylaminothiazol-4-yl)phenylamino]-6-(methylamino)-3-nitropyridine,
167) 2-[4-(2-dipropylaminothiazol-4-yl)phenylamino]-6-(isopropylamino)-3-nitropyridine,
168) 2-[4-(2-dipropylaminothiazol-4-yl)phenylamino]-6-(isobutylamino)-3-nitropyridine,
169) 2-[4-(2-dipropylaminothiazol-4-yl)phenylamino]-6-(4-hydroxypiperidino)-3-nitropyridine,
170) 2-[4-(2-dipropylaminothiazol-4-yl)phenylamino]-6-[(N-ethyl-2-hydroxyethyl)amino]-3-nitropyridine,
171) 2-[4-(2-dipropylaminothiazol-4-yl)phenylamino]-6-(piperazin-1-yl)-3-nitropyridine,
172) 2-[4-(2-dipropylaminothiazol-4-yl)phenylamino]-6-(4-methylpiperazin-1-yl)-3-nitropyridine,
173) 2-[4-(2-dipropylaminopropylthiazol-4-yl)phenylamino]-6-(4-aminopiperidino)-3-nitropyridine,
174) 2-[4-(2-dipropylaminothiazol-4-yl)phenylamino]-6-[(3-pyridyl)methylamino]-3-nitropyridine,
175) 2-[(3-fluoro-4-diethylamino)phenylamino]-6-(methylamino)-3-nitropyridine,
176) 2-[(3-fluoro-4-diethylamino)phenylamino]-6-(isopropylamino)-3-nitropyridine,
177) 2-[(3-fluoro-4-diethylamino)phenylamino]-6-(isobutylamino)-3-nitropyridine,
178) 2-[(3-fluoro-4-diethylamino)phenylamino]-6-(t-butylamino)-3-nitropyridine,
179) 2-[(3-fluoro-4-diethylamino)phenylamino]-6-(4-hydroxypiperidino)-3-nitropyridine,
180) 2-[(3-fluoro-4-diethylamino)phenylamino]-6-[(2-isopropyl)imidazol-1-yl]-3-nitropyridine,
181) 2-[(3-fluoro-4-diethylamino)phenylamino]-6-[(2-methyl-4,5-dihydro)imidazol-1-yl]-3-nitropyridine,
182) 2-[(3-fluoro-4-diethylamino)phenylamino]-6-(piperazin-1-yl)-3-nitropyridine,
183) 2-[(3-fluoro-4-diethylamino)phenylamino]-6-(4-methylpiperazin-1-yl)-3-nitropyridine,
184) 2-[(3-fluoro-4-diethylamino)phenylamino]-6-morpholino-3-nitropyridine,
185) 2-[(3-fluoro-4-diethylamino)phenylamino]-6-[(3-pyridyl)methylamino]-3-nitropyridine,
186) 2-[(3-fluoro-4-diethylamino)phenylamino]-6-[(4-pyridyl)methylamino]-3-nitropyridine,
187) 2-[(3-fluoro-4-diethylamino)phenylamino]-6-(4-aminopiperidino)-3-nitropyridine,
188) 2-[(3-fluoro-4-diethylamino)phenylamino]-6-[2-(morpholin-1-yl)ethylamino]-3-nitropyridine,
189) 2-[(3-fluoro-4-diethylamino)phenylamino]-6-[(3-imidazol-1-yl)propylamino]-3-nitropyridine,
190) 2-[(3-fluoro-4-diethylamino)phenylamino]-6-[(3-morpholin-1-yl)propylamino]-3-nitropyridine,
191) 2-[(3-fluoro-4-morpholino)phenylamino]-6-(methylamino)-3-nitropyridine,
192) 2-[(3-fluoro-4-morpholino)phenylamino]-6-(isopropylamino)-3-nitropyridine,
193) 2-[(3-fluoro-4-morpholino)phenylamino]-6-(isobutylamino)-3-nitropyridine,
194) 2-[(3-fluoro-4-morpholino)phenylamino]-6-(4-hydroxypiperidino)-3-nitropyridine,
195) 2-[(3-fluoro-4-morpholino)phenylamino]-6-[(2-methyl-4,5-dihydro)imidazol-1-yl]-3-nitropyridine,
196) 2-[(3-fluoro-4-morpholino)phenylamino]-6-[(2-isopropyl)imidazol-1-yl]-3-nitropyridine,
197) 2-[(3-fluoro-4-morpholino)phenylamino]-6-[(3-pyridyl)methylamino]-3-nitropyridine,
198) 2-[(3-fluoro-4-morpholino)phenylamino]-6-[(4-pyridyl)methylamino]-3-nitropyridine,
199) 2-[(3-fluoro-4-morpholino)phenylamino]-6-(t-butylamino)-3-nitropyridine,
200) 2-[(3-fluoro-4-morpholino)phenylamino]-6-(4-methylpiperazin-1-yl)-3-nitropyridine,
201) 2-[(3-fluoro-4-morpholino)phenylamino]-6-(piperazin-1-yl)-3-nitropyridine,
202) 2-[(3-fluoro-4-morpholino)phenylamino]-6-(4-aminopiperidino)-3-nitropyridine, 203) 2-[(3-fluoro-4-thiomorpholino)phenylamino]-6-(methylamino)-3-nitropyridine,
204) 2-[(3-fluoro-4-thiomorpholino)phenylamino]-6-(isopropylamino)-3-nitropyridine,
205) 2-[(3-fluoro-4-thiomorpholino)phenylamino]-6-(isobutylamino)-3-nitropyridine,
206) 2-[(3-fluoro-4-thiomorpholino)phenylamino]-6-(4-hydroxypiperidino)-3-nitropyridine,
207) 2-[(3-fluoro-4-thiomorpholino)phenylamino]-6-[(2-methyl-4,5-dihydro)imidazol-1-yl]-3-nitropyridine,
208) 2-[(3-fluoro-4-thiomorpholino)phenylamino]-6-[(2-isopropyl)imidazol-1-yl]-3-nitropyridine,
209) 2-[(3-fluoro-4-thiomorpholino)phenylamino]-6-[(3-pyridyl)methylamino]-3-nitropyridine,
210) 2-[(3-fluoro-4-thiomorpholino)phenylamino]-6-[(4-pyridyl)methylamino]-3-nitropyridine,
211) 2-[(3-fluoro-4-thiomorpholino)phenylamino]-6-(t-butylamino)-3-nitropyridine,
212) 2-[(3-fluoro-4-thiomorpholino)phenylamino]-6-(4-methylpiperazin-1-yl)-3-nitropyridine,
213) 2-[(3-fluoro-4-thiomorpholino)phenylamino]-6-(piperazin-1-yl)-3-nitropyridine,
214) 2-[(3-fluoro-4-thiomorpholino)phenylamino]-6-(4-aminopiperidino)-3-nitropyridine,
215) 2-[(3-fluoro-4-piperazino)phenylamino]-6-(methylamino)-3-nitropyridine,
216) 2-[(3-fluoro-4-piperazino)phenylamino]-6-(isopropylamino)-3-nitropyridine,
217) 2-[(3-fluoro-4-piperazino)phenylamino]-6-(isobutylamino)-3-nitropyridine,
218) 2-[(3-fluoro-4-piperazino)phenylamino]-6-(4-hydroxypiperidino)-3-nitropyridine,
219) 2-[(3-fluoro-4-piperazino)phenylamino]-6-[(2-isopropyl)imidazol-1-yl]-3-nitropyridine,
220) 2-[(3-fluoro-4-piperazino)phenylamino]-6-[(3-pyridyl)methylamino]-3-nitropyridine,

221) 2-[(3-fluoro-4-piperazino)phenylamino]-6-[(4-pyridyl)methylamino]-3-nitropyridine,
222) 2-[(3-fluoro-4-piperazino)phenylamino]-6-(t-butylamino)-3-nitropyridine,
223) 2-[(3-fluoro-4-piperidino)phenylamino]-6-(methylamino)-3-nitropyridine,
224) 2-[(3-fluoro-4-piperidino)phenylamino]-6-(isopropylamino)-3-nitropyridine,
225) 2-[(3-fluoro-4-piperidino)phenylamino]-6-(isobutylamino)-3-nitropyridine,
226) 2-[(3-fluoro-4-piperidino)phenylamino]-6-(4-hydroxypiperidino)-3-nitropyridine,
227) 2-[(3-fluoro-4-piperidino)phenylamino]-6-[(2-methyl-4,5-dihydro)imidazol-1-yl]-3-nitropyridine,
228) 2-[(3-fluoro-4-piperidino)phenylamino]-6-[(2-isopropyl)imidazol-1-yl]-3-nitropyridine,
229) 2-[(3-fluoro-4-piperidino)phenylamino]-6-[(3-pyridyl)methylamino]-3-nitropyridine,
230) 2-[(3-fluoro-4-piperidino)phenylamino]-6-[(4-pyridyl)methylamino]-3-nitropyridine,
231) 2-[(3-fluoro-4-piperidino)phenylamino]-6-(t-butylamino)-3-nitropyridine,
232) 2-[(3-fluoro-4-piperidino)phenylamino]-6-(4-methylpiperazin-1-yl)-3-nitropyridine,
233) 2-[(3-fluoro-4-piperidino)phenylamino]-6-(piperazin-1-yl)-3-nitropyridine,
234) 2-[(3-fluoro-4-piperidino)phenylamino]-6-(4-aminopiperidino)-3-nitropyridine,
235) 2-[(3-fluoro-4-piperidino)phenylamino]-6-morpholino-3-nitropyridine,
236) 2-{[3-fluoro-4-(4-hydroxypiperidino)]phenylamino}-6-(methylamino)-3-nitropyridine,
237) 2-{[3-fluoro-4-(4-hydroxypipendmo)]phenylamino}-6-(isopropylammo)-3-nitropyridine,
238) 2-{[3-fluoro-4-(4-hydroxypipendmo)]phenylamino}-6-(isobutylammo)-3-nitropyridine,
239) 2-{[3-fluoro-4-(4-hydroxypipendmo)]phenylammo}-6-(4-hydroxypipendmo)-3-nitropyridine,
240) 2-{[3-fluoro-4-(4-hydroxypipendmo)]phenylammo}-6-[(2-methyl-4,5-dihydro)imidazol-1-yl]-3-nitropyridine,
241) 2-{ [3-fluoro-4-(4-hydroxypipendino)]phenylammo}-6-[(3-pyridyl)methylamino]-3-nitropyridine,
242) 2-{ [3-Quoro-4-(4-hydroxypipendmo)]phenylamino}-6-[(4-pyridyl)methylamino]-3-nitropyridine,
243) 2-{[3-fluoro-4-(4-hydroxypipendino)]phenylammo}-6-(t-butylammo)-3-nitropyridine,
244) 2-{[3-fluoro-4-(4-hydroxypipendino)]phenylamino}-6-(4-methylpiperazin-1-yl)-3-nitropyridine,
245) 2-{ [3-Quoro-4-(4-hydroxypipendmo)]phenylammo}-6-(piperazin- l-yl)-3-nitropyridine,
246) 2-{[3-fluoro-4-(4-hydroxypipendino)]phenylamino}-6-(4-aminopipendino)-3-nitropyridine,
247) 2-{[3-fluoro-4-(4-hydroxypiperidino)]phenylamino}-6-morpholino-3-nitropyridine,
248) 2-{[3-fluoro-4-(4-aminopiperidino)]phenylamino}-6-(methylamino)-3-nitropyridine,
249) 2-{[3-fluoro-4-(4-aminopiperidino)]phenylamino}-6-(isopropylamino)-3-nitropyridine,
250) 2-{[3-fluoro-4-(4-aminopiperidino)]phenylamino}-6-(isobutylamino)-3-nitropyridine,
251) 2-{[3-fluoro-4-(4-aminopiperidino)]phenylamino}-6-(4-hydroxypiperidino)-3-nitropyridine,
252) 2-{[3-fluoro-4-(4-aminopiperidino)]phenylamino}-6-[(2-methyl-4,5-dihydro)imidazol-1-yl]-3-nitropyndine,
253) 2-{[3-fluoro-4-(4-aminopiperidino)]phenylamino}-6-(piperazin-1-yl)-3-nitropyridine,
254) 2-{[3-fluoro-4-(4-aminopipendmo)]phenylammo}-6-(4-methylpiperazm-1-yl)-3-nitropyridine,
255) 2-{[3-Quoro-4-(4-ammopipendmo)]phenylammo}-6-morpholmo-3-nitropyndme,
256) 2-{[3-fluoro-4-(4-ammopipendmo)]phenylammo}-6-(4-ammopipendmo-3-nitropyridine,
257) 2-{[3-fluoro-4-(4-aminopipendmo)]phenylammo}-6-[(3-pyndyl)methylammo]-3-nitropyridine,
258) 2-{[3-fluoro-4-(4-aminopipendmo)]phenylammo}-6-[(4-pyndyl)methylammo]-3-nitropyridine,
259) 2-{[3-Quoro-4-(4-ammopipendmo)]phenylammo}-6-[2-(morpholm-1-yl)ethylamino]-3-nitropyridine,
260) 2-{[3-Quoro-4-(4-aminopiperidino)]phenylamino}-6-[(3-morpholin-1-yl)propylamino]-3-nitropyridine,
261) 2-{[3-fluoro-4-(2-methylpiperidino)]phenylamino}-6-(methylamino)-3-nitropyridine,
262) 2-{[3-fluoro-4-(2-methylpiperidino)]phenylamino}-6-(isopropylamino)-3-nitropyridine,
263) 2-{[3-fluoro-4-(2-methylpiperidino)]phenylamino}-6-(isobutylamino)-3-nitropyridine,
264) 2-{[3-fluoro-4-(2-methylpiperidino)]phenylamino}-6-(t-butylamino)-3-nitropyridine,
265) 2-{[3-fluoro-4-(2-methylpiperidino)]phenylamino}-6-(4-hydroxypiperidino)-3-nitropyridine,
266) 2-{[3-fluoro-4-(2-methylpiperidino)]phenylamino}-6-[(2-methyl-4,5-dihydro)imidazol-1-yl]-3-nitropyndine,
267) 2-{[3-fluoro-4-(2-methylpiperidino)]phenylamino}-6-(piperazin-1-yl)-3-nitropyridine,
268) 2-{[3-fluoro-4-(2-methylpipendino)]phenylammo}-6-(4-methylpiperazm-1-yl)-3-nitropyridine,
269) 2-{[3-fluoro-4-(2-methylpiperidino)]phenylamino}-6-morpholino-3-nitropyridine,
270) 2-{[3-fluoro-4-(2-methylpiperidino)]phenylamino}-6-(4-aminopiperidino)-3-nitropyridine,
271) 2-{[3-fluoro-4-(2-methylpipendmo)]phenylamino}-6-[(4-pyndyl)methylammo]-3-nitropyridine
272) 2-{[3-fluoro-4-(2-methylpipendino)]phenylamino}-6-[(3-imidazol-1-yl)propylamino]-3-nitropyridine,
273) 2-{[3-Quoro-4-(2-methylpipendmo)]phenylamino}-6-[2-(morpholm-1-yl)ethylamino]-3-nitropyridine,
274) 2-{[3-Quoro-4-(2-methylpiperidino)]phenylamino}-6-[(3-morpholin-1-yl)propylamino]-3-nitropyridine,
275) 2-{[3-fluoro-4-(3-hydroxymethylpipendino)]phenylamino}-6-(methylamino)-3-nitropyridine,
276) 2-{[3-fluoro-4-(3-hydroxymethylpipendino)]phenylamino}-6-(isopropylamino)-3-nitropyridine,
277) 2-{[3-nuoro-4-(3-hydroxymethylpipendino)]phenylamino}-6-(isobutylamino)-3-nitropyridine,

278) 2-{[3-Quoro-4-(3-hydroxymethylpipendmo)]phenylammo}-6-(t-butylamino)-3-nitropyridine,
279) 2-{[3-Quoro-4-(3-hydroxymethylpipendmo)]phenylammo}-6-(4-hydroxypiperidino)-3-nitropyridine,
280) 2-{[3-fluoro-4-(3-hydroxymethylpipendino)]phenylammo}-6-[(2-methyl-4,5-dihydro)imidazol-1-yl]-3-nitropyridine,
281) 2-{[3-fluoro-4-(3-hydroxymethylpipendino)]phenylamino}-6-(piperazin-1-yl)-3-nitropyridine,
282) 2-{[3-fluoro-4-(3-hydroxymethylpipendino)]phenylamino}-6-(4-methylpiperazin-1-yl)-3-nitropyridine,
283) 2-{[3-fluoro-4-(3-hydroxymethylpipendino)]phenylamino}-6-morpholino-3-nitropyridine,
284) 2-{[3-fluoro-4-(3-hydroxymethylpipendino)]phenylamino}-6-(4-aminopiperidino)-3-nitropyridine,
285) 2-{[3-Quoro-4-(3-hydroxymethylpipendmo)]phenylamino}-6-[(3-pyridyl)methylamino]-3-nitropyridine,
286) 2-{[3-fluoro-4-(3-hydroxymethylpipendino)]phenylamino}-6-[(4-pyridyl)methylamino]-3-nitropyridine,
287) 2-{[3-nuoro-4-(3-hydroxymethylpipendmo)]phenylamino}-6-[2-(2-pyridyl)ethylamino]-3-nitropyridine,
288) 2-{[3-fluoro-4-(3-hydroxymethylpiperidino)]phenylamino}-6-(cyclopropylamino)-3-nitropyridine,
289) 2-{[3-fluoro-4-(4-carbamoylpiperidino)]phenylamino}-6-(methylamino)-3-nitropyridine,
290) 2-{[3-fluoro-4-(4-carbamoylpiperidino)]phenylamino}-6-(isopropylamino)-3-nitropyridine,
291) 2-{[3-fluoro-4-(4-carbamoylpiperidino)]phenylamino}-6-(isobutylamino)-3-nitropyridine,
292) 2-{[3-fluoro-4-(4-carbamoylpiperidino)]phenylamino}-6-(t-butylamino)-3-nitropyridine,
293) 2-{[3-Quoro-4-(4-carbamoylpipendmo)]phenylammo}-6-(4-hydroxypipendmo)-3-nitropyridine,
294) 2-{[3-Quoro-4-(4-carbamoylpipendmo)]phenylammo}-6-(piperazin-1-yl)-3-nitropyridine,
295) 2-{[3-Quoro-4-(4-carbamoylpipendmo)]phenylammo}-6-(4-methylpiperazm-1-yl)-3-nitropyridine,
296) 2-{[3-fluoro-4-(4-carbamoylpiperidino)]phenylamino}-6-morpholino-3-nitropyridine,
297) 2-{[3-fluoro-4-(4-carbamoylpipendmo)]phenylamino}-6-(4-ammopipendmo)-3-nitropyridine,
298) 2-{[3-fluoro-4-(4-carbamoylpiperidino)]phenylamino}-6-[(4-pyridyl)methylamino]-3-nitropyridine,
299) 2-{[3-fluoro-4-(4-carbamoylpipendmo)]phenylammo}-6-[(3-imidazol-1-yl)propylamino]-3-nitropyridine,
300) 2-{[3-fluoro-4-(4-carbamoylpipendino)]phenylammo}-6-[2-(morpholm-1-yl)ethylamino]-3-nitropyridine,
301) 2-{[3-fluoro-4-(3-carbamoylpiperidino)]phenylamino}-6-(methylamino)-3-nitropyridine,
302) 2-{[3-fluoro-4-(3-carbamoylpiperidino)]phenylamino}-6-(isopropylamino)-3-nitropyridine,
303) 2-{[3-fluoro-4-(3-carbamoylpiperidino)]phenylamino}-6-(isobutylamino)-3-nitropyridine,
304) 2-{[3-fluoro-4-(3-carbamoylpiperidino)]phenylamino}-6-(t-butylamino)-3-nitropyridine,
305) 2-{[3-fluoro-4-(3-carbamoylpipendino)]phenylammo}-6-(4-hydroxypipendmo-3-nitropyridine,
306) 2-{[3-fluoro-4-(3-carbamoylpipendino)]phenylammo}-6-(piperazm-1-yl)-3-nitropyridine,
307) 2-{[3-Quoro-4-(3-carbamoylpipendmo)]phenylammo}-6-(4-methylpiperazm-1-yl)-3-nitropyridine,
308) 2-{[3-fluoro-4-(3-carbamoylpiperidino)]phenylamino}-6-morpholino-3-nitropyridine,
309) 2-{[3-fluoro-4-(3-carbamoylpipendmo)]phenylamino}-6-(4-ammopipendmo)-3-nitropyridine,
310) 2-{ [3-fluoro-4-(3-carbamoylpipendino)]phenylammo}-6-[(3-pyridyl)methylamino]-3-nitropyridine,
311) 2-{[3-fluoro-4-(3-carbamoylpipendino)]phenylammo}-6-[(4-pyridyl)methylamino]-3-nitropyridine,
312) 2-{[3-fluoro-4-(3-carbamoylpipendino)]phenylammo}-6-[(3-imidazol-1-yl)propylamino]-3-nitropyridine,
313) 2-{[3-Quoro-4-(3-carbamoylpipendmo)]phenylammo}-6-[2-(morpholin-1-yl)ethylamino]-3-nitropyridine,
314) 2-{[3-fluoro-4-(3-carbamoylpipendino)]phenylammo}-6-[(3-morpholm-1-yl)propylamino]-3-nitropyridine,
315) 2-{[3-fluoro-4-(3-carbamoylpiperidino)]phenylamino}-6-(diethylamino)-3-nitropyridine,
316) 2-{[3-fluoro-4-(4-carboxylicpiperidino)]phenylamino}-6-(methylamino)-3-nitropyridine,
317) 2-{[3-fluoro-4-(4-carboxylicpiperidino)]phenylamino}-6-(isopropylamino)-3-nitropyridine,
318) 2-{[3-fluoro-4-(4-carboxylicpiperidino)]phenylamino}-6-(isobutylamino)-3-nitropyridine,
319) 2-{[3-Quoro-4-(4-carboxylicpipendmo)]phenylammo}-6-(4-hydroxypipendmo)-3-nitropyridine,
320) 2-{[3-Quoro-4-(4-carboxylicpipendmo)]phenylammo}-6-(4-methylpiperazm-1-yl)-3-nitropyridine,
321) 2-{[3-Quoro-4-(4-carboxylicpipendmo)]phenylamino}-6-[(3-pyridyl)methylamino]-3-nitropyridine,
322) 2-{[3-fluoro-4-(4-carboxylicpipendino)]phenylammo}-6-[(4-pyridyl)methylamino]-3-nitropyridine,
323) 2-{[3-fluoro-4-(4-carboxylicpipendino)]phenylammo}-6-[(3-imidazol-1-yl)propylamino]-3-nitropyridine,
324) 2-{[3-fluoro-4-(4-carboxylicpipendino)]phenylammo}-6-[2-(morpholm-1-yl)ethylamino]-3-nitropyridine, and
325) 2-{[3-fluoro-4-(4-carboxylicpipendino)]phenylamino}-6-[(3-morpholin-1-yl)propylamino]-3-nitropyridine.

**4.** The compound according to any one of claims 1 to 3, wherein the pharmaceutically acceptable salt is hydrochloride or methanesulfonate.

**5.** A method for preparing a 2,6-substituted-3-nitropyridine derivative compound of formula 1, comprising:

a) reacting 2,6-dichloro-3-nitropyridine with an aniline compound of formula 3 in the presence of a base to prepare a 6-chloro-3-nitropyridine derivative compound of formula 4; and

b) reacting the compound of formula 4 prepared in Step a) with an amine compound of formula 5 to prepare a 2,6-substituted-3-nitropyridine derivative compound of formula 1:

[Formula 3]

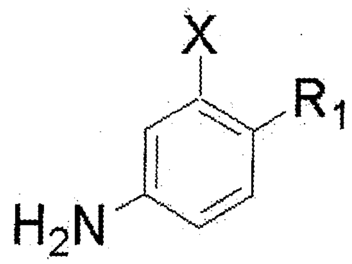

[Formula 4]

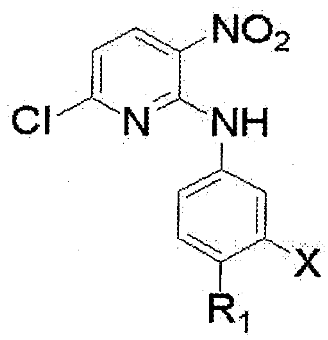

$HNR_5(CH_2)nR_6$ [Formula 5]

[Formula 1]

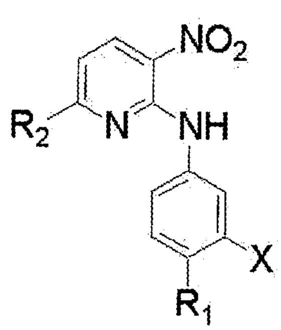

wherein $R_1$, $R_2$, $R_5$, $R_6$, n and X are as defined in claim 1.

6. The method according to claim 5, wherein the base of Step a) is at least one selected from triethylamine, N,N-diisopropylethylamine, N-methylmorpholine, N-methylpiperidine, 4-dimethylaminopyridine, N,N-dimethylaniline, 2,6-lutidine, pyridine, sodium hydroxide and sodium hydride.

7. The method according to claim 5, wherein the compound of formula 3 is prepared by a preparation method comprising:

a) subjecting a 4-nitrophenone compound of formula 6 to bromination at the alpha position with respect to the carboxyl group thereof to prepare a compound of formula 7;
b) reacting the compound of formula 7 prepared in Step a) with a thioamide compound of formula 8 to prepare a compound of formula 9; and
c) subjecting the compound of formula 9 prepared in Step b) to hydrogenation, thereby preparing the compound of formula 3:

[Formula 6]

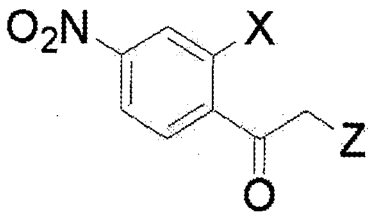

[Formula 7]

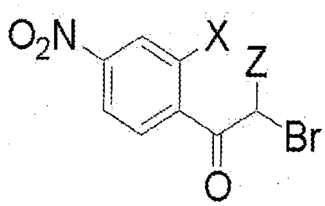

[Formula 8]

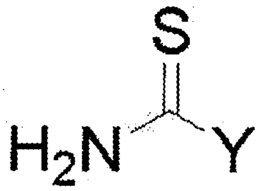

[Formula 9]

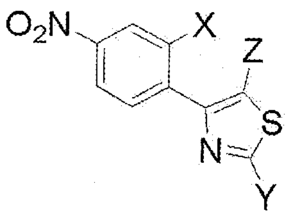

[Formula 3]

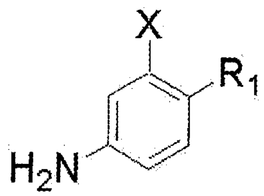

wherein X, Z and Y are as defined in claim 1, and $R_1$ represents a thiazolyl group

( 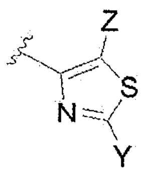 ).

**8.** The method according to claim 7, wherein the reagent used for the bromination reaction of Step a) is copper (II) bromide or bromine.

**9.** The method according to claim 7, wherein the compound of formula 8 in Step b) is thioacetamide, thiopropionamide, thioisobutyramide, trimethylthioacetamide, thiohexanoamide, cyclohexancarbothioic acid amide, piperidine-4-carbothioic acid amide, thiourea, N-methylthiourea, N-ethylthiourea, N,N-dipropylthiourea or thiobenzamide.

**10.** The method according to claim 7, wherein the hydrogenation reaction of Step c) is carried out under hydrogen gas in the presence of a Pd/C catalyst or a Raney nickel catalyst.

**11.** The method according to claim 5, wherein the compound of formula 3 is prepared by a preparation method comprising:

a) reacting a 3,4-difluoronitrobenzene compound with a compound of formula 10 in the presence of an organic base to prepare a nitrobenzene compound of formula 11; and
b) subjecting the compound of formula 11 prepared in Step a) to hydrogenation, thereby preparing the compound of formula 3:

$HR_1$ [Formula 10]

[Formula 11]

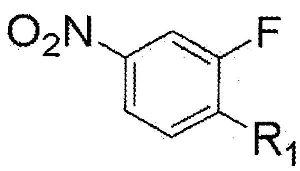

[Formula 3]

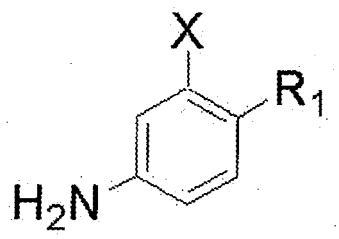

wherein $R_1$ represents $NR_3R_4$ wherein $R_3$ and $R_4$ taken together form a saturated or unsaturated 5-, 6- or 7-membered heterocyclic amino compound which contains 1 to 3 hetero atoms selected from N, O and S and is unsubstituted or substituted by a $C_1$-$C_3$ alkyl group, a hydroxyl group, a $C_1$-$C_3$ hydroxyalkyl group, an amino group, a carboxyl group or a carbamoyl group, and
X represents a fluoro group.

**12.** The method according to claim 11, wherein the compound of formula 10 in Step a) is diethylamine, morpholine, thiomorpholine, unsubstituted or substituted piperazine, piperidine, methylpiperidine, hydroxypiperidine, hydroxyethylpiperidine, aminopiperidine, 3- or 4-carbamoylpiperidine, carboxylicpiperidine or pyrrolidine

**13.** The method according to claim 11, wherein the organic base of Step a) is at least one selected from triethylamine, N,N-diisopropylethylamine, N-methylmorpholine, N-methylpiperidine, 4-dimethylaminopyridine, N,N-dimethylaniline, 2,6-lutidine and pyridine.

**14.** The method according to claim 11, wherein the hydrogenation reaction of Step b) is carried out under hydrogen gas in the presence of a Pd/C catalyst or a Raney nickel catalyst.

**15.** A pharmaceutical composition for the prevention or treatment of osteoporosis, comprising the 2,6-substituted-3-nitropyridine derivative of any one of claims 1 to 3 or a pharmaceutically acceptable salt thereof as an active ingredient.

**16.** The composition according to claim 15, wherein the pharmaceutically acceptable salt is hydrochloride or methanesulfonate.

**17.** A method for the prevention or treatment of osteoporosis, comprising administering an effective amount of the 2,6-substituted-3-nitropyridine derivative of any one of claims 1 to 3 or a pharmaceutically acceptable salt thereof to a mammal including a human.

**18.** The method according to claim 17, wherein the pharmaceutically acceptable salt is hydrochloride or methanesulfonate.

**19.** Use of the 2,6-substituted-3-nitropyridine derivative of any one of claims 1 to 3 or a pharmaceutically acceptable salt thereof, for manufacturing a pharmaceutical preparation for the prevention or treatment of osteoporosis.

**20.** The use according to claim 19, wherein the pharmaceutically acceptable salt is hydrochloride or methanesulfonate.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Remington's Pharmaceutical Sciences. Mack Publishing Company
- **E. Jimi.** *Endocrinology,* 1996, vol. 137, 2187-2190
- **Takahashi.** *Endocrinology,* 1988, vol. 123, 2600-2602